# EUROPEAN PATENT APPLICATION

(11) **EP 4 834 428 A2**
(43) Date of publication of application: **04.11.2026**
(21) Application number: 26204568.5
(22) Date of filing: 09.11.2020
(51) Int. Cl.: A61P 9/10

(54) **TRICYCLE DIHYDROIMIDAZOPYRIMIDONE DERIVATIVE, PREPARATION METHOD THEREOF, PHARMACEUTICAL COMPOSITION AND USE THEREOF**

(30) Priority: 09.11.2019 CN 201911104067
(62) Document number(s) of the earlier application(s) in accordance with Art. 76 EPC: 20885448.9 / 4 056 571
(71) Applicant: Shanghai Simr Biotechnology Co., Ltd., Shanghai 201321 (CN); Shanghai Simrd Biotechnology Co., Ltd, Shanghai 201306 (CN)
(72) Inventor: JIN, Yun, Shanghai, 201321 (CN); WU, Jinhua, Shanghai, 201321 (CN); PENG, Jun, Shanghai, 201321 (CN); SUN, Yong, Shanghai, 201321 (CN)
(74) Representative: Colombo, Stefano Paolo

(57) **Abstract**

Disclosed are a compound as represented by general formula (I), a cis-trans isomer thereof, an enantiomer thereof, a diastereoisomer thereof, a racemate thereof, a solvate thereof, a hydrate thereof, or a pharmaceutically acceptable salt thereof or a prodrug thereof, a preparation method therefor, a pharmaceutical composition comprising the compound and the use of the compound as an Lp-PLA₂ inhibitor

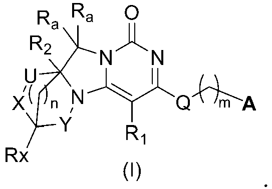

## Description

### Technical Field

The present invention relates to a novel tricyclic dihydroimidazopyrimidinone compound, a preparation method thereof and a pharmaceutical composition comprising the compound, as well as their use in the treatment of a disease mediated by Lp-PLA₂.

### Background Art

Lipoprotein-associated phospholipase A₂(Lp-PLA₂) is a phospholipase A₂ enzyme involved in the hydrolysis of lipoprotein lipids or phospholipids, also known as platelet-activating factor acetylhydrolase (PAF-AH). Lp-PLA₂ migrates with low-density lipoprotein (LDL) and rapidly cleaves oxidized phosphatidylcholine molecules resulting from the oxidation of LDL. Lp-PLA₂ hydrolyzes a sn-2 ester of oxidized phosphatidylcholine to yield a lipid mediator lysophosphatidylcholine (lysoPC) and an oxidized non-esterified fatty acid (NEFA). It has been reported in the literature that lysoPC and NEFA can induce inflammatory responses, so Lp-PLA₂ mediates oxidative inflammatory responses in vivo. (Zalewski A et al, Arterioscler. Thromb. Vasc. Biol., 25, 5, 923-31 (2005))

Literature (WO96/13484, WO96/19451, WO97/02242, WO97/12963, WO97/21675, WO97/21676, WO97/41098, WO97/41099, WO99/2442, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/87088, WO08/04886, US2008/0103156, US2008/0090851, US2008/0090852, WO08/048866, WO05/003118, WO06/063811, WO06/063813, WO2008/141176, WO2013013503A1, WO2013014185A1, WO2014114248A1, WO2014114694A1, WO2016011930A1, JP200188847 US2008/0279846A1, US 2010/0239565A1, US 2008/0280829A1) describe a number of Lp-PLA₂ inhibitors and/or their uses for the treatment of diseases involving or related to vascular endothelial dysfunction, diseases involving Lp-PLA₂ activity-associated (e.g., associated with formation of lysophosphatidylcholine and oxidized free fatty acids) lipid oxidation, and diseases involving activated monocytes, macrophages or lymphocytes or associated with increased involvement of monocytes, macrophages or lymphocytes. Examples of specific diseases include neurodegenerative diseases (e.g., Alzheimer's disease, Parkinson's disease, Huntington's disease, vascular dementia), various neuropsychiatric diseases such as schizophrenia and autism, peripheral and cerebral atherosclerosis , stroke, metabolic bone disease (e.g., bone marrow abnormalities), dyslipidemia, Paget's disease, type 2 diabetes, hypertension, angina pectoris, myocardial infarction, ischemia, reperfusion injury, metabolic syndrome, insulin resistance and parathyroid hyperfunction, diabetic complications (e.g., macular edema, diabetic retinopathy and posterior uveitis, diabetic ulcers, and diabetic nephropathy), diabetic peripheral neuropathy pain, inflammatory pain, neuropathic pain, various cancers (e.g., prostate cancer, colon cancer, breast cancer, kidney cancer, lung cancer and ovarian cancer, etc.), macular edema, wound healing, male erectile dysfunction, rheumatoid arthritis, chronic obstructive pulmonary disease (COPD), sepsis, acute and chronic inflammation, psoriasis and multiple sclerosis.

The scientific results further prove that Lp-PLA₂ inhibitors can be used to treat atherosclerosis. Wilensky et al demonstrated the effect of Lp-PLA₂ inhibitors on atherosclerotic plaque components in a porcine model of diabetes and hypercholesterolemia with accelerated coronary atherosclerosis (Wilensky et al, Nature Medicine, 10, 1015-1016 (2008)). Clinical studies have also found that Lp-PLA₂ inhibitors can stabilize sclerotic plaques in patients with atherosclerotic plaques, preventing further plaque development and rupture (Serruys et al., Circulation 118: 1172-1182 (2008)).

Studies have shown that high Lp-PLA₂ activity is associated with a high risk of dementia, including Alzheimer's disease (AD) and mixed dementia (Van Oijen et al., Annals of Neurology, 59, 139 (2006); Fitzpatrick et al., Atherosclerosis 235: 384-391 (2014)). Higher levels of oxidized LDL are observed in AD patients (Kassner et al, Current Alzheimer Research, 5, 358-366 (2008); Dildar et al, Alzheimer Dis Assoc Disord, 24, April-June (2010); Sinem et al, Current Alzheimer Research, 7, 463-469 (2010)).

In addition, US2008/0279846 describes that Lp-PLA₂ inhibitors reduce blood-brain barrier leakage and brain amyloid (Abeta) load, which can be used to treat diseases associated with blood-brain barrier leakage, such as Alzheimer's disease and vascular dementia. In clinical studies, Lp-PLA₂ inhibitors have been shown to prevent further deterioration of cognitive function in Alzheimer's patients (Maher-Edwards et al., Alzheimer's & Dementia: Translational Research & Clinical Interventions 1, 131-140 (2015)).

Neuroinflammation (involving the release of various cytotoxic cytokines) is a common feature of all neurodegenerative diseases (including multiple sclerosis, amyotrophic lateral sclerosis, Parkinson's disease, Alzheimer's disease, etc.) (Perry, Acta Neuropathol, 120, 277-286 (2010)). Lp-PLA₂ inhibitors reduce the release of various cytokines by inhibiting lysoPC production (Shi et al., Atherosclerosis 191, 54-62 (2007)). Therefore, the inhibition of Lp-PLA₂ is a potential treatment method for neurodegenerative diseases (including multiple sclerosis, amyotrophic lateral sclerosis, Parkinson's disease, etc).

LysoPC is also involved in leukocyte activation, inducing apoptosis and mediating vascular endothelial cell dysfunction (Wilensky et al., Current Opinion in Lipidology, 20, 415-420, (2009)). Therefore, Lp-PLA₂ inhibitors are believed to be useful in the treatment of diabetes-related tissue damage by reducing lysoPC production. High Lp-PLA₂ activity is associated with a high risk of developing diabetic retinopathy (Siddiqui et al., Diabetologia, 61, 1344-1353 (2018)). Lp-PLA₂ inhibitors can inhibit the main pathological changes of retinopathy in a diabetic rat model (Canning et al., P.N.A.S. 113, 7213-7218 (2016)). Clinical studies have also shown that Lp-PLA₂ inhibitors can improve the retina macular edema symptoms and visual acuity of diabetic retinopathy patients (Staurenghi et al, Ophthalmology 122, 990-996 (2015)). These studies demonstrate that Lp-PLA₂ inhibitors can be used in diabetic retinopathy.

Studies have shown that Lp-PLA₂ activity in diabetic patients is higher than in normal people (Serban et al. J. Cell. Mol. Med. 6:643-647, (2002); Garg et al. Indian J. Med. Res. 141:107- 114, (2015)). As mentioned above, the activity of Lp-PLA₂ mediates the oxidative inflammatory response. It is speculated that inhibiting the activity of Lp-PLA₂ can be used to treat various complications caused by oxidative inflammatory response in diabetic patients, such as diabetic nephropathy, diabetic peripheral nerve lesions and diabetic skin ulcers, etc.

Glaucoma and age-related macular degeneration (AMD) are retinal neurodegenerative diseases. Inflammation plays an important role in the pathogenesis of glaucoma and AMD (Buschini et al, Progress in Neurobiology, 95, 14-25 (2011); Tezel, Progress in Brain Research, vol. 173, ISSN0079-6123, Chapter 28). Therefore, Lp-PLA₂ inhibitors may offer potential therapeutic applications for glaucoma and AMD.

In male erectile dysfunction patients, the activity of Lp-PLA₂ in vivo was significantly higher than that of normal individuals, and it is believed that high Lp-PLA₂ activity can be used to predict early male erectile dysfunction (Otunctemur et al., Andrologia 47:706-710 (2015)), suggesting that Lp -PLA2 inhibitors can be used to treat male erectile dysfunction.

There is high expression of Lp-PLA₂ in prostate cancer tissue, and reducing Lp-PLA₂ can reduce prostate cell carcinogenesis and promote prostate cancer cell apoptosis in vitro (Vainio et al., Oncotarget, 2: 1176-1190 (2011)), suggesting that Lp-PLA₂ inhibitors can be used to treat prostate cancer.

However, there is still a strong need for new Lp-PLA₂ inhibitors in the prior art.

### Contents of the present invention

The purpose of the present invention is to provide a pyrimidone compound and a pharmaceutical composition thereof, which can be used as Lp-PLA₂ inhibitor.

In a first aspect, the present invention relates to a compound of Formula I, cis-trans isomer thereof, enantiomer thereof, diastereomer thereof, racemate thereof, solvate thereof, hydrate thereof or pharmaceutically acceptable salt thereof or prodrug thereof, 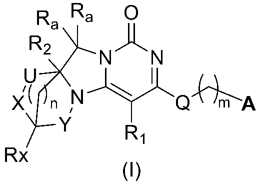 wherein
n is 0, 1 or 2, and when n is 0, R₂ is methyl or ethyl, and when n is 1 or 2, R₂ is absent;
R₁ is H, halogen, cyano, C₁₋₆ alkyl, C₁₋₆ alkoxy, C₃₋₈ cycloalkyl or 3- to 8-membered heterocyclyl, R₁ may be optionally substituted with one or more of the following substituents: halogen, cyano, C₁₋₆ alkoxy, C₃₋₈ cycloalkyl, 3- to 8-membered heterocyclyl or 6- to 10-membered heteroaryl;
Rₐ independently is H or D;
m is 1 or 2;
Rₓ is H, halogen, hydroxyl, carboxyl, cyano, amino, C₁₋₆ alkyl, C₁₋₆ alkoxy, C₃₋₈ cycloalkyl, 3- to 8-membered heterocyclyl, 6- to 10-membered aryl, 6- to 10-membered heteroaryl, - C(O)NR_{b}R_{c}, -S(O)₂NR_{b}R_{c}, Rₓ can be optionally substituted with one or more of the following substituents: halogen, hydroxyl, C₁₋₆ alkoxy, cyano, C₃₋₈ cycloalkyl, 3- to 8-membered heterocyclyl, 6- to 10-membered aryl or 6- to 10-membered heteroaryl;
Q is -O-, -S-, -CH₂- or -NR_{b}-;
X is -O-, -CH₂-, -NR_{c}-, -OCH₂- or absent;
R_{b} is H, C₁₋₆ alkyl or C₃₋₈ cycloalkyl or 3- to 8-membered heterocyclyl;
R_{c} is L, L-C(O)-, L-CH₂- or L-S(O)₂-, wherein L is H, C₁₋₆ alkyl, C₃₋₆ cycloalkyl, 3- to 8-membered heterocyclyl, 6- to 10-membered aryl or 6- to 10-membered heteroaryl, L may be optionally substituted with one or more of the following groups: halogen, hydroxyl, C₁₋₆ alkoxy, cyano, C₃₋₈ cycloalkyl, 3- to 8-membered heterocyclyl, 6- to 10-membered aryl or 6- to 10-membered heteroaryl;
Y is -CH₂-, -CH₂CH₂- or absent;
U is -CH₂-, -C(O)- or absent;
X and U are not absent at the same time;
Y and U may be optionally substituted with one or more of the following substituents: halogen, hydroxy, C₁₋₆ alkyl, C₁₋₆ alkoxy, cyano, C₃₋₈ cycloalkyl, 3- to 8-membered heterocyclyl , 6- to 10-membered aryl or 6- to 10-membered heteroaryl;
A is 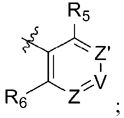
Z is N or CR₃;
Z' is N or CR₄;
R₃, R₄, R₅, R₆ are independently H, cyano, halogen or C₁₋₃ haloalkyl;
V is N or CR₉, wherein R₉ is H, cyano, halogen, C₁₋₃ alkyl, C₁₋₃ haloalkyl or -O-W;
W is phenyl or 5- or 6-membered heteroaryl, which may be optionally substituted with one or more of the following substituents: halogen, cyano, C₁₋₆ alkyl, C₁₋₃ alkoxy, C₁₋₃ haloalkyl and C₁₋₃ haloalkoxy.

In some embodiments, wherein n is 0; R₁ is H, cyano, halogen, C₁₋₆ alkyl, C₁₋₆ alkoxy, or C₁₋₃ haloalkyl; Rₐ is H; Rₓ is H, cyano, fluorine, difluoromethyl, amino, 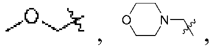 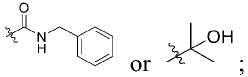 R₂ is methyl or ethyl; Q is -O-; X is -O-, -CH₂ or absent; Y is -CH₂-; U is -CH₂-, or absent; X and U are not absent at the same time.

Further, R₁ is H and Rₓ is H.

In some embodiments, wherein n=1 or 2, R₂ is absent. Further, wherein Rₓ is H. Further, wherein X is -O- or -CH₂-.

In some embodiments, wherein n is 1; Rₓ is H; and R₁ is H, cyano, amino, halogen, C₁₋₃ alkyl, C₁₋₃ haloalkyl, or C₁₋₆ alkoxy.

In some embodiments, wherein U is -CH₂-; X is -CH₂- or -O- and Q is -O-.

In some embodiments, wherein U is -CH₂-; X is -NR_{c}- and R_{c} is methyl, oxetanyl, trifluoroethyl, benzoyl, cyclobutyl, benzyl,

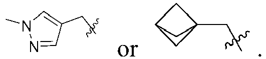

In some embodiments, wherein U is -C(O)-; R₁ and Rₓ are both H, Y is -CH₂-; X is -NR_{c}-, R_{c} is L or L-C(O)-, wherein L is methyl , trifluoroethyl, benzoyl, oxetane, cyclobutane, benzyl, 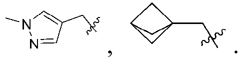 Further, L is methyl.

In some embodiments, wherein n is 1; X is -CH₂-; U is absent; Rₓ is H, hydroxy, halogen, cyano, amino, C₁₋₃ alkoxy, C₁₋₃ haloalkyl, 3- to 8-membered heterocyclyl, Rₓ may be optionally substituted with one or more of the following substituents: halogen, hydroxy, C₁₋₆ alkoxy, cyano, 3- to 8-membered heterocyclyl, 6- to 10-membered aryl or 6- to 10-membered heteroaryl.

In some embodiments, wherein Y is -CH₂-; R₁ is H, cyano, halo, C₁₋₃ alkyl, C₁₋₃ haloalkyl, C₁₋₆ alkoxy; Q is -O-.

In some embodiments, wherein Rₓ is H, halo, cyano, amino, difluoromethyl,

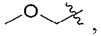

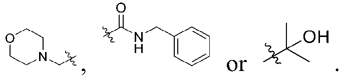

In some embodiments, wherein Y is -CH₂CH₂-; Rₓ is H, halogen, cyano, amino, difluoromethyl, 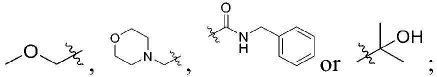 R₁ is H, cyano, halogen, C₁₋₃ alkyl, C₁₋₃ haloalkyl or C₁₋₆ alkoxy; Q is -O-. Further, Rₓ is H.

In some embodiments, wherein n is 2; U is -CH₂-; X is -CH₂- or -O-; Y is -CH₂- or absent; Rₓ is H, halogen, cyano, amino, difluoromethyl, 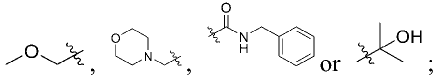 R₁ is H, cyano, halogen, C₁₋₃ alkyl, C₁₋₃ haloalkyl or C₁₋₆ alkoxy; and Q is -O-. Further, Rₓ is H and R₁ is H.

In some embodiments, wherein m=2; R₁ is H, cyano or C₁₋₃ haloalkyl; and Rₓ is H.

In some embodiments, wherein m=1, R₁ is H, cyano, halogen, C₁₋₃ alkyl, C₁₋₃ haloalkyl, or C₁₋₆ alkoxy; and Rₓ is H.

In some embodiments, wherein A is 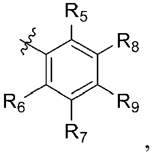 R₅, R₆, R₇, R₈, R₉ are independently H, F or cyano.

In some embodiments, wherein A is 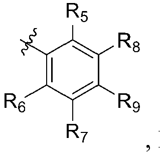 R₅, R₆, R₇ and R₈ are independently H, F or cyano; R₉ is -O-W, and W is a 5- or 6-membered heteroaryl or phenyl, which may be optionally substituted with one or more of the following substituents: C₁₋₃ haloalkyl, C₁₋₃ haloalkoxy, cyano, halogen and C₁₋₆ alkyl.

In some embodiments, wherein A is 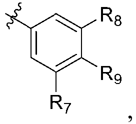 R₇ and R₈ are independently H, F or cyano; R₉ is -O-W; W is pyridyl, pyrimidinyl, pyrazolyl or phenyl, which may be optionally substituted with one or more substituents independently selected from the group consisting of halogen, cyano, CF₃, -OCF₃, CHF₂ and CH₃.

In some embodiments, the compound is of Formula I' 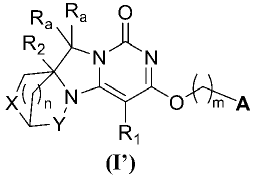 wherein R₁ is H, cyano, halogen, C₁₋₆ alkyl, C₁₋₃ alkoxy or C₁₋₃ haloalkyl; X is -O-, -CH₂-, - NR_{c}- or absent; R_{c} is L or L-C(O)-, where L is H, C₁₋₃ alkyl, C₃₋₆ cycloalkyl, C₃₋₆ heterocycloalkyl, C₁₋₃ haloalkyl, or benzyl; Y is -CH₂- or absent ; n, R₂, Rₐ, A, m are as defined in Formula (I) above.

In some embodiments, n is 0, R₂ is methyl or ethyl; R₁ is H; Rₐ is H; m is 1; X is -O- or -CH₂-.

In some embodiments, the compound is one of the following compounds: 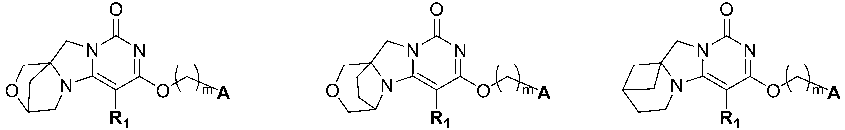 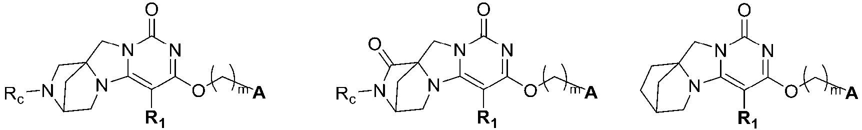 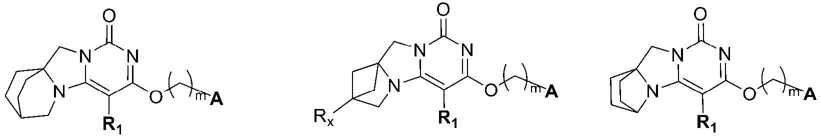 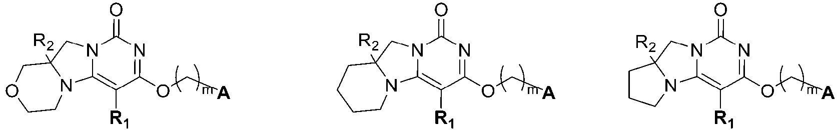 wherein, R₁ is H, halogen, cyano, C₁₋₆ alkyl, C₁₋₃ haloalkyl or C₁₋₆ alkoxy (further, the halogen is fluorine or chlorine, and the C₁₋₆ alkyl is methyl, ethyl or isopropyl, the C₁₋₃ haloalkyl is trifluoromethyl, and the C₁₋₆ alkoxy is methoxy); R₂ is methyl or ethyl; R_{c} is C₁₋₆ alkyl, 3- to 8-membered heterocyclyl, C₁₋₃ haloalkyl, benzoyl, C₃₋₈ cycloalkyl, benzyl or 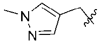 (further, the C₁₋₆ alkyl is methyl, the 3- to 8-membered heterocyclyl is oxetanyl, the C₁₋₃ haloalkyl is trifluoroethyl and the C₃₋₈ cycloalkyl is cyclobutyl); Rₓ is H, cyano, halogen, C₁₋₃ haloalkyl, amino, 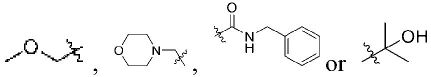 (further, the halogen is fluorine and the C₁₋₃ haloalkyl is difluoromethyl).

Further, the compound is one of the following compounds: 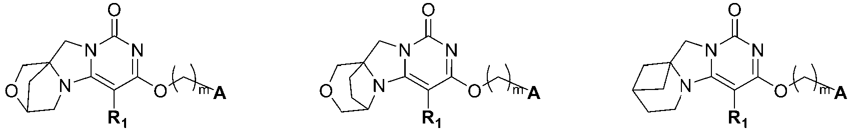 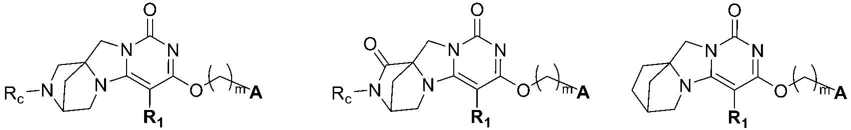 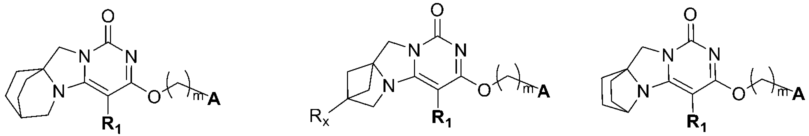 wherein, R₁ is H, halogen, cyano, C₁₋₆ alkyl, C₁₋₃ haloalkyl or C₁₋₆ alkoxy (further, the halogen is fluorine or chlorine, and the C₁₋₆ alkyl is methyl, ethyl or isopropyl, the C₁₋₃ haloalkyl is trifluoromethyl, and the C₁₋₆ alkoxy is methoxy); R₂ is methyl or ethyl; R_{c} is C₁₋₆ alkyl, 3- to 8-membered heterocyclyl, C₁₋₃ haloalkyl, benzoyl, C₃₋₈ cycloalkyl, benzyl or 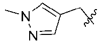 (further, the C₁₋₆ alkyl is methyl, the 3- to 8-membered heterocyclyl is oxetanyl, the C₁₋₃ haloalkyl is trifluoroethyl and the C₃₋₈ cycloalkyl is cyclobutyl); Rₓ is H, cyano, halogen, C₁₋₃ haloalkyl, amino, 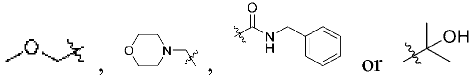 (further, the halogen is fluorine and the C₁₋₃ haloalkyl is difluoromethyl).

In some embodiments, in the compound of general Formula (I) above, A is selected from the following groups:

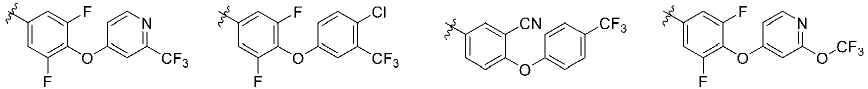

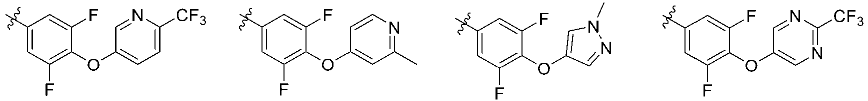

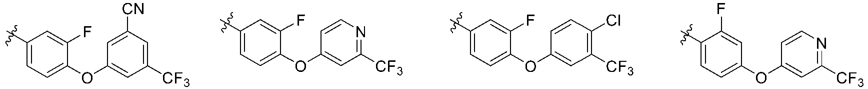

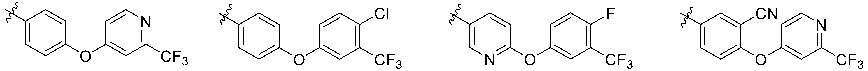

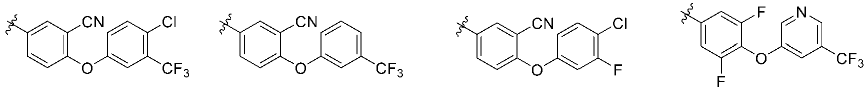

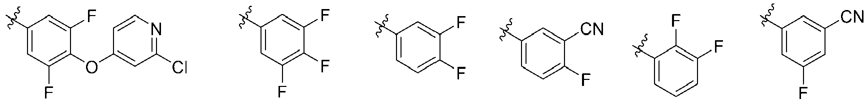

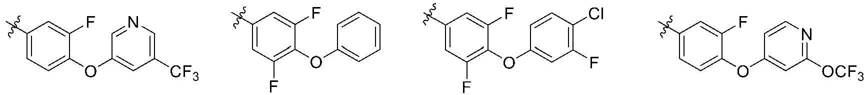

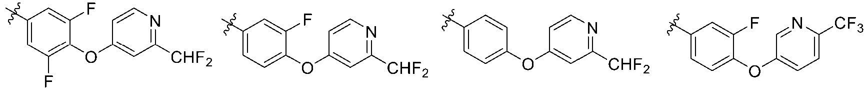

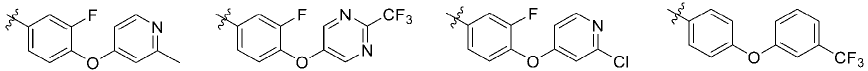

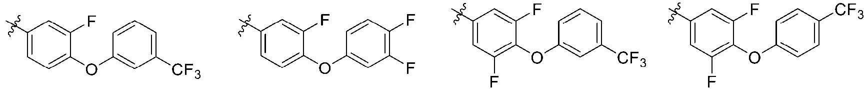

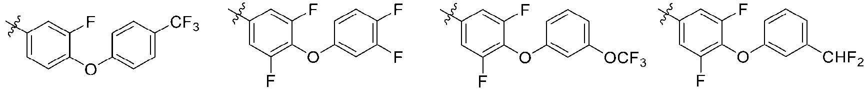

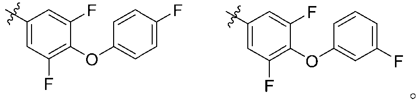

In some embodiments, the compound of general Formula (I) above is one of the following compound:

| Example | Structure | Example | Structure |
|---|---|---|---|
| **1** | 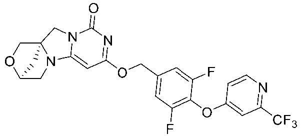 | **2** | 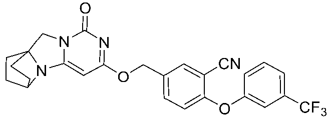 |
| **4** | 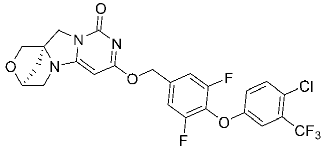 | **5** | 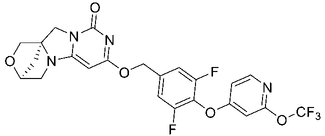 |
| **6** | 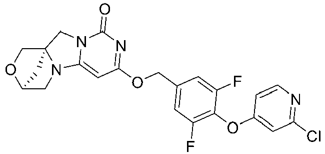 | **7** | 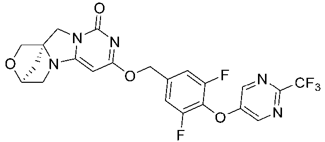 |
| **8** | 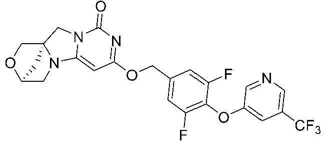 | **9** | 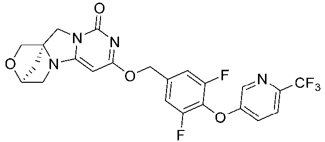 |
| **10** | 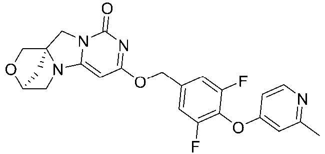 | **11** | 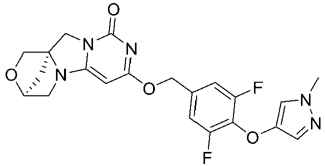 |
| **12** | 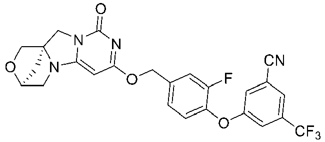 | **13** | 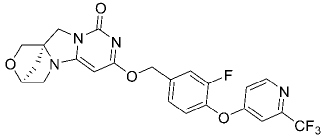 |
| **14** | 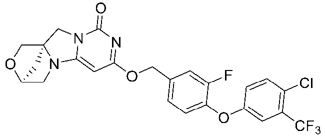 | **15** | 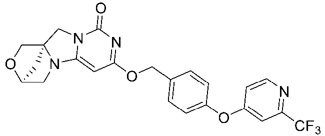 |
| **16** | 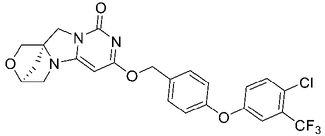 | **17** | 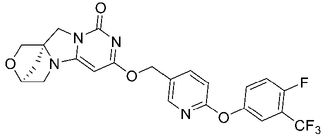 |
| **18** | 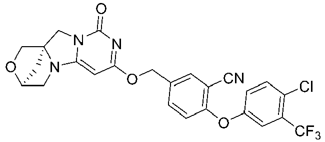 | **19** | 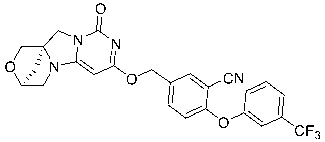 |
| **20** | 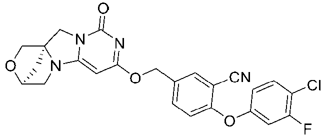 | **21** | 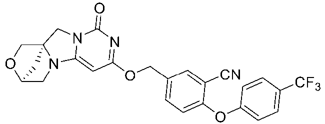 |
| **22** | 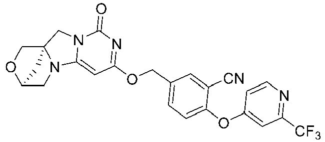 | **23** | 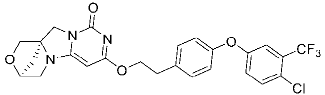 |
| **24** | 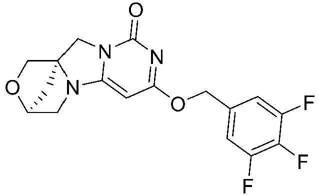 | **25** | 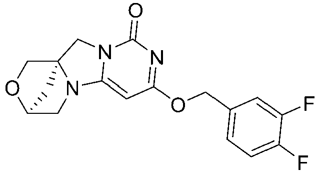 |
| **26** | 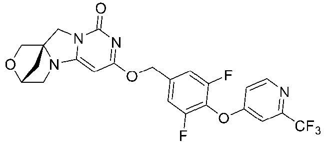 | **27** | 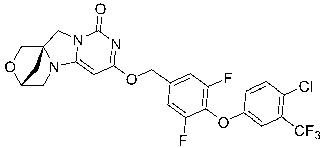 |
| **28** | 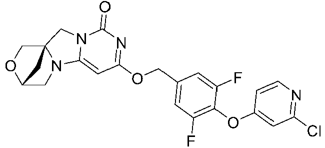 | **29** | 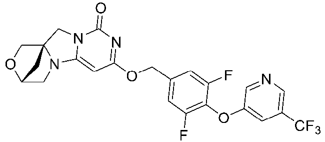 |
| **30** | 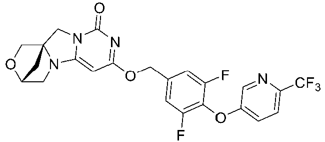 | **31** | 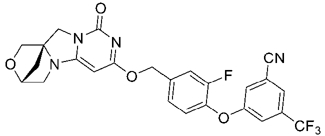 |
| **32** | 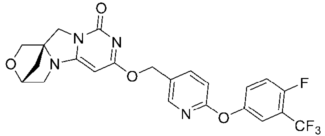 | **33** | 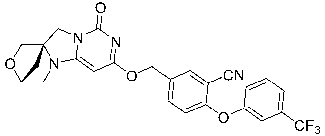 |
| **34** | 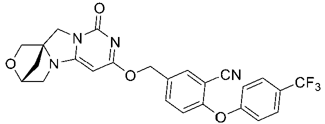 | **35** | 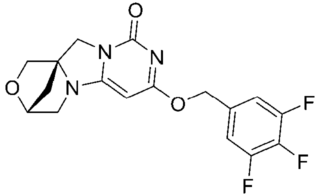 |
| **36** | 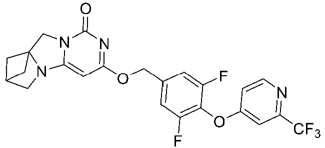 | **37** | 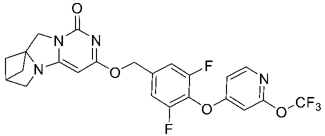 |
| **38** | 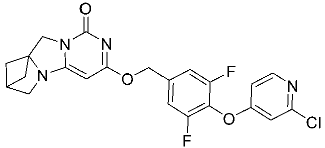 | **39** | 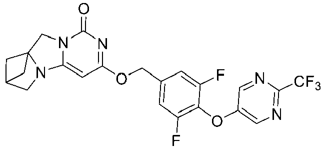 |
| **40** | 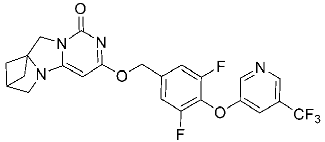 | **41** | 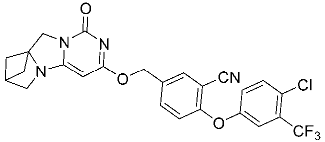 |
| **42** | 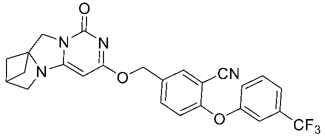 | **43** | 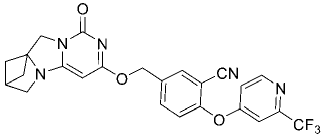 |
| **44** | 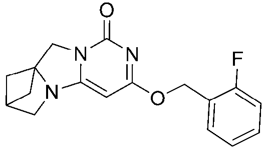 | **45** | 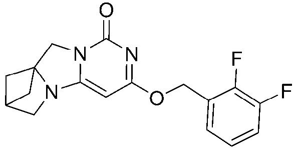 |
| **46** | 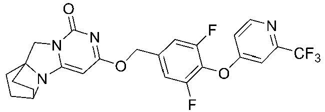 | **47** | 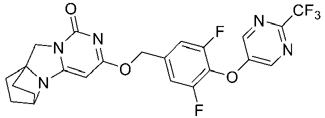 |
| **48** | 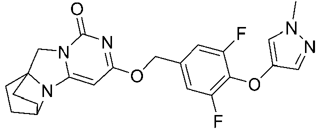 | **49** | 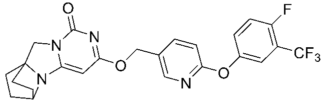 |
| **50** | 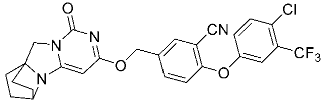 | **51** | 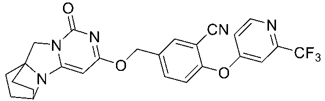 |
| **52** | 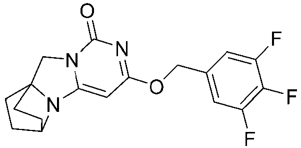 | **53** | 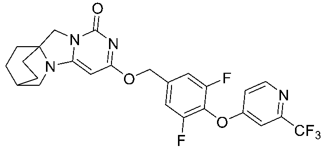 |
| **54** | 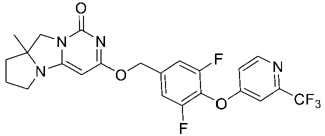 | **55** | 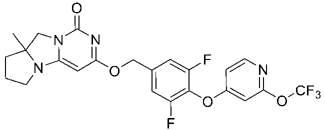 |
| **56** | 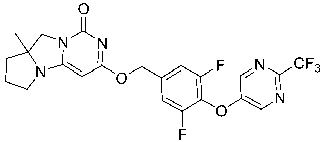 | **57** | 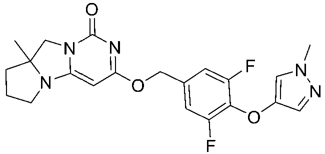 |
| **58** | 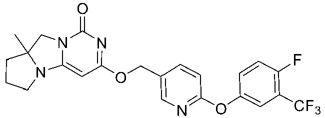 | **59** | 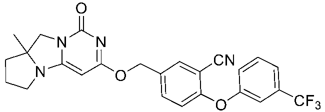 |
| **60** | 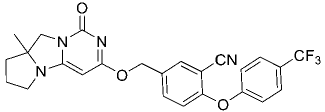 | **61** | 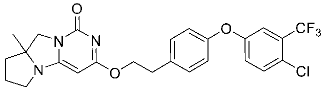 |
| **62** | 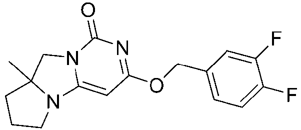 | **63** | 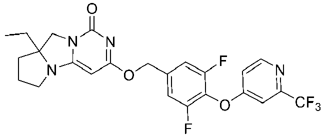 |
| **64** | 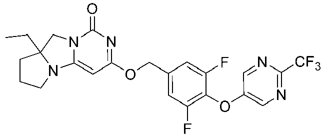 | **65** | 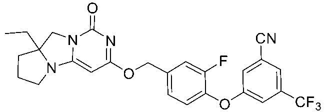 |
| **66** | 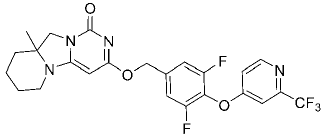 | **67** | 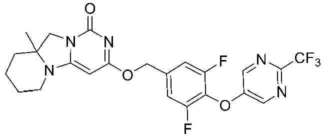 |
| **68** | 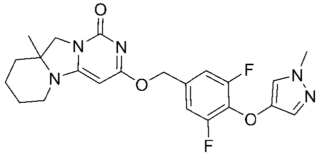 | **69** | 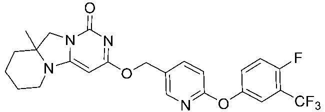 |
| **70** | 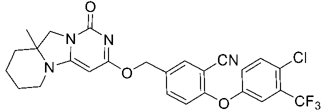 | **71** | 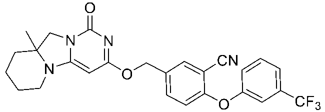 |
| **72** | 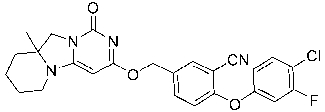 | **73** | 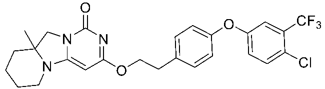 |
| **74** | 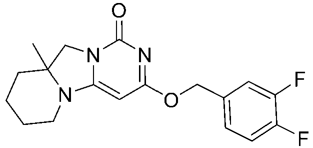 | **75** | 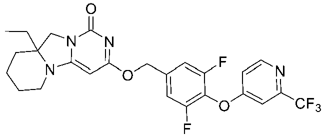 |
| **76** | 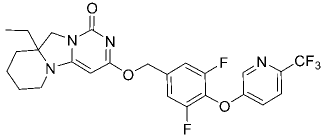 | **77** | 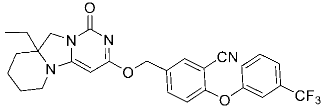 |
| **78** | 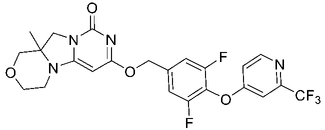 | **79** | 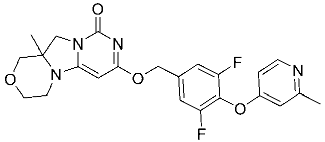 |
| **80** | 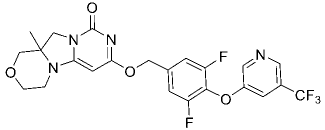 | **81** | 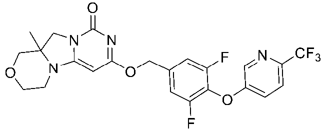 |
| **82** | 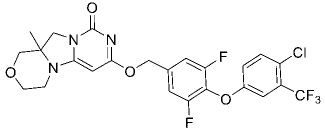 | **83** | 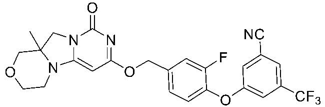 |
| **84** | 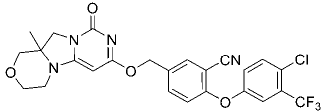 | **85** | 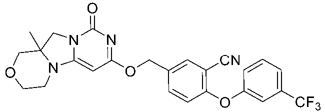 |
| **86** | 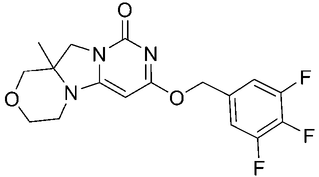 | **88** | 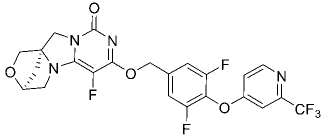 |
| **89** | 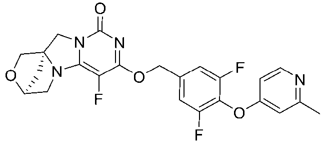 | **90** | 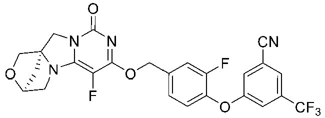 |
| **91** | 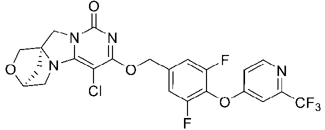 | **92** | 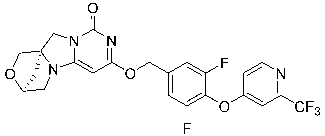 |
| **93** | 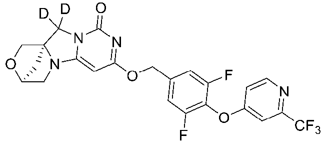 | **94** | 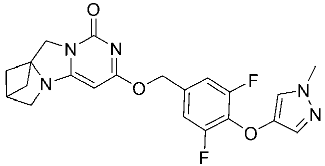 |
| **95** | 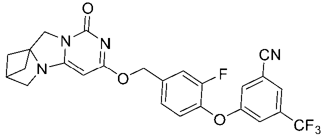 | **96** | 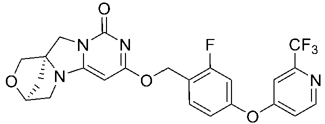 |
| **97** | 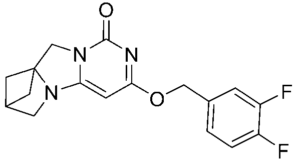 | **98** | 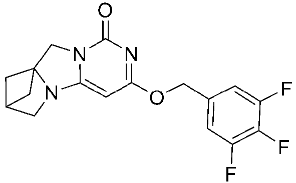 |
| **99** | 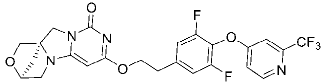 | **100** | 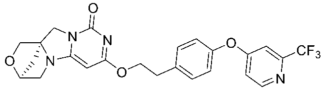 |
| **101** | 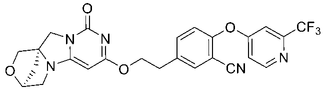 | **102** | 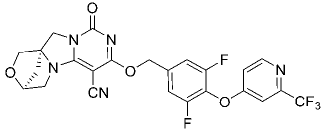 |
| **103** | 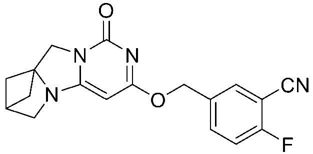 | **104** | 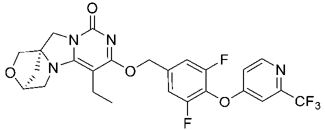 |
| **105** | 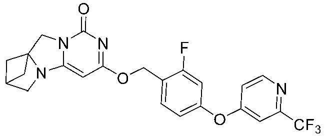 | **106** | 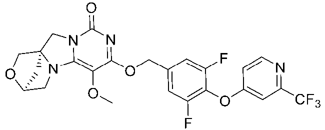 |
| **107** | 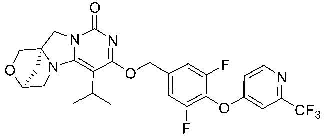 | **108** | 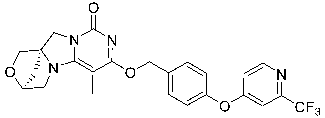 |
| **109** | 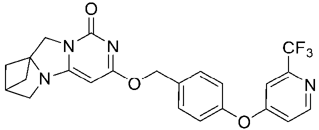 | **110** | 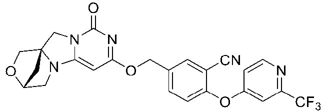 |
| **111** | 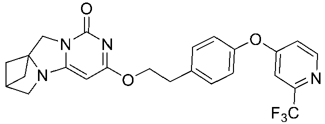 | **112** | 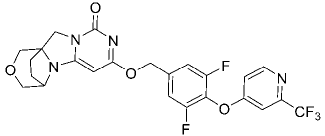 |
| **113** | 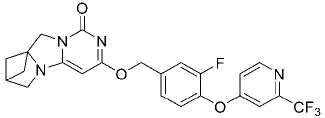 | **114** | 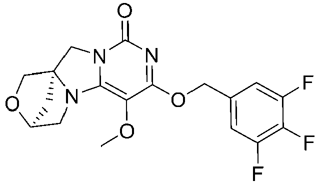 |
| **115** | 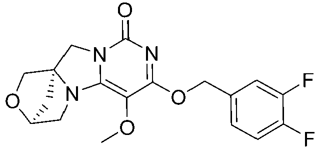 | **116** | 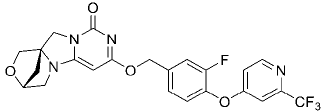 |
| **117** | 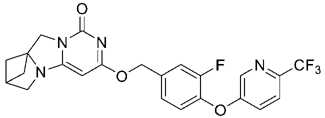 | **118** | 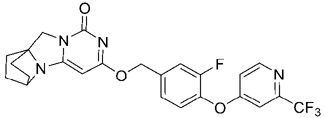 |
| **119** | 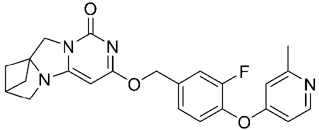 | **120** | 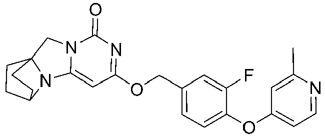 |
| **121** | 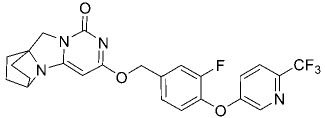 | **122** | 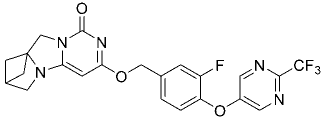 |
| **123** | 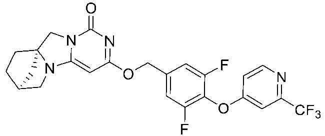 | **124** | 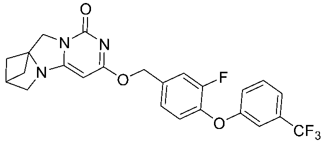 |
| **125** | 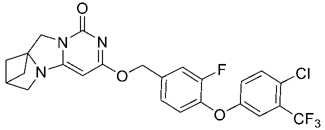 | **126** | 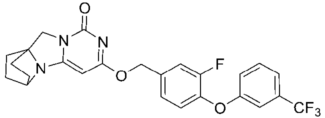 |
| **127** | 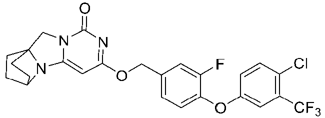 | **128** | 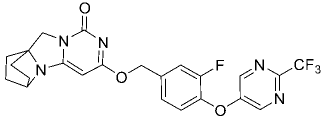 |
| **129** | 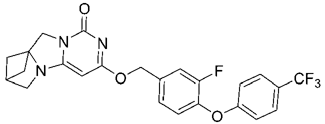 | **130** | 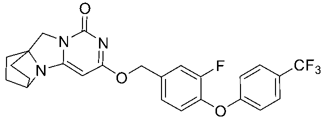 |
| **131** | 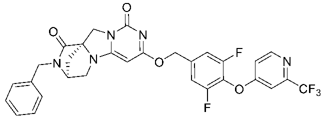 | **132** | 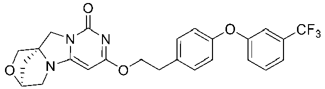 |
| **133** | 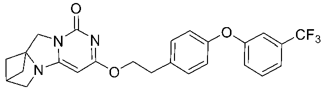 | **134** | 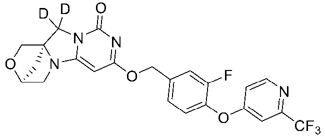 |
| **135** | 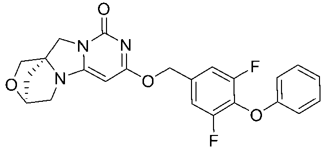 | **136** | 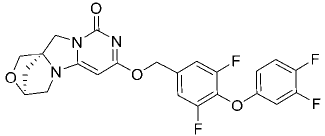 |
| **137** | 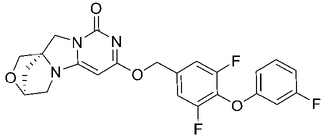 | **138** | 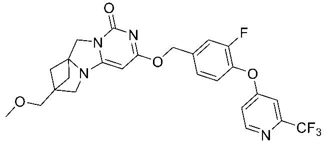 |
| **139** | 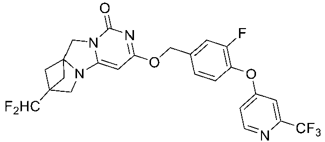 | **140** | 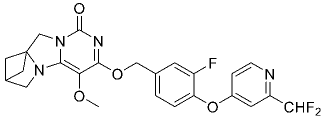 |
| **141** | 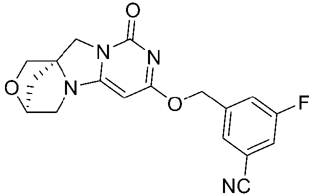 | **142** | 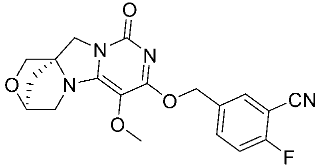 |
| **143** | 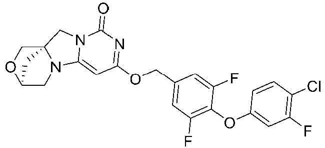 | **144** | 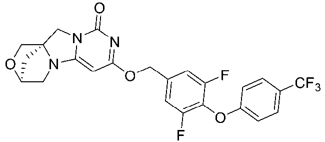 |
| **145** | 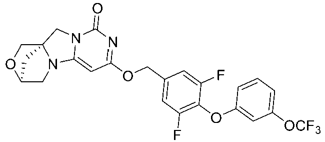 | **146** | 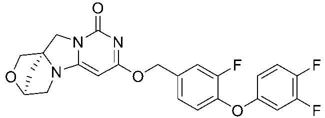 |
| **147** | 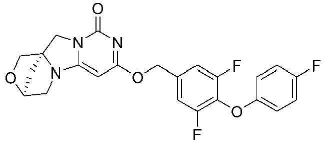 | **148** | 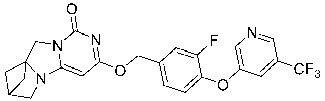 |
| **149** | 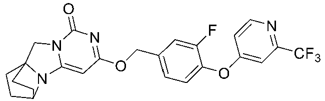 | **150** | 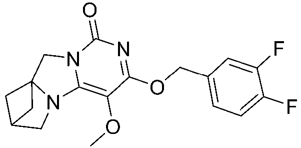 |
| **151** | 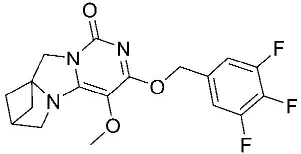 | **152** | 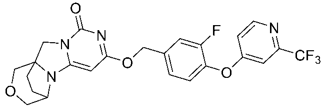 |
| **153** | 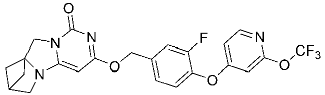 | **154** | 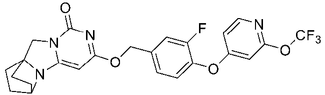 |
| **155** | 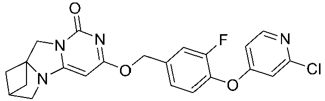 | **156** | 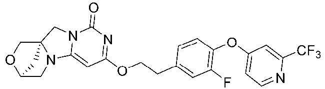 |
| **157** | 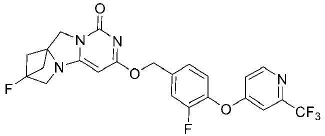 | **158** | 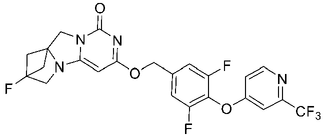 |
| **159** | 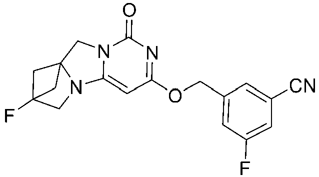 | **160** | 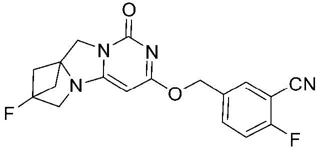 |
| **161** | 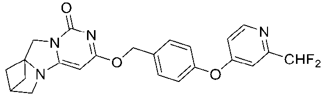 | **162** | 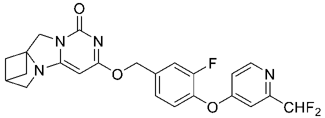 |
| **163** | 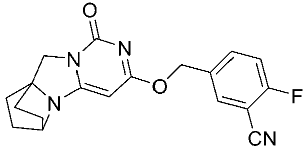 | **164** | 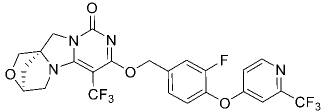 |
| **165** | 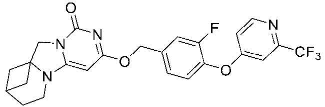 | **166** | 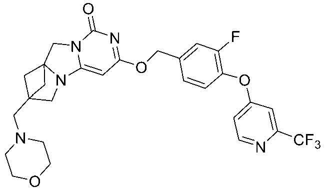 |
| **167** | 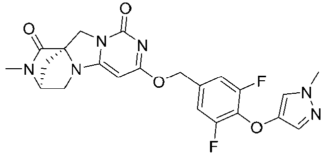 | **168** | 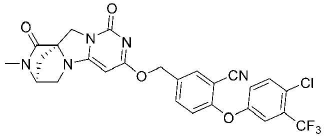 |
| **169** | 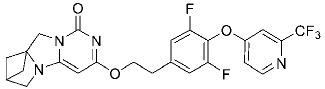 | **170** | 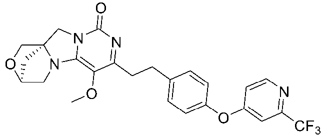 |
| **171** | 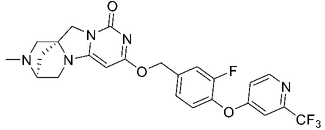 | **172** | 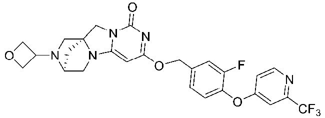 |
| **173** | 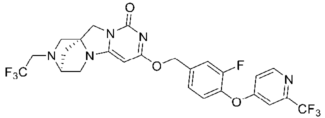 | **174** | 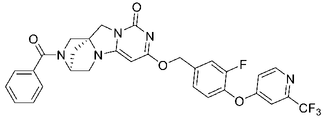 |
| **175** | 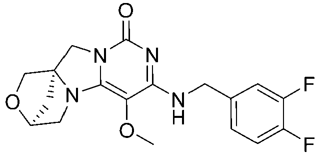 | **176** | 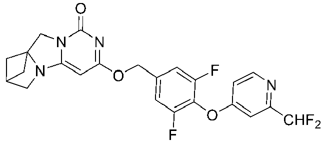 |
| **177** | 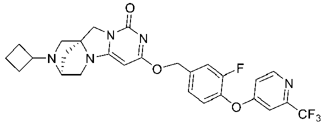 | **178** | 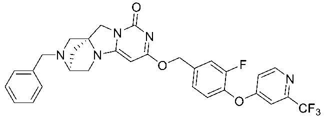 |
| **179** | 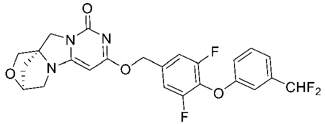 | **180** | 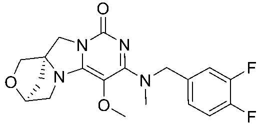 |
| **181** | 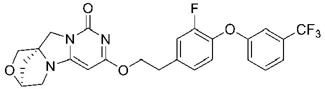 | **182** | 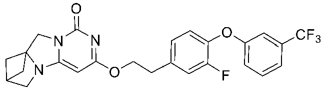 |
| **183** | 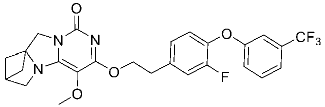 | **184** | 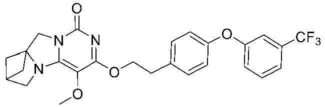 |
| **185** | 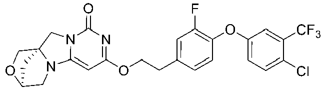 | **186** | 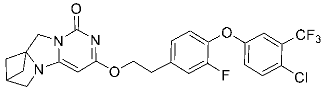 |
| **187** | 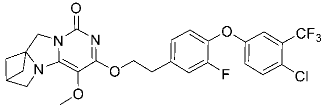 | **188** | 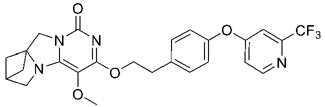 |
| **189** | 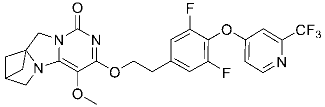 | **190** | 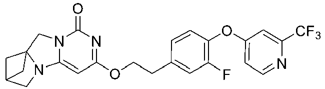 |
| **191** | 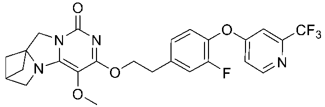 | **192** | 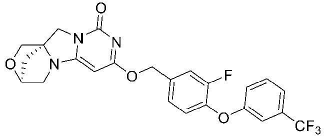 |
| **193** | 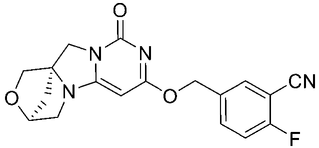 | **194** | 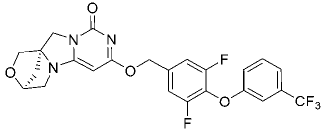 |
| **195** | 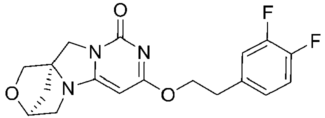 | **196** | 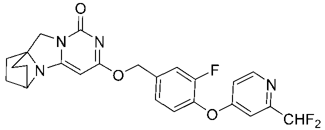 |
| **197** | 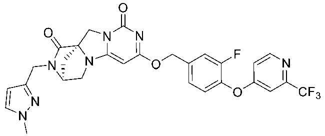 | **198** | 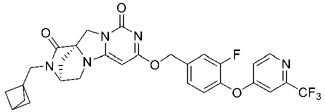 |
| **199** | 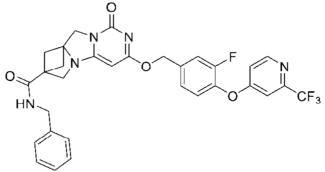 | **200** | 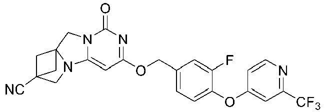 |
| **201** | 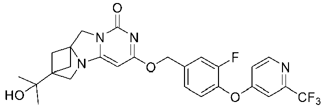 | **202** | 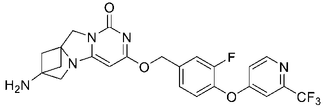 |

In the second aspect, the present invention relates to a compound represented by Formula I', its cis-trans isomer, its enantiomer, its diastereomer, its racemate, its solvate, its hydrate or its pharmaceutically acceptable salt or its prodrug, 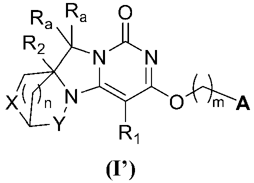 wherein
n is 0, 1 or 2, and when n is 0, R₂ is methyl or ethyl, and when n is not 0, R₂ is absent;
R₁ is H, halogen, C₁₋₆ alkyl or C₃₋₆ cycloalkyl;
Rₐ independently is H or D;
m is 1 or 2;
X is -O-, -CH₂-, -NR- or absent;
R is L-C(O)-, L-CH₂- or L-S(O)₂-, wherein L is H, C₁₋₃ alkyl, C₃₋₆ cycloalkyl or phenyl;
Y is -CH₂- or is absent;
A is 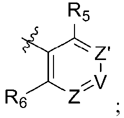
Z is N or CR₃;
Z' is N or CR₄;
R₃, R₄, R₅, R₆ are independently H, CN, halogen or C₁₋₃ haloalkyl;
V is N or CR₉, wherein R₉ is H, CN, halogen, C₁₋₃ alkyl, C₁₋₃ haloalkyl or -O-W;
W is 5- or 6-membered heteroaromatic ring or phenyl, which may be optionally substituted with one or more of the following substituents: C₁₋₃ haloalkyl, C₁₋₃ haloalkoxy, CN, halogen, and C₁₋₅ alkyl.

In some embodiments, the compound of general Formula (I') is characterized in that n is not 0, and has one or more of the following features:
(1) R₁ is H, Rₐ is H;
(2) X is O or is absent, Y is -CH₂-, and n is 1;
(3) X is -CH₂-, Y is -CH₂- or is absent, and n is 2;
(4) m is 1.

In some embodiments, the compound of general Formula (I') is characterized in that n is 0, and has one or more of the following features:
(1) R₁ is H, Rₐ is H;
(2) R₂ is methyl;
(3) X is absent, -O-, -CH₂-;
(4) m is 1.

In one embodiment, A is 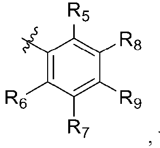 wherein R₅, R₆, R₇, R₈, R₉ are independently H, F or CN.

In one embodiment, A is 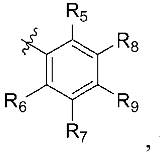 wherein R₅, R₆, R₇, R₈ are independently H, F or CN; R₉ is -O-W; W is 5- or 6-membered heteroaryl or phenyl, wherein the heteroaryl or phenyl is optionally substituted with one or more of the following substituents: C₁₋₃ haloalkyl, C₁₋₃ haloalkoxy, CN, halogen and C₁₋₅ alkyl.

In one embodiment, A is 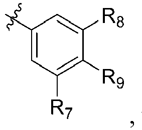 wherein R₇, R₈ are independently H, F or CN; R₉ is - O-W; W is pyridyl, pyrimidinyl, pyrazolyl and phenyl, wherein the pyridyl, pyrimidinyl, pyrazolyl or phenyl is optionally substituted with one or more of the following substituents: halogen, CN, CF₃, -OCF₃ and CH₃.

In some embodiments, A is

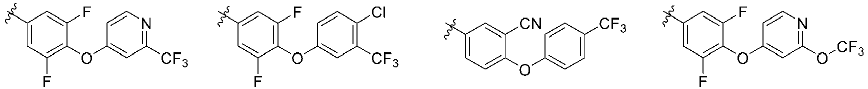

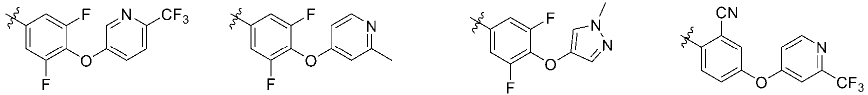

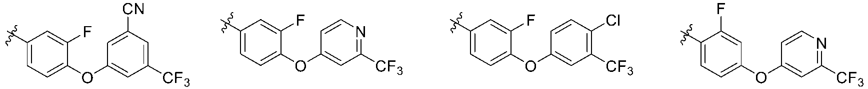

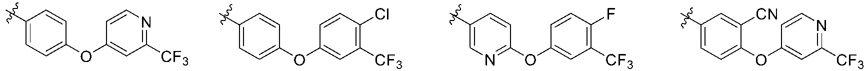

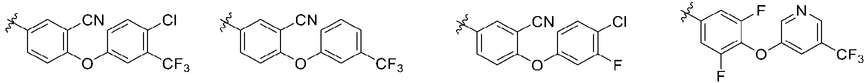

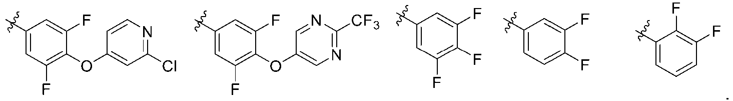

In one embodiment, the compound is any one of the following compounds:

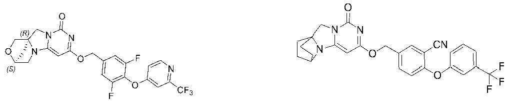

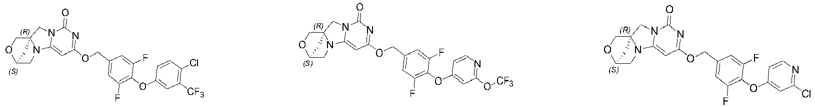

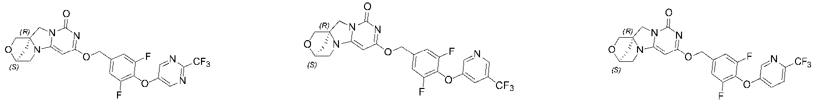

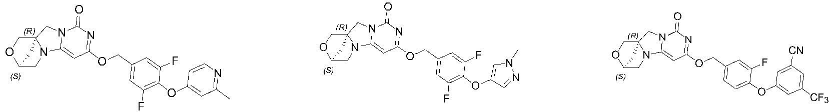

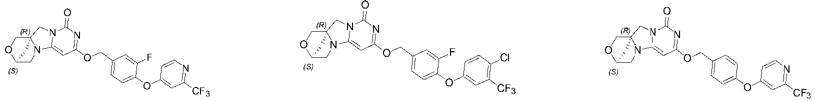

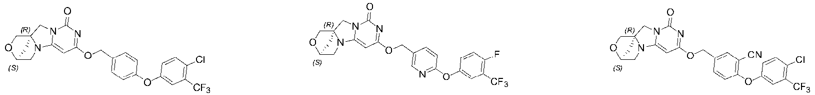

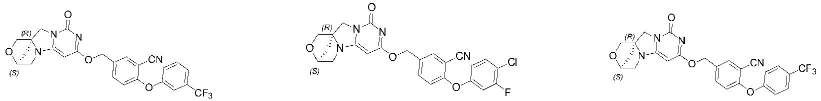

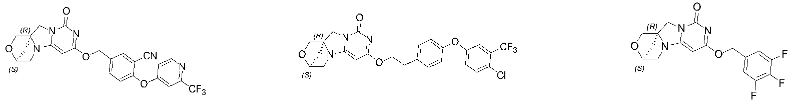

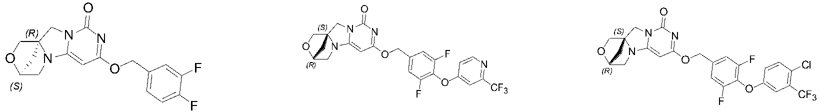

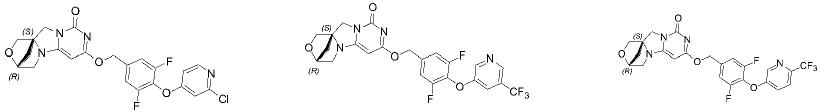

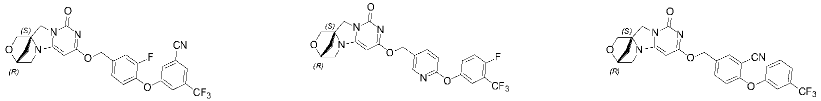

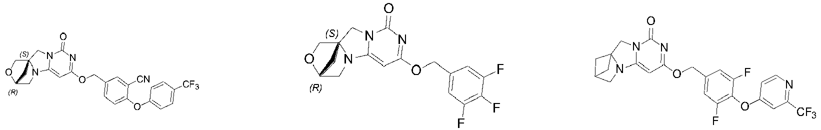

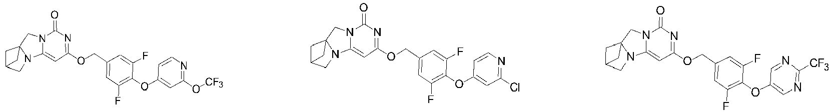

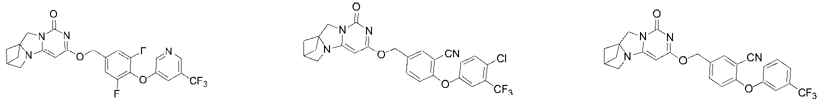

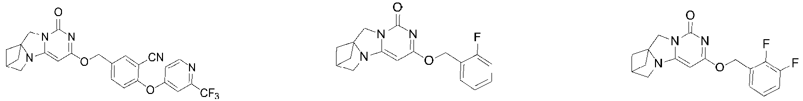

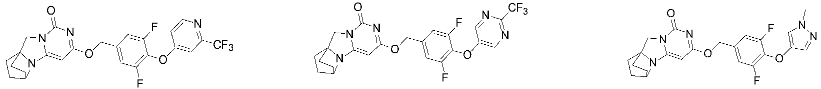

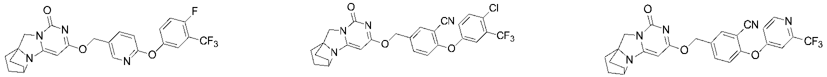

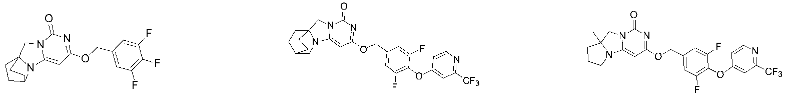

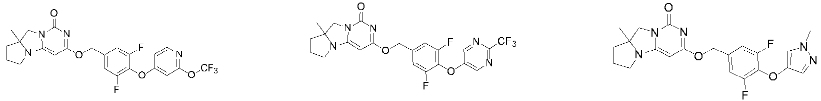

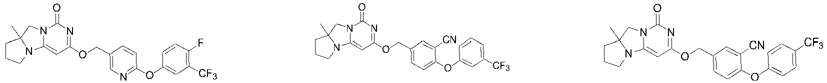

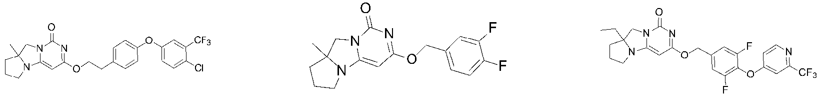

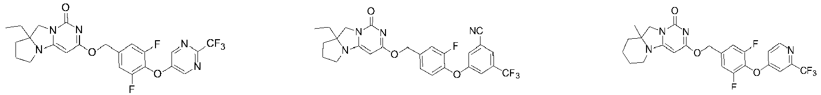

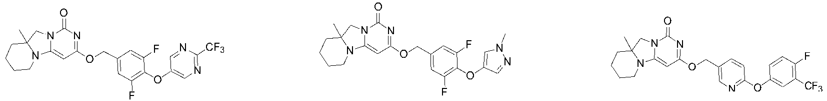

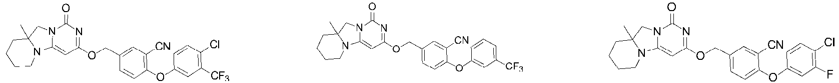

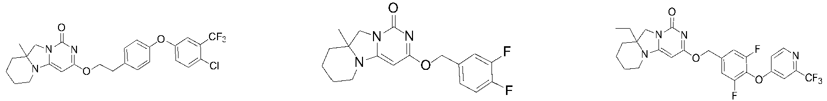

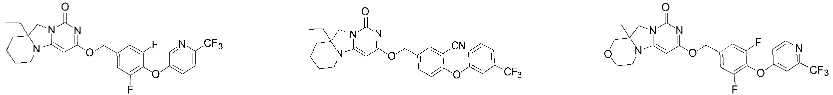

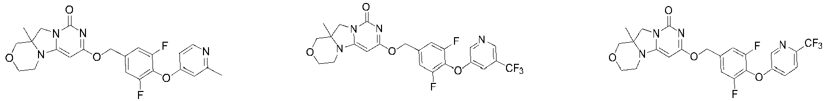

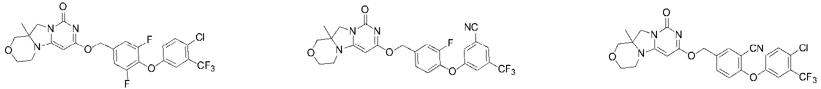

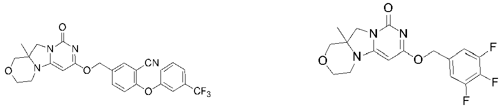

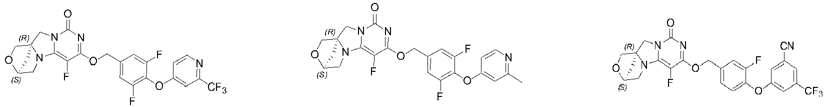

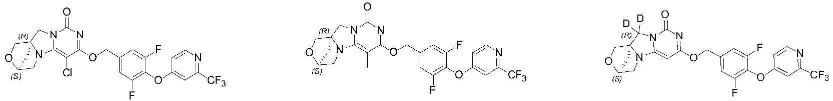

The compounds of the above formula, and salts thereof (e.g., pharmaceutically acceptable salts) may exist in stereoisomeric forms (e.g., which contain one or more asymmetric carbon atoms). The individual stereoisomers (enantiomers and diastereomers) and mixtures thereof are included within the scope of the present invention.

The present invention also comprises various deuterated forms of the compounds of the above formula, and salts thereof (e.g., pharmaceutically acceptable salts). Each available hydrogen atom attached to carbon atom can be independently replaced by a deuterium atom. One of ordinary skill in the art would know how to synthesize deuterated forms of the compounds of the above formula and salts thereof (e.g., pharmaceutically acceptable salts). Commercially available deuterated starting materials can be used in the preparation of deuterated forms of the compounds of the above formula and salts thereof (e.g., pharmaceutically acceptable salts); alternatively, conventional methods employing deuterated reagents such as lithium aluminum deuteride can be used to synthesize these compounds.

In addition to the free base or free acid forms of the compounds described herein, the salt forms of the compounds are also within the scope of the present invention. The salts or pharmaceutically acceptable salts of the compounds of the present invention can be prepared in situ during the final isolation and purification of the compounds, or prepared by separately reacting the purified compounds in free acid or free base form with a suitable base or acid, respectively. For reviews of suitable pharmaceutical salts, see: Berge et al., J. Pharm, Sci., 66, 1-19, 1977; PL Gould, International Journal of Pharmaceutics, 33 (1986), 201-217; and Bigley et al. , Encyclopedia of Pharmaceutical Technology, Marcel Dekker Inc, New York 1996, Volume 13, pages 453-497.

The compounds described herein, the salts thereof (e.g., pharmaceutically acceptable salts), deuterated forms, solvates or hydrates thereof can exist in one or more polymorphic forms. Thus, in another aspect, the present invention provides polymorphs of the compound and salt thereof (e.g., pharmaceutically acceptable salt) as defined herein, or polymorphs of the solvates or hydrates of the compound or salt thereof (e.g., a pharmaceutically acceptable salt) as described herein.

The present invention also comprises isotopically labeled compounds and salts, which are equivalent to the compounds of the above formula or salts thereof, except that one or more atoms have been replaced by an atom having an atomic mass or mass number different from that most often found in nature. Examples of isotopes that can be incorporated into the compounds of the above formula or salts thereof are isotopes of hydrogen, carbon, nitrogen, deuterium, such as ³H, ¹¹C, ¹⁴C and ¹⁸F. These isotopically labeled compounds of the above formula or salts thereof are useful in medicine and/or substrate tissue distribution assay. For example, ¹¹C and ¹⁸F isotopes can be used in PET (positron emission tomography). PET can be used for brain imaging. In some embodiments, the compounds of the above formula or salt thereof are non-isotopically labeled.

Accordingly, the compounds of the present invention comprises the compounds of the above formula or salts thereof, such as pharmaceutically acceptable salts thereof. Representative compounds of the present invention include the specific compounds described.

In a third aspect, the present invention also relates to a pharmaceutical composition comprising the compound of the present invention and a pharmaceutically acceptable excipient.

In a fourth aspect, the present invention also relates to a method of treating or preventing a disease associated with an activity of Lp-PLA₂, comprising administering to a subject in need thereof a therapeutically effective amount of the compound of the present invention described herein. The disease may be associated with the followings: increased involvement of monocytes, macrophages or lymphocytes, formation of lysophosphatidylcholine and oxidized free fatty acids, Lp-PLA₂ activity-associated lipid oxidation or endothelial dysfunction.

In some embodiments, the present invention also provides a method for treating or preventing a disease by inhibiting Lp-PLA₂ activity. Exemplary diseases include, but are not limited to, neurodegenerative diseases (e.g., Alzheimer's disease, Parkinson's disease, Huntington's disease, vascular dementia), various neuropsychiatric diseases such as schizophrenia and autism, peripheral and cerebral atherosclerosis, stroke, metabolic bone disease (e.g., bone marrow abnormalities), dyslipidemia, Paget's disease, type 2 diabetes, hypertension, angina pectoris, myocardial infarction, ischemia, reperfusion injury, metabolic syndrome, insulin resistance and hyperparathyroidism, diabetic complications (e.g., macular edema, diabetic retinopathy and posterior uveitis, diabetic ulcer and diabetic nephropathy), diabetic peripheral neuropathy pain, inflammatory pain, neuropathic pain, various cancers (e.g., prostate cancer, colon cancer, breast cancer, kidney cancer, lung cancer and ovarian cancer, etc.), macular edema, wound healing, male erectile dysfunction, rheumatoid arthritis, chronic obstructive pulmonary disease (COPD), sepsis, acute and chronic inflammation, psoriasis and multiple sclerosis. The method comprises administering to a subject in need thereof a therapeutically effective amount of the compound of the present invention. The present invention is not intended to be limited to any particular stage of disease (e.g., early or late).

In some embodiments, the present invention also provides a method for treating or preventing Alzheimer's disease. The method comprises administering to a subject in need thereof a therapeutically effective amount of the compound of the present invention.

In some embodiments, the present invention also provides a method for treating or preventing atherosclerosis. The method comprises administering to a subject in need thereof a therapeutically effective amount of the compound of the present invention.

In some embodiments, the present invention also provides a method for treating or preventing an ocular disease by administering the compound of the present invention. In some embodiments, the present invention provides a method for treating macular edema, which comprises administering to a subject a therapeutically effective amount of the compound of the present invention. In some embodiments, the macular edema is associated with a diabetic eye disease (e.g., diabetic macular edema or diabetic retinopathy). In one embodiment, the macular edema is associated with posterior uveitis.

In a fifth aspect, the present invention also provides a use of the compound of the present invention in the manufacture of a medicament for the treatment or prevention of a disease related to Lp-PLA₂. Exemplary diseases include, but are not limited to, neurodegenerative diseases (e.g., Alzheimer's disease, Parkinson's disease, Huntington's disease, vascular dementia), various neuropsychiatric diseases such as schizophrenia and autism, peripheral and cerebral atherosclerosis, stroke, metabolic bone disease (e.g., bone marrow abnormalities), dyslipidemia, Paget's disease, type 2 diabetes, hypertension, angina pectoris, myocardial infarction, ischemia, reperfusion injury, metabolic syndrome, insulin resistance and hyperparathyroidism, diabetic complications (e.g., macular edema, diabetic retinopathy and posterior uveitis, diabetic ulcers and diabetic nephropathy), diabetic peripheral neuropathy pain, inflammatory pain, neuropathic pain, various cancers (e.g., prostate cancer, colon cancer, breast cancer, kidney cancer, lung cancer and ovarian cancer, etc.), macular edema, wound healing, male erectile dysfunction, rheumatoid arthritis, chronic obstructive pulmonary disease (COPD), sepsis, acute and chronic inflammation, psoriasis and multiple sclerosis. The method comprises administering to a subject in need thereof a therapeutically effective amount of the compound of the present invention. The present invention is not intended to be limited to any particular stage of disease (e.g., early or late).

In a sixth aspect, the present invention also provides the compound of the present invention for use in the treatment or prevention of the diseases described herein.

As used herein, "and/or" is meant to include any and all possible combinations of one or more of the associated listed items. It will be further understood that the terms "comprising" and/or "including" used in this specification indicate the presence of the indicated feature, integer, step, operation, element and/or component, but do not exclude the presence or addition of one or more of other features, entities, steps, operations, elements, components, and/or combination thereof.

In general, the nomenclature used herein and the experimental procedures of organic chemistry, medicinal chemistry and biology described herein are well known and commonly employed in the art. Unless otherwise defined, all technical and scientific terms used herein generally have the same meaning as commonly understood by one of ordinary skill in the art to which this disclosure belongs. In the event that multiple definitions of terms used herein exist, the definitions in this section shall control unless otherwise indicated.

### Detailed Description of the present invention

### Definition

As used herein, unless stated otherwise, the term "disease" refers to any change in the state of body or some organs that interrupts or interferes with the performance of function and/or causes symptoms in a person who is ill or in contact with it (e.g., discomfort, dysfunction, adverse stress and even death).

As used herein, unless stated otherwise, "diabetic retinopathy" refers to chronic progressive retinal microvascular leakage and obstruction that results from diabetes. "Diabetic macular edema" refers to retinal thickening or hard exudative deposits caused by diabetes-induced accumulation of extracellular fluid within one optic disc diameter in the central fovea of macula.

As used herein, unless stated otherwise, "neurodegenerative disease" refers to various disorders of central nervous system characterized by a gradual, progressive loss of neural tissue and/or neural tissue function. Neurodegenerative diseases are a class of diseases of nervous system characterized by a gradual, progressive loss of neural tissue and/or alter neural function, usually show a decreased neural function caused by the gradual, progressive loss of neural tissue. In some embodiments, the neurodegenerative diseases described herein include neurodegenerative diseases in which a defective blood-brain barrier (e.g., permeable blood-brain barrier) exists. Examples of neurodegenerative diseases in which a defective blood-brain barrier exists include, but are not limited to, Alzheimer's disease, Huntington's disease, Parkinson's disease, vascular dementia, and so on.

As used herein, unless stated otherwise, "vascular dementia" is also referred to as "multi-infarct dementia", which refers to a group of symptoms caused by different mechanisms (all of which result in damage to blood vessels in the brain). For example, the main subtypes of vascular dementia are vascular mild cognitive impairment, multi-infarct dementia, vascular dementia due to major single infarct (affecting thalamus, anterior cerebral artery, parietal lobe, or cingulate gyrus), vascular dementia due to hemorrhagic lesions, small vessel disease (including, for example, vascular dementia due to lacunar lesions and Binswanger disease) and mixed dementia.

As used herein, unless stated otherwise, "neuropathic pain" is a pain provoked or caused by a primary damage and dysfunction of the nervous system.

As used herein, unless stated otherwise, "inflammatory pain" is a pain caused by localized acute inflammation or chronic inflammation that stimulates nerves.

As used herein, unless stated otherwise, "diabetic peripheral neuropathy pain" refers to a pain caused by a nerve damage associated with diabetes, which is at least in part due to the decreased blood flow and hyperglycemia.

As used herein, unless stated otherwise, "blood-brain barrier" or "BBB" are used interchangeably herein to refer to a permeable barrier present in blood vessels passing through brain tissue, which strictly limits and closely regulates the exchange of substances between blood and brain tissue. The components of blood-brain barrier comprise the endothelial cells that form the innermost lining of all blood vessels, the tight junctions between adjacent endothelial cells that serve as structure-associated substance of BBB, the basement membrane of endothelial cells, and the enlarged synapses that cover almost all exposed blood vessel outermost lining nearby astrocytes.

As used herein, unless stated otherwise, "metabolic bone disease" refers to a class of diverse bone diseases characterized by a gradual and progressive loss of bone tissue. Metabolic bone disease as described herein is a metabolic bone disease in which there is a condition of diffuse decreased bone density and/or decreased bone strength. Such diseases are characterized by histological appearance. Exemplary metabolic bone diseases include, but are not limited to, osteoporosis characterized by decreased minerals and bone matrix and osteomalacia characterized by decreased minerals but with intact bone matrix.

As used herein, unless stated otherwise, "osteopenic disease" or "osteopenia" are used interchangeably herein to refer to a condition with decreased calcification and/or bone density, and are used as a descriptive term to denote a condition in which all skeletal systems are observed to have a decreased calcification and/or bone density. Osteopenia also refers to osteopenia due to insufficient synthesis of osteoid.

As used herein, unless stated otherwise, "osteoporosis" refers to a disorder in which minerals and/or bone matrix are reduced and/or bone matrix is reduced.

As used herein, unless stated otherwise, "alkyl" is a monovalent, saturated hydrocarbon chain having the specified number of carbon atoms. For example, C₁₋₃ alkyl refers to an alkyl group having 1 to 3 carbon atoms. C₁₋₅ alkyl refers to an alkyl group having 1 to 5 carbon atoms. C₁₋₆ alkyl refers to an alkyl group having 1 to 6 carbon atoms. Alkyl can be straight or branched. In some embodiments, a branched alkyl may have one, two, or three branches. Exemplary alkyl includes, but is not limited to, methyl, methylethyl, ethyl, propyl (n-propyl and isopropyl), butyl (n-butyl, isobutyl and tert-butyl), pentyl, hexyl.

As used herein, unless stated otherwise, "alkoxy" substituent is a group of formula "R-O-", wherein R is an alkyl as defined above. For example, C₁₋₃ alkoxy refers to an alkoxy substituent containing 1 to 3 carbons. Exemplary alkoxy substituent includes, but is not limited to, methoxy, ethoxy, n-propoxy, n-butoxy, n-pentoxy, n-hexyloxy, isopropoxy, isobutoxy , sec-butoxy, tert-butoxy, isopentyloxy and neopentyloxy.

As used herein, "cycloalkyl" refers to a monovalent saturated cyclic hydrocarbon group including bridged and spiro rings, preferably having 3 to 8 ring carbon atoms (C₃₋₈ cycloalkyl), 3 to 7 ring carbon atoms (C₃₋₇ cycloalkyl) or 3 to 6 ring carbon atoms (C₃₋₆ cycloalkyl), for example cyclopropyl, cyclobutyl, cyclopentyl or [1,1,1]propellanyl, and those groups that are specifically exemplified below. Unless stated otherwise, "C₃₋₆ cycloalkyl" is a monovalent group obtained by removing one hydrogen atom from a 3-, 4-, 5-, or 6-membered monocyclic cycloalkane. Exemplary cycloalkyl includes, but is not limited to, cyclopropyl, cyclobutyl, cyclopentyl, and cyclohexyl.

As used herein, unless stated otherwise, "aryl" refers to a hydrocarbon group containing one or more aromatic rings, such as phenyl or naphthyl, and the like.

As used herein, in some embodiments, "heteroaryl" is a monovalent group obtained by removing one hydrogen atom from a monocyclic 5- or 6-membered heteroaromatic ring, the ring consists of ring-carbon atoms and ring-heteroatoms selected from nitrogen, oxygen and sulfur, and the ring is aromatic. For example, a heteroaryl is a monocyclic heteroaryl consisting of 5 or 6 ring atoms, of which 1 to 3 are ring-heteroatoms. Exemplary heteroaryl groups include, but are not limited to, furyl, thienyl, pyrrolyl, oxazolyl, thiazolyl, imidazolyl, pyrazolyl, isoxazolyl, isothiazolyl, oxadiazolyl, triazolyl, thiadiazolyl, pyridyl, pyridazinyl, pyrimidinyl, pyrazinyl, triazinyl, azepinyl, oxazepinyl, thiazepinyl, and diazepinyl. In other embodiments, "heteroaryl" refers to a stable monocyclic, bicyclic, or tricyclic ring having up to 7 atoms in each ring, wherein at least one ring is aromatic and at least one ring contains 1 to 4 heteroatoms selected from O, N and S. Heteroaryl groups within the scope of this definition include, but are not limited to, acridinyl, carbazolyl, cinnolinyl, quinoxalinyl, quinazolinyl, pyrazolyl, indolyl, isoindolyl, 1H,3H-1-oxoisoindolyl, benzotriazolyl, furanyl, thienyl, pyridomorpholinyl, pyridopiperidinyl, pyridopyrrolidinyl, benzothienyl, benzofuranyl, benzodioxanyl, benzodioxyphenyl, quinolinyl, isoquinolinyl, oxazolyl, isoxazolyl, benzoxazolyl, imidazolyl, pyrazinyl, pyridazinyl, pyridyl, pyrimidinyl, pyrrolyl, tetrahydroquinolinyl, thiazolyl, isothiazolyl, 1,2,3-triazolyl, 1,2,4-triazolyl, 1,2,4-oxadiazolyl, 1,2,4-thiadiazolyl, 1,3,5-triazinyl, 1,2,4-triazinyl, 1,2,4,5-tetrazinyl, tetrazolyl, xanthyl, phenazinyl, phenothiazinyl, phenoxazinyl, azepinyl, oxazepinyl, and thiazepinyl. Particular heteroaryl groups have 5- or 6-membered rings, examples thereof including furyl, pyridyl, pyridazinyl, pyrimidinyl, pyrazinyl, thienyl, isoxazolyl, oxazolyl, diazolyl, imidazolyl, pyrrolyl , pyrazolyl, triazolyl, tetrazolyl, thiazolyl, isothiazolyl, thiadiazolyl, pyridomorpholinyl, pyridopiperidinyl, pyridopyrrolidinyl. As used herein, in some embodiments, "heterocyclyl" is a monovalent group obtained by removing one hydrogen atom from a 3-, 4-, 5-, or 6-membered saturated monocyclic heterocycle consisting of ring-carbon atoms and ring-heteroatoms selected from nitrogen, oxygen and sulfur.

Exemplary monocyclic saturated heterocyclyl substituents include, but are not limited to, pyrrolidinyl, dioxolanyl, imidazolidinyl, pyrazolidinyl, piperidinyl, dioxanyl, morpholino, dithianyl, thiomorpholino and piperazinyl. In other embodiments, "heterocycle" or "heterocyclyl" refers to a cyclic hydrocarbon in which 1 to 4 carbon atoms have been independently replaced by a heteroatom selected from N, N(R), S, S(O), S(O) and O. Heterocycles can be saturated or unsaturated, but are not aromatic. Heterocyclyl groups may also contain 1, 2 or 3 rings, including bridged and spiro structures. Examples of suitable heterocyclyl groups include, but are not limited to: azetidinyl, oxetanyl, tetrahydrofuranyl, tetrahydrothienyl, pyrrolidinyl, 2-oxopyrrolidinyl, pyrrolinyl, pyranyl, dioxolanyl, piperidinyl, 2-oxopiperidinyl, pyrazolinyl, imidazolinyl, thiazolinyl, dithiolanyl, oxathiolanyl, dioxanyl, dioxenyl, dioxazolyl, oxathiozolyl, oxazolonyl, piperazinyl, morpholino, thiomorpholinyl, 3-oxomorpholinyl, dithianyl, trithianyl and oxazinyl.

As used herein, unless stated otherwise, a "bridged ring compound" refers to a compound in which one or more atoms (i.e., C, O, N, or S) connect(s) two non-adjacent carbon or nitrogen atoms. In preferred bridged ring, it includes, but is not limited to, one carbon atom, two carbon atoms, one nitrogen atom, two nitrogen atoms and one carbon-nitrogen group. It is worth noting that a bridge always converts a single ring into a triple ring. In a bridged ring, a substituent on the ring may also appears on the bridge.

The term "spirocyclic compound" refers to a polycyclic compound in which two monocyclic rings share one carbon atom, which is referred to as a spiro atom.

As used herein, unless stated otherwise, "halogen" refers to fluorine (F), chlorine (Cl), bromine (Br), or iodine (I). Halo refers to a halogen group: fluorine (-F), chlorine (-Cl), bromine (-Br), or iodine (-I).

As used herein, unless stated otherwise, "haloalkyl" is an alkyl substituted with one or more halogen substituents, which may be the same or different. For example, C₁₋₃ haloalkyl refers to a haloalkyl substituent containing 1 to 3 carbons. Exemplary haloalkyl substituents include, but are not limited to, monofluoromethyl, difluoromethyl, trifluoromethyl, 1-chloro-2-fluoroethyl, trifluoropropyl, 3-fluoropropyl, and 2-fluoroethyl.

As used herein, and unless stated otherwise, when two substituents on a ring are joined together with their interconnecting atoms to form another ring, the ring can be spiro-fused or unilaterally-fused. A spiro-fused ring system consists of two rings that have only one carbon atom in common. A unilaterally-fused ring system consists of two rings that share only two atoms and one bond.

As used herein, unless stated otherwise, "optionally substituted" means that a group or ring may be unsubstituted, or the group or ring may be substituted with one or more substituents as defined herein.

As used herein, unless stated otherwise, "4-, 5- or 6-membered saturated ring optionally containing a heteroatom selected from N or O" refers to a 4-, 5- or 6-membered saturated carbocyclic ring and one carbon atom as ring member may be optionally replaced by a heteroatom selected from N or O, and example thereof including cyclobutyl, cyclopentyl, cyclohexyl, azitidinyl, pyrrolidinyl, piperidinyl, oxetanyl, tetrahydrofuranyl and tetrahydro-2H-pyranyl.

As used herein, unless stated otherwise, "treat", "treating" or "treatment" in reference to a disease means: (1) alleviating a disease, or alleviating one or more biological manifestations of a disease, (2) interfering with (a) one or more points in a biological cascade that causes or contributes to a disease, or (b) one or more biological manifestations of a disease, (3) alleviating one or more symptoms or effects associated with a disease, and/or (4) slowing down a progression of a disease, or one or more biological manifestations of a disease, and/or (5) reducing a severity of a disease, or a likelihood of a biological manifestation of a disease.

As used herein, unless stated otherwise, "prevention" refers to a prophylactic administration of a drug to reduce a likelihood or delay an occurrence of a disease or its biological manifestations.

As used herein, unless stated otherwise, "subject" refers to a mammalian subject (e.g., dog, cat, horse, cow, sheep, goat, monkey, etc.), and especially a human subject.

As used herein, unless stated otherwise, "pharmaceutically acceptable salt" refers to a salt that retains a desired biological activity of a subject compound and exhibits minimal undesired toxicological effects. These pharmaceutically acceptable salts can be prepared in situ during the final isolation and purification of the compound, or by separately reacting a free acid or free base form of the purified compound with an appropriate base or acid, respectively.

As used herein, unless stated otherwise, the term "therapeutically effective amount" refers to an amount that results in the treatment or prevention of disease as compared to a corresponding subject who does not receive the amount, but the amount is low enough within the scope of sound medical judgment to avoid serious side effects (at a reasonable benefit/risk ratio). The therapeutically effective amount of a compound will vary with the particular compound chosen (e.g., taking into account the potency, efficacy, and half-life of the compound); route of administration chosen; disease being treated; severity of disease being treated; age, size, weight and physical condition of patient being treated: medical history of patient being treated; duration of treatment; nature of concurrent treatment; desired therapeutic effect, etc., but can still be determined in a routine manner by one skilled in the art.

### Compound synthesis

It will be understood by those skilled in the art that if a substituent described herein is incompatible with the synthetic methods described herein, the substituent may be protected with a suitable protecting group that is stable under the reaction conditions. The protecting group can be removed at a suitable point in the reaction sequence to yield the desired intermediate or target compound. Suitable protecting groups and methods for protecting and deprotecting various substituents using such suitable protecting groups are well known to those skilled in the art; and examples of this can be found in, I. Greene and P. Wuts, Protecting Groups in Chemical Synthesis (3rd ed.), John Wiley & Sons, NY (1999). In some cases, substituents that are reactive under the reaction conditions employed can be specifically selected. In these cases, the reaction conditions transform the selected substituent into another substituent that can be used as an intermediate compound or another substituent that is a desired substituent in the target compound.

### General Scheme:

The general scheme provides general synthetic routes for compounds of Formula 1.5 and 2.5, wherein R₁, R₂, Rₓ, U, X, Y, m, n, Q, A are as defined in formula (I),

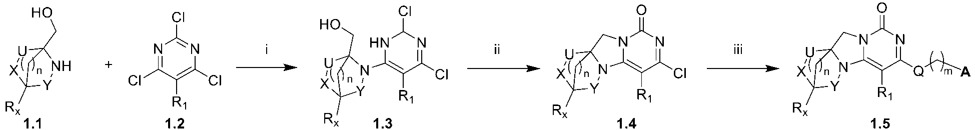

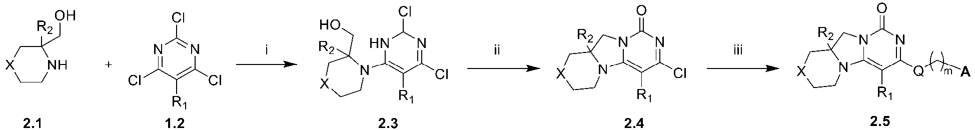

Step (i) can be used as a S_{N}Ar reaction, in which Compounds **1.1** and **1.2** are reacted by using a suitable reagent (e.g., triethylamine) in a suitable solvent (e.g., acetonitrile) at a suitable temperature (e.g., room temperature) to give Compound **1.3.** In Step (ii), Compound **1.3** reacts with a suitable reagent (e.g., triethylamine) and methanesulfonyl chloride or thionyl chloride at a suitable temperature (e.g., 0°C or room temperature) to convert the hydroxyl to a mesylate or chloride compound, then without purification, it further undergoes a ring-closing reaction in the presence of a base (e.g., potassium carbonate) in a suitable solvent (e.g., acetonitrile) at reflux to obtain Compound **1.4.** In Step (iii), **1.4** reacts with a corresponding alcohol or amine HQ-(CH₂)ₘ-A (Q is -O- or -NR_{b}-) in the presence of a suitable base (e.g., NaH or DIPEA) in a suitable solvent (e.g., acetonitrile or 1,4-dioxane) to obtain the final product **1.5.** Compound **2.5** is prepared starting from alcohol **2.1** and a R₁-substituted trichloropyrimidine. Variations in reaction conditions and reactants will be apparent to those skilled in the art. When Q is -CH₂-, see Example 170 for a specific synthesis scheme.

In addition, the general scheme provides general synthetic routes for compounds of Formula **1.5** and **2.5,** wherein R₁, R₂, X, Y, m, n, A are as defined in formula (I')

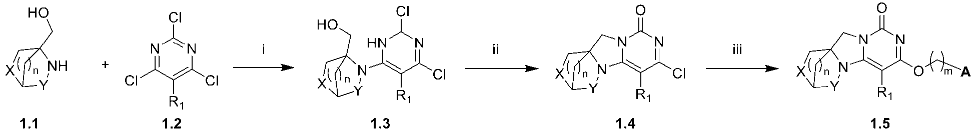

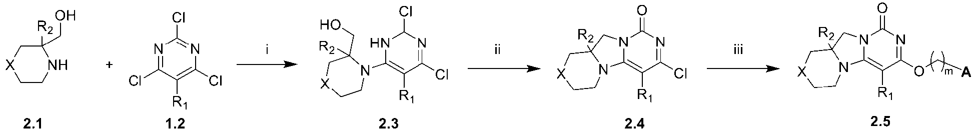

Step (i) can be used as a S_{N}Ar reaction, in which Compounds **1.1** and **1.2** are reacted by using a suitable reagent (e.g., triethylamine) in a suitable solvent (e.g., acetonitrile) at a suitable temperature (e.g., room temperature) to give Compound **1.3.** In Step (ii), Compound **1.3** reacts with a suitable reagent (e.g., triethylamine) and methanesulfonyl chloride or thionyl chloride at a suitable temperature (e.g., 0°C or room temperature) to convert the hydroxyl to a mesylate or chloride compound, then without purification, it further undergoes a ring-closing reaction in the presence of a base (e.g., potassium carbonate) in a suitable solvent (e.g., acetonitrile) at reflux to obtain Compound **1.4.** In Step (iii), **1.4** reacts with a corresponding alcohol HO-(CH₂)ₘ-A in the presence of a suitable base (e.g., NaH) in a suitable solvent (e.g., acetonitrile) to obtain the final product **1.5.** Compound **2.5** is prepared starting from alcohol **2.1** and a R₁-substituted trichloropyrimidine. Variations in reaction conditions and reactants will be apparent to those skilled in the art.

### Use

The compounds of the present invention are Lp-PLA₂ inhibitors. Accordingly, these compounds are useful in the treatment of diseases, for example treatment or prevention of diseases associated with the activity of Lp-PLA₂, which comprises treating a subject in need of such treatment with a therapeutically effective amount of an Lp-PLA₂ inhibitor. Accordingly, one aspect of the present invention relates to a method for treating or preventing a disease associated with Lp-PLA₂ activity. As will be appreciated by those of skill in the art, a particular disease or treatment thereof may involve one or more underlying mechanisms, including one or more of the mechanisms described herein, which are related to Lp-PLA₂ activity.

In some embodiments, the present invention provides a use of the compound of the present invention in the manufacture of a medicament for the treatment or prevention of any of the diseases disclosed in the following published patent applications: WO96/13484, WO96/19451, WO97/02242, WO97/12963, WO97/21675, WO97/21676, WO97/41098, WO97/41099, WO99/24420, WO00/10980, WO00/66566, WO00/66567, WO00/68208, WO01/60805, WO02/30904, WO02/30911, WO03/015786, WO03/016287, WO03/041712, WO03/042179, WO03/042206, WO03/042218, WO03/086400, WO03/87088, WO08/048867, US2008/0103156, US2008/0090851, US2008/0090852, WO08/048866, WO05/003118 (CA2530816A1), WO06/063811, WO06/063813, WO2008/141176, WO2013013503A1, WO2013014185A1, WO2014114248A1, WO2014114694A1, WO2016011930A1, JP200188847, US2008/0279846 A1, US2010/0239565 A1, and US 2008/0280829 A1.

In some embodiments, the present invention provides a use of the compound of the present invention in the manufacture of a medicament for the treatment of an eye disease. The eye diseases applicable in the present invention may be associated with disruption of the inner blood retinal barrier (iBRB). Exemplary eye diseases relate to diabetic eye diseases, which include macular edema, diabetic retinopathy, posterior uveitis, retinal vein occlusion, and the like. Additional eye diseases include, but are not limited to, central retinal vein occlusion, branch retinal vein occlusion, Irvine-Gass syndrome (post-cataract and post-surgery), retinitis pigmentosa, pars planitis, shotgun retinochoroidopathy, retinal outer membrane, choroidal tumor, cystic macular edema, parafoveal telangiectasia, traction maculopathy, vitreomacular traction syndrome, retinal detachment, neuroretinitis, idiopathic macular edema, etc. More details on the use of Lp-PLA₂ inhibitors for the treatment of eye diseases are provided in WO2012/080497, which is incorporated herein by reference.

Furthermore, some embodiments of the present invention provide a use of the compound of the present invention in the manufacture of a medicament for the treatment or prevention of a diabetic macular edema in a subject. In some embodiments, the present invention provides a use of the compound of the present invention for the treatment of a diabetic macular edema in a subject.

In some embodiments, the present invention provides a use of the compound of the present invention in the manufacture of a medicament for the treatment or prevention of a subject having or at risk of having a macular edema. In some embodiments, the present invention provides a use of the compound of the present invention in the manufacture of a medicament for the treatment of a subject suffering from or at risk of developing a macular edema. In other embodiments, the macular edema is associated with a diabetic eye disease, such as diabetic macular edema or diabetic retinopathy. In other embodiments, the macular edema is associated with posterior uveitis.

In some embodiments, the present invention provides a use of the compound of the present invention in the manufacture of a medicament for the treatment or prevention of a glaucoma or macular degeneration. In some embodiments, the present invention provides a use of the compound of the present invention in the manufacture of a medicament for the treatment of a glaucoma or macular degeneration.

In some embodiments, the present invention provides a use of the compound of the present invention in the manufacture of a medicament for the treatment or prevention of a disorder associated with a disruption of blood retinal inner barrier in a subject in need of such treatment. In some embodiments, the present invention provides a use of the compound of the present invention in the manufacture of a medicament for the treatment of a disorder associated with a disruption of blood retinal inner barrier in a subject in need of such treatment.

In some embodiments, the present invention provides a use of the compound of the present invention in the manufacture of a medicament for the treatment or prevention of any of the following diseases involving endothelial dysfunction, for example, atherosclerosis (e.g., peripheral vascular atherosclerosis and cerebrovascular atherosclerosis), diabetes, hypertension, angina pectoris, conditions after ischemia and reperfusion.

In some embodiments, the present invention provides a use of the compound of the present invention in the manufacture of a medicament for the treatment or prevention of any of the following diseases involving lipid oxidation associated with enzymatic activity, for example, diseases other than diseases such as atherosclerosis and diabetes, and for example rheumatoid arthritis, stroke, inflammatory disorders of brain (e.g., Alzheimer's disease), various neuropsychiatric disorders (e.g., schizophrenia, autism), myocardial infarction, ischemia, reperfusion injury, sepsis, and acute and chronic inflammation.

In some embodiments, the present invention provides a use of the compound of the present invention in the manufacture of a medicament for reducing a chance of a cardiovascular event (e.g., heart attack, myocardial infarction, or stroke) in a patient suffering from coronary heart disease.

In some embodiments, the present invention provides a use of the compound of the present invention in the manufacture of a medicament for the treatment or prevention of a disease as follows, in which the disease involves an activated monocyte, macrophage or lymphocyte, as all these cell types express Lp-PLA₂, which includes a disease involving an activated macrophage (e.g., M1, dendritic and/or other oxidative stress-producing macrophage). Exemplary disorders include, but are not limited to, psoriasis, rheumatoid arthritis, wound healing, chronic obstructive pulmonary disease (COPD), cirrhosis, atopic dermatitis, emphysema, chronic pancreatitis, chronic gastritis, aortic aneurysm, atherosclerosis, multiple sclerosis, Alzheimer's disease and autoimmune diseases such as lupus.

In other embodiments, the present invention provides a use of the compound of the present invention in the manufacture of a medicament for the primary or secondary prevention of an acute coronary event (e.g., caused by atherosclerosis); the prevention of restenosis adjuvant therapy; or the delay of development of diabetes or hypertensive renal insufficiency. The prevention comprises treatment of a subject at risk of such disorder.

In some embodiments, the present invention provides a method of treating or preventing a nervous system disease associated with blood-brain barrier (BBB) dysfunction, inflammation, and/or microglial activation in a subject in need of such treatment. In some embodiments, the present invention provides a method for treating or preventing a nervous system disease associated with blood-brain barrier (BBB) dysfunction, inflammation, and/or microglial activation in a subject in need of such treatment. The method comprises administering to a subject a therapeutically effective amount of the compound of the present invention. In other embodiments, the BBB dysfunction is an osmotic BBB. In other embodiments, the disease is a neurodegenerative disease. Such neurodegenerative disease is, for example, but not limited to, vascular dementia, Alzheimer's disease, Parkinson's disease and Huntington's disease. In some embodiments, the present invention provides a method for treating or preventing a disease associated with a blood-brain barrier (BBB) leakage in a subject. In some embodiments, the present invention provides a method for treating a disorder associated with blood-brain barrier (BBB) leakage in a subject. Exemplary diseases include, but are not limited to, cerebral hemorrhage, cerebral amyloid angiopathy. In some embodiments, the neurodegenerative disease is Alzheimer's disease. In specific embodiments, the neurodegenerative disease is vascular dementia. In some embodiments, the neurodegenerative disease is multiple sclerosis (MS).

In some embodiments, the compound of the present invention is useful in the treatment or prevention of a neurodegenerative disease in a subject. The method comprises administering the compound of the present invention (e.g., in the form of pharmaceutical composition comprising the compound of the present invention) to a subject in need of such treatment. In some embodiments, the compound of the present invention is useful in the treatment of a neurodegenerative disease in a subject. Exemplary neurodegenerative diseases include, but are not limited to, Alzheimer's disease, vascular dementia, Parkinson's disease, and Huntington's disease. In a specific embodiment, the neurodegenerative disease as mentioned in the present invention is associated with an abnormal blood-brain barrier. In some embodiments, the subject to which the agent that inhibits Lp-PLA₂ activity is administered is a human.

In some embodiments, the present invention provides a method for treating or preventing a subject having or at risk of developing a vascular dementia. The method comprises administering to a subject the compound of the present invention (e.g., a pharmaceutical composition comprising a therapeutically effective amount of the compound of the present invention). In some embodiments, the present invention provides a method for treating a subject having or at risk of developing a vascular dementia. In a specific embodiment, the vascular dementia is associated with Alzheimer's disease.

In some embodiments, the present invention relates to a method for treating or preventing a metabolic bone disease by administering to a subject in need of such treatment a therapeutically effective amount of the compound of the present invention. In some embodiments, the present invention relates to a method for treating a metabolic bone disease by administering to a subject in need of such treatment a therapeutically effective amount of the compound of the present invention. Exemplary metabolic bone diseases include diseases associated with loss of bone mass and bone density, including but not limited to osteoporosis and osteopenia. Exemplary osteoporosis and osteopenia diseases include, but are not limited to, myeloid abnormality, dyslipidemia, Paget's disease, type II diabetes, metabolic syndrome, insulin resistance, hyperparathyroidism, and related diseases. In other embodiments, the subject in need of such treatment is a human.

It is believed that the method for preventing osteoporosis and/or osteopenia described herein may be influenced by inhibiting the expression of Lp-PLA₂ and/or inhibiting the protein activity of Lp-PLA₂. Accordingly, some embodiments of the present invention provide a method for inhibiting Lp-PLA₂ by blocking enzymatic activity. In other embodiments, a method for inhibiting Lp-PLA₂ by reducing and/or down-regulating Lp-PLA₂ RNA expression is provided. In other embodiments, preventing and/or reducing bone mass loss and/or bone density loss results in preventing or reducing a symptom associated with a metabolic bone disease such as osteoporosis and/or osteopenia.

In specific embodiments, the method further comprises administering to a subject in need of treatment an additional therapeutic agent for the treatment of metabolic bone disease. For example, when the metabolic bone disease is osteoporosis, an additional therapeutic agent such as bisphosphate (e.g., alendronate, ibandronate, risedronate, calcovarin, raloxifene), selective estrogen modulator (SERM), estrogen therapy, hormone replacement therapy (ET/HRT), and teriparatide can be used.

In some embodiments, systemic inflammatory diseases such as juvenile rheumatoid arthritis, inflammatory bowel disease, Kawasaki disease, multiple sclerosis, sarcoidosis, polyarteritis, psoriatic arthritis, reactive arthritis, systemic lupus erythematosus, Fuku-Koyanagi-Harada syndrome, Lyme disease, Behcet disease, ankylosing spondylitis, chronic granulomatous disease, enthesitis may be the root cause of posterior uveitis affecting the retina, and it may lead to macular edema. The present invention relates to a method for treating or preventing posterior uveitis or any one of these systemic inflammatory diseases by administering a therapeutically effective amount of the compound of the present invention. In some embodiments, the present invention provides a method for treating posterior uveitis or any one of these systemic inflammatory diseases by administering a therapeutically effective amount of the compound of the present invention.

The treatment and/or prevention of a disease associated with Lp-PLA₂ activity can be obtained by using the compounds of the present invention in monotherapy or in dual or multiple combination therapy. For example, the compound of the present invention may be used in combination with an antihyperlipidemic, antiatherosclerotic, antidiabetic, antianginal, antiinflammatory or antihypertensive agent, or an agent for lowering lipoprotein (a) (Lp(a)) for the treatment or prevention of the diseases described herein. Examples of such agents include, but are not limited to, cholesterol synthesis inhibitor such as statins; antioxidant such as probucol; insulin sensitizer; calcium channel antagonist and anti-inflammatory drug such as non-steroidal anti-inflammatory drug (NSAID). The agents for lowering Lp(a) include phosphoramidates described in WO 97/02037, WO 98/28310, WO 98/28311 and WO 98/28312. In some embodiments, the compound of the present invention may be used with one or more statins. Statins are well known cholesterol lowering agents and include atorvastatin, simvastatin, pravastatin, cerivastatin, fluvastatin, lovastatin and rosuvastatin. In some embodiments, the compound of the present invention may be used with an antidiabetic agent or insulin sensitizer. In some embodiments, the compound of the present invention can be used with PPAR γ activator such as GI262570 (GlaxoSmithKline) and glitazone compound such as rosiglitazone, troglitazone and pioglitazone. The agent may be administered, for example, in a therapeutically effective amount known in the art or in a smaller or greater amount than known in the art to provide effective treatment.

The combination therapy comprises administration of the therapeutic agents together in separate dosage forms or in a single dosage form. The combination therapy may comprises simultaneous or separate administration of the therapeutic agents, which may be substantially simultaneous or substantially separated administration. Typically, the combination therapy comprises administering each agent such that a therapeutically effective amount of each agent is present in the subject for at least an overlapping period of time.

### Method of use

The therapeutically effective amount of the compound of the present invention will depend on many factors including, for example, the age and weight of the intended recipient, the precise condition to be treated and its severity, the nature of the formulation and the route of administration, and will ultimately depend on the prescribing physician's discretion. However, the therapeutically effective amount of the compound of the present invention for the treatment of the diseases described herein will generally be in the range of 0.1 to 100 mg/kg recipient body weight/day, more usually 1 to 10 mg/kg body weight/day. Thus, for example, for a 70 kg adult mammal, the actual amount per day is usually 70 to 700 mg, and this amount may be administered in a single dose per day or in multiple sub-doses per day, such as two, three, four, five or six doses per day. Or, the administration can be done intermittently, for example, every other day, once per week, or once per month. It is anticipated that similar doses may be suitable for the treatment of the other conditions described above.

The pharmaceutical compositions of the present invention may contain one or more compounds of the present invention. In some embodiments, the pharmaceutical composition may comprise more than one compound of the present invention. For example, in some embodiments, the pharmaceutical composition may contain two or more compounds of the present invention. In addition, the pharmaceutical composition may also optionally contain one or more additional pharmaceutically active compounds.

"Pharmaceutically acceptable excipient" as used in the present invention refers to a pharmaceutically acceptable raw material, component or carrier that participates in imparting form or consistency to the pharmaceutical composition. When mixed, each excipient is compatible with the other ingredients of the pharmaceutical composition, thereby avoiding an interaction that would significantly reduce the efficacy of the compound of the present invention when administered to a subject and avoiding an interaction that would result in a pharmaceutically unacceptable pharmaceutical ingredient.

The compound of the present invention and one or more pharmaceutically acceptable excipients can be formulated into a dosage form suitable for administration to a subject by the desired route of administration. For example, the dosage form comprises those suitable for the following routes of administration: (1) for oral (including buccal or sublingual) administration, examples including tablet, capsule, caplet, pill, lozenge, powder, syrup, brew, suspension, solution, emulsion, sachet and cachet; (2) for parenteral (including subcutaneous, intramuscular, intravenous or intradermal) administration, examples including sterile solution, suspension and powder for reconstitution; (3) for transdermal administration, examples including transdermal patch; (4) for rectal administration, examples including suppository; (5) for nasal inhalation, examples including dry powder, aerosol, suspension and solution; and (6) for topical (including buccal, sublingual, or transdermal) administration, examples including cream, ointment, lotion, solution, paste, spray, foam, and gel. Such compositions can be prepared by any method known in the art of pharmacy, for example by combining the compound of the above formulae with a carrier or excipient.

The pharmaceutical composition suitable for oral administration may be presented as a discrete unit such as capsule or tablet; powder or granule; solution or suspension in aqueous or non-aqueous liquid form; edible foam or whip; oil-in-water liquid emulsion or water-in-oil liquid emulsion.

The suitable pharmaceutically acceptable excipients may vary depending upon the particular dosage form chosen. Furthermore, the appropriate pharmaceutically acceptable excipients can be selected according to the particular function they serve in the composition. For example, some pharmaceutically acceptable excipients may be selected for their ability to facilitate the production of uniform dosage forms. Some pharmaceutically acceptable excipients may be selected for their ability to facilitate the production of stable dosage forms. Certain pharmaceutically acceptable excipients may be selected for their ability to facilitate the delivery or transport of one or more compounds of the present invention from one organ or part of the body to another organ or part of the body when administered to a subject. Some pharmaceutically acceptable excipients may be selected for their ability to increase patient compliance.

The suitable pharmaceutically acceptable excipients include the following types of excipients: diluent, filler, binder, disintegrant, lubricant, glidant, granulating agent, coating agent, wetting agent, solvent, co-solvent, suspending agent, emulsifier, sweetener, flavoring agent, taste masking agent, coloring agent, anti-caking agent, humectant, chelating agent, plasticizer, tackifier, antioxidant, preservative, stabilizer, surfactant and buffer. It will be understood by those of skill in the art that some pharmaceutically acceptable excipients may provide more than one function, and may provide an additional function depending on how much of the excipient is present in the formulation and what other ingredient is present in the formulation.

The one skilled in the art possesses the knowledge and skill in the art to be able to select an appropriate amount of an appropriate pharmaceutically acceptable excipient for use in the present invention. In addition, the one skilled in the art has access to many resources that describe pharmaceutically acceptable excipients and can be used to select an appropriate pharmaceutically acceptable excipient. Examples include Remington's Pharmaceutical Sciences, Mack Publishing, Inc., The Handbook of Pharmaceutical Additives, Gower Publishing, Inc., and Handbook of Pharmaceutical Excipients, American Pharmaceutical Association and Pharmaceutical Press, Inc..

The pharmaceutical composition of the present invention is prepared using a technique and method known to those skilled in the art. Some methods commonly used in the art are described in Remington Pharmaceutical Sciences (Mack Press).

In one aspect, the present invention relates to a solid oral dosage form, such as tablet or capsule, comprising a therapeutically effective amount of the compound of the present invention and a diluent or filler. The suitable diluent and filler includes lactose, saccharide, dextrose, mannitol, sorbitol, starch (e.g., corn starch, potato starch and pregelatinized starch), cellulose and derivative thereof (e.g., microcrystalline cellulose), calcium sulfate and calcium hydrogen phosphate. The oral solid dosage form may also comprise binder. The suitable binder includes starch (e.g., corn starch, potato starch and pregelatinized starch), gelatin, acacia, sodium alginate, alginic acid, xanthan gum, guar gum, povidone and cellulose and derivative thereof (e.g., microcrystalline cellulose). The oral solid dosage form may also comprise a disintegrant. The suitable disintegrant includes crospovidone, sodium starch glycolate, croscarmelose, alginic acid and sodium carboxymethylcellulose. The oral solid dosage form may also comprise a lubricant. The suitable lubricant includes stearic acid, magnesium stearate, calcium stearate and talc.

In particular embodiments, the present invention relates to a pharmaceutical composition comprising 0.01 mg to 1000 mg of one or more of the compounds of formulae described herein or pharmaceutically acceptable salt thereof, and 0.01 g to 5 g of one or more pharmaceutically acceptable excipients.

### Intermediate 1

### (1S, 4R)-4-(hydroxymethyl)-2-oxa-5-azabicyclo[2.2.1]heptane-5-carboxylic acid tert-butyl ester

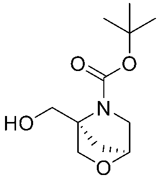

(1*S*,4*S*)-5-tert-butoxycarbonyl-2-oxa-5-azabicyclo[2.2.1]heptane-4-carboxylic acid methyl ester (0.88 g, 3.4 mmol, Chemistry Letters, 2017, 566-568) was added to anhydrous tetrahydrofuran (30 mL), cooled to 0°C, slowly added with lithium borohydride (222 mg, 10.2 mmol), and stirred at room temperature overnight. The reaction solution was cooled to 0°C and added with sodium sulfate decahydrate for quenching. The reaction solution was poured into dichloromethane, dried over anhydrous sodium sulfate, filtered, and the filter cake was washed with dichloromethane/methanol (20/1), and the filtrate was concentrated to obtain the crude title compound (1.5 g).

LC-MS: *m*/*z* [M+H-tBu]⁺ = 174.

### ((1S, 4R)-2-oxa-5-azabicyclo[2.2.1]hept-4-yl)methanol hydrochloride

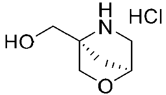

(1S,4R)-4-(hydroxymethyl)-2-oxa-5-azabicyclo[2.2.1]heptane-5-carboxylic acid tert-butyl ester (1.5g of crude product) was dissolved in a mixed solvent of dichloromethane (5 mL) and methanol (5 mL), a solution of hydrogen chloride in ethyl acetate (4.0 M, 5 mL) was added, and the reaction mixture was stirred at 40-50°C overnight. The reaction solution was concentrated to obtain the crude title compound (900 mg).

LC-MS: *m*/*z* [M+H]⁺ = 130.

### ((1S, 4R)-5-(2, 6-dichloropyrimidin-4-yl)-2-oxa-5-azabicyclo[2.2.1]hept-4-yl)methanol

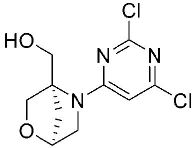

((1*S*,4*R*)-2-oxa-5-azabicyclo[2.2.1]hept-4-yl)methanol hydrochloride (0.9 g, 5.4 mmol) was dissolved in dichloromethane (50 mL); after cooling to 0°C, triethylamine (1.1 g, 1.5 mL, 10.8 mmol) and 2,4,6-trichloropyrimidine (1.2 g, 5.76 mmol) were sequentially added, and the mixture was stirred at room temperature for 4 hours. The reaction was quenched with saturated sodium bicarbonate solution, extracted with dichloromethane, the organic phase was concentrated, and purified by silica gel chromatography to obtain the title compound (0.36 g, yield 38% over three steps).

LC-MS: *m*/*z* [M+H]⁺ = 276.

### (3S, 11aR)-7-chloro-3, 4-dihydro-1H, 9H, 11H-3, 11a-methanopyrimido[6', 1':2,3]imidazo[5, 1-C] [1, 4]oxazin-9-one

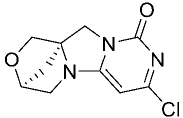

((1*S*,4*R*)-5-(2,6-dichloropyrimidin-4-yl)-2-oxa-5-azabicyclo[2.2.1]heptan-4-yl)methanol (360 mg, 1.3 mmol) and triethylamine (395 mg, 3.9 mmol) were added to anhydrous tetrahydrofuran (10 mL) and cooled to 0°C. Methylsulfonyl chloride (229 mg, 2.0 mmol) was added dropwise, and the mixture was stirred at 0°C for 30 minutes. The reaction solution was concentrated, potassium carbonate (898 mg, 6.5 mmol) and acetonitrile (15 mL) were added, and the mixture was stirred at 80°C overnight. The reaction solution was concentrated and separated by preparative thin layer chromatography (dichloromethane/methanol = 20/1) to obtain the title compound (230 mg, 74%).

LC-MS: *m*/*z* [M+H]⁺ = 240.

¹H NMR (400 MHz, CDCl₃) δ5.60 (s, 1H), 4.78 (br. s, 1H), 4.49 (d, *J =* 13.2 Hz, 1H), 4.09 - 3.88 (m, 3H), 3.61 (d, *J =* 9.8 Hz, 1H), 3.32 (d, *J* = 9.8 Hz, 1H), 2.10 (d, *J* = 10.8 Hz, 1H), 1.84 (d, *J* = 10.3 Hz, 1H).

### Intermediate 2

### (1R, 4S)-4-(hydroxymethyl)-2-oxa-5-azabicyclo[2.2.1]heptane-5-carboxylic acid tert-butyl ester

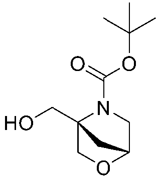

(1*R*,4*R*)-5-tert-butoxycarbonyl-2-oxa-5-azabicyclo[2.2.1]heptane-4-carboxylic acid methyl ester (430 mg, 1.67 mmol, Chemistry Letters, 2017, 566-568) was added to anhydrous tetrahydrofuran (10 mL), cooled to 0°C, slowly added with lithium borohydride (430 mg, 1.67 mmol), and stirred at room temperature overnight. The reaction solution was cooled to 0°C and added with water for quenching. The reaction solution was diluted with ethyl acetate, dried over anhydrous sodium sulfate, filtered, the filter cake was washed with ethanol, and the filtrate was concentrated. Dichloromethane was added to dissolve the residue, filtered, and the filtrate was concentrated to give the crude title compound (380 mg, 99%).

LC-MS: *m*/*z* [M+H-tBu]⁺ = 174.

### ((1R, 4S)-2-oxa-5-azabicyclo[2.2.1]hept-4-yl)methanol

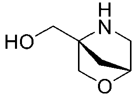

(1*R*,4*S*)-4-(hydroxymethyl)-2-oxa-5-azabicyclo[2.2.1]heptane-5-carboxylic acid tert-butyl ester (380 mg, 1.67 mmol) was added to dichloromethane (10 mL) and trifluoroacetic acid (5 mL), and stirred at room temperature overnight. The reaction solution was concentrated and purified by column chromatography (dichloromethane/methanol = 20/1~2/1) to obtain the crude title compound (800 mg).

LC-MS: *m*/*z* [M+H]⁺ = 130.

### ((1R, 4S)-5-(2, 6-dichloropyrimidin-4-yl)-2-oxa-5-azabicyclo[2.2.1]hept-4-yl)methanol

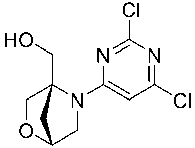

2,4,6-Trichloropyrimidine (1055 mg, 5.76 mmol) was added to acetonitrile (30 mL) and cooled to 0°C. ((1*R*,4*S*)-2-oxa-5-azabicyclo[2.2.1]hept-4-yl)methanol (700 mg, 2.88 mmol) and a solution of triethylamine (872 mg, 8.64 mmol) in acetonitrile (30 mL) was added dropwise, and stirred at 0°C for 2 hours. The reaction solution was poured into water, extracted with ethyl acetate, the organic phase was concentrated, and purified by silica gel column chromatography (petroleum ether/ethyl acetate = 10/1~4/1) to obtain the title compound (120 mg, 15%).

LC-MS: *m*/*z* [M+H]⁺ = 276.

### (3R,11aS)-7-chloro-3, 4-dihydro-1H, 9H, 11H-3, 11a-methanopyrimido[6', 1':2, 3]imidazo[5, 1-C] [1, 4]oxazin-9-one

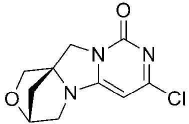

((1*R*,4*S*)-5-(2,6-dichloropyrimidin-4-y1)-2-oxa-5-azabicyclo[2.2.1]heptan-4-y1)methanol (110 mg, 0.40 mmol) and triethylamine (121 mg, 1.20 mmol) were added to anhydrous tetrahydrofuran (3 mL) and cooled to 0°C. Methylsulfonyl chloride (69 mg, 0.60 mmol) was added dropwise, and the mixture was stirred at 0°C for 10 minutes. The reaction solution was concentrated, and potassium carbonate (166 mg, 1.20 mmol) and acetonitrile (2 mL) were added, and stirred at 90°C for 5 hours. The reaction solution was concentrated and separated by preparative thin layer chromatography (dichloromethane/methanol = 20/1) to obtain the title compound (50 mg, 52 %).

LC-MS: *m*/*z* [M+H]⁺ = 240.

¹H NMR (400 MHz, CDCl₃) δ5.60 (s, 1H), 4.78 (br. s, 1H), 4.49 (d, *J* = 12.7 Hz, 1H), 4.07 - 3.98 (m, 3H), 3.60 (d, *J =* 10.3 Hz, 1H), 3.32 (d, *J* = 9.8 Hz, 1H), 2.10 (d, *J =* 9.8 Hz, 1H), 1.87 (d, *J =* 10.3 Hz, 1H).

### Intermediate 3

### (2-(2, 6-dichloropyrimidin-4-yl)-2-azabicyclo[2.1.1]hex-1-yl)methanol

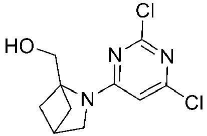

(2-Azabicyclo[2.1.1]hex-1-yl)methanol (3.5 g, 30.98 mmol, Journal of Organic Chemistry, 2018, 83, 14350-14361) was added into acetonitrile (100 mL), added with sodium carbonate (12.6 g, 93 mmol), the reaction solution was cooled to below 0°C, 2,4,6-trichloropyrimidine (28.3 g, 154.9 mmol) was added dropwise, the reaction solution was stirred at room temperature overnight, and the reaction solution was filtered through celite, the filtrate was concentrated and purified by silica gel column chromatography to obtain the title compound (5.7 g, 71 %) as a white solid.

LC-MS: *m*/*z* [M+H]⁺ = 260.

### 3-Chloro-7, 8-dihydro-1H, 6H, 9H-7, 8a-methanopyrrolo[1', 2':3, 4]imidazo[1, 2-c]pyrimidin-1-one

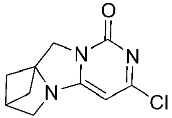

(2-(2,6-Dichloropyrimidin-4-yl)-2-azabicyclo[2.1.1]hex-1-yl)methanol (5.1 g, 19.6 mmol) was dissolved in dichloromethane (100 mL), added with thionylchloride (7 g, 58.8 mmol), stirred at room temperature for 1 hour, the reaction solution was concentrated, the residue was dissolved in acetonitrile (100 mL), potassium carbonate (8.11 g, 58.8 mmol) was added, stirred at 85°C overnight, the reaction solution was filtered through celite, the filtrate was concentrated, and separated and purified by column chromatography to obtain the title compound (3.9 g, 89 %). LC-MS: *m*/*z* [M+H]⁺ = 224.

¹H NMR (400 MHz, DMSO-*d*₆) δ5.93 (s, 1H), 3.92 (s, 2H), 3.40 (s, 2H), 2.93 (br. s, 1H), 2.06 (br. s, 2H), 1.66 - 1.61 (m, 2H).

### Intermediate 4

### (7-(2, 6-dichloropyrimidin-4-yl)-7-azabicyclo[2.2.1]heptan-1-yl)methanol

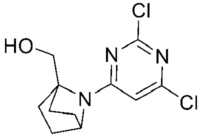

1-(Hydroxymethyl)-7-azabicyclo[2.2.1]heptane-7-carboxylic acid tert-butyl ester (4.3 g, 18.9 mmol, Tetrahedron Letters, 2010, 51, 6741-6744) was dissolved in a mixed solvent of dichloromethane (20 mL) and methanol (20 mL), added with a solution of hydrogen chloride in ethyl acetate (4.0 M, 20 mL), and stirred at 40-50°C overnight. The reaction solution was concentrated. The residue was dissolved in dichloromethane (80 mL), cooled to 0°C, followed by adding triethylamine (5.7 g, 7.8 mL, 56.7 mmol) and 2,4,6-trichloropyrimidine (6.9 g, 4.3 mL, 37.8 mmol) in sequence, stirred at room temperature for 4 hours. The reaction was quenched with saturated sodium bicarbonate solution, extracted with dichloromethane, the organic phase was concentrated, and purified by silica gel column chromatography to give the title compound (1.13 g, 21 %).

LC-MS: *m*/*z* [M+H]⁺ = 274.

### 3-chloro-7,8-dihydro-1H,6H,9H-6,8a-ethanopyrrolo[1',2':3,4]imidazo[1,2-c] pyrimidin-1-one

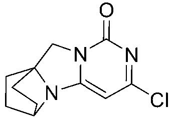

(7-(2,6-Dichloropyrimidin-4-yl)-7-azabicyclo[2.2.1]heptan-1-yl)methanol (360 mg, 1.3 mmol) was dissolved in dichloromethane (30 mL), cooled to 0°C. Thionyl chloride (4.56 g, 38.35 mmol) was added dropwise, and the mixture was stirred at room temperature for 1 hour. The reaction solution was concentrated, the residue and potassium carbonate (3.18 g, 23.01 mmol) were added to acetonitrile (30 mL), and the mixture was stirred at 80°C overnight. The reaction solution was filtered through celite, the filter cake was washed several times with ethyl acetate, and the combined filtrates were concentrated to obtain the title compound (0.76 g, 94%). LC-MS: *m*/*z* [M+H]⁺ = 238.

¹H NMR (400 MHz, CDCl₃) δ5.68 (s, 1H), 4.19 (t, *J =* 4.6 Hz, 1H), 4.03 (s, 2H), 2.10 (dd, *J* = 4.6, 10.5 Hz, 2H), 1.87 - 1.76 (m, 6H)..

### Intermediate 5

### (2-(2, 6-dichloropyrimidin-4-yl)-2-azabicyclo[2.2.2]oct-1-yl)methanol

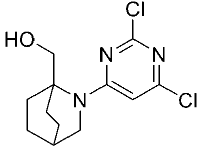

2,4,6-Trichloropyrimidine (584 mg, 3.19 mmol) was added to acetonitrile (5 mL) and cooled to 0°C. (2-Azabicyclo[2.2.2]oct-1-yl)methanol (300 mg, 2.12 mmol, J. Org. Chem., 2007, 72, 3112 - 3115) and a solution of triethylamine (429 mg, 4.25 mmol) in acetonitrile (5 mL) was added dropwise, stirred at 0°C for 2 hours. The reaction solution was poured into water, extracted with ethyl acetate, the organic phase was concentrated, and purified by silica gel column chromatography (petroleum ether/ethyl acetate = 10/1~4/1) to obtain the title compound (198 mg, 32%).

LC-MS: *m*/*z* [M+H]⁺ = 288.

### 3-chloro-6,7,8,9-tetrahydro-1H,10H-7,9a-ethanopyrido[1',2':3,4]imidazo[1,2-c]pyrimidin-1-one

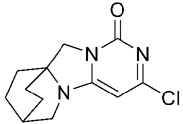

(2-(2,6-Dichloropyrimidin-4-yl)-2-azabicyclo[2.2.2]oct-1-yl)methanol (198 mg, 0.69 mmol) was added to dichloromethane (5 mL), cooled to 0°C. Thionyl chloride (409 mg, 3.44 mmol) was added dropwise, and the mixture was stirred at room temperature for 30 minutes. The reaction was concentrated, and potassium carbonate (379 mg, 2.75 mmol) and acetonitrile (5 mL) were added, stirred at 90°C overnight. The reaction solution was concentrated and separated by preparative thin layer chromatography (dichloromethane/methanol = 20/1) to obtain the title compound (170 mg, 98 %).

LC-MS: *m*/*z* [M+H]⁺ = 252.

### Intermediate 6

### Benzylproline methyl ester

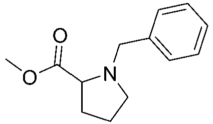

1-Boc-2-pyrrolidinecarboxylic acid methyl ester (25 g, 109.04 mmol) was added to a solution of hydrogen chloride in ethyl acetate (4.0 M, 100 mL), and the mixture was stirred at room temperature for 4 hours. The reaction solution was concentrated, and the residue, benzyl bromide (22.38 g, 130.85 mmol), and anhydrous potassium carbonate (30.1 g, 218.08 mmol) were added to acetonitrile (100 mL), and the mixture was heated to reflux and stirred overnight. The reaction solution was poured into water, extracted with ethyl acetate, the combined organic phases were concentrated, and purified by silica gel column chromatography (petroleum ether/ethyl acetate = 200/1~80/1) to obtain the title compound (18 g, 75%).

LC-MS: *m*/*z* [M+H]⁺ = 220.

### 1-benzyl-2-methylpyrrolidine-2-carboxylic acid methyl ester

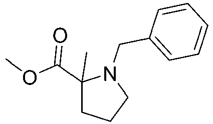

Benzylproline methyl ester (8 g, 36.48 mmol) was added to tetrahydrofuran (100 mL) and cooled to -20°C under argon protection. Iodomethane (15.5 g, 109.44 mmol) was added to the reaction solution, the temperature was lowered to -50°C, the temperature was controlled between -50°C and -40°C, and a solution of lithium diisopropylamide in tetrahydrofuran (2.0 M, 63.84 mL, 127.68 mmol) was added dropwise, after the dropwise addition was completed, the temperature was controlled at -50°C to -40°C, and stirring was carried out for 3 hours. After adding methanol (50 mL) to the reaction solution for quenching, the reaction solution was poured into water, extracted with ethyl acetate, the organic phase was concentrated, and purified by silica gel column chromatography (petroleum ether/ethyl acetate=200/1~100/1) to give the title compound (5 g, 59%).

LC-MS: *m*/*z* [M+H]⁺ = 234.

### (1-Benzyl-2-methylpyrrolidin-2-yl)methanol

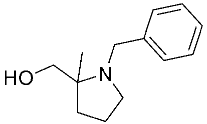

Lithium aluminum hydride (1.63 g, 42.90 mmol) was added to anhydrous tetrahydrofuran (25 mL), and the temperature was lowered to 0°C under argon protection. 1-benzyl-2-methylpyrrolidine-2-carboxylic acid methyl ester (5 g, 21.45 mmol) in anhydrous tetrahydrofuran (25 mL) was added dropwise, and stirred at room temperature overnight. Sodium sulfate decahydrate (20 g) was added to the reaction solution to quench the reaction, and the mixture was stirred at room temperature for 30 minutes. The reaction solution was filtered, and the filtrate was concentrated to obtain the crude title compound (3.8 g, 86%).

LC-MS: *m*/*z* [M+H]⁺ = 206.

### (2-Methylpyrrolidin-2-yl)methanol

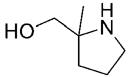

(1-Benzyl-2-methylpyrrolidin-2-yl)methanol (3.8 g, 18.52 mmol) and Pd/C (10%, 400 mg) were added to methanol (40 mL). Hydrogenation was performed at 50°C under atmospheric pressure overnight. The reaction solution was filtered, and the filtrate was concentrated to obtain the crude title compound (2.16 g, 101%).

LC-MS: *m*/*z* [M+H]⁺ = 116.

### (1-(2, 6-Dichloropyrimidin-4-yl)-2-methylpyrrolidin-2-yl)methanol

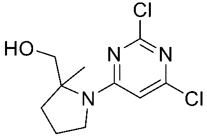

2,4,6-Trichloropyrimidine (5.16 g, 28.15 mmol) and triethylamine (3.8 g, 37.54 mmol) were added to acetonitrile (80 mL). After the reaction solution was cooled to 0°C, (2-methylpyrrolidin-2-yl)methanol (2.16 g, 18.77 mmol) was added, and the mixture was stirred at 0°C for 2 hours. The reaction solution was poured into water, extracted with ethyl acetate, the organic phase was concentrated, and purified by silica gel column chromatography (petroleum ether/ethyl acetate = 5/1~2/1) to obtain the title compound (2 g, 41%).

LC-MS: *m*/*z* [M+H]⁺ = 262.

### 3-Chloro-8a-methyl-7,8,8a,9-tetrahydro-1H,6H-pyrrolo[1',2':3,4]imidazo[1,2-c]pyrimidin-1-one

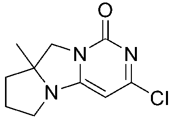

(1-(2,6-Dichloropyrimidin-4-yl)-2-methylpyrrolidin-2-yl)methanol (2 g, 7.67 mmol) was added to dichloromethane (30 mL) and cooled to 0°C. Thionyl chloride (4.56 g, 38.35 mmol) was added dropwise, and the mixture was stirred at room temperature for 30 minutes. The reaction solution was concentrated, and the concentrated residue and potassium carbonate (3.18 g, 23.01 mmol) were added to acetonitrile (30 mL), stirred at 90°C overnight. The reaction solution was poured into water (100 mL), extracted with ethyl acetate and dichloromethane, respectively, and the combined organic phases were concentrated to obtain the title compound (1.5 g, 88%).

¹H NMR (400 MHz, DMSO-*d*₆) δ5.96 (s, 1H), 4.06 (d*, J =* 12.2 Hz, 1H), 3.79 (d, *J =* 12.2 Hz, 1H), 3.48 - 3.35 (m, 2H), 2.10 - 1.89 (m, 2H), 1.87 - 1.68 (m, 2H), 1.32 (s, 3H).

### Intermediate 7

### 1-Benzyl-2-ethylpyrrolidine-2-carboxylic acid methyl ester

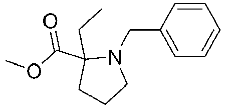

Benzylproline methyl ester (8 g, 36.48 mmol) was added to tetrahydrofuran (100 mL) and cooled to -20°C under argon protection. Iodoethane (17.1 g, 109.45 mmol) was added to the reaction solution, cooled to -50°C. The temperature was controlled at -50°C to -40°C, and a solution of lithium diisopropylamide in tetrahydrofuran (2.0 M, 63.84 mL, 127.68 mmol) was added dropwise. After the dropwise addition was completed, the temperature was controlled at - 50°C to -40°C and stirring was carried out for 3 hours. After methanol was added to the reaction solution for quenching, the reaction solution was poured into water, and extracted with ethyl acetate. The combined organic phases were concentrated, and purified by silica gel column chromatography (petroleum ether/ethyl acetate=200/1~100/1) to obtain the title compound (4.9 g, 54%).

LC-MS: *m*/*z* [M+H]⁺ = 248.

### (1-Benzyl-2-ethylpyrrolidin-2-yl)methanol

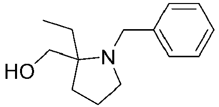

Lithium aluminum hydride (1.51 g, 39.66 mmol) was added to anhydrous tetrahydrofuran (25 mL), and cooled to 0°C under argon protection. A solution of 1-benzyl-2-ethylpyrrolidine-2-carboxylic acid methyl ester(4.9 g, 19.83 mmol) in dry tetrahydrofuran (25 mL) was added dropwise. Stirring was carried out overnight at room temperature. Sodium sulfate decahydrate (5 g) was added to the reaction solution to quench the reaction, and the mixture was stirred at room temperature for 30 minutes. The reaction solution was filtered, and the filtrate was concentrated to obtain the crude title compound (3.5 g, 81%).

LC-MS: *m*/*z* [M+H]⁺ = 220.

### (2-Ethylpyrrolidin-2-yl)methanol

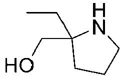

(1-Benzyl-2-ethylpyrrolidin-2-yl)methanol (3.5 g, 15.97 mmol), Pd/C (10%, 350 mg) were added to methanol (50 mL). Hydrogenation was performed at 50°C under atmospheric pressure overnight. The reaction solution was filtered, and the filtrate was concentrated to obtain the crude title compound (2.1 g, 100%).

LC-MS: m/z [M+H]⁺ = 130.

### (1-(2, 6-Dichloropyrimidin-4-yl)-2-ethylpyrrolidin-2-yl)methanol

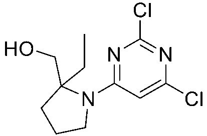

2,4,6-Trichloropyrimidine (3.5 g, 19.16 mmol) and triethylamine (3.23 g, 31.94 mmol) were added to acetonitrile (80 mL) and cooled to 0°C. (2-Ethylpyrrolidin-2-yl)methanol (2.1 g, 15.97 mmol) was added to the reaction solution, followed by stirring at 0°C for 2 hours. The reaction solution was poured into water, extracted with ethyl acetate, the organic phase was concentrated, and purified by column chromatography (petroleum ether/ethyl acetate = 5/1~2/1) to obtain the title compound (1.5 g, 28%).

LC-MS: *m*/*z* [M+H]⁺ = 276.

### 3-Chloro-8a-ethyl-7,8,8a,9-tetrahydro-1H,6H-pyrrolo[1',2':3,4]imidazo[1,2-c]pyrimidin-1-one

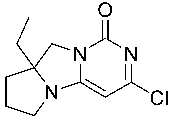

(1-(2,6-Dichloropyrimidin-4-yl)-2-ethylpyrrolidin-2-yl)methanol (1.5 g, 5.45 mmol) was added to dichloromethane (30 mL) and cooled to 0°C. Thionyl chloride (3.24 g, 27.27 mmol) was added dropwise, and the mixture was stirred at room temperature for 30 minutes. The reaction solution was concentrated, and the concentrated residue and potassium carbonate (2.26 g, 16.35 mmol) were added to acetonitrile (30 mL), and the mixture was stirred at 90°C overnight. The reaction solution was poured into water (30 mL), extracted with ethyl acetate and dichloromethane, respectively, the combined organic phases were concentrated, and separated by preparative thin layer chromatography (dichloromethane/methanol=20/1) to obtain the title compound (260 mg, 20%).

LC-MS: *m*/*z* [M+H]⁺ = 240.

### Intermediate 8

### N-Boc-2-methylpiperidine-2-carboxylic acid methyl ester

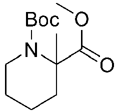

N-BOC-piperidine-2-carboxylic acid methyl ester (10.0 g, 41.15 mmol) and iodomethane (16.2 g, 123.4 mmol) were added to tetrahydrofuran (60 mL), cooled to 0°C, and lithium diisopropylamine in tetrahydrofuran (61.7 mL, 2.0 M, 123.4 mmol) was added dropwise. After stirring for 3 hours, the reaction solution was poured into saturated ammonium chloride solution, extracted with ethyl acetate, the organic phase was concentrated, and purified by column chromatography (petroleum ether/ethyl acetate = 20/1) to obtain the title compound (10.0 g, 95 %).

LC-MS: *m*/*z* [M+H-Boc]⁺ = 158.

### (2-Methylpiperidin-2-yl)methanol

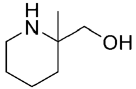

N-Boc-2-methylpiperidine-2-carboxylic acid methyl ester (5.0 g, 19.45 mmol) was dissolved in dichloromethane (15 mL), hydrogen chloride in ethyl acetate (30 mL) was added, and the reaction solution was concentrated after 20 minutes. Dichloromethane (20 mL) and solid sodium carbonate were added to adjust the pH to 7-8. After filtration, the filtrate was concentrated to give a crude product of 2-methylpiperidine-2-carboxylic acid methyl ester, which was dissolved in tetrahydrofuran (20 mL). The solution was added dropwise to a solution of lithium aluminum hydride in tetrahydrofuran (30 mL, 23.3 mmol) at 0°C, and after stirring at 0°C for 2 hours, the reaction was quenched with sodium sulfate decahydrate, stirred at room temperature for 30 minutes, filtered, and the filtrate was concentrated to obtain the title compound (1.8 g, 72%).

LC-MS: *m*/*z* [M+H]⁺ = 130.

### (1-(2, 6-Dichloropyrimidin-4-yl)-2-methylpiperidin-2-yl)methanol

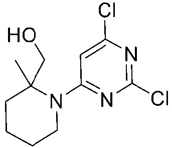

2,4,6-Trichloropyrimidine (3.8 g, 20.93 mmol) was added to acetonitrile (60 mL) and cooled to 0°C. After adding sodium carbonate (2.95 g, 27.90 mmol) and stirring for 10 minutes, a solution of (2-methylpiperidin-2-yl)methanol (1.8 g, 13.95 mmol) in acetonitrile (20 mL) was added dropwise, and the mixture was stirred at room temperature overnight. After filtration, the filtrate was concentrated, and purified by silica gel column chromatography (petroleum ether/ethyl acetate = 10/1~5/1) to give the title compound (1.8 g, 47%).

LC-MS: *m*/*z* [M+H]⁺ = 276.

### 3-Chloro-9a-methyl-6,7,8,9,9a,10-hexahydro-1H-pyrido[1',2':3,4]imidazo[1,2-c]pyrimidin-1-one

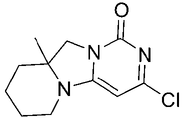

(1-(2,6-Dichloropyrimidin-4-yl)-2-methylpiperidin-2-yl)methanol (1.8 g, 6.54 mmol) was added to dichloromethane (20 mL), and thionyl chloride (1.56 g, 13.0 mmol) was added dropwise at room temperature. After stirring at room temperature for 10 minutes, the reaction solution was concentrated, and potassium carbonate (2.7 g, 19.62 mmol) and acetonitrile (40 mL) were added. Stirring was carried out overnight at 85°C. The reaction solution was filtered, the filtrate was concentrated, and purified by column chromatography (dichloromethane/methanol = 20/1) to obtain the title compound (1.2 g, 77%).

LC-MS: *m*/*z* [M+H]⁺ = 240.

¹H NMR (400 MHz, CDCl₃) δ5.47 (s, 1H), 3.91 - 3.78 (m, 2H), 3.40 (dd, *J =* 3.2, 13.5 Hz, 1H), 3.25 - 3.07 (m, 1H), 1.98 - 1.56 (m, 7H), 1.51 (dd, *J* = 4.2, 9.0 Hz, 2H).

### Intermediate 9

### N-Boc-2-ethylpiperidine-2-carboxylic acid methyl ester

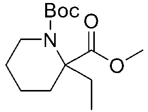

N-BOC-piperidine-2-carboxylic acid methyl ester (5.1 g, 20.98 mmol) and iodoethane (3.2 mL, 41.0 mmol) were added to tetrahydrofuran (60 mL), cooled to 0°C, and a solution of lithium diisopropylamine in tetrahydrofuran (21.0 mL, 2.0 M, 42.0 mmol) was added. After stirring at 0°C for 0.5 hours, the reaction solution was poured into saturated ammonium chloride solution (100 mL), extracted with ethyl acetate, the organic phase was concentrated, and purified by column chromatography (petroleum ether/ethyl acetate = 20/1~10/1 ) to give the crude title compound (4.0 g, 70%).

LC-MS: m/z [M+H-Boc]⁺ = 172.

### 2-Ethyl-2-hydroxymethylpiperidine-1-carboxylic acid tert-butyl ester

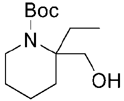

N-Boc-2-ethylpiperidine-2-carboxylic acid methyl ester (3.0 g, 11.0 mmol) was dissolved in tetrahydrofuran (30 mL), and lithium aluminum hydride (970 mg, 25.5 mmol) was added in batches at 0°C. After stirring at the temperature for 15 minutes, sodium sulfate decahydrate was added to quench the reaction, the mixture was stirred at room temperature for 30 minutes, filtered, the filtrate was concentrated, and purified by column chromatography (petroleum ether/ethyl acetate = 10/1) to obtain the title compound (300 mg , 11%).

LC-MS: *m*/*z* [M+H-Boc]⁺ = 144.

### (1-(2, 6- Dichloropyrimidin-4-yl)-2-ethylpiperidin-2-yl)methanol

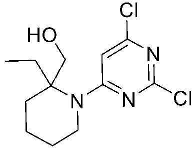

2-Ethyl-2-hydroxymethylpiperidine-1-carboxylic acid tert-butyl ester (400 mg, 1.65 mmol) was added to dichloromethane (3 mL), and a solution of hydrogen chloride in ethyl acetate (4.0 M, 6 mL) was added. After stirring at room temperature for 10 minutes, the mixture was concentrated to give (2-ethylpiperidin-2-yl)methanol (320 mg). (2-Ethylpiperidin-2-yl)methanol (320 mg, 1.10 mmol) and 2,4,6-trichloropyrimidine (600 mg, 3.33 mmol) were sequentially added to acetonitrile (16 mL), cooled to 0°C. Sodium carbonate (530 mg, 5.01 mmol) and (2-ethylpiperidin-2-yl)methanol obtained above were added separately, and the reaction mixture was stirred at room temperature overnight. After filtration, the filtrate was concentrated, and separated by preparative thin layer chromatography (petroleum ether/ethyl acetate = 5/1) to give the title compound (200 mg, 41 %).

LC-MS: *m*/*z* [M+H]⁺ = 290.

### 3-Chloro-9a-ethyl-6,7,8,9,9a,10-hexahydro-1H-pyrido[1',2':3,4]imidazo[1,2-c]pyrimidin-1-one

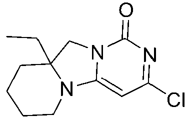

(1-(2,6-Dichloropyrimidin-4-yl)-2-ethylpiperidin-2-yl)methanol (50 mg, 0.17 mmol) was added to dichloromethane (6 mL), thionyl chloride (40 mg, 0.34 mmol) was added dropwise at room temperature, and the reaction solution was stirred at room temperature for 5 minutes after the addition. The reaction solution was concentrated, potassium carbonate (70 mg, 0.51 mmol) and acetonitrile (6 mL) were added, and the mixture was stirred at 85°C overnight. The reaction solution was filtered, the filtrate was concentrated, and separated by preparative thin-layer chromatography (dichloromethane/methanol = 10/1) to obtain the title compound (30 mg, 77 %). LC-MS: *m*/*z* [M+H]⁺ = 254.
¹H NMR (400 MHz, CDCl₃): δ5.49 (s, 1H), 3.95 (d, *J* = 12.2 Hz, 1H), 3.75 (d, *J* = 12.2 Hz, 1H), 3.41 (d, *J=* 13.7 Hz, 1H), 3.13 (t, *J* = 13.0 Hz, 1H), 1.70 - 2.05 (m, 6H), 1.52 (d, *J =* 12.7 Hz, 2H), 0.89 (t, *J* = 7.3 Hz, 3H);

### Intermediate 10

### N-Boc-morpholine-3-carboxylic acid methyl ester

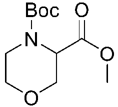

N-Boc-morpholine-3-carboxylic acid (10 g, 43.3 mmol) was dissolved in dichloromethane (200 mL) and methanol (20 mL), and trimethylsilyldiazomethane (43 mL, 2.0 M in n-hexane solution, 86.7 mmol) was added in batches. After the addition, the mixture was stirred at room temperature for 1 hour, and the reaction solution was directly concentrated to obtain the crude title compound (11.4 g).

LC-MS: m/z [M+H-Boc]⁺ = 146.

### N-Boc-3-methyl-morpholine-3-carboxylic acid methyl ester

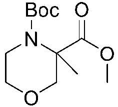

N-Boc-morpholine-3-carboxylic acid methyl ester (11 g, 44.9 mmol) was dissolved in tetrahydrofuran (120 mL), and iodomethane (10.5 g, 67.3 mmol) was added under argon protection. The temperature of the reaction solution was cooled to below 0°C, and a solution of sodium bis(trimethylsilyl)amide in tetrahydrofuran (2.0 M, 45 mL, 89.8 mmol) was added dropwise. After the addition, the mixture was stirred at room temperature for 3 hours, and methanol was added dropwise to quench the reaction. The reaction solution was poured into water, extracted with ethyl acetate, the combined organic phases were dried, filtered, and the filtrate was concentrated and separated by column chromatography to obtain the title compound (8.6 g, 74%).

LC-MS: m/z [M+H-Boc]⁺ = 160.

### 3-Hydroxymethyl-3-methyl-morpholine-4-carboxylic acid tert-butyl ester

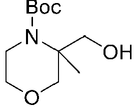

N-Boc-3-methylmorpholine-3-carboxylic acid methyl ester (1 g, 3.86 mmol) was dissolved in tetrahydrofuran (10 mL), and the reaction solution was cooled to below 0°C. Lithium aluminum hydride (220 mg, 5.8 mmol) was added in batches, and stirred at room temperature for 30 minutes. The reaction solution was added with sodium sulfate decahydrate for quenching, stirred at room temperature for 0.5 hours, dried over anhydrous sodium sulfate, filtered, and the filtrate was concentrated and purified by column chromatography to obtain the title compound (550 mg, 61%). LC-MS: m/z [M+H-Boc]⁺ = 132.

### (3-Methyl-morpholin-3-yl)methanol hydrochloride

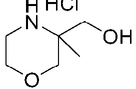

3-Hydroxymethyl-3-methyl-morpholine-4-carboxylic acid tert-butyl ester (3.2 g, 13.85 mmol) was dissolved in dichloromethane (50 mL), and a solution of hydrogen chloride in ethyl acetate (4.0 M, 50 mL) was added under argon protection, stirred at room temperature for 1 hour, and the reaction solution was concentrated to obtain the crude title compound (1.7 g).

LC-MS: *m*/*z* [M+H]⁺ = 132.

### (4-(2, 6-Dichloropyrimidin-4-yl)-3-methyl-morpholin-3-yl)methanol

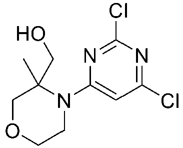

(3-Methylmorpholin-3-yl)methanol hydrochloride (1.5 g, 11.45 mmol) was dissolved in acetonitrile (50 mL), sodium carbonate (3.64 g, 34.35 mmol) was added, and the reaction solution was cooled to below 0°C. 2,4,6-Trichloropyrimidine (10.5 g, 57.25 mmol) was added dropwise, stirred at 50°C overnight, filtered through celite, the filtrate was concentrated, and purified by column chromatography to obtain the title compound (2.1 g, 66%).

LC-MS: *m*/*z* [M+H]⁺ = 278.

### 7-Chloro-11a-methyl-3,4,11,11a-tetrahydro-1H,9H-pyrimido[6',1':2,3]imidazo[5,1-c][1,4]oxazin-9-one

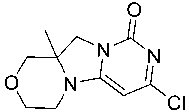

(4-(2,6-Dichloropyrimidin-4-yl)-3-methyl-morpholin-3-yl)methanol (1.6 g, 5.76 mmol) was dissolved in dichloromethane (50 mL), thionyl chloride (2.05 g, 17.3 mmol) was added, stirred at room temperature for 1 hour, the reaction solution was concentrated, the concentrated residue was dissolved in acetonitrile (50 mL), potassium carbonate (2.1 g, 15.2 mmol) was added. After stirring at 85°C overnight, the reaction solution was filtered through celite, the filtrate was concentrated, and purified by silica gel column chromatography to obtain the title compound (1.2 g, 98%).
LC-MS: *m*/*z* [M+H]⁺ = 242.
¹H NMR (400 MHz, DMSO-*d*₆) δ5.99 (s, 1H), 3.79 (d, *J* = 7.3 Hz, 1H), 3.73 (s, 2H), 3.66 - 3.58 (m, 2H), 3.57 - 3.50 (m, 1H), 3.47 - 3.39 (m, 2H), 1.47 (s, 3H);

### Intermediate 11

### ((1S, 4R)-2-oxa-5-azabicyclo[2.2.1]hept-4-yl)methan-d2-ol

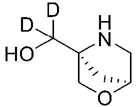

(1S,4S)-5-tert-butoxycarbonyl-2-oxa-5-azabicyclo[2.2.1]heptane-4-carboxylic acid methyl ester (470 mg, 1.83 mmol, Chemistry Letters, 2017, 566 - 568) was added to a solution of hydrogen chloride in ethyl acetate (4.0 M, 10 mL), and the reaction mixture was stirred at room temperature for 5 hours. The reaction solution was concentrated, acetonitrile (20 mL) and sodium carbonate (1 g) were added, and the mixture was stirred at room temperature for 1 hour. The reaction solution was filtered and concentrated. Anhydrous tetrahydrofuran (15 mL) and lithium aluminum deuteride (138.9 mg, 3.66 mmol) were added to the residue, and the mixture was stirred at room temperature overnight. The reaction solution was added with sodium sulfate decahydrate for quenching, filtered, and the filtrate was concentrated to obtain the crude title compound (200 mg). LC-MS: *m*/*z* [M+H]⁺ = 132.

### (((1S, 4R)-5-(2, 6-dichloropyrimidin-4-yl)-2-oxa-5-azabicyclo[2.2.1]hept-4-yl)methan-d2-ol

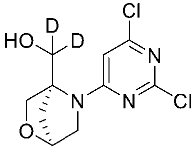

Triethylamine (307 mg, 3.04 mmol) and 2,4,6-trichloropyrimidine (335.6 mg, 1.83 mmol) were added to acetonitrile (20 mL), and cooled to 0°C. ((1S,4R)-2-oxa-5-azabicyclo[2.2.1]hept-4-yl)methan-d2-ol (200 mg, 1.52 mmol) was slowly added to the reaction system, and the reaction solution was stirred at room temperature overnight. The reaction solution was concentrated and purified by silica gel column chromatography to obtain the title compound (130 mg, 31 %).

LC-MS: *m*/*z* [M+H]⁺ = 278.

### (3S,11aR)-7-chloro-3,4-dihydro-1H,9H,11H-3,11a-methanopyrimido[6',1':2,3]imidazo[5,1-c] [1, 4 ]oxazin-9-one-11,11-d2

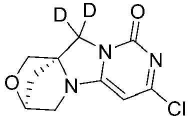

((1S,4R)-5-(2,6-dichloropyrimidin-4-yl)-2-oxa-5-azabicyclo[2.2.1]heptan-4-yl) methan-d2-ol (130 mg, 0.47 mmol) was added to dichloromethane (10 mL) and cooled to 0°C. Thionyl chloride (279.6 mg, 2.35 mmol) was added dropwise, and the mixture was stirred at 0°C for 30 minutes. The reaction solution was concentrated, and potassium carbonate (194.6 mg, 1.41 mmol) and acetonitrile (15 mL) were added, and the mixture was stirred at reflux overnight. The reaction solution was diluted with water and extracted with dichloromethane. The organic phase was washed with saturated brine, dried over anhydrous sodium sulfate, and concentrated. The residue was separated by preparative thin layer chromatography (dichloromethane/methanol = 20/1) to give the title compound (63 mg, 74 %).

LC-MS: *m*/*z* [M+H]⁺ = 242.

### Intermediate 12

### ((1S,4R)-5-(2,6-dichloro-5-fluoropyrimidin-4-yl)-2-oxa-5-azabicyclo[2.2.1]hept-4-yl)methanol

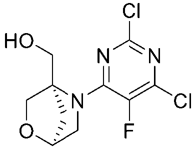

2,4,6-Trichloro-5-fluoropyrimidine (872 mg, 4.4 mmol) and triethylamine (795 mg, 7.9 mmol) were added to tetrahydrofuran (10 mL) and cooled to 0°C. ((1S,4R)-2-oxa-5-azabicyclo[2.2.1]hept-4-yl)methanol (650 mg, 4.0 mmol) was added to the reaction solution, and the mixture was stirred at 0°C for 2 hours. The reaction solution was concentrated and purified by silica gel column chromatography (petroleum ether/ethyl acetate = 5/1~2/1) to obtain the title compound (700 mg, 60%).

LC-MS: *m*/*z* [M+H]⁺ = 294.

### (3S,11aR)-7-chloro-6-fluoro-3,4-dihydro-1H,9H,11H-3,11a-methanopyrimido[6',1':2,3limidazo[5,1-cl [1, 4]oxazin-9-one

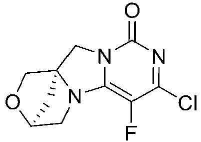

((1*S*,4*R*)-5-(2,6-dichloro-5-fluoropyrimidin-4-yl)-2-oxa-5-azabicyclo[2.2.1]hept-4-yl)methanol (700 mg, 2.4 mmol) was added to thionyl chloride (10 mL) and stirred at room temperature for 30 minutes. The reaction solution was concentrated, and potassium carbonate (660 mg, 4.8 mmol) and acetonitrile (20 mL) were added. Stirring was carried out overnight at 80°C. The reaction solution was concentrated, and separated by preparative thin layer chromatography to obtain the title compound (560 mg, 91 %).

LC-MS: *m*/*z* [M+H]⁺ = 258.

¹H NMR (400 MHz, CDCl₃) δ4.80 (br. s, 1H), 4.53 *(d, J=* 12.7 Hz, 1H), 4.15 - 4.06 (m, 1H), 4.05 - 3.95 (m, 2H), 3.80 (d, *J* = 10.3 Hz, 1H), 3.67 (d, *J* = 10.8 Hz, 1H), 2.22 - 2.13 (m, 1H), 2.12 - 2.05 (m, 1H).

### Intermediate 13

### ((1S, 4R)-5-(2,5,6-trichloropyrimidin-4-yl)-2-oxa-5-azabicyclo[2.2.1]hept-4-yl)methanol

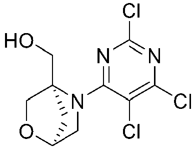

Perchloropyrimidine (333 mg, 1.71 mmol) was dissolved in acetonitrile (16 mL), solid sodium carbonate (362 mg, 3.42 mmol) and ((1S,4R)-2-oxa-5-azabicyclo[2.2.1]hept-4-yl)methanol hydrochloride (200 mg, 1.14 mmol) were added in sequence, stirred at room temperature overnight. After filtration, the filtrate was concentrated, and purified by preparative thin layer chromatography (petroleum ether/ethyl acetate = 4/1) to give the title compound (280 mg).

LC-MS: *m*/*z* [M+H]⁺ = 310.

### (3S,11aR)-6,7-dichloro-3,4-dihydro-1H,9H,11H-3,11a-methanopyrimido[6',1':2,3limidazo[5,1-cl [1, 4]oxazin-9-one

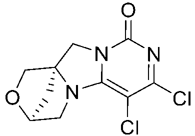

((1*S*,4*R*)-5-(2,5,6-trichloropyrimidin-4-yl)-2-oxa-5-azabicyclo[2.2.1]hept-4-yl)methanol (280 mg, 0.9 mmol) and thionyl chloride (650 mg, 4.5 mmol) were added to dichloromethane (8 mL) and stirred for 30 minutes. The reaction solution was concentrated, potassium carbonate (620 mg, 4.8 mmol) and acetonitrile (16 mL) were added, and the mixture was stirred at 90°C overnight. The reaction solution was filtered, the filtrate was concentrated, and separated and purified by preparative thin layer chromatography (dichloromethane/methanol=25/1) to obtain the title compound (160 mg, 65%).

LC-MS: *m*/*z* [M+H]⁺ = 274.

¹H NMR (400 MHz, CDCl₃) δ4.78 (br. s, 1H), 4.51 (d, *J =* 13.2 Hz, 1H), 4.08 (d, *J =* 7.3 Hz, 1H), 3.95 - 4.04 (m, 2H), 3.89 (s, 2H), 1.96 - 2.29 (m, 2H).

### Intermediate 14

### ((1S,4R)-5-(2, 6-dichloro-5-methyl-pyrimidin)-4-yl-2-oxa-5-azabicyclo[2.2.1lhept-4-yl)methanol

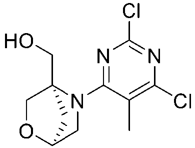

2,4,6-Trichloro-5-methylpyrimidine (113 mg, 0.4 mmol) was dissolved in acetonitrile (8 mL), and solid sodium carbonate (130 mg, 1.2 mmol) and ((1S,4R)-2-oxa-5-azabicyclo[2.2.1]hept-4-yl)methanol hydrochloride (70 mg, 0.6 mmol) were added separately, stirred at room temperature overnight. After filtration, the filtrate was concentrated, and purified by preparative thin layer chromatography (petroleum ether/ethyl acetate = 4/1) to give the title compound (80 mg).

LC-MS: *m*/*z* [M+H]⁺ = 290.

### (3S,11aR)-7-chloro-6-methyl-3,4-dihydro-1H,9H,11H-3,11a-methanopyrimido[6',1':2,3]imidazo[5, 1-cl [1, 4]oxazin-9-one

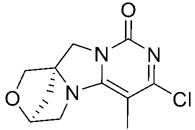

((1*S*,4*R*)-5-(2,6-dichloro-5-methylpyrimidin)-4-yl-2-oxa-5-azabicyclo[2.2.1]hept-4-yl)methanol (80 mg, 0.27 mmol) and thionyl chloride (160 mg, 1.35 mmol) were added to dichloromethane (3 mL) and stirred for 30 minutes. The reaction solution was concentrated, and potassium carbonate (112 mg, 0.81 mmol) and acetonitrile (12 mL) were added, and the mixture was stirred at 90°C overnight. The reaction solution was filtered, the filtrate was concentrated, and separated by preparative thin-layer chromatography (dichloromethane/methanol = 25/1) to obtain the title compound (20 mg, 29 %).

LC-MS: *m*/*z* [M+H]⁺ = 254.

### Intermediate 15

### 5-Formyl-2-(3-(trifluoromethyl)phenoxy)benzonitrile

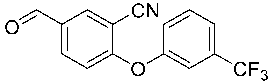

2-Fluoro-5-formyl-benzonitrile (15.0 g, 0.1 mol), 3-(trifluoromethyl)phenol (16.0 g, 0.1 mol) and potassium carbonate (13.8 g, 0.1 mol) were added to N,N-dimethylformamide(100.0 mL). After reacting at 105°C for 8 hours, the reaction solution was poured into water, extracted with dichloromethane, the organic phase was concentrated, and the concentrated residue was slurried with ethanol to obtain the title compound (22.1 g, 75.5 %).

¹H NMR (400 MHz, DMSO-*d*₆) δ9.96 (s, 1H), 8.49 (s, 1H), 8.14 (br. s, 1H), 7.88 (br. s, 2H), 7.47 (br. s , 2H), 7.19 (br. s, 1H).

### 5-(Hydroxymethyl)-2-(3-(trifluoromethyl)phenoxy)benzonitrile

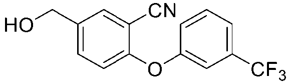

5-Formyl-2-(3-(trifluoromethyl)phenoxy)benzonitrile (24.3 g, 83.44 mmol) was added to methanol (250.0 mL), and sodium borohydride (4.86 g, 127.9 mmol) was added in batches. After reacting at room temperature for 0.5 hours, the reaction solution was poured into water, extracted with dichloromethane, and the organic phase was concentrated to obtain the title compound (24.2 g, 99%).

¹H NMR (400 MHz, DMSO-*d*₆) δ7.80 (s, 1H), 7.73 - 7.53 (m, 3H), 7.52 - 7.27 (m, 2H), 7.09 (s, 1H), 5.41 (s, 1H), 4.51 (s, 2H).

Referring to the table below, the following intermediates were prepared with reference to the method for the preparation of Intermediate 15, except that the starting materials described in the column "starting materials" were used in place of the corresponding starting materials.

| **Interme diate No.** | **Intermediate name** | **Structural formula** | **¹HNMR (400MHz)** | **Starting materials** |
|---|---|---|---|---|
| **16** | (3,5-Difluoro-4-((2-(trifluoromethyl)pyridi n-4-yl)oxy)phenyl)methanol | 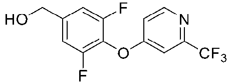 | DMSO-*d*₆:δ8.68(d,*J*=5.9Hz, 1H),7. 60(d,*J*=2.4Hz, 1H),7.32(s,1 H),7.29(s,1H),7.27(dd,*J*=2. 4,5.9Hz, 1H),5.57(t,*J*=5.6H z,1H),4.57(d,*J*=5.4Hz,2H) | 3,4,5-trifluorobenzaldehyde ; 4-hydroxy-2-(trifluoromethyl)pyrid ine |
| **17** | (4-(4-chloro-3-(trifluoromethyl)pheno xy)-3,5-difluorophenyl)methan ol | 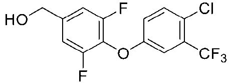 | DMSO-*d*₆:δ7.70(d,*J*=8.8Hz,1H),7. 47(d,*J*=2.0Hz,1H),7.27(d,*J* =9.3Hz,3H),5.53(br.s,1H), 4.55(d,*J*=2.9Hz,2H) | 3,4,5-trifluorobenzaldehyde ; 4-chloro-3-(trifluoromethyl)phen ol |
| **18** | (4-((2-chloropyridin-4-yl)oxy)-3,5-difluorophenyl)methan ol | 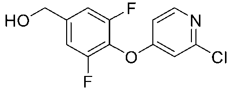 | CDCl₃:δ8.28(br.s,1H),7.11 (d,*J*=8.3Hz,2H),6.85(br.s,2 H),4.85-4.67(m,2H),2.09(br.s,1H) | 3,4,5-trifluorobenzaldehyde ; 2-chloro-4-hydroxypyridine |
| **19** | (3,5-difluoro-4-((2-(trifluoromethyl)pyrimi din-5-yl)oxy)phenyl)methano l | 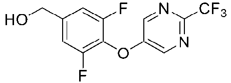 | CDCl₃:δ8.56(br.s.,2H),7.1 3(d,*J*=8.8Hz,2H),4.77(br.s. ,2H),2.08(br.s.,1H) | 3,4,5-trifluorobenzaldehyde ; 2-(trifluoromethyl)pyri midin-5-ol |
| **20** | (3,5-difluoro-4-((5-(trifluoromethyl)pyridi n-3-yl)oxy)phenyl)methano l | 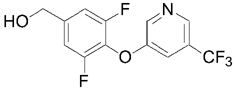 | CDCl₃:δ8.61(br.s,1H),8.55 (br.s,1H),7.42(br.s,1H),7.1 0(d,*J*=8.3Hz,2H),4.75(br.s, 2H),2.47(br.s,1H) | 3,4,5-trifluorobenzaldehyde ; 3-hydroxy-5-(trifluoromethyl)pyrid ine |
| **21** | (3,5-difluoro-4-((6-(trifluoromethyl)pyridi n-3-yl)oxy)phenyl)methano l | 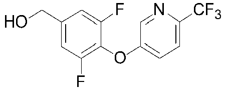 | CDCl₃:δ8.48(br.s,1H),7.64 (d,*J*=8.3Hz,1H),7.30(d,*J*=7 .8Hz,1H),7.1 0(d,*J*=8.8Hz,2 H),4.74(br.s,2H), 2.33(br.s,1H) | 3,4,5-trifluorobenzaldehyde ; 3-hydroxy-6-trifluoromethylpyridine |
| **22** | (3,5-difluoro-4-((2-methyl-pyridin-4-yl)oxy)phenyl)methano l | 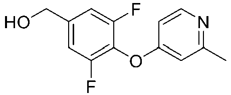 | CDCl₃:δ8.40-8.26(m,1H),7.09(d*,J*=8.3H z,2H),6.67(s,1H),6.70(s,1H ),4.74(br.s,2H),2.52(br.s,3 H),2.39(br.s,1H) | 3,4,5-trifluorobenzaldehyde ; 4-hydroxy-2-methylpyridine |
| **23** | (3,5-difluoro-4-((1-methyl-lH-pyrazol-4-yl)oxy)phenyl)methano l | 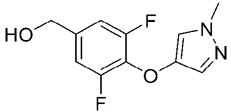 | CDCl₃:δ7.25(br.s,1H),7.20 (br.s,1H),7.00(d,*J*=7.8Hz,2 H),4.68(br.s,2H),3.82(br.s, 3H) | 3,4,5-trifluorobenzaldehyde ; 1-methyl-4-hydroxy-1H-pyrazole |
| **24** | 3-(2-fluoro-4-(hydroxymethyl)benzyl )-5-(trifluoromethyl)benzo nitrile | 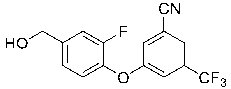 | DMSO-*d*₆:δ8.06(br.s,1H),7.77(br.s, 1H),7.62(br.s,1H),7.44-7.27(m,2H),7.23(d,*J*=7.8H z,1H),5.47(br.s,1H),4.53(d, *J*=4.9Hz,2H) | 3-fluoro-4-hydroxybenzaldehyde ; 3-fluoro-5-(trifluoromethyl)benz onitrile |
| **25** | (3-fluoro-4-((2-(trifluoromethyl)pyridi n-4-yl)oxy)phenyl)methano l | 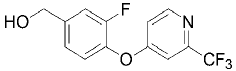 | DMSO-*d*₆:δ8.64 (d, *J =* 5.4 Hz, 1H ), 7.44 (br. s., 1H), 7.42 - 7. 35 (m, 2H), 7.28 (d, *J* = 8.3 Hz, 1H), 7.16 (d, *J* = 3.4 Hz, 1H), 5.44 (br. s., 1H), 4.57 (br. s., 2H) | 3,4-difluorobenzaldehyde; 4-hydroxy-2-(trifluoromethyl)pyrid ine |
| **26** | (4-(4-chloro-3-(trifluoromethyl)pheno xy)-3-fluorophenyl)methanol | 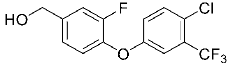 | DMSO-*d*₆:δ7.70(d,*J*=8.8Hz,1H),7. 45-7.19(m,5H),5.39(br.s,1H),4 .52(br.s,2H) | 3,4-difluorobenzaldehyde; 4-chloro-3-(trifluoromethyl)phen ol |
| **27** | (4-((2-(trifluoromethyl)pyridi n-4-yl)oxy)phenyl)methano l | 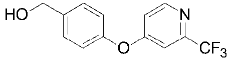 | DMSO-*d*₆:δ8.62(d,*J=*5.4Hz,1H),7. 46(d,*J*=7.8Hz,2H),7.37(br. s,1H),7.21(d,*J=*8.3Hz,2H), 7.13(d,J=3.4Hz,1H),5.29(t, *J=*5.4Hz,1H),4.54(d,*J*=5.4 Hz,2H). | p-fluorobenzaldehyde; 4-hydroxy-2-(trifluoromethyl)pyrid ine |
| **28** | (4-(4-chloro-3-(trifluoromethyl)pheno xy)phenyl)methanol | 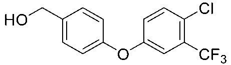 | DMSO-*d*₆:37.69(d,*J*=8.8Hz,1H),7. 49-7.32(m,3H),7.24(d,*J=*8.3H z, 1H),7.09(d,*J*=8.3Hz,2H), 5.23(br.s,1H),4.51(br.s,2H) | p-fluorobenzaldehyde; 4-chloro-3-(trifluoromethyl)phen ol |
| **29** | (6-(4-fluoro-3-(trifluoromethyl)pheno xy)pyridin-3-yl)methanol | 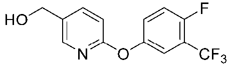 | DMSO-*d*₆:δ8.06(br.s,1H),7.83(d,*J* =7.8Hz,1H),7.55(d,*J*=8.3H z,3H),7.09(d,*J*=7.8Hz,1H), 5.29(br.s, 1H),4.48(br.s,2H) | 4-fluoro-3-(trifluoromethyl)phen ol; 6-chloronicotinaldehyde |
| **30** | 2-(4-chloro-3-(trifluoromethyl)pheno xy)-5-(hydroxymethyl)benzo nitrile | 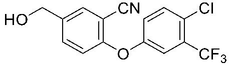 | CDCl₃:δ7.73(s,1H),7.53(d, *J*=8.8Hz,1H),7.57(d,*J*=8.8 Hz,1H),7.40(d,*J*=2.4Hz,1H ),7.18(dd,*J*=2.4,8.8Hz,1H), 6.95(d,*J*=8.3Hz,1H),4.74(b r.s,2H) | 4-fluoro-3-cyanobenzaldehyde; 4-chloro-3-(trifluoromethyl)phen ol |
| **31** | 2-(4-chloro-3-fluorophenoxy)-5-(hydroxymethyl)benzo nitrile | 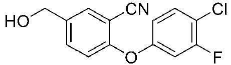 | DMSO-d6:δ7.78(br.s,1H),7.62(br.s ,2H),7.32(br.s,1H),7.12(br. s,1H),6.97(br.s,1H),5.41(br .s,1H),4.50(br.s,2H) | 4-fluoro-3-cyanobenzaldehyde; 4-chloro-3-fluorophenol |
| **32** | 5-(hydroxymethyl)-2-(4-(trifluoromethyl)pheno xy)benzonitrile | 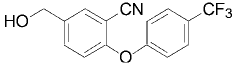 | DMSO-*d*₆:δ7.89-7.73(m,3H),7.67(s,1H),7.3 6-7.12(m,3H),5.44(s,1H),4.5 3(s,2H) | 4-fluoro-3-cyanobenzaldehyde; 4-(trifluoromethyl)phen ol |
| **33** | 5-(hydroxymethyl)-2-((2-(trifluoromethyl)pyridi n-4-yl)oxy)benzonitrile | 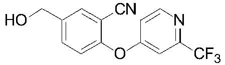 | DMSO-*d*₆:88.71(d,*J*=5.4Hz,1H),7. 90(br.s, 1H),7.77(d,*J*=8.3H z,1H),7.61(br.s,1H),7.46(d, *J=*8.3Hz,1H),7.30(d,*J=*3.4 Hz,1H),5.50(t,*J*=5.6Hz,1H ),4.58(d,*J*=5.4Hz,2H). | 4-fluoro-3-cyanobenzaldehyde; 4-hydroxy-2-(trifluoromethyl)pyrid ine |

### Intermediate 34

### 4-(Benzyloxy)-2-(trifluoromethoxy)pyridine

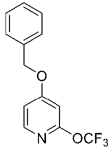

4-(Benzyloxy)pyridin-2-ol (1.91 g, 9.48 mmol) and 1-trifluoromethyl- 1,2-benziodoxol-3(H)-one (1 g, 3.16 mmol) were added into nitromethane (25 mL) and stirred at 100°C overnight. The reaction solution was concentrated, and the residue was purified by column chromatography (petroleum ether/ethyl acetate = 10/1) to obtain the title compound (530 mg, 47%).

LC-MS: *m*/*z* [M+H]⁺ = 270.

### 2-(Trifluoromethoxy)pyridin-4-ol

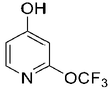

4-(Benzyloxy)-2-(trifluoromethoxy)pyridine (530 mg, 1.97 mmol) and Pd/C (10%, 125 mg) were added to methanol (20 mL) at 60°C, stirred overnight under hydrogen atmosphere at a pressure of 3.0 bar. The reaction solution was filtered, and the filtrate was concentrated to obtain the crude title compound (330 mg, 94%).

LC-MS: *m*/*z* [M+H]⁺ = 180.

### (3, 5-Difluoro-4-((2-(trifluoromethoxy)pyridin-4-yl)oxy)phenyl)methanol

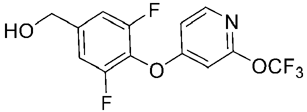

3,4,5-Trifluorobenzaldehyde (294.4 mg, 1.84 mol), 2-(trifluoromethoxy)pyridin-4-ol (330 mg, 1.84 mol) and potassium carbonate (330.6 mg, 2.39 mol) were added to N,N-dimethylformamide (10 mL), stirred at 120°C for 2 hours. The reaction solution was poured into water, extracted with ethyl acetate, and the organic phase was concentrated to obtain 3,5-difluoro-4-((2-(trifluoromethoxy)pyridin-4-yl)oxy)benzaldehyde as a crude product, the crude product was added to ethanol (250.0 mL), sodium borohydride (69.61 mg, 1.84 mmol) was added, and the mixture was stirred at room temperature for 1 hour. The reaction was quenched with water, extraction was performed with ethyl acetate, and the organic phase was dried and concentrated to give the title compound (400 mg, 68%).

LC-MS: *m*/*z* [M+H]⁺ = 322.

¹H NMR (400 MHz, DMSO-*d*₆) δ8.30 (d, *J =* 5.9 Hz, 1H), 7.29 (d, *J =* 9.3 Hz, 2H), 7.07 (d, *J =* 3.9 Hz, 1H), 6.95 (s, 1H), 5.56 (br. s, 1H), 4.56 (d, *J= 4.9* Hz, 2H).

### Intermediate 35

### 4-((2-(Trifluoromethyl)pyridin-4-yl)oxy)benzaldehyde

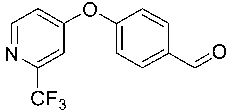

2-(Trifluoromethyl)pyridin-4-ol (1.0 g, 5.6 mmol), 4-fluorobenzaldehyde (0.7 g, 5.5 mmol) and potassium carbonate (1.7 g, 12.0 mmol) were added to N,N-dimethylformamide (10.0 mL). After stirring at 120°C for 14 h, the reaction solution was poured into water, extracted with ethyl acetate, the organic phase was concentrated, and purified by silica gel column to obtain the title compound (0.8 g, 54 %).

LC-MS: *m*/*z* [M+H]⁺ = 268.

### 2-(Trifluoromethyl)-4-(4-vinylphenoxy)pyridine

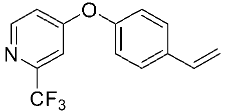

4-((2-(Trifluoromethyl)pyridin-4-yl)oxy)benzaldehyde (0.8 g, 2.9 mmol) and methyltriphenylphosphonium bromide (1.28 g, 3.6 mmol) were added to tetrahydrofuran (20 mL), sodium hydride (173 mg, 7.2 mmol, 60%) was added to the system, and the mixture was stirred for 16 h under argon protection. The reaction solution was poured into ice water, extracted with dichloromethane, the organic phase was concentrated, and purified by column chromatography to obtain the title compound (0.2 g, 26%).

LC-MS: *m*/*z* [M+H]⁺ = 266.

### 2-(4-((2-(Trifluoromethyl)pyridin-4-yl)oxy)phenyl)ethan-1-ol

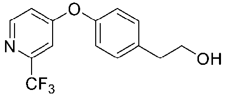

At 0°C, 2-(trifluoromethyl)-4-(4-vinylphenoxy)pyridine (0.2 g, 0.75 mmol) was dissolved in tetrahydrofuran (2 mL), and a solution of 9-borabicyclo[3.3.1]nonane in tetrahydrofuran (0.5 M, 15 mL, 7.5 mmol) was added, naturally warmed to room temperature and stirred for 16 h. Water (1 mL), hydrogen peroxide (30 %, 5 mL), sodium hydroxide aqueous solution (3.0 M, 10 mL) were added to the reaction solution, and the reaction was carried out at 50°C for 2 h. The reaction solution was diluted with water, extracted with dichloromethane, the combined organic phases were concentrated, and separated by preparative thin layer chromatography to obtain the title compound (116 mg, 55%).

LC-MS: *m*/*z* [M+H]⁺ = 284.

¹H NMR (400 MHz, DMSO-*d*₆) δ8.61 (d, *J =* 5.4 Hz, 1H), 7.38 (br. s, 2H), 7.35 (br. s, 1H), 7.16 (d, *J= 8.3* Hz, 2H), 7.12 (d, *J* = 3.4 Hz, 1H), 4.67 (t, *J* = 4.9 Hz, 1H), 3.71 - 3.55 (m, 2H), 2.77 (t, *J= 6.6* Hz, 2H).

Referring to the table below, the following intermediates were prepared with reference to the method for the preparation of Intermediate 35, except that the starting materials described in the "Starting materials" column were used in place of the corresponding starting materials.

| **Intermed iate No.** | **Intermediate name** | **Structural formula** | **¹H NMR (400 MHz)** | **Starting materials** |
|---|---|---|---|---|
| **36** | 2-(4-(4-chloro-3-(trifluoromethyl)phe noxy)phenyl)ethan-1-ol | 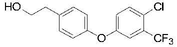 | CDCl₃: δ7.41 (d, *J =* 8.8 Hz, 1H), 7.31 (br. s, 1H), 7.25 (br. s, 1H), 7.23 (br. s, 1H), 7.05 (d, *J = 6.8* Hz, 1H), 6.96 (d, *J =* 8.3 Hz, 2H), 3.87 (t, *J* = 5.6 Hz, 2H), 2.87 (t, *J* = 6.4 Hz, 2H) | 4-chloro-3-(trifluoromethyl)phen ol; 4-fluorobenzaldehyde |
| **37** | 2-(3,5-difluoro-4-((2-(trifluoromethyl)pyri din-4-yl)oxy)phenyl)ethan-1-ol | 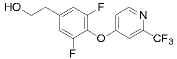 | DMSO-*d*₆: δ8.65 (br. s, 1H), 7.56 (br. s, 1H), 7.22 (s, 2H), 7.25 (s, 1H), 4.69 (br. s, 1H), 3.65 (br. s, 2H), 2.76 (br. s, 2H) | 3,4,5-trifluorobenzaldehyde ; 4-hydroxy-2-(trifluoromethyl)pyrid ine |
| **38** | 5-(2-hydroxyethyl)-2-((2-(trifluoromethyl)pyri din-4-yl)oxy)benzonitrile | 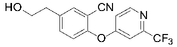 | DMSO-*d*₆: δ8.70 (d, *J* = 5.4 Hz, 1H), 7.87 (br. s, 1H), 7.69 (d, *J =* 8.3 Hz, 1H), 7.61 (br. s, 1H), 7.41 (d, *J* = 8.8 Hz, 1H), 7.26 (d, *J* = 2.9 Hz, 1H), 4.75 - 4.67 (m, 1H), 3.66 (br. s, 2H), 2.81 (t, *J =* 6.1 Hz, 2H) | 4-fluoro-3-cyanobenzaldehyde; 4-hydroxy-2-(trifluoromethyl)pyrid ine |

Referring to the table below, the following intermediates were prepared with reference to the preparation method of Intermediate 13, except that the starting materials described in the column "Starting materials" were used in place of the corresponding starting materials.

| **Intermed iate No.** | **Intermediate name** | **Structural formula** | **LC-MS (m/z [M+H]⁺)** | **Starting materials** |
|---|---|---|---|---|
| **39** | (3*S*,11a*R*)-7-chloro-6-ethyl-3,4-dihydro-1H,9H,11H-3,1la-methanopyrimido[6',1':2,3]imi dazo[5,1-c][1,4]oxazin-9-one | 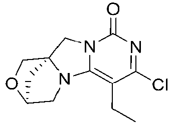 | 268 | 2,4,6-trichloro-5-ethylpyrimidine; ((1*S*,4*R*)-2-oxa-5-azabicyclo[2.2. 1Jhept-4-yl)methanol hydrochloride |
| **40** | (3*S*,11aR)-7-chloro-6-isopropyl-3,4-dihydro-1H,9H,11H-3,11a-methanopyrimido[6',1':2,3]imi dazo[5,1-c][1,4]oxazin-9-one | 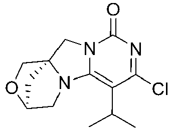 | 282 | 2, 4, 6-trichloro-5-isopropylpyrimidine; ((1*S*,4*R*)-2-oxa-5-azabicyclo[2.2.1]hept-4-yl)methanol hydrochloride |

Referring to the table below, the following intermediates were prepared with reference to the method for the preparation of Intermediate 15, except that the starting materials described in the column "Starting materials" were used in place of the corresponding starting materials.

| **Interme diate No.** | **Intermediate name** | **Structural formula** | **Spectrum: ¹H NMR (400MHz) or LC-MS (*m*/*z* [M+H]⁺)** | **Starting materials** |
|---|---|---|---|---|
| **41** | (2-fluoro-4-((2-(trifluoromethyl)pyrid in-4-yl)oxy)phenyl)methan ol | 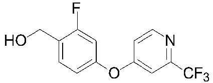 | CDCl₃: δ8.56 (br. s, 1H), 7.54 (t, *J =* 7.8 Hz, 1H), 7.22 (br. s, 1H), 6.99 (br. s, 1H), 6.92 (d, *J* = 7.8 Hz, 1H), 6.85 (d, *J* = 9.8 Hz, 1H), 4.79 (br. s, 2H) | 2,4-difluorobenzaldehyd e; 4-hydroxy-2-(trifluoromethyl)pyri dine |
| **42** | (3-fluoro-4-((6-(trifluoromethyl)pyrid in-3-yl)oxy)phenyl)methan ol | 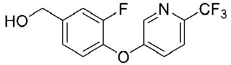 | CDCl₃: δ8.41 (d, *J* = 2.9 Hz, 1H), 7.62 (d, *J =* 8.3 Hz, 1H), 7.32 - 7.24 (m, 2H), 7.21 - 7.12 (m, 2H), 4.72 (d, *J* = 4.9 Hz, 2H) | 3,4-difluorobenzaldehyd e; 6-(trifluoromethyl)pyri dine-3-ol |
| **43** | (3-fluoro-4-((5-(trifluoromethyl)pyrid in-3-yl)oxy)phenyl)methan ol | 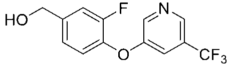 | 288 | 3,4-difluorobenzaldehyd e; 5-(trifluoromethyl)pyri dine-3-ol |
| **44** | (3-fluoro-4-((2-methyl-pyridin-4-yl)oxy)phenyl)methan ol | 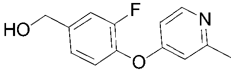 | CDCl₃: δ8.24 (d, *J* = 5.9 Hz, 1H), 7.24 (d, *J =* 4.9 Hz, 1H), 7.18 - 7.07 (m, 2H), 6.67 - 6.58 (m, 2H), 4.71 (s, 2H), 2.47 (s, 3H) | 3,4-difluorobenzaldehyd e; 2-methyl-pyridin-4-ol |
| **45** | (4-((2-chloropyridin-4-yl)oxy)-3-fluorophenyl)methano l | 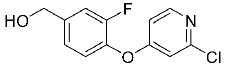 | 254 | 3,4-difluorobenzaldehyd e; 2-chloropyridin-4-ol |
| **46** | (3-fluoro-4-(3-(trifluoromethyl)phen oxy)phenyl)methanol | 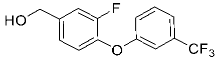 | DMSO-*d*₆: δ8.04 (s, 1H), 7.51 - 7.43 (m, 1H), 7.37 (d, *J* = 7.8 Hz, 1H), 7.34 - 7.21 (m, 2H), 7.21 - 7.09 (m, 3H), 4.76 (s, 2H) | 3,4-difluorobenzaldehyd e; 3-(trifluoromethyl)phe nol |
| **47** | (3,5-difluoro-4-(3-(trifluoromethyl)phen oxy)phenyl)methanol | 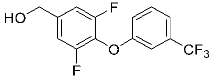 | CDCl₃: δ7.46 - 7.39 (m, 1H), 7.38 - 7.31 (m, 1H), 7.19 (br. s, 1H), 7.12 (d, *J* = 8.3 Hz, 1H), 7.07 (d, *J* = 8.3 Hz, 2H), 4.73 (s, 2H) | 3,4,5-trifluorobenzaldehyd e; m-trifluoromethylphen ol |
| **48** | (3,5-difluoro-4-(4-(trifluoromethyl)phen oxy)phenyl)methanol | 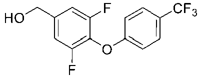 | CDCl₃: δ7.58 (d, *J* = 8.3 Hz, 2H), 7.52 (t, *J =* 7.8 Hz, 1H), 7.11 - 6.99 (m, 3H), 4.73 (s, 2H) | 3,4,5-trifluorobenzaldehyd e; 4-(trifluoromethyl)phe nol |
| **49** | (3-fluoro-4-(4-(trifluoromethyl)phen oxy)phenyl)methanol | 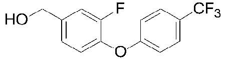 | CDCl₃: δ7.62 - 7.48 (m, 2H), 7.29 - 7.23 (m, 1H), 7.18 - 7.09 (m, 2H), 7.04 - 6.98 (m, *J* = 8.3 Hz, 2H), 4.72 (s, 2H) | 3,4-difluorobenzaldehyd e; 4-(trifluoromethyl)phe nol |
| **50** | (3,5-difluoro-4-(3-(trifluoromethoxy)phe noxy)phenyl)methano l | 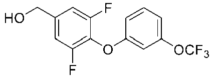 | CDCl₃: δ7.31 (t, *J* = 8.3 Hz, 1H), 7.28 (s, 1H), 7.25 (m, 1H), 7.07 (d, *J* = 8.3 Hz, 1H), 6.95 (d, *J* = 8.3 Hz, 1H), 6.90 - 6.77 (m, 1H), 4.72 (s, 2H) | 3,4,5-trifluorobenzaldehyd e; 3-(trifluoromethoxy)ph enol |
| **51** | (4-(3-(difluoromethyl)phen oxy)-3,5-difluorophenyl)metha nol | 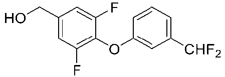 | CDCl₃: δ7.37 (t, *J* = 8.1 Hz, 1H), 7.20 (d, *J =* 7.3 Hz, 1H), 7.12 - 6.97 (m, 4H), 6.77 - 6.41 (m, 1H), 4.67 (s, 1H) | 3,4,5-trifluorobenzaldehyd e; 3-(difluoromethyl)phe nol |
| **52** | (3,5-difluoro-4-(4-fluorophenoxy)phenyl )methanol | 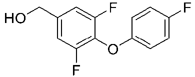 | CDCl₃: δ7.07 - 6.95 (m, 4H), 6.94 - 6.87 (m, 2H), 4.69 (s, 2H) | 3,4,5-trifluorobenzaldehyd e; 4-fluorophenol |
| **53** | (3,5-difluoro-4-(3-fluorophenoxy)phenyl )methanol | 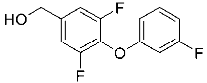 | CDCl₃: δ7.25 - 7.19 (m, 1H), 7.05 (d, *J* = 7.8 Hz, 2H), 6.83 - 6.71 (m, 2H), 6.66 (d, *J =* 9.8 Hz, 1H), 4.71 (s, 2H) | 3,4,5-trifluorobenzaldehyd e; 3-fluorophenol |
| **54** | (4-(3,4-difluorophenoxy)-3,5-difluorophenyl)metha nol | 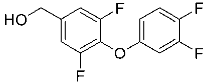 | CDCl₃: δ7.15 - 7.01 (m, 3H), 6.85 - 6.75 (m, 1H), 6.67 (d, *J* = 8.8 Hz, 1H), 4.71 (s, 2H) | 3,4,5-trifluorobenzaldehyd e; 3,4-difluorophenol |
| **55** | (4-(3,4-difluorophenoxy)-3-fluorophenyl)methano l | 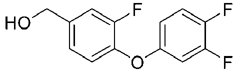 | CDCl₃: δ7.23 (d, *J =* 11.2 Hz, 1H), 7.15 - 7.02 (m, 3H), 6.85 - 6.75 (m, 1H), 6.69 (d, *J =* 8.8 Hz, 1H), 4.69 (s, 2H) | 3,4-difluorobenzaldehyd e; 3,4-difluorophenol |
| **56** | (3,5-difluoro-4-phenoxyphenyl)metha nol | 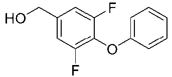 | CDCl₃: δ7.36 - 7.28 (m, 2H), 7.12 - 6.99 (m, 3H), 6.96 (d, *J* = 7.8 Hz, 2H), 4.68 (s, 2H) | 3,4,5-trifluorobenzaldehyd e; phenol |
| **57** | (4-(4-chloro-3-fluorophenoxy)-3,5-difluorophenyl)metha nol | 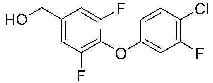 | CDCl₃: δ7.34 - 7.30 (m, 1H), 7.29 - 7.26 (m, 1H), 7.06 (d, *J* = 8.3 Hz, 2H), 6.81 - 6.65 (m, 2H), 4.71 (s, 2H). | 3,4,5-trifluorobenzaldehyd e; 4-chloro-3-fluorophenol |
| **58** | (3-fluoro-4-(((2-(trifluoromethyl)pyri midin-5-yl)oxy)phenyl)methan ol | 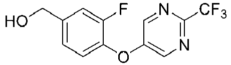 | CDCl₃: δ8.53 (s, 2H), 7.33 (d, *J* = 11.7 Hz, 1H), 7.24 - 7.21 (m, 2H), 4.76 (s, 2H) | 3,4-difluorobenzaldehyd e; 2-(trifluoromethyl)pyri midin-5-ol |

Referring to the table below, the following intermediates were prepared with reference to the method for the preparation of Intermediate 34, except that the starting materials described in the "Starting materials" column were used in place of the corresponding starting materials.

| **Interme diate No.** | **Intermediate name** | **Structural formula** | **LC-MS (*m*/*z* [M+H]⁺)** | **Starting materials** |
|---|---|---|---|---|
| **59** | (3-fluoro-4-(((2-(trifluoromethoxy)pyridin-4-yl)oxy)phenyl)methanol | 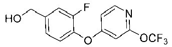 | 304 | 3,4-difluorobenzaldehyde; 2-(trifluoromethoxy)pyridin-4-ol |

Referring to the table below, the following intermediates were prepared with reference to the method for the preparation of Intermediate 35, except that the starting materials described in the "Starting materials" column were used in place of the corresponding starting materials.

| **Intermedi ate No.** | **Intermediate name** | **Structural formula** | **¹H NMR (400 MHz)** | **Starting materials** |
|---|---|---|---|---|
| **60** | 2-(4-(3-(trifluoromethyl)phe noxy)phenyl)ethan-1-ol | 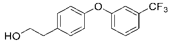 | DMSO-*d*₆: δ7.63 - 7.54 (m, 1H), 7.44 (d, *J =* 7.3 Hz, 1H), 7.32 - 7.19 (m, 4H), 7.01 (d, *J* = 7.8 Hz, 2H), 4.65 (t, *J* = 4.6 Hz, 1H), 3.62 (q, *J =* 5.9 Hz, 2H), 2.73 (t, *J* = 6.8 Hz, 2H) | 3-(trifluoromethyl)phenol; 4-fluorobenzaldehyde |
| **61** | 2-(3-fluoro-4-(3-(trifluoromethyl)phe noxy)phenyl)ethan-1-ol | 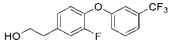 | DMSO-*d*₆: δ7.64 - 7.55 (m, 1H), 7.46 (d, *J =* 7.3 Hz, 1H), 7.36 - 7.16 (m, 4H), 7.12 (d, *J* = 8.3 Hz, 1H), 4.68 (t, *J =* 4.9 Hz, 1H), 3.65 (q, *J* = 5.9 Hz, 2H), 2.76 (t, J = 6.6 Hz, 2H) | 3-(trifluoromethyl)phenol; 3,4-difluorobenzaldehyde |
| **62** | 2-(4-(4-chloro-3-(trifluoromethyl)phe noxy)-3-fluorophenyl)ethan-1-ol | 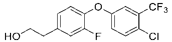 | DMSO-*d*₆: δ7.69 (d, *J =* 8.8 Hz, 1H), 7.47 - 7.38 (m, 1H), 7.30 (d, *J* = 12.2 Hz, 1H), 7.26 - 7.17 (m, 2H), 7.13 (d, *J* = 8.3 Hz, 1H), 4.68 (t, *J* = 5.1 Hz, 1H), 3.64 (q, *J =* 6.4 Hz, 2H), 2.76 (t, *J =* 6.6 Hz, 2H) | 3,4-difluorobenzaldehyde; 4-chloro-3-(trifluoromethyl)phenol |

### Intermediate 63

### 2,4-Dichloro-6-((1S,4R)-4-((hydroxymethyl)-2-oxa-5-azabicyclo[2.2.1]hept-5-yl)pyrimidine-5-carbonitrile

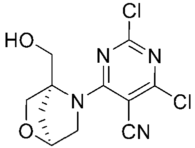

2,4,6-Trichloropyrimidine-5-carbonitrile (400 mg, 1.94 mmol) was dissolved in acetonitrile (12 mL), then solid sodium carbonate (318 mg, 3.0 mmol) and ((1S,4R)-2-oxa-5-azabicyclo[2.2.1]hept-4-yl)methanol hydrochloride (166 mg, 1.0 mmol) were added in sequence, stirred at room temperature overnight. After filtration, the filtrate was concentrated, and purified by preparative thin layer chromatography (petroleum ether/ethyl acetate = 4/1) to obtain the title compound (40 mg, 13%).

LC-MS: *m*/*z* [M+H]⁺ = 301.

### (3S,11aR)-7-chloro-9-oxo-3,4-dihydro-1H,9H,11H-3,11a-methanopyrimido[6',1':2,3]limidazo[5,1-c] [1, 4loxazine-6-carbonitrile

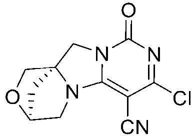

2,4-Dichloro-6-((1*S*,4*R*)-4-((hydroxymethyl)-2-oxa-5-azabicyclo[2.2.1]hept-5-yl)pyrimidine-5-carbonitrile (40 mg, 0.12 mmol) and thionyl chloride (0.2 mL) were added to dichloromethane (3 mL), and stirred at room temperature for 20 min. The reaction solution was concentrated, and sodium carbonate (40 mg, 0.37 mmol) and acetonitrile (6 mL) were added and stirred overnight at 85°C. The reaction solution was filtered, the filtrate was concentrated, and separated by preparative thin layer chromatography (dichloromethane/methanol=25/1) to obtain the title compound (30 mg, 91 %).

LC-MS: *m*/*z* [M+H]⁺ = 265.

Referring to the table below, the following intermediates were prepared with reference to the preparation method of Intermediate 63, except that the starting materials described in the column "Starting materials" were used in place of the corresponding starting materials.

| **Inter media te No.** | **Intermediate name** | **Structural formula** | **LC-MS (m/z [M+H]⁺)** | **Starting materials** |
|---|---|---|---|---|
| **64** | (3*S*,11aR)-7-chloro-6-methoxy-3,4-dihydro-1H,9H,11H-3,11a-methanopyrimido[6',1':2,3]imidaz o[5,1-c][1,4]oxazin-9-one | 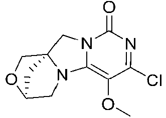 | 270 | 2,4,6-trichloro-5-methoxypyrimidine; ((1*S*,4*R*)-2-oxa-5-azabicyclo[2.2.1]hept-4-yl)methanol hydrochloride |

### Intermediate 65

### (2-(2, 6-Dichloro-5-methoxyprimidin-4-yl)-2-azabicyclo[2.1.1]hex-1-yl)methanol

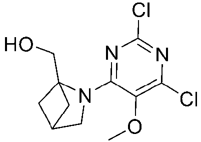

(2-Azabicyclo[2.1.1]hex-1-yl)methanol (500 mg, 4.42 mmol, Journal of Organic Chemistry, 2018, 83, 14350-14361) was dissolved in acetonitrile (10 mL), and sodium carbonate (3.64 g, 34.35 mmol) was added. The reaction solution was cooled to 0°C, and 2,4,6-trichloro-5-methoxypyrimidine (1.4 g, 6.46 mmol) was added. The reaction solution was reacted overnight at room temperature, and the reaction solution was filtered through celite, the filtrate was concentrated, and separated and purified by silica gel column chromatography to obtain the title compound (1.1 g, 72 %) as a white solid.

LC-MS: *m*/*z* [M+H]⁺ = 290.

### 3-Chloro-4-methoxy-7,8-dihydro-1H,6H,9H-7,8a-methanopyrrolo[1',2':3,4]imidazo[1,2-c]pyrimidin-1-one

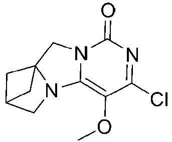

(2-(2,6-Dichloro-5-methoxypyrimidin-4-yl)-2-azabicycllo[2.1.1]hex-1-yl)methanol (1.1 g, 3.79 mmol) was dissolved in dichloromethane (10 mL), thionyl chloride (1.35 g, 11.38 mmol) was added, and the reaction was carried out at room temperature for 4 hours. The reaction solution was concentrated and dissolved in acetonitrile (10 mL), and potassium carbonate (1.5 g, 10.9 mmol) was added. The mixture was stirred at 85°C overnight, the reaction solution was cooled to room temperature, filtered through celite, and the filtrate was concentrated and purified by chromatography to obtain the solid title compound (230 mg, 25%).

LC-MS: *m*/*z* [M+H]⁺ = 254.

¹H NMR (400 MHz, CDCl₃) δ4.03 (s, 2H), 3.74 (s, 3H), 3.67 (s, 2H), 3.07 (br. s, 1H), 2.13 (br. s, 2H), 1.75 (d, *J =* 4.4 Hz, 2H).

### Intermediate 66

### (3-Oxa-8-azabicyclo[3.2.1]octan-1-yl)methanol hydrochloride

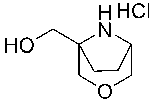

1-(Hydroxymethyl)-3-oxa-8-azabicyclo[3.2.1]octane-8-carboxylic acid tert-butyl ester (200 mg, 0.82 mmol, Tetrahedron Letters, 2016, 57, 599 - 602) was dissolved in dichloromethane (5 mL), a solution of hydrogen chloride in ethyl acetate (4.0 M, 50 mL) was added under argon protection, and the mixture was stirred at room temperature for 3 hours. The reaction solution was concentrated to obtain the crude title compound (140 mg).

LC-MS: *m*/*z* [M+H]⁺ = 144.

### (8-(2, 6-Dichloropyrimidin-4-yl)-3-oxa-8-azabicyclo[3.2.1]oct-1-yl)methanol

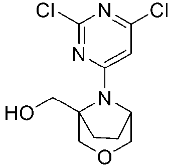

(3-Oxa-8-azabicyclo[3.2.1]octan-1-yl)methanol hydrochloride (140 mg, 0.98 mmol) was dissolved in acetonitrile (10 mL), and sodium carbonate (311 mg, 2.94 mmol) was added under stirring. The reaction solution was cooled to below 0°C, 2,4,6-trichloropyrimidine (537 mg, 2.94 mmol) was added dropwise, and the mixture was stirred at 50°C overnight. The reaction solution was cooled to room temperature and filtered through celite. The filtrate was concentrated and purified by silica gel column chromatography to obtain the title compound (190 mg, 67%).

LC-MS: *m*/*z* [M+H]⁺ = 290.

### 7-Chloro-3,4-dihydro-1H,9H,1H-4,11a-ethanopyrimido[6',1':2,3]imidazo[5,1-c][1,4]oxazin-9-one

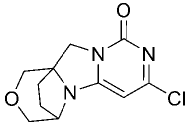

(8-(2,6-Dichloropyrimidin-4-yl)-3-oxa-8-azabicyclo[3.2.1]oct-1-yl)methanol (110 mg, 0.59 mmol) was dissolved in dichloromethane (2 mL), thionyl chloride (209 mg, 1.76 mmol) was added dropwise under stirring, and the mixture was stirred at room temperature for 3 hours. The reaction solution was concentrated, acetonitrile (10 mL) and potassium carbonate (243 mg, 1.76 mmol) were sequentially added to the residue, and the mixture was stirred at 85°C overnight. The reaction solution was cooled to room temperature and filtered through celite. The filtrate was concentrated and purified by silica gel column chromatography to obtain the title compound (85 mg, 57 %).

LC-MS: *m*/*z* [M+H]⁺ = 254.

¹H NMR (400 MHz, CDCl₃) δ5.69 (s, 1H), 4.27 (d, *J =* 12.2 Hz, 1H), 4.05 (d, *J =* 6.4 Hz, 1H), 3.95 (d, *J* = 10.3 Hz, 1H), 3.75 (br. s, 4H), 2.41 - 2.26 (m, 1H), 2.20 - 2.05 (m, 1H), 1.94 (td, *J* = 6.2, 12.0 Hz, 1H), 1.82 - 1.68 (m, 1H).

### Intermediate 67

### (1R,4S)-1-(hydroxymethyl)-2-azabicyclo[2.2.1]heptane-2-carboxylic acid benzyl ester

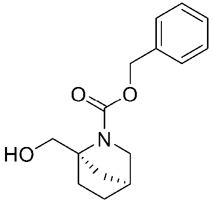

2-Benzyl-1-methyl(1R,4S)-2-azabicyclo[2.2.1]heptane-1,2-dicarboxylate (300 mg, 1.04 mmol, Tetrahedron, 2009, 1433-1436 ) was added to a mixed solvent of anhydrous tetrahydrofuran (10 mL) and methanol (1 mL), sodium borohydride (117.8 mg, 3.11 mmol) was slowly added, and the mixture was stirred at room temperature for 2 hours. Lithium borohydride (67.74 mg, 3.11 mmol) was added and stirred at room temperature overnight. The reaction solution was quenched with saturated aqueous ammonium chloride solution and extracted with ethyl acetate. The organic phase was concentrated and purified by silica gel column chromatography (petroleum ether/ethyl acetate = 7/1~1/1) to obtain the title compound (230 mg, 85%).

LC-MS: *m*/*z* [M+H]⁺ = 262.

### ((1R, 4S)-2-azabicyclo[2.2.1]hept-1-yl)methanol

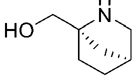

(1R,4S)-1-(hydroxymethyl)-2-azabicyclo[2.2.1]heptane-2-carboxylic acid benzyl ester (230 mg, 0.88 mmol) and Pd/C (10%, 25 mg) were added to methanol (1 mL), and hydrogenation was performed at room temperature under atmospheric pressure for 2 hours. The reaction solution was filtered, and the filtrate was concentrated to obtain the crude title compound (230 mg).

LC-MS: *m*/*z* [M+H]⁺ = 128.

### ((1R,4S)-2-(2, 6-dichloropyrimidin-4-yl)-2-azabicyclo[2.2.1lhept-1-yl)methanol

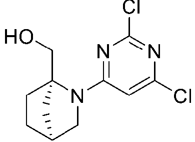

((1*R*,4*S*)-2-azabicyclo[2.2.1]hept-1-yl)methanol (125 mg, crude) was added to acetonitrile (10 mL) and cooled to 0°C under stirring, and triethylamine (197.96 mg, 1.96 mmol) and 2,4,6-trichloropyrimidine (215.7 mg, 1.18 mmol) were added to the reaction solution in sequence, and stirred at room temperature overnight. The reaction solution was concentrated, and separated by preparative thin layer chromatography (petroleum ether/ethyl acetate = 2/1) to give the title compound (140 mg, 58% yield over two steps).

LC-MS: *m*/*z* [M+H]⁺ = 274.

### (7S,9aR)-3-chloro-6,7,8,9-tetrahydro-1H,10H-7,9a-methanopyrido[1,2':3,4]imidazo[1,2-clpyrimidin-1-one

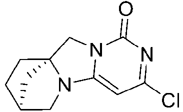

((1*R*,4*S*)-2-(2,6-dichloropyrimidin-4-yl)-2-azabicyclo[2.2.1]hept-1-yl)methanol (140 mg, 0.51 mmol) was added in dichloromethane (5 mL) and cooled to 0°C. Thionyl chloride (303.77 mg, 0.19 mL, 2.0 mmol) was added dropwise, and the mixture was stirred at 0°C for 1 hour. The reaction solution was concentrated, potassium carbonate (422.28 mg, 3.06 mmol) and acetonitrile (10 mL) were sequentially added to the residue, and the mixture was refluxed and stirred overnight. The reaction solution was quenched by adding water, extracted with dichloromethane, the organic phase was concentrated, and separated by preparative thin layer chromatography (dichloromethane/methanol = 25/1) to obtain the title compound (110 mg, 91 %).

LC-MS: *m*/*z* [M+H]⁺ = 238.

¹H NMR (400 MHz, CDCl₃) δ5.54 (s, 1H), 4.37 (d, *J =* 12.7 Hz, 1H), 3.92 (d, *J* = 12.7 Hz, 1H), 3.21 (s, 2H), 2.77 (br. s, 1H), 2.04 - 1.86 (m, 3H), 1.69 - 1.54 (m, 3H).

### Intermediate 68

### (2-Benzyl-4-fluoro-2-azabicyclo[2.1.1]hex-1-yl)methanol

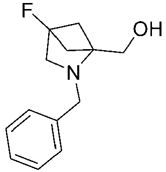

2-Benzoyl-4-fluoro-2-azabicyclo[2.1.1]hexane-1-carboxylic acid methyl ester (880 mg, 3.35 mmol, Journal of Organic Chemistry, 2017, 8831-8841) was dissolved in tetrahydrofuran (10 mL) and cooled to 0°C, lithium aluminum hydride (381 mg, 10.04 mmol) was added in batches; after the addition was completed, the mixture was stirred at 50°C for 3 hours. The reaction solution was quenched by adding sodium sulfate decahydrate. After filtration, the filtrate was concentrated, and purified by silica gel column chromatography to obtain the title compound (370 mg, 50%).

LC-MS: *m*/*z* [M+H]⁺ = 222.

### (4-Fluoro-2-azabicyclo[2.1.1]hex-1-yl)methanol

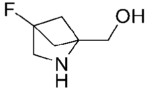

(2-Benzyl-4-fluoro-2-azabicyclo[2.1.1]hex-1-yl)methanol (370 mg, 1.67 mmol) was dissolved in methanol (100 mL), Pd/C (10 %, 74 mg) was added, and hydrogenation was performed at 50°C under atmospheric pressure overnight. The reaction solution was filtered, and the filtrate was concentrated to obtain the crude title compound (220 mg, 100%).

LC-MS: *m*/*z* [M+H]⁺ = 132.

### (2-(2,6-Dichloropyrimidin-4-yl)-4-fluoro-2-azabicyclo[2.1.1]hex-1-yl)methanol

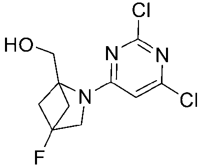

(4-Fluoro-2-azabicyclo[2.1.1]hex-1-yl)methanol (210 mg, 1.59 mmol), 4,6-dichloro-2-methoxypyrimidine (427 mg, 2.39 mmol) and solid sodium carbonate (674 mg, 6.36 mmol) were added to isopropanol (10 mL), and stirred at 85°C for 3 days. The reaction solution was filtered, the filtrate was concentrated, and purified by silica gel column chromatography to obtain the title compound (432 mg, 99%).

LC-MS: *m*/*z* [M+H]⁺ = 275.

### 3-Chloro-7-fluoro-7,8-dihydro-1H,6H,9H-7,8a-methanopyrrolo[1',2':3,4]imidazo[1.2-clpyrimidin-1-one

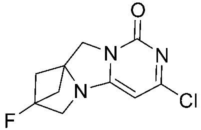

(2-(2,6-Dichloropyrimidin-4-yl)-4-fluoro-2-azabicyclo[2.1.1]hex-1-yl)methanol (332 mg, 1.21 mmol) was added to dichloromethane (5 mL), thionyl chloride (288 mg, 2.42 mmol) was added dropwise under stirring, and the mixture was stirred at room temperature for 1 hour. The reaction solution was concentrated, the residue was added to water, adjusted to pH=14 with a 15% sodium hydroxide aqueous solution under stirring, and stirred at room temperature for 10 minutes. After extraction with dichloromethane, the organic phase was concentrated, and purified by silica gel column chromatography to obtain the title compound (213 mg, 73 %).

LC-MS: *m*/*z* [M+H]⁺ = 242.

### Intermediate 69

### (5-Chloromethyl-2-oxo-3-oxabicyclo[3.1.1]heptan-1-yl)carbamic acid tert-butyl ester

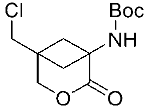

5-Chloromethyl-2-oxo-3-oxabicyclo[3.1.1]heptane-1-carboxylic acid (4.0 g, 19.5 mmol, Tetrahedron Letters, 2014, 466-468) was added to toluene (60 mL) ), triethylamine (3.9 g, 39.0 mmol) and diphenylphosphoryl azide (8.0 g, 29.2 mmol) were sequentially added under stirring, and the mixture was stirred at room temperature for 2 hours. The reaction solution was concentrated and purified by silica gel column chromatography (petroleum ether/ethyl acetate = 10/1) to obtain a yellow oil (3.8 g). The oily substance was added to tert-butanol (80 mL), di-tert-butyl dicarbonate (21.9 g, 97.5 mmol) was added under stirring, and the mixture was stirred at 90°C overnight. The reaction solution was concentrated and purified by column chromatography (petroleum ether/ethyl acetate = 10/1) to obtain the title compound (2.8 g, 52%).

LC-MS: m/z [M+H-Boc]⁺ = 176.

### 2-(tert-Butoxycarbonyl)-4-hydroxymethyl-2-azabicyclo[2.1.1]hexane-1-carboxylic acid

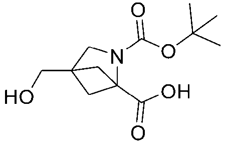

(5-Chloromethyl-2-oxo-3-oxabicyclo[3.1.1]hept-1-yl)carbamic acid tert-butyl ester (4.5 g, 16.3 mmol) was added to dichloromethane (20 mL), a solution of hydrogen chloride in ethyl acetate (4.0 M, 40 mL) was added under stirring, and the mixture was stirred at room temperature for 1.5 hours. The reaction solution was concentrated, sodium hydroxide aqueous solution (0.6 M, 140 mL) was added to the residue, and the mixture was stirred at 95°C for 0.5 hour. The reaction solution was cooled to room temperature and adjusted to pH=7 with hydrochloric acid (6.0 M). The reaction solution was concentrated, tetrahydrofuran (40 mL) and sodium hydroxide aqueous solution (5%, 35 mL) were added to the residue, di-tert-butyl dicarbonate (10.6 g, 48.9 mmol) was added under stirring, and the mixture was stirred at room temperature overnight. The reaction solution was concentrated, the residue was poured into water, the pH of the reaction solution was adjusted to 2 with concentrated hydrochloric acid; after extraction with ethyl acetate, the organic phase was concentrated, and purified by silica gel column chromatography (dichloromethane/methanol=50/1~10/1) to obtain the title compound (1.6 g, 38 %).

LC-MS: m/z [M+H-Boc]⁺ = 158.

### 2-tert-Butoxycarbonyl-4-hydroxymethyl-2-azabicyclo[2.1.1]hexane-1-carboxylic acid methyl ester

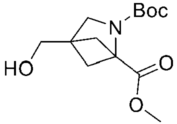

2-(tert-Butoxycarbonyl)-4-hydroxymethyl-2-azabicyclo[2.1.1]hexane-1-carboxylic acid (1.6 g, 6.22 mmol) and solid potassium carbonate (2.57 g, 18.66 mmol) ) were added to N,N-dimethylformamide (30 mL), and stirred at room temperature for 15 minutes under argon protection. Iodomethane (2.65 g, 18.66 mmol) was added to the reaction solution, and the mixture was stirred at room temperature overnight. The reaction solution was poured into water, extracted with ethyl acetate, the organic phase was concentrated, and purified by silica gel column chromatography (dichloromethane/methanol = 50/1~10/1) to obtain the title compound (1.1 g, 65%).

LC-MS: m/z [M+H-Boc]⁺ = 172.

### 2-tert-Butoxycarbonyl-4-methoxymethyl-2-azabicyclo[2.1.1]hexane-1-carboxylic acid methyl ester

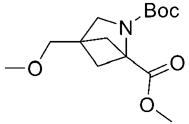

2-tert-Butoxycarbonyl-4-hydroxymethyl-2-azabicyclo[2.1.1]hexane-1-carboxylic acid methyl ester (400 mg, 1.47 mmol) was added to N,N-dimethylformamide (16 mL), sodium hydride (60%, 177 mg, 4.43 mmol) was added under stirring at room temperature, and after stirring at room temperature for 15 minutes, iodomethane (630 mg, 4.43 mmol) was added, and the mixture was stirred at room temperature for 3 hours. The reaction solution was poured into water, extracted with ethyl acetate, the organic phase was concentrated, and purified by silica gel column chromatography (petroleum ether/ethyl acetate = 10/1~3/1) to obtain the title compound (290 mg, 69%).

LC-MS: *m*/*z* [M+H-Boc]⁺ = 186.

### (4-(Methoxymethyl)-2-azabicyclo[2.1.1]hex-1-yl)methanol

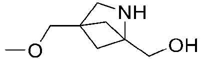

2-tert-Butoxycarbonyl-4-methoxymethyl-2-azabicyclo[2.1.1]hexane-1-carboxylic acid ester (290 mg, 1.02 mmol) was added to dichloromethane (2 mL), a solution of hydrogen chloride in ethyl acetate (4.0 M, 6 mL) was added under stirring, and the mixture was stirred at room temperature for 0.5 h. The reaction solution was concentrated, tetrahydrofuran (6 mL) and lithium aluminum hydride (76 mg, 2.0 mmol) were added to the residue, and the mixture was stirred at room temperature for 1 hour. The reaction solution was quenched by adding sodium sulfate decahydrate, stirred at room temperature for 0.5 hours, filtered, and the filtrate was concentrated to obtain the title compound (155 mg, 97%).

LC-MS: *m*/*z* [M+H]⁺ = 158.

### (2-(6-Chloro-2-methoxyprimidin-4-yl)-4-(methoxymethyl)-2-azabicyclo[2.1.1]hex-1-yl)methanol

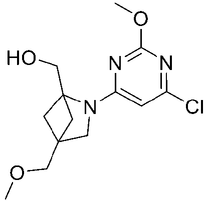

(4-(Methoxymethyl)-2-azabicyclo[2.1.1]hex-1-yl)methanol (155 mg, 1.0 mmol), 4,6-dichloro-2-methoxypyrimidine (267 mg, 1.5 mmol) and solid sodium carbonate (318 mg, 3.0 mmol) were added to isopropanol (10 mL) and stirred at reflux overnight. The reaction solution was cooled to room temperature, filtered, and the filtrate was concentrated, and separated by preparative thin layer chromatography (dichloromethane/methanol = 50/1) to give the title compound (260 mg, 87 %).

LC-MS: *m*/*z* [M+H]⁺ = 300.

### 3-Chloro-7-(methoxymethyl)-7,8-dihydro-1H,6H,9H-7,8a-methanopyrrolo[1',2':3,4]imidazo[1,2-c]pyrimidin-1-one

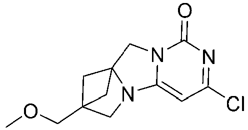

(2-(6-Chloro-2-methoxypyrimidin-4-yl)-4-(methoxymethyl)-2-azabicyclo[2.1.1]hex-1-yl)methanol (260 mg, 0.87 mmol) was added to dichloromethane (4 mL), thionyl chloride (154 mg, 1.3 mmol) was added dropwise under stirring, and the mixture was stirred at room temperature for 0.5 hours. The reaction solution was concentrated, water (4 mL) and sodium hydroxide aqueous solution (1.0 M, 4 mL) were sequentially added to the residue, and the mixture was stirred at room temperature for 0.5 hours. After extraction with dichloromethane, the combined organic phases were concentrated to give the title compound (180 mg, 78 %).

LC-MS: *m*/*z* [M+H]⁺ = 268.

### Intermediate 70

### 2-tert-Butoxycarbonyl-4-formyl-2-azabicyclo[2.1.1]hexane-1-carboxylic acid methyl ester

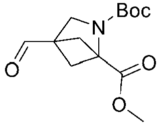

2-tert-Butoxycarbonyl-4-(hydroxymethyl)-2-azabicyclo[2.1.1]hexane-1-carboxylic acid methyl ester (400 mg, 1.47 mmol) was added to dichloromethane (20 mL), Dess-Martin reagent (1.23 g, 2.94 mmol) was added under stirring, and the mixture was stirred at room temperature overnight. After filtration, the filtrate was poured into saturated sodium bicarbonate solution, extracted with dichloromethane, the organic phase was concentrated, and purified by silica gel column chromatography (dichloromethane/methanol = 50/1) to obtain the title compound (250 mg, 63%).

LC-MS: *m*/*z* [M+H-Boc]⁺ = 170.

### 2-tert-Butoxycarbonyl-4-(difluoromethyl)-2-azabicyclo[2.1.1]hexane-1-carboxylic acid methyl ester

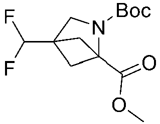

2-(tert-Butoxycarbonyl)-4-formyl-2-azabicyclo[2.1.1]hexane-1-carboxylic acid methyl ester (250 mg, 0.86 mmol) was added to dichloromethane (12 mL), cooled to 0°C, diethylaminosulfur trifluoride (741 mg, 4.6 mmol) was added, stirred at 0°C for 2 hours, and stirred at room temperature overnight. The reaction solution was poured into saturated sodium bicarbonate solution, extracted with dichloromethane, the organic phase was concentrated, and purified by silica gel column chromatography (petroleum ether/ethyl acetate = 5/1) to obtain the title compound (150 mg, 56%).

LC-MS: m/z [M+H-Boc]⁺ = 192.

### (4-(Difluoromethyl)-2-azabicyclo[2.1.1]hex-1-yl)methanol

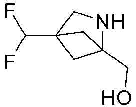

2-(tert-Butoxycarbonyl)-4-(difluoromethyl)-2-azabicyclo[2.1.1]hexane-1-carboxylic acid methyl ester (150 mg, 0.51 mmol) was added to dichloromethane (4 mL), a solution of hydrogen chloride in ethyl acetate (4.0 M, 6 mL) was added under stirring, and the mixture was stirred at room temperature for 0.5 h. The reaction solution was concentrated, tetrahydrofuran (6 mL) and lithium aluminum hydride (286 mg, 0.75 mmol) were sequentially added to the residue, and the mixture was stirred at room temperature for 1 hour. The reaction solution was quenched by adding sodium sulfate decahydrate, and stirred at room temperature for 0.5 hours. After filtration, the filtrate was concentrated to obtain the crude title compound (100 mg).

LC-MS: *m*/*z* [M+H]⁺ = 164.

### 2-(6-Chloro-2-methoxyprimidin-4-yl)-1-(difluoromethyl)-4-(methoxymethyl)-2-azabicyclo[2.1.1]hexane

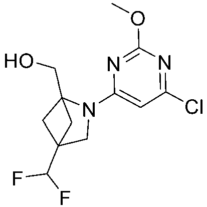

(4-(Difluoromethyl)-2-azabicyclo[2.1.1]hex-1-yl)methanol (100 mg, 0.61 mmol), 4,6-dichloro-2-methoxypyrimidine (163 mg, 0.92 mmol) and solid sodium carbonate (190 mg, 1.83 mmol) were added to isopropanol (10 mL) and stirred at reflux overnight. The reaction solution was cooled to room temperature, filtered, and the filtrate was concentrated and separated by preparative thin layer chromatography (petroleum ether/ethyl acetate = 2/1) to obtain the title compound (60 mg, two-step yield: 38%).

LC-MS: *m*/*z* [M+H]⁺ = 306.

### 3-Chloro-7-(difluoromethyl)-7,8-dihydro-1H,6H,9H-7,8a-methanopyrrolo[1',2':3,4]imidazo[1, 2-c]pyrimidin-1-one

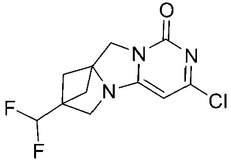

2-(6-Chloro-2-methoxypyrimidin-4-yl)-1-(difluoromethyl)-4-(methoxymethyl)-2-azabicyclo[2.1.1]hexane (60 mg, 0.20 mmol) was added to dichloromethane (4 mL), thionyl chloride (47 mg, 0.40 mmol) was added dropwise under stirring, and the mixture was stirred at room temperature for 0.5 hours. The reaction solution was concentrated, water (2 mL) and sodium hydroxide aqueous solution (1.0 M, 0.5 mL) were sequentially added to the residue, and the mixture was stirred at room temperature for 0.5 hours. The reaction solution was extracted with dichloromethane, and the organic phase was concentrated to obtain the title compound (45 mg, 82 %).

LC-MS: *m*/*z* [M+H]⁺ = 274.

### Intermediate 71

### 2-(tert-Butoxycarbonyl)-4-(((methylsulfonyl)oxy)methyl)-2-azabicyclo[2.1.1]hexane-1-carboxylic acid methyl ester

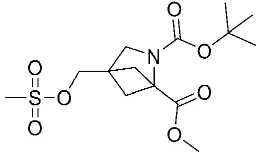

2-(tert-Butoxycarbonyl)-4-(hydroxymethyl)-2-azabicyclo[2.1.1]hexane-1-carboxylic acid methyl ester (270 mg, 1.0 mmol) and triethylamine (300 mg, 3.0 mmol) were added to dichloromethane (10 mL), cooled to 0°C, methanesulfonyl chloride (3434 mg, 3.0 mmol) was added dropwise, and the mixture was stirred at room temperature overnight. The reaction solution was poured into saturated sodium bicarbonate solution, extracted with dichloromethane, the organic phase was concentrated, and purified by preparative thin layer chromatography (dichloromethane/methanol = 20/1) to obtain the title compound (180 mg, 52 %).
LC-MS: m/z [M+H-Boc]⁺ = 250

### 2-(tert-Butoxycarbonyl)-4-(morpholinomethyl)-2-azabicyclo[2.1.1]hexane-1-carboxylic acid methyl ester

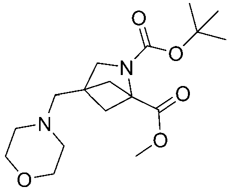

2-(tert-Butoxycarbonyl)-4-(((methylsulfonyl)oxy)methyl)-2-azabicyclo[2.1.1]hexane-1-carboxylic acid methyl ester (180 mg, 0.52 mmol), morpholine (226 mg, 2.6 mmol), solid potassium carbonate (215 mg, 1.56 mmol), potassium iodide (17 mg, 0.1 mmol) were added to acetonitrile (12 mL), and stirred at 85°C overnight. The reaction solution was cooled to room temperature, filtered, the filtrate was concentrated, and purified by thin-layer preparative chromatography (dichloromethane/methanol = 20/1) to obtain the title compound (140 mg, 80%). LC-MS: *m*/*z* [M+H]⁺ = 341

### (4-(Morpholinomethyl)-2-azabicyclo[2.1.1]hex-1-yl)methanol

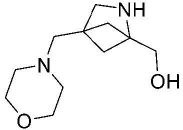

2-(tert-Butoxycarbonyl)-4-(morpholinomethyl)-2-azabicyclo[2.1.1]hexane-1-carboxylic acid methyl ester (140 mg, 0.41 mmol) was added to dichloromethane (3 mL), hydrogen chloride in ethyl acetate (4.0 M, 3 mL) was added under stirring, and the mixture was stirred at room temperature for 0.5 hours. The reaction solution was concentrated, tetrahydrofuran (6 mL) and lithium aluminum hydride (31 mg, 0.82 mmol) were sequentially added to the residue, and the mixture was stirred at room temperature for 0.5 hours. The reaction solution was quenched by adding solid sodium sulfate decahydrate, and stirred at room temperature for 0.5 hours. After filtration, the filtrate was concentrated to obtain the crude title compound (80 mg).

LC-MS: *m*/*z* [M+H]⁺ = 213.

### (2-(6-Chloro-2-methoxyprimidin-4-yl)-4-(morpholinomethyl)-2-azabicyclo[2.1.1]hex-1-yl)methanol

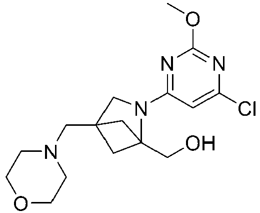

(4-(Morpholinomethyl)-2-azabicyclo[2.1.1]hex-1-yl)methanol (80 mg, 0.38 mmol), 4,6-dichloro-2-methoxypyrimidine (135 mg, 0.76 mmol) and solid sodium carbonate (120 mg, 1.14 mmol) were added to isopropanol (12 mL) and stirred at reflux overnight. The reaction solution was cooled to room temperature, filtered, and the filtrate was concentrated and separated by preparative thin layer chromatography (petroleum ether/ethyl acetate = 2/1) to obtain the title compound (80 mg, two-step yield: 55%).

LC-MS: *m*/*z* [M+H]⁺ = 355.

### 3-Chloro-7-(morpholinomethyl)-7,8-dihydro-1H,6H,9H-7,8a-methanopyrrolo[1',2':3,4limidazo[1, 2-c]pyrimidin-1-one

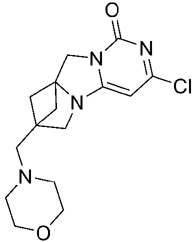

(2-(6-Chloro-2-methoxypyrimidin-4-yl)-4-(morpholinomethyl)-2-azabicyclo[2.1.1]hex-1-yl)methanol (80 mg, 0.23 mmol) was added to dichloromethane (3 mL), thionyl chloride (81 mg, 0.69 mmol) was added dropwise under stirring, and the mixture was stirred at room temperature for 0.5 hours. The reaction solution was concentrated, water (3 mL) and sodium hydroxide aqueous solution (1.0 M, 3 mL) were sequentially added to the residue, and the mixture was stirred at room temperature for 0.5 hours. After extraction with dichloromethane, the organic phase was concentrated to obtain the title compound (45 mg, 61 %).

LC-MS: *m*/*z* [M+H]⁺ = 323.

### Intermediate 72

### 2-(tert-Butoxycarbonyl)-4-((((tert-butyldimethylsilyl)oxy)methyl)-2-azabicyclo[2.1.1]hexane-1-carboxylic acid methyl ester

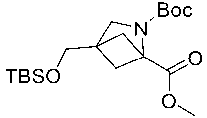

2-(tert-Butoxycarbonyl)-4-(hydroxymethyl)-2-azabicyclo[2.1.1]hexane-1-carboxylic acid methyl ester (1.0 g, 3.69 mmol) was added to dichloromethane (10 mL), imidazole (0.5 g, 7.35 mmol) and tert-butyldimethylsilyl chloride (0.66 g, 4.37 mmol) were sequentially added under stirring, and the mixture was stirred at room temperature for 2 hours. The reaction solution was poured into saturated aqueous ammonium chloride solution, extracted with ethyl acetate, the organic phase was concentrated, and purified by silica gel column chromatography (petroleum ether/ethyl acetate = 5/1) to obtain the title compound (1.2 g, 84%).

LC-MS: m/z [M+H-Boc]⁺ = 286.

### 4-((((tert-Butyldimethylsilyl)oxy)methyl)-1-(hydroxymethyl)-2-azabicyclo[2.1.1]hexane-2-carboxylic acid tert-butyl ester

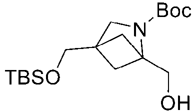

2-(tert-Butoxycarbonyl)-4-((((tert-butyldimethylsilyl)oxy)methyl)-2-azabicyclo[2.1.1]hexane-1-carboxylic acid methyl ester (1.2 g, 3.11 mmol) was added to methanol (15 mL), the temperature of the reaction solution was increased to 65°C, sodium borohydride (1.18 g, 31.16 mmol) was added in batches, and the mixture was stirred at 65°C for 0.5 hours. The reaction solution was cooled to room temperature, the reaction solution was quenched with saturated aqueous ammonium chloride solution, extracted with ethyl acetate, the organic phase was concentrated, and purified by silica gel column chromatography (petroleum ether/ethyl acetate=5/1) to obtain the title compound (1 g, 90%).

LC-MS: *m*/*z* [M+H-Boc]⁺ = 258.

### 1-(Benzyloxy)methyl)-4-((((tert-butyldimethylsilyl)oxy)methyl)-2-azabicyclo[2.1.1]hexane-2-carboxylic acid tert-butyl ester

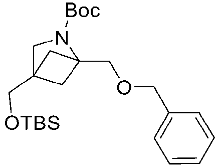

4-((((tert-Butyldimethylsilyl)oxy)methyl)-1-(hydroxymethyl)-2-azabicyclo[2.1.1]hexane-2-carboxylic acid tert-butyl ester (1.0 g, 2.80 mmol) was added to N,N-dimethylformamide (16 mL), the temperature was lowered to 0°C under argon protection, and sodium hydride (60%, 224 mg, 5.60 mmol) was added under stirring; after stirring at room temperature for 10 minutes, benzyl bromide (718 mg, 4.20 mmol) was added, and the mixture was stirred at room temperature overnight. The reaction solution was poured into water, extracted with ethyl acetate, and the organic phase was concentrated to obtain the title compound (1.2 g, 96%).

LC-MS: m/z [M+H-Boc]⁺ = 348.

### 1-((Benzyloxy)methyl)-4-(hydroxymethyl)-2-azabicyclo[2.1.1]hexane-2-carboxylic acid tert-butyl ester

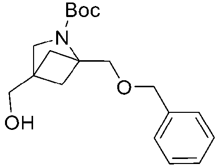

1-((Benzyloxy)methyl)-4-((((tert-butyldimethylsilyl)oxy)methyl)-2-azabicyclo[2.1.1]hexane-2-carboxylic acid tert-butyl ester (1.2 g, 2.68 mmol) was added to tetrahydrofuran (10 mL), a solution of tetrabutylammonium fluoride in tetrahydrofuran (1.0 M, 4 mL) was added under stirring, and stirred at room temperature for 2 hours. The reaction solution was poured into saturated aqueous ammonium chloride solution, extracted with ethyl acetate, the organic phase was concentrated, and purified by silica gel column chromatography (dichloromethane/methanol=20/1) to obtain the title compound (600 mg, 67%).

LC-MS: *m*/*z* [M+H-Boc]⁺ = 234.

### 1-((Benzyloxy)methyl)-2-(tert-butoxycarbonyl)-2-azabicyclo[2.1.1]hexane-4-carboxylic acid

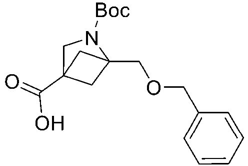

1-((Benzyloxy)methyl)-4-(hydroxymethyl)-2-azabicyclo[2.1.1]hexane-2-carboxylic acid tert-butyl ester (600 mg, 1.80 mmol) was dissolved in acetone (6 mL), slowly dropped into Jones reagent (2.0 M, 2 mL), and stirred at room temperature for 0.5 h. Isopropanol (6 mL) was added to the reaction solution, stirred at room temperature for 10 minutes, filtered, and the filtrate was concentrated. The residue was dissolved in water, the pH was adjusted to weakly alkaline with saturated sodium bicarbonate solution, and then adjusted to pH=2 with hydrochloric acid (6.0 M), extracted with ethyl acetate, the organic phase was concentrated, and purified by preparative thin layer chromatography to obtain the crude title compound (400 mg, 64%).

LC-MS: m/z [M+H-Boc]⁺ = 248.

### 2-(tert-Butoxycarbonyl)-1-((benzyloxy)methyl)-2-azabicyclo[2.1.1]hexane-4-carboxylic acid methyl ester

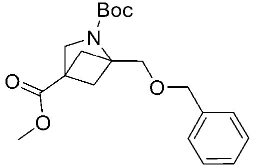

1-((Benzyloxy)methyl)-2-(tert-butoxycarbonyl)-2-azabicyclo[2.1.1]hexane-4-carboxylic acid (400 mg, 1.15 mmol) and solid potassium carbonate (476 mg, 3.45 mmol) were added to N,N-dimethylformamide (5 mL). After stirring at room temperature for 10 minutes, iodomethane (326 mg, 2.30 mmol) was added, and stirred at room temperature overnight. The reaction solution was poured into water, extracted with ethyl acetate, and the organic phase was concentrated, and purified by thin layer preparative chromatography (petroleum ether/ethyl acetate = 5/1) to obtain the title compound (230 mg, 56%).

LC-MS: m/z [M+H-Boc]⁺ = 262.

### 2-(tert-Butoxycarbonyl)-1-(hydroxymethyl)-2-azabicyclo[2.1.1]hexane-4-carboxylic acid methyl ester

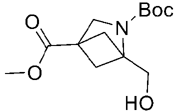

2-(tert-Butoxycarbonyl)-1-((benzyloxy)methyl)-2-azabicyclo[2.1.1]hexane-4-carboxylic acid methyl ester (230 mg, 0.64 mmol) and Pd /C (10%, 25 mg) were added to methanol (10 mL), and hydrogenation was performed at room temperature under atmospheric pressure overnight. After filtration, the filtrate was concentrated to give the title compound (150 mg, 96%). LC-MS: m/z [M+H-Boc]⁺ = 172.

### 2-(2,6-Dichloropyrimidin-4-yl)-1-(hydroxymethyl)-2-azabicyclo[2.1.1]hexane-4-carboxylic acid methyl ester

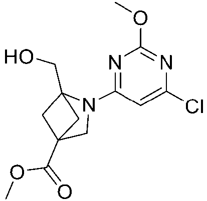

2-(tert-Butoxycarbonyl)-1-(hydroxymethyl)-2-azabicyclo[2.1.1]hexane-4-carboxylic acid methyl ester (80 mg, 0.29 mmol) was added to a solution of hydrogen chloride in dioxane (4.0 M, 6 mL), and stirred at 50°C for 30 min. The reaction solution was concentrated, and 4,6-dichloro-2-methoxypyrimidine (103 mg, 0.58 mmol), solid sodium carbonate (92 mg, 0.87 mmol) and acetonitrile (6 mL) were sequentially added to the residue, stirred at 85°C overnight. The reaction solution was cooled to room temperature and filtered. The filtrate was concentrated and purified by preparative thin layer chromatography (dichloromethane/methanol = 30/1) to give the title compound (60 mg, 66%).

LC-MS: *m*/*z* [M+H]⁺ = 314.

### 3-Chloro-1-oxo-1H,6H,9H-7,8a-methanopyrrolo[1',2':3,4]imidazo[1,2-c]pyrimidine-7(8H)-carboxylic acid methyl ester

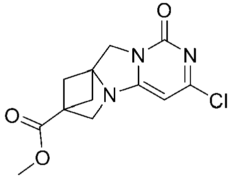

2-(2,6-Dichloropyrimidin-4-yl)-1-(hydroxymethyl)-2-azabicyclo[2.1.1]hexane-4-carboxylicacid methyl ester (60 mg, 0.87 mmol) was added to dichloromethane (3 mL), then thionyl chloride (154 mg, 1.3 mmol) was added dropwise, and the mixture was stirred at room temperature for 0.5 hours. The reaction solution was concentrated. Water (4 mL) and sodium hydroxide aqueous solution (1.0 M, 2 mL) were sequentially added to the residue, and the mixture was stirred at room temperature for 0.5 hours. After extraction with dichloromethane, the organic phase was concentrated to give the title compound (30 mg, 56%).

LC-MS: *m*/*z* [M+H]⁺ = 282.

### 3-((3-Fluoro-4-((2-(trifluoromethyl)pyridin-4-yl)oxy)benzyl)oxy)-1-oxo-1H,6H,9H-7, 8a-methanopyrrolo[1',2':3,4]imidazo[1,2-c]pyrimidine-7(8H)-carboxylic acid methyl ester

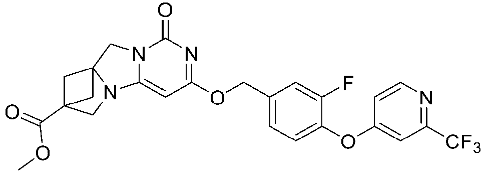

3-Chloro-1-oxo-1H,6H,9H-7,8a-methanopyrolo[1',2':3,4]imidazo[1,2-c]pyrimidine-7(8H)-carboxylic acid methyl ester (30 mg, 0.106 mmol), (3-fluoro-4-((2-(trifluoromethyl)pyridin-4-yl)oxy)phenyl)methanol (45 mg, 0.156 mmol) and cesium carbonate (103 mg, 0.318 mmol) were added to toluene (6 mL), and the mixture was stirred at 130°C for 4 hours. The reaction solution was cooled to room temperature, filtered, and the filtrate was concentrated, and purified by preparative thin layer chromatography (dichloromethane/methanol = 20/1) to obtain the title compound (20 mg, 35 %).

LC-MS: *m*/*z* [M+H]⁺ = 533.

### 3-((3-Fluoro-4-((2-(trifluoromethyl)pyridin-4-yl)oxy)benzyl)oxy)-1-oxo-1H,6H,9H-7, 8a-methanopyrrolo[1', 2 ':3, 4]imidazo[1, 2-clpyrimidine-7(8H)-carboxylic acid

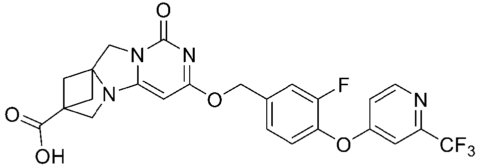

3-((3-Fluoro-4-((2-(trifluoromethyl)pyridin-4-yl)oxy)benzyl)oxy)-1-oxo-1H,6H,9H-7,8a-methanopyrrolo[1',2':3,4]imidazo[1,2-c]pyrimidine-7(8H)-carboxylic acid methyl ester (1.1 g, 2.07 mmol) was added into acetonitrile (10 mL) and sodium hydroxide aqueous solution (1.0 M, 20 mL), and stirred at room temperature for 1 hour. The pH of the reaction solution was adjusted to 3-4 with aqueous hydrochloric acid (1.0 M). After extraction with dichloromethane, the organic phase was concentrated to obtain the title compound (1.07 g, 100%).

LC-MS: *m*/*z* [M+H]⁺ = 519.

### (3-((3-Fluoro-4-((2-(trifluoromethyl)pyridin-4-yl)oxy)benzyl)oxy)-1-oxo-1H,6H,9H-7, 8a-methanopyrrolo[1',2':3,4]imidazo[1,2-c]pyrimidine-7(8H)-yl)carbamic acid tert-butyl ester

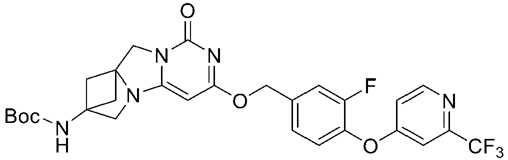

3-((3-Fluoro-4-((2-(trifluoromethyl)pyridin-4-yl)oxy)benzyl)oxy)-1-oxo-1H,6H,9H-7,8a-methanopyrrolo[1',2':3,4]imidazo[1,2-c]pyrimidine-7(8H)-carboxylic acid (1.07 g, 2.07 mmol) and triethylamine (418 mg, 4.14 mmol) were added to dichloromethane (20 mL) and cooled to 0°C, diphenylphosphoryl azide (628 mg, 2.28 mmol) was added, and the mixture was stirred at room temperature for 4 hours. The reaction solution was concentrated, di-tert-butyl dicarbonate (1.35 g, 6.21 mmol) and tert-butanol (20 mL) were sequentially added to the residue, and the mixture was stirred at 90°C overnight. The reaction solution was concentrated and purified by column chromatography (dichloromethane/methanol=200/1~50/1) to obtain the title compound (350 mg, 29%).

LC-MS: *m*/*z* [M+H]⁺ = 590.

### Intermediate 73

### 4-Aminoformyl-1-(hydroxymethyl)-2-azabicyclo[2.1.1]hexane-2-carboxylic acid tert-butyl ester

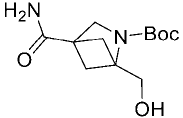

4-Methoxycarbonyl-1-(hydroxymethyl)-2-azabicyclo[2.1.1]hexane-2-carboxylic acid tert-butyl ester (200 mg, 0.74 mmol) was added to methanol (3 mL) and ammonia (6 mL), stirred at room temperature for 3 hours. The reaction solution was concentrated to obtain the title compound (120 mg, 63%).

LC-MS: m/z [M+H-Boc]⁺ = 157.

### 4-Cyano-1-(hydroxymethyl)-2-azabicyclo[2.1.1]hexane-2-carboxylic acid tert-butyl ester

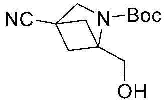

4-Aminoformyl-1-(hydroxymethyl)-2-azabicyclo[2.1.1]hexane-2-carboxylic acid tert-butyl ester (220 mg, 0.86 mmol) was added to dichloromethane (16 mL), triethylamine (434 mg, 4.30 mmol) and trifluoroacetic anhydride (722 mg, 3.44 mmol) were sequentially added under stirring, and the mixture was stirred at room temperature for 1 hour. The reaction solution was poured into ethyl acetate, the organic phase was washed successively with saturated ammonium chloride solution and saturated sodium carbonate solution, the organic phase was concentrated, and purified by preparative thin layer chromatography (petroleum ether/ethyl acetate=5/1) to obtain the title compound (100 mg, 49%).

LC-MS: *m*/*z* [M+H-Boc]⁺ = 139.

### 2-(6-Chloro-2-methoxyprimidin-4-yl)-1-(hydroxymethyl)-2-azabicyclo[2.1.1]hexane-4-carbonitrile

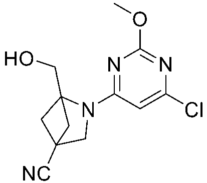

4-Cyano-1-(hydroxymethyl)-2-azabicyclo[2.1.1]hexane-2-carboxylic acid tert-butyl ester (100 mg, 0.42 mmol) was added to dichloromethane (4 mL), hydrochloric acid/dioxane (4.0 M, 3 mL) was added under stirring, and the mixture was stirred at 50°C for 30 minutes. The reaction solution was concentrated, and 4,6-dichloro-2-methoxypyrimidine (146 mg, 0.82 mmol), solid sodium carbonate (133 mg, 1.26 mmol) and acetonitrile (8 mL) were sequentially added to the residue, and stirred at 90°C overnight. The reaction solution was cooled to room temperature, filtered, and the filtrate was concentrated and purified by preparative thin layer chromatography (petroleum ether/ethyl acetate = 4/1) to obtain the title compound (20 mg, 17%).

LC-MS: *m*/*z* [M+H]⁺ = 281.

### 3-Chloro-1-oxo-1H,6H,9H-7,8a-methanopyrrolo[1',2':3,4]imidazo[1,2-c]pyrimidine-7(8H)-carbonitrile

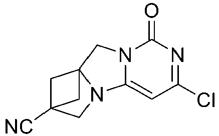

2-(6-Chloro-2-methoxypyrimidin-4-yl)-1-(hydroxymethyl)-2-azabicyclo[2.1.1]hexane-4-carbonitrile (20 mg, 0.07 mmol ) was added to dichloromethane (2 mL), thionyl chloride (42 mg, 0.35 mmol) was added under stirring, and the mixture was stirred at room temperature for 0.5 hours. The reaction solution was concentrated, the residue was added to water, adjusted to pH=10 with solid potassium carbonate, and stirred at room temperature for 0.5 hours. The reaction solution was extracted with dichloromethane, and the organic phase was concentrated to obtain the title compound (12 mg, 69 %).

LC-MS: *m*/*z* [M+H]⁺ = 249.

### Intermediate 74

### 4-(Benzylcarbamoyl)-1-((benzyloxy)methyl)-2-azabicyclo[2.1.1]hexane-2-carboxylic acid tert-butyl ester

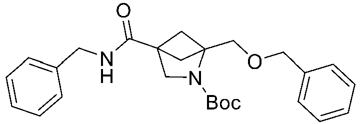

1-((Benzyloxy)methyl)-2-(tert-butoxycarbonyl)-2-azabicyclo[2.1.1]hexane-4-carboxylic acid (750 mg, 2.16 mol), benzylamine (347.4 mg, 3.24 mmol), HATU (1.64 g, 4.32 mmol) and triethylamine (873 mg, 8.64 mmol) were added to dichloromethane (20 mL) and stirred at room temperature for 2 hours. The reaction solution was concentrated and purified by column chromatography (dichloromethane/methanol=20/1) to obtain the title compound (800 mg, 85%). LC-MS: *m*/*z* [M+H]⁺ = 437.

### 4-(Benzylcarbamoyl)-1-(hydroxymethyl)-2-azabicyclo[2.1.1]hexane-2-carboxylic acid tert-butyl ester

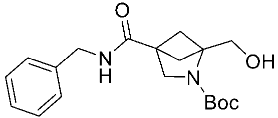

4-(Benzylcarbamoyl)-1-((benzyloxy)methyl)-2-azabicyclo[2.1.1]hexane-2-carboxylic acid tert-butyl ester (800 mg, 1.83 mmol) and Pd/C (10 %, 80 mg) were added to methanol (10 mL), and the reaction solution underwent hydrogenation at 60°C under normal pressure overnight. The reaction solution was filtered, and the filtrate was concentrated to obtain the crude title compound (480 mg).

LC-MS: m/z [M+H-tBu]⁺ = 291.

### N-Benzyl-2-(2,6-dichloropyrimidin-4-yl)-1-(hydroxymethyl)-2-azabicyclo[2.1.1]hexane-4-carboxamide

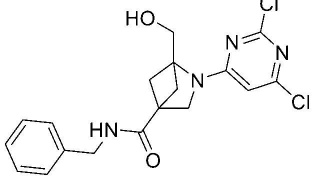

4-(Benzylcarbamoyl)-1-(hydroxymethyl)-2-azabicyclo[2.1.1]hexane-2-carboxylic acid tert-butyl ester (480 mg, 1.39 mmol) was added to hydrochloric acid/dioxane (4.0 M, 5 mL) and stirred at room temperature for 1 hour. The reaction solution was concentrated, 2,4,6-trichloropyrimidine (254.5 mg, 1.39 mmol), solid sodium carbonate (294.7 mg, 2.78 mmol) and acetonitrile (10 mL) were sequentially added to the residue, and the mixture was stirred at room temperature for 2 hours. The reaction solution was poured into water, extracted with ethyl acetate, the organic phase was concentrated, and purified by column chromatography (petroleum ether/ethyl acetate = 1/1) to obtain the title compound (150 mg, two-step yield: 27%).

LC-MS: *m*/*z* [M+H]⁺ = 393.

### N-Benzyl-3-chloro-1-oxo-1H,6H,9H-7,8a-methanopyrrolo[1',2':3,4]imidazo[1,2-c]pyrimidine-7(8H)-carboxamide

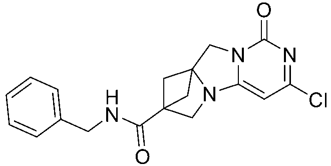

N-Benzyl-2-(2,6-dichloropyrimidin-4-yl)-1-(hydroxymethyl)-2-azabicyclo[2.1.1]hexane-4-carboxamide (150 mg , 0.38 mmol) was added to dichloromethane (6 mL), thionyl chloride (2 mL) was added dropwise under stirring, and the mixture was stirred at room temperature for 30 minutes. The reaction solution was concentrated, potassium carbonate (263.4 mg, 1.91 mmol) and acetonitrile (15 mL) were sequentially added to the residue, and the mixture was stirred under reflux overnight. The reaction solution was poured into water, extracted with dichloromethane, the organic phase was concentrated, and purified by column chromatography (dichloromethane/methanol = 20/1) to obtain the title compound (25 mg, 18%).

LC-MS: *m*/*z* [M+H]⁺ = 357.

### Intermediate 75

### 1-(tert-Butoxycarbonyl)- (4R)-4-((trimethylsilyl)oxy)pyrrolidine-2-carboxylic acid methyl ester

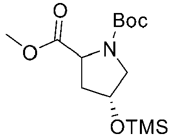

1-(tert-Butoxycarbonyl)-(4R)-4-hydroxypyrrolidine-2-dicarboxylic acid methyl ester (5 g, 20.39 mmol) and triethylamine (2.88 g, 28.55 mmol) were added to dichloromethane (50 mL). The temperature was lowered to 0°C under argon protection, trimethylchlorosilane (2.88 g, 26.51 mmol) was added dropwise, and the mixture was stirred at room temperature overnight. The reaction solution was poured into dichloromethane, and the organic phase was washed successively with water and saturated sodium bicarbonate solution. The organic phase was concentrated to give the title compound (6.34 g, 98%).

LC-MS: m/z [M+H-tBu]⁺ = 262.

### 1-(tert-Butoxycarbonyl)-(4R)-2-((benzyloxy)methyl)-4-((trimethylsilyl)oxy)pyrrolidine-2-carboxylic acid methyl ester

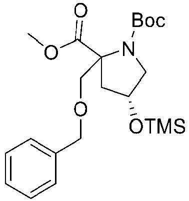

1-(tert-Butoxycarbonyl)-(4*R*)-4-((trimethylsilyl)oxy)pyrrolidine-2-carboxylic acid methyl ester (6.5 g, 20.5 mmol) and benzyl chloromethyl ether (6.4 g, 41 mmol) was dissolved in tetrahydrofuran (100 mL). Under argon protection, the temperature was lowered to 0°C and a solution of lithium diisopropylamide in tetrahydrofuran (2.0 M, 20 mL, 40 mmol) was added dropwise. After stirring at room temperature for 3 hours, the reaction solution was quenched with methanol, poured into water, and extracted with ethyl acetate. The organic phase was concentrated and purified by silica gel column chromatography to give the title compound (3.6 g, 40%). LC-MS: *m*/*z* [M+H]⁺ = 438.

### 1-(tert-Butoxycarbonyl)-(4R)-2-((benzyloxy)methyl)-4-hydroxypyrrolidine-2-carboxylic acid methyl ester

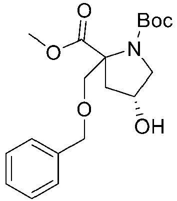

1-(tert-Butoxycarbonyl)-(4*R*)-2-((benzyloxy)methyl)-4-((trimethylsilyl)oxy)pyrrolidine-2-carboxylic acid methyl ester (3 g, 6.86 mmol) was added to a solution of citric acid in methanol (10%, 10 mL), and stirred at room temperature for 3 hours. The reaction solution was filtered, and the filtrate was concentrated to obtain the title compound (2.3 g, 89%).

LC-MS: *m*/*z* [M+H]⁺ = 366.

### 1-(tert-Butoxycarbonyl)-(4R)-2-((benzyloxy)methyl)-4-((methylsulfonyl)oxy)pyrrolidine-2-carboxylic acid methyl ester

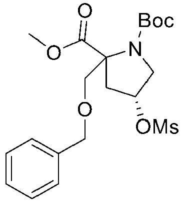

1-(tert-Butoxycarbonyl)-(4*R*)-2-((benzyloxy)methyl)-4-hydroxypyrrolidine-2-carboxylic acid methyl ester (2.3 g, 6.3 mmol) and triethylamine (1.28 g, 12.6 mmol) were added to dichloromethane (15 mL) in sequence. The reaction solution was cooled to 0°C, methanesulfonic anhydride (2.2 g, 10.1 mmol) was dissolved in dichloromethane (15 mL) and dropped into the reaction solution, and the mixture was stirred at room temperature overnight. The reaction solution was poured into saturated sodium bicarbonate solution and extracted with dichloromethane. The organic phase was concentrated to give the title compound (2.6 g, 100%).

LC-MS: *m*/*z* [M+H]⁺ = 444.

### 1-(tert-Butoxycarbonyl)-(4S)-4-(benzylamino)-2-((benzyloxy)methyl)pyrrolidine-2-carboxylic acid methyl ester

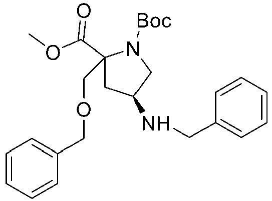

1-(tert-Butoxycarbonyl)-(4*R*)-2-((benzyloxy)methyl)-4-((methylsulfonyl)oxy)pyrrolidine-2-carboxylic acid methyl ester (2.6 g, 5.87 mmol) was dissolved in benzylamine (5 mL) and stirred at 85°C overnight, and purified by silica gel column chromatography to obtain the title compound (2.32 g, 87%).

LC-MS: *m*/*z* [M+H-Boc]⁺ = 355.

### (1R,4S)-5-Benzyl-1-((benzyloxy)methyl)-6-oxo-2,5-diazabicyclo[2.2.1]heptane-2-carboxylic acid tert-butyl ester

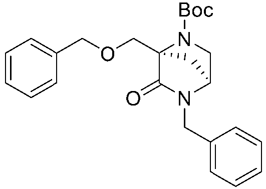

1-(tert-Butoxycarbonyl)-(4*S*)-4-(benzylamino)-2-((benzyloxy)methyl)pyrrolidine-2-carboxylic acid methyl ester (519 mg, 114 mmol) and lithium hydroxide (54.8 mg, 2.28 mmol) were added to water (2 mL) and methanol (10 mL), and the mixture was stirred at 30°C for 2 days. The reaction solution was concentrated, HATU (865 mg, 2.28 mmol), triethylamine (460 mg, 4.56 mmol) and N,N-dimethylformamide (35 mL) were sequentially added to the residue, and the mixture was stirred at room temperature overnight. The reaction solution was poured into ethyl acetate, washed successively with water and saturated brine, and dried over anhydrous sodium sulfate. The organic phase was concentrated, and purified by silica gel column chromatography (petroleum ether/ethyl acetate = 20/1) to obtain the title compound (150 mg, 31 %).

LC-MS: m/z [M+H-Boc]⁺ = 323.

### (1S,4R)-2-Benzyl-4-(hydroxymethyl)-2, 5-diazabicyclo[2.2.1]heptan-3-one

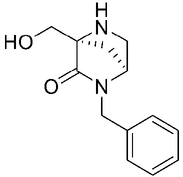

(1R,4S)-5-Benzyl-1-((benzyloxy)methyl)-6-oxo-2,5-diazabicyclo[2.2.1]heptane-2-carboxylic acid tert-butyl ester (150 mg, 0.36 mmol) and Pd/C (10 %, 20 mg) were added to methanol (10 mL), and underwent hydrogenation at 40°C under atmospheric pressure overnight. The reaction solution was filtered and concentrated. The residue was added with a solution of hydrogen chloride in dioxane (4.0 M, 5 mL) and stirred at room temperature for 2 hours. The reaction solution was concentrated to obtain the crude title compound, which was directly used in the next step.

LC-MS: *m*/*z* [M+H]⁺ = 233.

### (1S,4R)-2-Benzyl-5-(2,6-dichloropyrimidin-4-yl)-4-(hydroxymethyl)-2,5-diazabicyclo [2.2.1]heptan-3-one

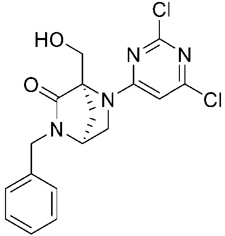

(1*S*,4*R*)-2-Benzyl-4-(hydroxymethyl)-2,5-diazabicyclo[2.2.1]heptan-3-one (crude) was dissolved in acetonitrile (10 mL), cooled to 0°C. Triethylamine (71 mg, 0.71 mmol) and 2,4,6-trichloropyrimidine (98 mg, 0.53 mmol) were added sequentially, and the mixture was stirred at room temperature overnight. The reaction solution was concentrated, and separately by preparative thin layer chromatography (petroleum ether/ethyl acetate = 2/1) to obtain the title compound (40 mg, 30% yield for two steps).

LC-MS: *m*/*z* [M+H]⁺ = 379.

### (3S,11aR)-2-Benzyl-7-chloro-3,4-dihydro-9H,11H-3,11a-methanopyrazino[1',2':3,4]imidazo[1,2-c]pyrimidine-1,9(2H)-dione

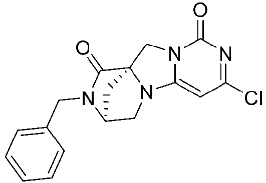

(1*S*,4*R*)-2-Benzyl-5-(2,6-dichloropyrimidin-4-yl)-4-(hydroxymethyl)-2,5-diazabicyclo[2.2.1]heptane-3-one (40 mg, 0.11 mmol) was added to dichloromethane (5 mL). After cooling to 0°C, thionyl chloride (0.5 mL) was added dropwise, and the mixture was stirred at 0°C for 0.5 hours. The reaction solution was concentrated, and potassium carbonate (45.54 mg, 0.33 mmol) and acetonitrile (5 mL) were added, and the mixture was refluxed and stirred overnight. The reaction solution was poured into water, extracted with dichloromethane, the organic phase was concentrated, and separated by preparative thin layer chromatography (dichloromethane/methanol = 25/1) to obtain the title compound (15 mg, 40 %).

LC-MS: *m*/*z* [M+H]⁺ = 343.

### Intermediate 76

### 1-(tert-Butoxycarbonyl)-(4S)-2-((benzyloxy)methyl)-4-((3,5-dimethoxybenzyl)amino)pyrrolidine-2-carboxylic acid methyl ester

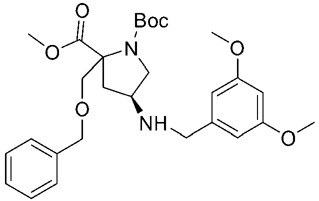

1-(tert-Butoxycarbonyl)-(4*R*)-2-((benzyloxy)methyl)-4-((methylsulfonyl)oxy)pyrrolidine-2-carboxylic acid methyl ester (100 g) , 0.23 mol) was added to 2,4-dimethoxybenzylamine (200 mL), and stirred at 90°C overnight. The reaction solution was directly purified by column chromatography to obtain the title compound (75.8 g, 64%).

LC-MS: *m*/*z* [M+H]⁺ = 515.

### (1R,4S)-1-((Benzyloxy)methyl)-5-(3,5-dimethoxybenzyl)-6-oxo-2,5-diazabicyclo[2.2.1]heptane-2-carboxylic acid tert-butyl ester

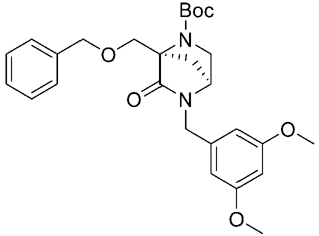

1-(tert-Butoxycarbonyl)-(4*S*)-2-((benzyloxy)methyl)-4-((3,5-dimethoxybenzyl)amino)pyrrolidine-2-carboxylic acid methyl ester (75.8 g, 0.148 mol) and sodium hydroxide (17.76 g, 0.444 mol) were added to a mixed solvent of water (28 mL) and methanol (280 mL), and stirred at 40°C overnight. The reaction solution was concentrated to dry, HATU (112.26 g, 0.296 mol), triethylamine (59.8 g, 0.592 mol) and N,N-dimethylformamide (90 mL) were sequentially added to the residue, and the mixture was stirred at room temperature overnight. The reaction solution was poured into water, extracted with ethyl acetate, the organic phase was concentrated, and purified by silica gel column chromatography (petroleum ether/ethyl acetate = 5/1) to obtain the title compound (23 g, 32%).

LC-MS: *m*/*z* [M+H-Boc]⁺ = 383.

### (1R,4S)-1-((Benzyloxy)methyl)-6-oxo-2,5-diazabicyclo[2.2.1]heptane-2-carboxylic acid tert-butyl ester

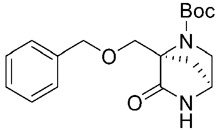

*(1R,4S)-1-((benzyloxy)methyl)-5-(3,5-dimethoxybenzyl)-6-oxo-2,5-*diazabicyclo[2.2.1]heptane-2-carboxylic acid tert-butyl ester (21 g, 0.044 mol) and DDQ (29.63 g, 0.13 mol) were added to dichloromethane (100 mL) and water (5 mL), and stirred at room temperature overnight. The reaction solution was poured into water and extracted with dichloromethane. The organic phase was concentrated and purified by silica gel column chromatography (petroleum ether/ethyl acetate = 1/1) to obtain the title compound (5.6 g, 38%).

LC-MS: *m*/*z* [M+H-Boc]⁺ = 233.

### (1R,4S)-1-((Benzyloxy)methyl)-5-methyl-6-oxo-2, 5-diazabicyclo[2.2.1]heptane-2-carboxylic acid tert-butyl ester

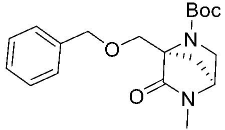

(1*R*,4*S*)-1-((Benzyloxy)methyl)-6-oxo-2,5-diazabicyclo[2.2.1]heptane-2-carboxylic acid tert-butyl ester (1.8 g, 5.42 mmol) was added to tetrahydrofuran (20 mL), and cooled to 0°C under argon protection. Under stirring, Sodium hydride (325 mg, 8.12 mmol) was added to the reaction solution, and the mixture was stirred at 0°C for 30 minutes. Iodomethane (1.15 g, 8.12 mmol) was added dropwise to the reaction solution, and the mixture was stirred at room temperature overnight. The reaction solution was poured into water and extracted with ethyl acetate. The organic phase was concentrated and purified by silica gel column chromatography (dichloromethane/methanol = 15/1) to obtain the title compound (800 mg, 43 %).

LC-MS: *m*/*z* [M+H]⁺ = 247.

### (1R,4S)-1-(Hydroxymethyl)-5-methyl-6-oxo-2,5-diazabicyclo[2.2.1]heptane-2-carboxylic acid tert-butyl ester

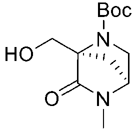

(1*R*,4*S*)-1-((Benzyloxy)methyl)-5-methyl-6-oxo-2,5-diazabicyclo[2.2.1]heptane-2-carboxylic acid tert-butyl ester (800 mg, 2.3 mmol) and Pd/C (10 %, 80 mg) were added to methanol (8 mL), and the reaction solution underwent hydrogenation at 60°C under atmospheric pressure overnight. The reaction solution was filtered, and the filtrate was concentrated to the crude title compound (700 mg).

LC-MS: *m*/*z* [M+H]⁺ = 257.

### (1S,4R)-5-(6-Chloro-2-methoxyprimidin-4-yl)-4-(hydroxymethyl)-2-methyl-2,5-diazabicyclo [2.2.1]heptan-3-one

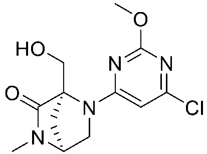

(1*R*,4*S*)-1-(hydroxymethyl)-5-methyl-6-oxo-2,5-diazabicyclo[2.2.1]heptane-2-carboxylic acid tert-butyl ester (700 mg, 2.7 mmol) was added to trifluoroacetic acid (5 mL) and dichloromethane (5 mL), and stirred at room temperature for 2 hours. The reaction solution was concentrated, 4,6-dichloro-2-methoxypyrimidine (489.4 mg, 2.7 mmol), solid sodium carbonate (1.43 g, 13.5 mmol) and acetonitrile (15 mL) were sequentially added to the residue, and the mixture was stirred under reflux for 3 days. The reaction solution was poured into water, extracted with ethyl acetate, and the organic phase was concentrated, and purified by silica gel column chromatography (petroleum ether/ethyl acetate = 2/1) to obtain the title compound (520 mg, 75% yield over two steps).

LC-MS: *m*/*z* [M+H]⁺ = 299.

### (3R,10aR)-6-Chloro-2-methyl-2,3,4,4a-tetrahydro-10H-3,10a-methanopyrido[4',3':3,4]pyrrolo[1,2-c]pyrimidin-1, 8-dione

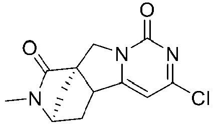

(1*S*,4*R*)-5-(6-Chloro-2-methoxypyrimidin-4-yl)-4-(hydroxymethyl)-2-methyl-2,5-diazabicyclo[2.2.1]heptan-3-one (420 mg, 1.4 mmol) was added to dichloromethane (7 mL), and thionyl chloride (836 mg, 7.0 mmol) was slowly dropped into the system, and stirred at room temperature for 1 hour. The reaction solution was concentrated, the residue was added to saturated aqueous sodium bicarbonate solution, extracted with dichloromethane, and the organic phase was concentrated to obtain the title compound (220 mg, 59%).

LC-MS: *m*/*z* [M+H]⁺ = 267.

### Intermediate 77

### (1R,4S)-1-((Benzyloxy)methyl)-5-((1-methyl-1H-pyrazol-3-yl)methyl)-6-oxo-2,5-diazabicyclo[2.2.1]heptane-2-carboxylic acid tert-butyl ester

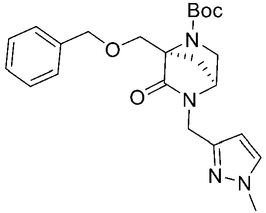

(1*R*,4*S*)-1-((Benzyloxy)methyl)-6-oxo-2,5-diazabicyclo[2.2.1]heptane-2-carboxylic acid tert-butyl ester (1 g , 3 mmol) and 3-(chloromethyl)-1-methyl-1H-pyrazole (600 mg, 4.5 mmol) were added to tetrahydrofuran (20 mL). Sodium hydride (200 mg, 4.5 mmol) was added to the reaction solution under stirring, and the mixture was stirred at 50°C for 3 days. The reaction solution was poured into water, extracted with ethyl acetate, the organic phase was concentrated, and purified by column chromatography (petroleum ether/ethyl acetate = 1/1) to obtain the title compound (800 mg, 62%).

LC-MS: m/z [M+H-Boc]⁺ = 327.

### (1R,4S)-1-(Hydroxymethyl)-5-((1-methyl-1H-pyrazol-3-yl)methyl)-6-oxo-2,5-diazabicyclo[2.2.1]heptane-2-carboxylic acid tert-butyl ester

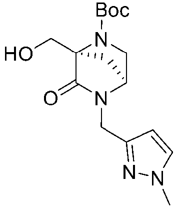

(1*R*,4*S*)-1-((Benzyloxy)methyl)-5-((1-methyl-1H-pyrazol-3-yl)methyl)-6-oxo-2,5-diazabicyclo[2.2.1]heptane-2-carboxylic acid tert-butyl ester (800 mg, 1.88 mmol) and Pd/C (10%, 180 mg) were added to methanol (15 mL), the reaction solution underwent hydrogenation at 60°C under atmospheric pressure overnight. The reaction solution was filtered, and the filtrate was concentrated to obtain the crude title compound (600 mg).

LC-MS: m/z [M+H-Boc]⁺ = 237.

### (1S,4R)-5-(6-Chloro-2-methoxyprimidin-4-yl)-4-(hydroxymethyl)-2-((1-methyl-1H-pyrazol-3-yl)methyl)-2, 5-diazabicyclo[2.2.1]heptan-3-one

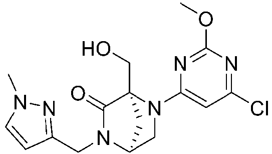

(1*R*,4*S*)-1-(Hydroxymethyl)-5-((1-methyl-1H-pyrazol-3-yl)methyl)-6-oxo-2,5-diazabicyclo[2.2.1]heptane-2-carboxylic acid tert-butyl ester (300 mg, 1.88 mmol) was added to hydrochloric acid/ethyl acetate (4.0 M, 6 mL) and ethyl acetate (5 mL), and stirred at room temperature for 3 hours. The reaction solution was concentrated, 4,6-dichloro-2-methoxypyrimidine (159.3 mg, 0.89 mmol), solid sodium carbonate (471.7 mg, 4.45 mmol) and acetonitrile (15 mL) were sequentially added to the residue, and the mixture was stirred under reflux for 3 days. The reaction solution was poured into water, extracted with ethyl acetate, and the organic phase was concentrated, and purified by column chromatography (petroleum ether/ethyl acetate = 1/1) to obtain the title compound (220 mg, two-step yield: 65%).

LC-MS: *m*/*z* [M+H]⁺ = 379.

### (3R,10aR)-6-Chloro-2-((1-methyl-1H-pyrazol-4-yl)methyl)-2,3,4,4a-tetrahydro-10H-3,10a-methanopyrazino [4', 3':3, 4]pyrrolo[1, 2-c]pyrimidin-1, 8-dione

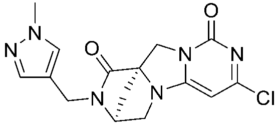

(1*S*,4*R*)-5-(6-Chloro-2-methoxypyrimidin-4-yl)-4-(hydroxymethyl)-2-((1-methyl-1H-pyrazol-3-yl)methyl)-2,5-diazabicyclo[2.2.1]heptan-3-one (220 mg, 1.4 mmol) was added to dichloromethane (10 mL), and methanesulfonyl chloride (99.65 mg, 0.87 mmol) was slowly dropped into the system, and stirred at room temperature for 2 hours. The reaction solution was concentrated, the residue was added to saturated aqueous sodium bicarbonate solution, extracted with dichloromethane, and the organic phase was concentrated to obtain the title compound (120 mg, 60%).

LC-MS: *m*/*z* [M+H]⁺ = 347.

### Intermediate 78

### (1R,4S)-1-((Benzyloxy)methyl)-5-(bicyclo[1.1.1]pent-1-ylmethyl)-6-oxo-2,5-diazabicyclo[2.2.1]heptane- 2-carboxylic acid tert-butyl ester

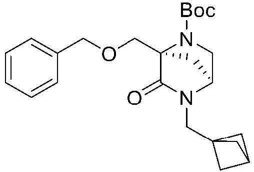

(1*R*,4*S*)-1-((Benzyloxy)methyl)-6-oxo-2,5-diazabicyclo[2.2.1]heptane-2-carboxylic acid tert-butyl ester (1 g , 3 mmol) and 1-(chloromethyl)bicyclo[1.1.1]pentane (1.05 g, 9.0 mmol) were added to tetrahydrofuran (20 mL). Under stirring, sodium hydride (600 mg, 15 mmol) was added to the reaction solution, and the mixture was stirred at 50°C for 3 days in a stuffy tank. The reaction solution was poured into water and extracted with ethyl acetate. The organic phase was concentrated and purified by column chromatography (petroleum ether/ethyl acetate = 5/1) to give the title compound (780 mg, 63%).

LC-MS: *m*/*z* [M+H -Boc]⁺ = 313.

### (1R,4S)-5-(bicyclo[1.1.1]pent-1-ylmethyl)-1-(hydroxymethyl)-6-oxo-2,5-diazabicyclo[2.2.1]heptane-2-carboxylic acid tert-butyl ester

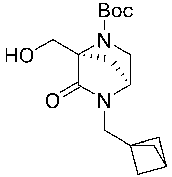

(1*R*,4*S*)-1-((benzyloxy)methyl)-5-(bicyclo[1.1.1]pent-1-ylmethyl)-6-oxo-2,5-diazabicyclo[2.2.1]heptane-2-carboxylic acid tert-butyl ester (780 mg, 1.9 mmol) and Pd/C (10%, 200 mg) were added to methanol (20 mL), and the reaction solution was hydrogenated (70°C, 0.5 MPa) overnight. The reaction solution was filtered, and the filtrate was concentrated to obtain the crude title compound (430 mg).

LC-MS: m/z [M+H-tBu]⁺ = 267.

### (1S,4R)-2-(bicyclo[1.1.1]pent-1-ylmethyl)-5-(2,6-dichloropyrimidin-4-yl)-4-(hydroxymethyl)-2,5-diazabicyclo[2.2.1]heptan-3-one

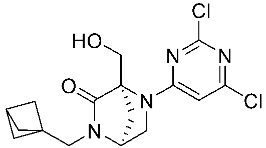

(1*R*,4*S*)-5-(bicyclo[1.1.1]pent-1-ylmethyl)-1-(hydroxymethyl)-6-oxo-2,5-diazabicyclo[2.2.1]heptane-2-carboxylic acid tert-butyl ester (480 mg, 1.88 mmol) was added to hydrochloric acid/dioxane (4.0 M, 5 mL) and dichloromethane (5 mL), and stirred at room temperature for 30 minutes. The reaction solution was concentrated, 2,4,6-trichloropyrimidine (372.2 mg, 1.44 mmol), solid sodium carbonate (458 mg, 4.32 mmol) and acetonitrile (15 mL) were sequentially added to the residue, and the mixture was stirred at room temperature overnight. The reaction solution was filtered, the filtrate was concentrated, and purified by column chromatography (petroleum ether/ethyl acetate = 1/1) to obtain the title compound (175 mg, two-step yield: 33%).

LC-MS: *m*/*z* [M+H]⁺ = 369.

### (3S,11aR)-2-(Bicyclo[1.1.1]pent-1-ylmethyl)-7-chloro-3,4-dihydro-9H,11H-3,11a-methanopyrazino[1',2':3,4]imidazo[1,2-c]pyrimidin-1,9(2H)-dione

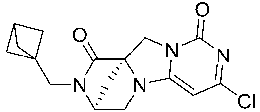

(1*S*,4*R*)-2-(bicyclo[1.1.1]pentyl-1-ylmethyl)-5-(2,6-dichloropyrimidin-4-yl)-4-(hydroxymethyl)-2,5-diazabicyclo[2.2.1]heptan-3-one (50 mg, 1.4 mmol) was added to dichloromethane (6 mL), and thionyl chloride (2 mL) was added dropwise under stirring, and stirred at room temperature for 30 minutes. The reaction solution was concentrated, sodium carbonate (43.2 mg, 0.41 mmol) and acetonitrile (10 ml) were sequentially added to the residue, and the mixture was refluxed and stirred overnight. The reaction solution was poured into water, extracted with dichloromethane, the organic phase was concentrated, and purified by column chromatography (dichloromethane/methanol = 20/1) to obtain the title compound (42 mg, 93%). LC-MS: *m*/*z* [M+H]⁺ = 333.

### Intermediate 79

### (1S,4S)-2-Benzyl-5-(tert-butoxycarbonyl)-2,5-diazabicyclo[2.2.1]heptane-1-carboxylic acid

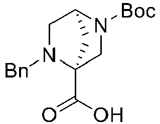

(1*S*,4*S*)-5-Benzyl-4-cyano-2,5-diazabicyclo[2.2.1]heptane-2-carboxylic acid tert-butyl ester (6.3 g, 20 mmol, Synthesis, 2015, 1123 - 1130), sodium hydroxide aqueous solution (1.0 M, 50 mL) and solid sodium hydroxide (8.3 g, 207 mmol) were added to methanol (150 mL), and stirred at 100°C overnight. The reaction solution was concentrated to remove methanol, and the residue was adjusted to pH=3.8~4.4 with aqueous hydrochloric acid (1.0 M), extracted with dichloromethane, and the organic phase was concentrated to give the title compound (5.4 g, 81%). LC-MS: *m*/*z* [M+H]⁺ = 333.

### (1S,4S)-5-Benzyl-4-(hydroxymethyl)-2, 5-diazabicyclo[2.2.1]heptane-2-carboxylic acid tert-butyl ester

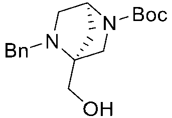

(1*S*,4*S*)-2-Benzyl-5-(tert-butoxycarbonyl)-2,5-diazabicyclo[2.2.1]heptane-1-carboxylic acid (3.0 g, 9 mmol) and a solution of borane-dimethyl sulfide complex in tetrahydrofuran (2.0 M, 9 mL, 18 mmol) were added to tetrahydrofuran (30 mL), and stirred at 80°C overnight. The reaction solution was quenched with methanol, concentrated, and purified by silica gel column chromatography to obtain the title compound (2.0 g, 70 %).

LC-MS: *m*/*z* [M+H]⁺ = 319.

### (1S,4S)-4-(Hydroxymethyl)-2, 5-diazabicyclo[2.2.1]heptane-2-carboxylic acid tert-butyl ester

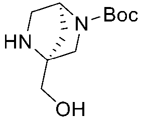

(1*S*,4*S*)-5-Benzyl-4-(hydroxymethyl)-2,5-diazabicyclo[2.2.1]heptane-2-carboxylic acid tert-butyl ester (1.7 g 5.3 mmol) and Pd/C (10 %, 100 mg) were added to methanol (17 mL), and hydrogenated at room temperature under atmospheric pressure overnight. The reaction solution was filtered through celite, and the filtrate was concentrated to give the crude title compound (1.4 g, 115%), which was used in the next step without further purification.

### (1S,4S)-5-(2,6-Dichloropyrimidin-4-yl)-4-(hydroxymethyl)-2,5-diazabicyclo[2.2.1]heptane-2-carboxylic acid tert-butyl ester

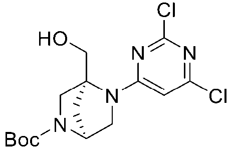

(1*S*,4*S*)-4-(Hydroxymethyl)-2,5-diazabicyclo[2.2.1]heptane-2-carboxylic acid tert-butyl ester (1.4 g, 6.1 mmol), solid sodium carbonate (1.3 g, 12 mmol) and 2,4,6-trichloropyrimidine (1.5 g, 8.0 mmol) were added to acetonitrile (14 mL) and stirred at room temperature overnight. The reaction solution was filtered, the filtrate was concentrated, and purified by silica gel column chromatography to obtain the title compound (1.3 g, 56%).

LC-MS: *m*/*z* [M+H]⁺ = 374.

### (3S,11aR)-7-Chloro-9-oxo-3,4-dihydro-9H,11H-3,11a-methanopyrazino[1',2':3,4]imidazo[1,2-c]pyrimidine-2(1H)-carboxylic (1H)-carboxylic acid tert-butyl ester

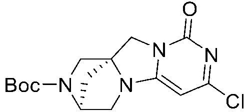

(1*S*,4*S*)-5-(2,6-Dichloropyrimidin-4-yl)-4-(hydroxymethyl)-2,5-diazabicyclo[2.2.1]heptane-2-carboxylic acid tert-butyl ester (1.3 g, 3.5 mmol) and triethylamine (700 mg, 7.0 mmol) were added to dichloromethane (13 mL), cooled to 0°C, then added with methanesulfonic anhydride (900 mg, 5.2 mmol), and stirred at room temperature for 1 hour. The reaction solution was concentrated, the residue and solid potassium carbonate (970 mg, 7.0 mmol) were added to acetonitrile (13 mL), and the mixture was stirred at 80°C overnight. The reaction solution was poured into water, extracted with dichloromethane, and concentrated to obtain the title compound (1.1 g, 97 %).

LC-MS: *m*/*z* [M+H]⁺ = 339.

¹H NMR (400 MHz, CDCl₃) δ5.59 (br. s, 1H), 4.45 (d, *J* = 12.7 Hz, 1H), 3.99 (br. s, 1H), 3.65 - 3.54 (m, 3H), 3.32 (d, *J* = 8.8 Hz, 1H), 3.17 - 3.06 (m, 1H), 2.09 - 1.98 (m, 1H), 1.86 (d, *J =* 9.3 Hz, 1H), 1.49 (br. s, 9H).

### (3S,11aR)-7-(((3-Fluoro-4-((2-(trifluoromethyl)pyridin-4-yl)oxy)benzyl)oxy)-9-oxo-3,4-dihydro-9H,11H-3,11a-methanopyrazino[1',2':3,4]imidazo[1,2-c]pyrimidine-2(1H)-carboxylic acid tert-butyl ester

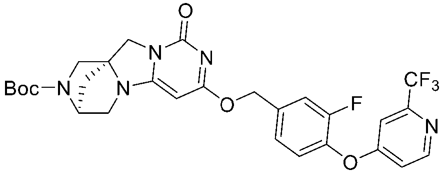

(3*S*,11a*R*)-7-Chloro-9-oxo-3,4-dihydro-9H,11H-3,11a- methanopyrazino [1',2':3,4]imidazo[1,2-c]pyrimidine-2(1H)-carboxylic acid tert-butyl ester (1.1 g, 3.4 mmol), (3-fluoro-4-((2-(trifluoromethyl)pyridin-4-yl)oxy)phenyl)methanol (1.2 g, 4.4 mmol) and solid cesium carbonate (3.3 g, 10 mmol) were added to toluene (11 mL), and stirred at 120°C overnight. The reaction solution was filtered, and the filtrate was concentrated and purified by silica gel column chromatography to obtain the title compound (1.3 g, 65%).

LC-MS: *m*/*z* [M+H]⁺ = 590.

¹H NMR (400 MHz, CDCl₃) δ8.58 (d, *J =* 5.4 Hz, 1H), 7.33 (d, *J =* 11.2 Hz, 1H), 7.27 - 7.16 (m, 3H), 6.96 (d, *J* = 4.9 Hz, 1H), 5.44 (d, *J* = 4.9 Hz, 2H), 5.15 - 5.06 (m, 1H), 4.43 (d, *J* = 12.7 Hz, 1H), 4.02 - 3.90 (m, 1H), 3.66 - 3.47 (m, 4H), 3.23 (d, *J* = 9.8 Hz, 1H), 1.96 (d, *J =* 10.3 Hz, 1H), 1.71 (d, *J* = 9.8 Hz, 1H), 1.49 (br. s, 9H).

### Intermediate 80

### 2-Fluoro-4-(2-hydroxyethyl)phenol

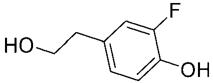

2-(3-Fluoro-4-hydroxyphenyl)acetic acid (2 g, 11.6 mmol) was added to tetrahydrofuran (10 mL), borane-dimethyl sulfide complex (3.5 mL, 34.8 mmol) was added under stirring, and the mixture was stirred at room temperature for 3 hours, the reaction solution was concentrated and diluted with water, extracted with ethyl acetate, and the organic phase was concentrated to obtain the crude title compound (2.2 g, 120%).

LCMS: *m*/*z* [M+H]⁺ = 157.

### 2-(3-Fluoro-4-(((2-(trifluoromethyl)pyridin-4-yl)oxy)phenyl)ethan-1-ol

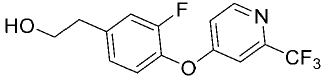

2-Fluoro-4-(2-hydroxyethyl)phenol (2.1 g, 13.29 mmol), 4-chloro-2-(trifluoromethyl)pyridine (2.35 g, 13.29 mmol) and potassium carbonate (3.67 g, 26.58 mmol) were added to N,N-dimethylformamide (20 mL) and stirred at 120°C for 3 hours. The reaction solution was poured into water, extracted with ethyl acetate, the organic phase was concentrated, and separated by silica gel column chromatography to obtain the title compound (1.63 g, 40.6%). LCMS: *m*/*z* [M+H]⁺ = 302.

### Intermediate 81

### 4-Bromo-2-(difluoromethyl)pyridine

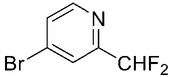

4-Bromopyridylaldehyde (10 g, 53.76 mmol) was added to dichloromethane (100 mL), and the temperature was lowered to 0°C. Under stirring, diethylaminosulfur trifluoride (17.3 g, 107.52 mmol) was added dropwise to the reaction solution, and the mixture was stirred at room temperature overnight. The reaction solution was slowly dropped into water, extracted with dichloromethane, and the organic phase was concentrated to obtain the crude title compound, which was directly used in the next reaction.

LC-MS: *m*/*z* [M+H]⁺ = 208.

### 2-(Difluoromethyl)-4-methoxypyridine

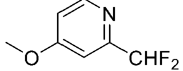

4-Bromo-2-(difluoromethyl)pyridine (11.18 g, 53.76 mmol), sodium methoxide (5.81 g, 107.52 mmol) were added to methanol (100 mL), and stirred at 90°C overnight. The reaction solution was cooled to room temperature, poured into water (300 mL), and extracted with ethyl acetate. The organic phase was concentrated to obtain the crude title compound, which was directly used in the next reaction.

LC-MS: *m*/*z* [M+H]⁺ = 160.

### 2-(Difluoromethyl)pyridin-4-ol

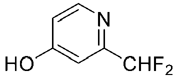

2-(Difluoromethyl)-4-methoxypyridine (9.54 g, 59.95 mmol) was added to hydrobromic acid (40% aqueous solution, 58 mL), and stirred at 90°C for 2 days. The reaction solution was concentrated, the residue was diluted with water, solid sodium bicarbonate was added under stirring until no bubbles were generated in the system; after extraction with ethyl acetate, the organic phase was concentrated, and purified by column chromatography (petroleum ether/ethyl acetate=10/1~1 /1) to obtain the title compound (2.5 g, 26% overall yield over three steps). LC-MS: *m*/*z* [M+H]⁺ = 146.

### 4-((2-(Difluoromethyl)pyridin-4-yl)oxy)benzaldehyde

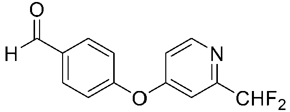

4-Fluorobenzaldehyde (200 mg, 1.6 mmol), 2-(difluoromethyl)pyridin-4-ol (289 mg, 1.8 mmol) and solid potassium carbonate (441 mg, 3.2 mmol) were added to N,N-dimethylformamide (5 mL), stirred at 100°C overnight, and separated by preparative thin layer chromatography to obtain the title compound (180 mg, 45%).

LC-MS: *m*/*z* [M+H]⁺ = 250.

### (4-((2-(Difluoromethyl)pyridin-4-yl)oxy)phenyl)methanol

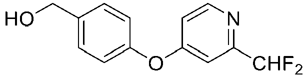

4-((2-(Difluoromethyl)pyridin-4-yl)oxy)benzaldehyde (180 mg, 0.7 mmol) was added to absolute ethanol (5 mL), and sodium borohydride (41 mg, 1 mmol) was added under stirring, and stirred at room temperature for 2 hours. The reaction solution was poured into water, extracted with dichloromethane, and the organic phase was concentrated to obtain the title compound (100 mg, 62 %).

LC-MS: *m*/*z* [M+H]⁺ = 252.

Referring to the table below, the following intermediates were prepared with reference to the method for the preparation of Intermediate **81,** except that the starting materials described in the "starting materials" column were used in place of the corresponding starting materials.

| **Intermedi ate No.** | **Intermediate name** | **Structural formula** | **Spectrum:¹H NMR (400 MHz) or LC-MS(m/z [M+H]⁺)** | **Starting materials** |
|---|---|---|---|---|
| **82** | (4-((2-(difluoromethyl)pyridin-4-yl)oxy)-3,5-difluorophenyl)methanol | 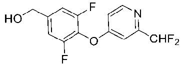 | CDCl₃: δ8.51 (d, *J =* 5.4 Hz, 1H), 7.16 (s, 1H), 7.10 (d, *J =* 8.3 Hz, 2H), 6.95 (d, *J* = 4.4 Hz, 1H), 6.75 - 6.45 (m, 1H), 4.74 (s, 2H) | 3,4,5-trifluorobenzaldeh yde; 4-bromopyridylaldeh yde |
| **83** | (4-((2-(difluoromethyl)pyridin-4-yl)oxy)-3-fluorophenyl)methanol | 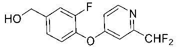 | 270 | 3,4-diflulorobenzaldeh yde; 4-bromopyridylaldeh yde |

### Intermediate 84:

### 4-(4-Bromophenoxy)-2-(trifluoromethyl)pyridine

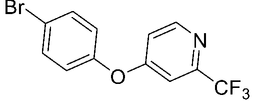

4-Bromophenol (9.4 g, 54.53 mmol), 4-chloro-2-(trifluoromethyl)pyridine (9 g, 49.58 mmol) and potassium carbonate (13.7 g, 99.16 mmol) were added to N,N-dimethyl acetamide (100 mL), stirred at 120°C for 2 hours, the reaction solution was poured into water, extracted with ethyl acetate, the organic phase was concentrated, and purified by silica gel column chromatography (petroleum ether/ethyl acetate = 10/1) to obtain the title Compound (4.3 g, 25 %).

LCMS: *m*/*z* [M+H]⁺ = 318.

### 2-(Trifluoromethyl)-4-(4-((trimethylsilyl)ethynyl)phenoxy)pyridine

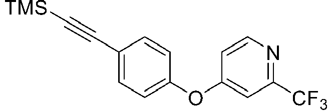

4-(4-Bromophenoxy)-2-(trifluoromethyl)pyridine (4.3 g, 13.52 mmol), ethynyltrimethylsilane (3.32 g, 33.8 mmol), tetrakis(triphenylphosphine)palladium (1.56 g, 1.352 mmol) and cuprous iodide (275 mg, 1.352 mmol) were added to a mixed solvent of diisopropylethylamine (15 mL) and toluene (30 mL). The reaction solution was filtered, the filtrate was poured into water, extracted with ethyl acetate, the organic phase was concentrated, and purified by silica gel column chromatography (petroleum ether/ethyl acetate = 100/1~20/1) to obtain the title compound (1.5 g, 33%).

LCMS: *m*/*z* [M+H]⁺ = 336.

### 4-(4-Ethynylphenoxy)-2-(trifluoromethyl)pyridine

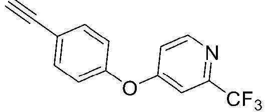

2-(Trifluoromethyl)-4-(4-((trimethylsilyl)ethynyl)phenoxy)pyridine (1 g, 2.98 mmol) and potassium carbonate (822.9 mg, 5.96 mmol) were added into methanol (10 mL) and stirred at room temperature overnight. The reaction solution was quenched with water, extracted with ethyl acetate, and the organic phase was concentrated to obtain the title compound (750 mg, 96 %).

LCMS: *m*/*z* [M+H]⁺ = 264.

### Intermediate 85:

### (3,4-Difluorobenzyl)carbamic acid tert-butyl ester

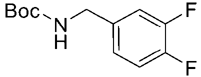

(3,4-Difluorophenyl)methanamine (1.0 g, 6.99 mmol), di-tert-butyl dicarbonate (2.3 g, 10.5 mmol) and triethylamine (1.4 g, 14.0 mmol) were added to dichloromethane (10 mL) and stirred at room temperature overnight. The reaction solution was concentrated and purified by silica gel column chromatography to obtain the title compound (1.6 g, 94%).

LCMS: *m*/*z* [M+H-Boc]⁺ = 144.

### 1-(3,4-Difluorophenyl)-N-methyl-methylamine

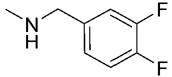

(3,4-Difluorobenzyl)carbamic acid tert-butyl ester (300 mg, 1.2 mmol) and lithium aluminum tetrahydride (47 mg, 1.2 mmol) were added to tetrahydrofuran (3 mL), the temperature was raised to reflux and stirred for 1 hour. The reaction solution was quenched with sodium sulfate decahydrate, filtered, and the filtrate was concentrated to obtain the title compound (170 mg, 88 %).

¹H NMR (400 MHz, DMSO-*d*₆) δ7.63 (t, *J* = 9.5 Hz, 1H), 7.56 - 7.45 (m, 1H), 7.37 (br. s, 1H), 4.15 (br. s, 1H), 3.88 (s, 2H), 2.52 (s, 3H).

### Examples

### Example 1

### Method A

### (3S,11aR)-7-((3,5-difluoro-4-((2-(trifluoromethyl)pyridin-4-yl)oxy)benzyl)oxy)-3,5-difluorobenzyl)oxy)-3,4-dihydro-1H,9H,11H-3,11a-methanopyrimido[6'-,1':2,3]imidazo[5,1-c][1,4]oxazin-9-one

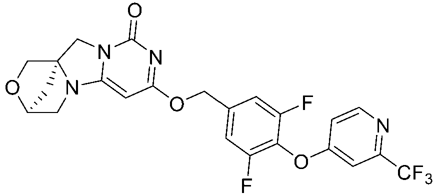

(3,5-Difluoro-4-((2-(trifluoromethyl)pyridin-4-yl)oxy)phenyl)methanol (51 mg, 0.167 mmol) was dissolved in dry tetrahydrofuran (4 mL), lithium hexamethyldisilazide (0.167 mL, 1.0 M solution in tetrahydrofuran, 0.167 mmol) was added at room temperature, and the mixture was stirred for 15 minutes. (3*S*,11a*R*)-7-chloro-3,4-dihydro-1H,9H,11H-3,11a-methanopyrimido[6',1':2,3]imidazo[5,1-c][1,4]oxazin-9-one (20 mg, 0.083 mmol) was added, the reaction mixture was stirred at 120°C for 16 hours, added with methanol to quench the reaction, and separated by preparative thin layer chromatography to obtained the title compound (10.7 mg, 25%).

LC-MS: *m*/*z* [M+H]⁺ = 509.

¹H NMR (400 MHz, DMSO-*d*₆) δ8.68 (d, *J* = 4.4 Hz, 1H), 7.65 (br. s, 1H), 7.45 (d, *J* = 8.8 Hz, 2H), 7.30 (br. s, 1H), 5.34 (br. s, 2H), 5.31 (br. s, 1H), 4.68 (br. s, 1H), 4.29 (d, *J =* 11.7 Hz, 1H), 3.99 - 3.76 (m, 3H), 3.32 - 3.22 (m, 2H), 1.90 (d, *J =* 9.3 Hz, 1H), 1.79 (d, *J* = 9.8 Hz, 1H).

### Example 2

### Method B

### 5-(((1-Oxo-7,8-dihydro-1H,6H,9H-6,8a-ethanopyrrolo[1',2':3,4]imidazo[1,2-c]pyrimidin-3-yl)oxy)methyl)-2-(3-(trifluoromethyl)phenoxy)benzonitrile

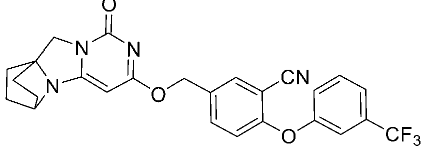

5-(Hydroxymethyl)-2-(3-(trifluoromethyl)phenoxy)benzonitrile (233 mg, 0.80 mmol) was dissolved in acetonitrile (12 mL), cooled to 0°C, and sodium hydride (42 mg, 1.06 mmol, 60 %) and 3-chloro-7,8-dihydro-1H,6H,9H-6,8a-ethanopyrrolo[1',2':3,4]imidazo[1,2-c]pyrimidin-1-one (125 mg, 0.53 mmol) were added, and stirred for 0.5 h. The reaction was quenched with saturated ammonium chloride solution and extracted with ethyl acetate. The organic phase was washed with saturated brine and dried over anhydrous sodium sulfate. The organic phase was concentrated and separated by preparative thin layer chromatography (dichloromethane/methanol = 25/1) to give the title compound (128 mg, 49 %).

LC-MS: *m*/*z* [M+H]⁺ = 495.

¹H NMR (400 MHz, DMSO-*d*₆) δ7.98 (s, 1H), 7.79 - 7.40 (m, 5H), 7.10 (d, *J* = 8.8 Hz, 1H), 5.44 (s, 1H), 5.27 (s, 2H), 4.27 (br. s, 1H), 3.88 (s, 2H), 1.92 (d, *J* = 7.8 Hz, 2H), 1.81 - 1.55 (m, 6H).

Examples 4 to 168 listed in the table below were prepared by steps similar to those described in Examples 1 and 2, starting from the corresponding intermediates:

| **Exa mple #** | **Name** | **Structure** | **Metho d used** | ***m*/*z*** | **¹H NMR (400 MHz)** |
|---|---|---|---|---|---|
| **4** | (3*S*,11a*R*)-7-((4-(4-chloro-3-(trifluoromethyl)phenoxy)-3,5-difluorobenzyl)oxy)-3,4-dihydro-1H,9H,11H-3,11a-methanopyrimido[6'-,1':2,3]imidazo[5,1-c][1,4]oxazin-9-one | 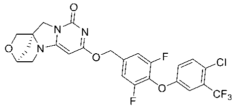 | B | 542 | CDCl₃: δ7.42 (d, *J* = 8.8 Hz, 1H), 7.30 (br. s, 1H), 7.09 (d, *J* = 7.8 Hz, 2H), 7.02 (d, *J* = 7.8 Hz, 1H), 5.50 - 5.29 (m, 2H), 5.12 (s, 1H), 4.75 (br. s, 1H), 4.47 (d, *J* = 12.2 Hz, 1H), 4.09 - 3.86 (m, 3H), 3.59 (d, *J* = 9.8 Hz, 1H), 3.25 (d, *J* = 9.8 Hz, 1H), 2.05 (d, *J* = 8.8 Hz, 1H), 1.75 (d, *J=* 10.3 Hz, 1H) |
| **5** | (3*S*,11a*R*)-7-((3,5-difluoro-4-((2-(trifluoromethoxy)pyridin-4-yl)oxy)benzyl)oxy)-3,4-dihydro-1H,9H,11H-3,11a-methanopyrimido[6', 1':2,3]i midazo[5, 1-c][1,4]oxazin-9-one | 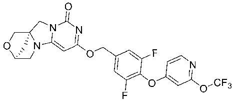 | B | 525 | CDCl₃: δ8.21 (d, *J* = 5.4 Hz, 1H), 7.12 (d, *J* = 8.3 Hz, 2H), 6.82 (d, *J* = 4.4 Hz, 1H), 6.50 (s, 1H), 5.34 - 5.51 (m, 2H), 5.12 (s, 1H), 4.75 (br. s, 1H), 4.48 (d, *J* = 12.2 Hz, 1H), 4.08 - 3.89 (m, 3H), 3.59 (d, *J* = 9.8 Hz, 1H), 3.26 (d, *J* = 9.3 Hz, 1H), 2.05 (d, *J* = 9.3 Hz, 1H), 1.75 (d, *J* = 9.8 Hz, 1H) |
| **6** | (3*S*,11a*R*)-7-((4-((2-chloropyridin-4-yl)oxy)-3,5-difluorobenzyl)oxy)-3,4-dihydro-1H,9H,11H-3,11a-methanopyrimido[6',1':2,3]i midazo[5,1-c][1,4]oxazin-9-one | 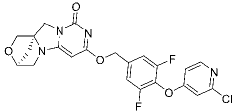 | B | 475 | CDCl₃: δ8.28 (d, *J* = 4.4 Hz, 1H), 7.12 (d, *J* = 7.8 Hz, 2H), 6.85 (br. s, 2H), 5.42 (d, *J* = 6.8 Hz, 2H), 5.13 (br. s, 1H), 4.75 (br. s, 1H), 4.47 (d, *J* = 12.2 Hz, 1H), 4.09 - 3.89 (m, 3H), 3.59 (d, *J* = 9.8 Hz, 1H), 3.26 (d, *J* = 9.3 Hz, 1H), 2.05 (d, *J* = 9.8 Hz, 1H), 1.75 (d, *J =* 9.8 Hz, 1H) |
| **7** | (3*S*,11a*R*)-7-((3,5-difluoro-4-((2-(trifluoromethyl)pyrimidin-5-yl)oxy)benzyl)oxy)-3,4-dihydro-1H,9H,11H-3,11a-methanopyrimido[6', 1':2,3]i midazo[5, 1-c][1,4]oxazin-9-one | 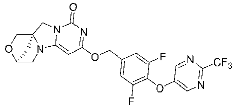 | B | 510 | DMSO-*d*₆: δ9.28 - 8.70 (m, 2H), 7.44 (d, *J* = 9.3 Hz, 2H), 5.32 (d, *J* = 10.8 Hz, 3H), 4.72 - 4.60 (m, 1H), 4.29 (d, *J* = 11.7 Hz, 1H), 4.03 - 3.73 (m, 3H), 3.57 (br. s, 1H), 3.22 - 3.09 (m, 1H), 1.90 (d, *J* = 8.8 Hz, 1H), 1.79 (d, *J* = 9.3 Hz, 1H) |
| **8** | (3*S*,11a*R*)-7-((3,5-difluoro-4-((5-(trifluoromethyl)pyridin-3-yl)oxy)benzyl)oxy)-3,4-dihydro-1H,9H,11H-3,11a-methanopyrimido[6', 1':2,3]i midazo[5, 1-c][1,4]oxazin-9-one | 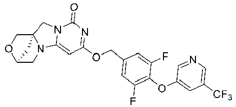 | B | 509 | DMSO-*d*₆: δ8.76 (s, 2H), 7.92 (br. s, 1H), 7.41 (d, *J* = 8.8 Hz, 2H), 5.38 - 5.24 (m, 3H), 4.68 (br. s., 1H), 4.28 (d, *J* = 12.2 Hz, 1H), 3.97 - 3.80 (m, 3H), 3.39 (br. s., 1H), 3.27 - 3.19 (m, 1H), 1.94 - 1.76 (m, 2H) |
| **9** | (3*S*,11a*R*)-7-(((3,5-difluoro-4-((6-(trifluoromethyl)pyridin-3-yl)oxy)benzyl)oxy)-3,4-dihydro-1H,9H,11H-3,11a-methanopyrimido[6', 1':2,3]i midazo[5, 1-c][1,4]oxazin-9-one | 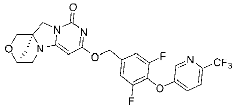 | B | 509 | DMSO-*d*₆: δ8.66 (br. s, 1H), 7.89 (d, *J* = 8.8 Hz, 1H), 7.61 (d, *J* = 8.3 Hz, 1H), 7.42 (d, *J* = 9.3 Hz, 2H), 5.37 - 5.25 (m, 3H), 4.67 (br. s, 1H), 4.27 (d, *J* = 12.2 Hz, 1H), 3.96 - 3.77 (m, 3H), 3.36 (br. s, 1H), 3.25 (br. s, 1H), 1.89 (d, *J* = 9.8 Hz, 1H), 1.77 (d, *J* = 9.8 Hz, 1H) |
| **10** | (3*S*,11a*R*)-7-((3,5-difluoro-4-((2-methyl-pyridin-4-yl)oxy)benzyl)oxy)-3,4-dihydro-1H,9H,11H-3,11a-methanopyrimido[6', 1':2,3]i midazo[5,1-c][1,4]oxazin-9-one | 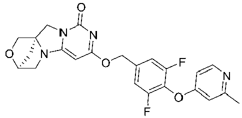 | B | 455 | CDCl₃: δ8.37 (d, *J* = 5.9 Hz, 1H), 7.09 (d, *J* = 8.3 Hz, 2H), 6.73 - 6.62 (m, 2H), 5.48 - 5.36 (m, 2H), 5.12 (s, 1H), 4.75 (s, 1H), 4.47 (d, *J* = 12.7 Hz, 1H), 4.05 - 3.90 (m, 3H), 3.59 (d, *J* = 10.3 Hz, 1H), 3.25 (d, *J* = 9.8 Hz, 1H), 2.52 (s, 3H), 2.05 (d, *J =* 9.8 Hz, 1H), 1.75 (d, *J =* 9.8 Hz, 1H) |
| **11** | (3*S*,11a*R*)-7-((3,5-difluoro-4-((1-methyl-1H-pyrazol-4-yl)oxy)benzyl)oxy)-3,4-dihydro-1H,9H,11H-3,11a-methanopyrimido[6',1':2,3]i midazo[5,1-c][1,4]oxazin-9-one | 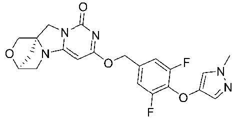 | B | 444 | DMSO-*d*₆: δ7.60 (s, 1H), 7.35 - 7.21 (m, 3H), 5.27 (br. s, 3H), 4.68 (br. s, 1H), 4.27 (d, *J* = 12.2 Hz, 1H), 3.98 - 3.79 (m, 3H), 3.72 (s, 3H), 3.39 (br. s, 1H), 3.29 - 3.25 (m, 1H), 1.92 - 1.75 (m, 2H) |
| **12** | 3-(2-fluoro-4-((((((3*S*,11a*R*)-9-oxo-3,4-dihydro-1H,9H,11H-3,11a-methanopyrimido[6', 1':2,3]i midazo[5, 1-c][1,4]oxazin-7-yl)oxy)methyl)phenoxy)-5-(trifluoromethyl)benzonitrile | 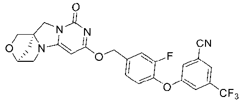 | B | 515 | CDCl₃: δ7.61 (br. s, 1H), 7.45 (br. s, 1H), 7.37 - 7.28 (m, 3H), 7.17 (d, *J* = 7.8 Hz, 1H), 5.44 (br. s, 2H), 5.12 (br. s, 1H), 4.74 (br. s, 1H), 4.47 (d, *J* = 12.2 Hz, 1H), 4.01 - 3.93 (m, 3H), 3.58 (d, *J* = 9.8 Hz, 1H), 3.24 (d, *J* = 9.3 Hz, 1H), 2.04 (d, *J* = 9.3 Hz, 1H), 1.75 - 1.73 (m, 1H) |
| **13** | (3*S*,11a*R*)-7-((3-fluoro-4-((2-(trifluoromethyl)pyridin-4-yl)oxy)benzyl)oxy)-3,4-dihydro-1H,9H,11H-3,11a-methanopyrimido[6',1':2,3]i midazo[5, 1-c][1,4]oxazin-9-one | 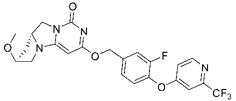 | B | 491 | CDCl₃: δ8.58 (d, *J* = 5.9 Hz, 1H), 7.33 (d, *J* = 10.8 Hz, 1H), 7.28 (br. s, 1H), 7.25 - 7.15 (m, 2H), 6.96 (dd, *J* = 2.0, 5.4 Hz, 1H), 5.52 - 5.39 (m, 2H), 5.12 (s, 1H), 4.75 (s, 1H), 4.48 (d, *J* = 12.7 Hz, 1H), 4.05 - 3.91 (m, 3H), 3.59 (d, *J* = 10.3 Hz, 1H), 3.24 (d, *J* = 9.3 Hz, 1H), 2.05 (d, *J* = 8.8 Hz, 1H), 1.74 (d, *J* = 9.8 Hz, 1H) |
| **14** | (3*S*,11a*R*)-7-((4-(4-chloro-3-(trifluoromethyl)phenoxy)-3-fluorobenzyl)oxy)-3,4-dihydro-1H,9H,11H-3,11a-methanopyrimido[6',1':2,3]i midazo[5,1-c][1,4]oxazin-9-one | 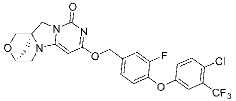 | B | 524 | CDCl₃: δ7.42 (d, *J* = 8.3 Hz, 1H), 7.28 (d, *J* = 12.7 Hz, 2H), 7.20 (d, *J* = 7.8 Hz, 1H), 7.13 - 6.99 (m, 2H), 5.48 - 5.34 (m, 2H), 5.09 (s, 1H), 4.74 (br. s, 1H), 4.46 (d, *J* = 12.2 Hz, 1H), 4.06 - 3.90 (m, 3H), 3.57 (d, *J* = 9.8 Hz, 1H), 3.23 (d, *J* = 9.8 Hz, 1H), 2.04 (d, *J* = 9.8 Hz, 1H), 1.73 (d, *J* = 9.8 Hz, 1H) |
| **15** | (3*S*,11a*R*)-7-((4-((2-(trifluoromethyl)pyridin-4-yl)oxy)benzyl)oxy)-3,4-dihydro-1H,9H,11H-3,11a-methanopyrimido[6',1':2,3]i midazo[5,1-c][1,4]oxazin-9-one | 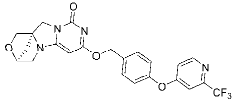 | B | 473 | CDCl₃: δ8.56 (d, *J* = 5.4 Hz, 1H), 7.52 (d, *J* = 7.8 Hz, 2H), 7.24 (br. s., 1 H), 7.17 - 6.92 (m, 3 H), 5.44 (br. s., 2H), 5.10 (s, 1H), 4.74 (br. s., 1H), 4.47 (d, *J* = 12.7 Hz, 1H), 4.07 - 3.86 (m, 3H), 3.57 (d, *J* = 9.8 Hz, 1H), 3.23 (d, *J* = 9.8 Hz, 1H), 2.04 (d, *J* = 9.3 Hz, 1H), 1.73 (d, *J* = 9.8 Hz, 1H) |
| **16** | (3*S*,11a*R*)-7-((4-(4-chloro-3-(trifluoromethyl)phenoxy)be nzyl)oxy)-3,4-dihydro-1H,9H,11H-3,11a-methanopyrimido[6',1':2,3]i midazo[5, 1-c][1,4]oxazin-9-one | 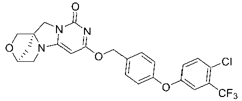 | B | 506 | DMSO-*d*₆: δ7.76 - 7.66 (m, 1H), 7.46 (d, *J* = 6.8 Hz, 3H), 7.28 (br. s., 1H), 7.14 (d, *J* = 7.3 Hz, 2H), 5.35 - 5.20 (m, 3H), 4.67 (br. s., 1H), 4.28 (d, *J* = 11.7 Hz, 1H), 3.97 - 3.77 (m, 3H), 3.45 - 3.37 (m, 1H), 3.28 (br. s., 1H), 1.88 (br. s., 1H), 1.78 (d, *J* = 9.3 Hz, 1H) |
| **17** | (3*S*,11a*R*)-7-((6-(4-fluoro-3-(trifluoromethyl)phenoxy)py ridin-3-yl)methoxy)-3,4-dihydro-1H,9H,11H-3,11a-methanopyrimido[6',1':2,3]i midazo[5, 1-c][1,4]oxazin-9-one | 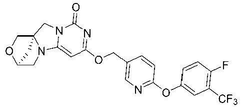 | B | 491 | CDCl₃: δ8.18 (br. s., 1H), 7.83 (d, *J* = 6.4 Hz, 1H), 7.45 - 7.29 (m, 1H), 7.26 - 7.12 (m, 1H), 6.96 (d, *J* = 8.3 Hz, 1H), 5.51 - 5.24 (m, 2H), 5.04 (s, 1H), 4.78 - 4.77 (m, 1H), 4.73 (br. s., 1H), 4.45 (d, *J* = 12.7 Hz, 1H), 4.09 - 3.82 (m, 3H), 3.56 (d, *J* = 9.8 Hz, 1H), 3.21 (d, *J* = 9.8 Hz, 1H), 2.03 (d, *J* = 8.8 Hz, 1H), 1.72 (d, *J* = 9.8 Hz, 1H) |
| **18** | 5-((((3*S*,11a*R*)-9-oxo-3,4-dihydro-1H,9H,11H-3,11a-methanopyrimido[6', 1':2,3]i midazo[5, 1-c][1,4]oxazin-7-yl)oxy)methyl)-2-(4-chloro-3-(trifluoromethyl)phenoxy)be nzonitrile | 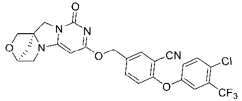 | A | 531 | DMSO-*d*₆: δ7.97 (s, 1H), 7.80 (d, *J =* 8.8 Hz, 1H), 7.72 (br. s., 2H), 7.50 (d, *J* = 6.4 Hz, 1H), 7.16 (d, *J* = 8.8 Hz, 1H), 5.29 (s, 2H), 5.26 (s, 1H), 4.67 (br. s., 1H), 4.27 (d, *J =* 12.2 Hz, 1H), 3.91 (d, *J* = 12.2 Hz, 1H), 3.88 - 3.74 (m, 2H), 3.30 - 3.19 (m, 2H), 1.89 (d, *J* = 10.3 Hz, 1H), 1.78 (d, *J =* 10.3 Hz, 1H) |
| **19** | 5-((((3*S*,11a*R*)-9-oxo-3,4-dihydro-1H,9H,11H-3,11a-methanopyrimido[6',1':2,3]i midazo[5,1-c][1,4]oxazin-7-yl)oxy)methyl)-2-(3-(trifluoromethyl)phenoxy)be nzonitrile | 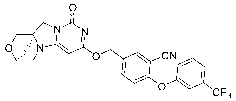 | B | 497 | CDCl₃: δ7.74 (br. s., 1H), 7.64 - 7.44 (m, 3H), 7.34 (br. s., 1H), 7.27 (br. s., 1H), 6.90 (d, *J* = 8.3 Hz, 1H), 5.52 - 5.31 (m, 2H), 5.09 (s, 1H), 4.75 (br. s., 1H), 4.46 (d, *J* = 12.2 Hz, 1H), 4.11 - 3.86 (m, 3H), 3.58 (d, *J* = 9.8 Hz, 1H), 3.24 (d, *J* = 9.8 Hz, 1H), 2.04 (d, *J* = 9.8 Hz, 1H), 1.75 (d, *J =* 9.8 Hz, 1H) |
| **20** | 2-(4-chloro-3-fluorophenoxy)-5-((((((3*S*,11a*R*)-9-oxo-3,4-dihydro-1H,9H,11H-3,11a-methanopyrimido[6',1':2,3]imidazo[5,1-c][1,4]oxazin-7-yl)oxy)methyl)benzonitrile | 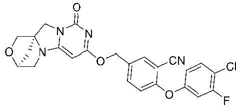 | B | 481 | DMSO-*d*₆: δ7.96 (br. s., 1H), 7.76 - 7.62 (m, 2H), 7.41 (d, *J* = 10.3 Hz, 1H), 7.15 (d, *J* = 8.8 Hz, 1H), 7.05 (d, *J =* 8.3 Hz, 1H), 5.35 - 5.20 (m, 3H), 4.67 (br. s., 1H), 4.27 (d, *J* = 12.2 Hz, 1H), 3.96 - 3.79 (m, 3H), 3.40 (br. s., 1H), 3.28 (br. s., 1H), 1.89 (d, *J* = 9.8 Hz, 1H), 1.78 (d, *J* = 9.8 Hz, 1H) |
| **21** | 5-((((((3*S*,11a*R*)-9-oxo-3,4-dihydro-1H,9H,11H-3,11a-methanopyrimido[6',1':2,3]i midazo[5,1-c][1,4]oxazin-7-yl)oxy)methyl)-2-(4-(trifluoromethyl)phenoxy)be nzonitrile | 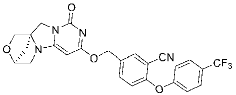 | B | 497 | DMSO-*d*₆: δ7.99 (br. s., 1H), 7.89 -7.70 (m, 3H), 7.31 (d, *J =* 8.3 Hz, 2H), 7.23 (d, *J* = 8.3 Hz, 1H), 5.40 - 5.19 (m, 3H), 4.68 (br. s., 1H), 4.28 (d, *J* = 12.2 Hz, 1H), 3.97 - 3.77 (m, 3H), 3.48 - 3.36 (m, 1H), 3.26 (br. s., 1H), 1.89 (d, *J* = 9.3 Hz, 1H), 1.78 (d, *J =* 9.8 Hz, 1H) |
| **22** | 5-((((3*S*,11a*R*)-9-oxo-3,4-dihydro-1H,9H,11H-3,11a-methanopyrimido[6', 1':2,3]i midazo[5, 1-c][1,4]oxazin-7-yl)oxy)methyl)-2-((2-(trifluoromethyl)pyridin-4-yl)oxy)benzonitrile | 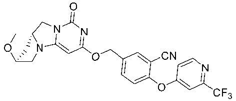 | B | 498 | CDCl₃: δ8.66 (d, *J* = 5.4 Hz, 1H), 7.81 (br. s., 1H), 7.72 (d, *J* = 8.3 Hz, 1H), 7.30 (br. s., 1H), 7.18 (d, *J* = 8.8 Hz, 1H), 7.05 (d, *J* = 3.4 Hz, 1H), 5.47 (d, *J =* 11.2 Hz, 2H), 5.12 (s, 1H), 4.76 (br. s., 1H), 4.48 (d, *J* = 12.2 Hz, 1H), 3.89 - 4.07 (m, 3H), 3.60 (d, *J* = 9.3 Hz, 1H), 3.26 (d, *J* = 9.8 Hz, 1H), 2.06 (d, *J* = 9.8 Hz, 1H), 1.76 (d, *J* = 9.8 Hz, 1H) |
| **23** | (3*S*,11a*R*)-7-(4-(4-chloro-3-(trifluoromethyl)phenoxy)ph enethoxy)-3,4-dihydro-1H,9H,11H-3,11a-methanopyrimido[6',1':2,3]i midazo[5,1-c][1,4]oxazin-9-one | 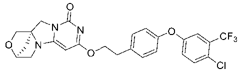 | A | 520 | DMSO-*d*₆: δ7.68 (d, *J* = 6.8 Hz, 1H), 7.40 (br. s., 1H), 7.39 - 7.27 (m, 2H), 7.24 (br. s., 1H), 7.06 (br. s., 2H), 5.14 (br. s., 1H), 4.65 (br. s., 1H), 4.39 (br. s., 2H), 4.26 (d, *J* = 11.7 Hz, 1H), 3.84 (d, *J* = 15.7 Hz, 3H), 3.25 (br. s., 2H), 2.97 (br. s., 2H), 1.87 (br. s., 1H), 1.80 - 1.65 (m, 1H) |
| **24** | (3*S*,11a*R*)-7-((3,4,5-trifluorobenzyl)oxy)-3,4-dihydro-1H,9H,11H-3,11a-methanopyrimido[6',1':2,3]i midazo[5,1-c][1,4]oxazin-9-one | 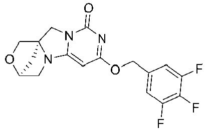 | A | 366 | DMSO-*d*₆: δ7.37 (br. s., 2H), 5.28 (br. s., 1H), 5.25 (br. s., 2H), 4.67 (br. s., 1H), 4.27 (d, *J* = 11.2 Hz, 1H), 3.98 - 3.73 (m, 3H), 3.29 - 3.15 (m, 2H), 1.88 (br. s., 1H), 1.79 (br. s., 1H) |
| **25** | (3*S*,11a*R*)-7-((3,4-trifluorobenzyl)oxy)-3,4-dihydro-1H,9H,11H-3,11a-methanopyrimido[6',1':2,3]i midazo[5,1-c][1,4]oxazin-9-one | 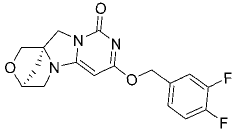 | A | 348 | DMSO-*d*₆: δ7.58 - 7.35 (m, 2H), 7.27 (br. s., 1H), 5.25 (br. s., 3H), 4.67 (br. s., 1H), 4.27 (d, *J* = 12.2 Hz, 1H), 4.00 - 3.71 (m, 3H), 3.33 - 3.12 (m, 2H), 1.89 (d, *J =* 9.8 Hz, 1H), 1.78 (d, *J =* 10.3 Hz, 1H) |
| **26** | (3*R*,11a*S*)-7-((3,5-difluoro-4-((2-(trifluoromethyl)pyridin-4-yl)oxy)benzyl)oxy)-3,4-dihydro-1H,9H,11H-3,11a-methanopyrimido[6',1':2,3]i midazo[5,1-c][1,4]oxazin-9-one | 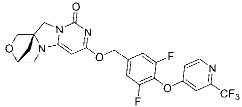 | A | 509 | DMSO-*d*₆: δ8.68 (d, *J* = 5.4 Hz, 1H), 7.67 (br. s., 1H), 7.45 (d, *J* = 9.3 Hz, 2H), 7.31 (d, *J* = 2.9 Hz, 1H), 5.34 (s, 2H), 5.32 (s, 1H), 4.68 (br. s., 1H), 4.29 (d, *J* = 12.2 Hz, 1H), 4.06 - 3.77 (m, 3H), 3.40 - 3.35 (m, 1H), 3.27 (br. s., 1H), 1.93 - 1.72 (m, 2H) |
| **27** | (3*R*,11a*S*)-7-((4-(4-chloro-3-(trifluoromethyl)phenoxy)-3,5-difluorobenzyl)oxy)-3,4-dihydro-1H,9H,11H-3,11a-methanopyrimido[6',1':2,3]i midazo[5,1-c][1,4]oxazin-9-one | 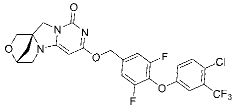 | B | 542 | DMSO-*d*₆: δ7.70 (d, *J* = 8.8 Hz, 1H), 7.52 (br. s., 1H), 7.40 (d, *J* = 8.8 Hz, 2H), 7.28 (d, *J =* 8.3 Hz, 1H), 5.38 - 5.26 (m, 3H), 4.68 (br. s., 1H), 4.28 (d, *J =* 12.2 Hz, 1H), 3.98 - 3.75 (m, 3H), 3.41 (br. s., 1H), 3.27 (br. s., 1H), 1.90 (d, *J =* 9.3 Hz, 1H), 1.79 (d, *J* = 9.8 Hz, 1H) |
| **28** | (3*R*,11a*S*)-7-((4-((2-chloropyridin-4-yl)oxy)-3,5-difluorobenzyl)oxy)-3,4-dihydro-1H,9H,11H-3,11a-methanopyrimido[6',1':2,3]i midazo[5,1-c][1,4]oxazin-9-one | 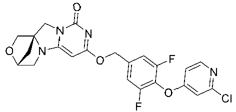 | B | 475 | CDCl₃: δ8.27 (br. s., 1H), 7.12 (d, *J* = 7.8 Hz, 2H), 6.85 (br. s., 2H), 5.42 (d, *J* = 6.4 Hz, 2H), 5.12 (br. s., 1H), 4.75 (br. s., 1H), 4.47 (d, *J* = 11.2 Hz, 1H), 4.02 - 3.90 (m, 3H), 3.59 (d, *J* = 9.3 Hz, 1H), 3.25 (d, *J* = 9.3 Hz, 1H), 2.05 (d, *J =* 9.3 Hz, 1H), 1.75 (d, *J* = 9.3 Hz, 1H) |
| **29** | (3*R*,11a*S*)-7-((3,5-difluoro-4-((5-(trifluoromethyl)pyridin-3-yl)oxy)benzyl)oxy)-3,4-dihydro-1H,9H,11H-3,11a-methanopyrimido[6',1':2,3]i midazo[5,1-c][1,4]oxazin-9-one | 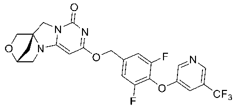 | B | 509 | CDCl₃: δ8.78 - 8.44 (m, 2H), 7.42 (br. s., 1H), 7.13 (d, *J* = 8.3 Hz, 2H), 5.52 - 5.35 (m, 2H), 5.12 (s, 1H), 4.75 (br. s., 1H), 4.47 (d, *J* = 12.2 Hz, 1H), 4.08 - 3.90 (m, 3H), 3.59 (d, *J* = 9.8 Hz, 1H), 3.25 (d, *J* = 9.8 Hz, 1H), 2.05 (d, *J* = 9.8 Hz, 1H), 1.75 (d, *J =* 10.3 Hz, 1H) |
| **30** | (3*R*,11a*S*)-7-((3,5-difluoro-4-((6-(trifluoromethyl)pyridin-3-yl)oxy)benzyl)oxy)-3,4-dihydro-1H,9H,11H-3,11a-methanopyrimido[6',1':2,3]i midazo[5,1-c][1,4]oxazin-9-one | 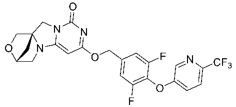 | B | 509 | CDCl₃: δ8.49 (br. s., 1H), 7.64 (d, *J* = 8.8 Hz, 1H), 7.30 (d, *J* = 8.3 Hz, 1H), 7.12 (d, *J* = 8.3 Hz, 2H), 5.50 - 5.33 (m, 2H), 5.12 (s, 1H), 4.75 (br. s., 1H), 4.47 (d, *J* = 12.7 Hz, 1H), 4.07 - 3.89 (m, 3H), 3.59 (d, *J* = 9.8 Hz, 1H), 3.25 (d, *J* = 9.8 Hz, 1H), 2.05 (d, *J* = 9.3 Hz, 1H), 1.75 (d, *J =* 10.3 Hz, 1H) |
| **31** | 3-(2-fluoro-4-(((((((3*R*,11a*S*)-9-oxo-3,4-dihydro-1H,9H,11H-3,11a-methanopyrimido[6',1':2,3]i midazo[5,1-c][1,4]oxazin-7-yl)oxy)methyl)phenoxy)-5-(trifluoromethyl)benzonitrile | 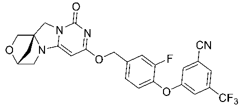 | B | 515 | DMSO-*d*₆: δ8.11 (br. s., 1H), 7.86 (br. s., 1H), 7.72 (br. s., 1H), 7.50 (d, *J =* 11.7 Hz, 1H), 7.39 - 7.30 (m, 2H), 5.39 - 5.17 (m, 3H), 4.68 (br. s., 1H), 4.28 (d, *J* = 11.7 Hz, 1H), 4.04 - 3.71 (m, 3H), 3.37 (br. s., 1H), 3.29 (br. s., 1H), 1.90 (d, *J* = 9.8 Hz, 1H), 1.78 (d, *J* = 9.8 Hz, 1H) |
| **32** | (3*R*,11a*S*)-7-((6-(4-fluoro-3-(trifluoromethyl)phenoxy)py ridin-3-yl)methoxy)-3,4-dihydro-1H,9H,11H-3,11a-methanopyrimido[6',1':2,3]i midazo[5, 1-c][1,4]oxazin-9-one | 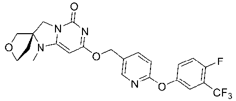 | B | 491 | DMSO-*d*₆: δ8.20 (br. s., 1H), 7.95 (d, *J* = 8.3 Hz, 1H), 7.66 - 7.53 (m, 3H), 7.14 (d, *J* = 8.8 Hz, 1H), 5.29 - 5.18 (m, 3H), 4.66 (br. s., 1H), 4.27 (d, *J* = 12.2 Hz, 1H), 3.94 - 3.79 (m, 3H), 3.39 (br. s., 1H), 3.27 (br. s., 1H), 1.91 - 1.75 (m, 2H) |
| **33** | 5-((((((3*R*,11a*S*)-9-oxo-3,4-dihydro-1H,9H,11H-3,11a-methanopyrimido[6', 1':2,3]i midazo[5, 1-c][1,4]oxazin-7-yl)oxy)methyl)-2-(3-(trifluoromethyl)phenoxy)be nzonitrile | 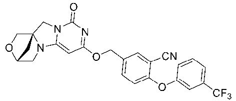 | B | 497 | DMSO-*d*₆: δ7.97 (br. s., 1H), 7.77 - 7.67 (m, 2H), 7.67 - 7.61 (m, 1H), 7.56 (br. s., 1H), 7.46 (d, *J* = 7.3 Hz, 1H), 7.10 (d, *J* = 8.3 Hz, 1H), 5.45 - 5.15 (m, 3H), 4.67 (br. s., 1H), 4.28 (d, *J* = 12.2 Hz, 1H), 3.96 - 3.77 (m, 3H), 3.57 - 3.41 (m, 1H), 3.28 - 3.22 (m, 1H), 1.89 (d, *J* = 9.3 Hz, 1H), 1.78 (d, *J* = 9.8 Hz, 1H) |
| **34** | 5-((((3*R*,11a*S*)-9-oxo-3,4-dihydro-1H,9H,11H-3,11a-methanopyrimido[6', 1':2,3]i midazo[5, 1-c][1,4]oxazin-7-yl)oxy)methyl)-2-(4-(trifluoromethyl)phenoxy)be nzonitrile | 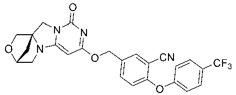 | B | 497 | CDCl₃: δ7.83 - 7.51 (m, 4H), 7.16 (br. s., 2H), 6.97 (br. s., 1H), 5.41 (d, *J* = 13.2 Hz, 2H), 5.09 (br. s., 1H), 4.75 (br. s., 1H), 4.47 (d, *J* = 11.7 Hz, 1H), 4.12 - 3.89 (m, 3H), 3.58 (d, *J =* 8.3 Hz, 1H), 3.25 (d, *J* = 8.3 Hz, 1H), 2.05 (d, *J* = 7.8 Hz, 1H), 1.75 (d, *J =* 8.3 Hz, 1H) |
| **35** | (3*R*,11a*S*)-7-((3,4,5-trifluorobenzyl)oxy)-3,4-dihydro-1H,9H,11H-3,11a-methanopyrimido[6',1':2,3]i midazo[5,1-c][1,4]oxazin-9-one | 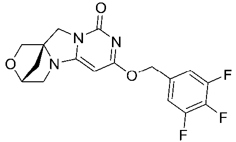 | A | 366 | DMSO-*d*₆: δ7.38 (t, *J* = 7.6 Hz, 2H), 5.28 (s, 1H), 5.25 (s, 2H), 4.67 (br. s., 1H), 4.27 (d, *J* = 12.2 Hz, 1H), 3.95 - 3.78 (m, 3H), 3.39 - 3.37 (m, 1H), 3.28 - 3.25 (m, 1H), 1.93 - 1.74 (m, 2H) |
| **36** | 3-((3,5-difluoro-4-((2-(trifluoromethyl)pyridin-4-yl)oxy)benzyl)oxy)-7,8-dihydro-1H,6H,9H-7,8a-methanopyrrolo[1',2': 3,4] imi dazo[1,2-c]pyrimidin-1-one | 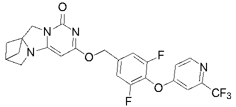 | B | 493 | DMSO-*d*₆: δ8.69 (br. s., 1H), 7.66 (br. s., 1H), 7.45 (d, *J* = 9.3 Hz, 2H), 7.31 (br. s., 1H), 5.33 (br. s., 3H), 3.87 (br. s., 2H), 2.95 (br. s., 1H), 2.05 (br. s., 2H), 1.58 (br. s., 2H), 1.23 (br. s., 2H) |
| **37** | 3-((3,5-difluoro-4-((2-(trifluoromethoxy)pyridin-4-yl)oxy)benzyl)oxy)-7,8-dihydro-1H,6H,9H-7,8a-methanopyrrolo[1',2':3,4]imi dazo[1,2-c]pyrimidin-1-one | 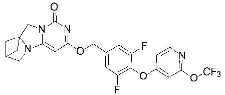 | B | 509 | DMSO-*d*₆: δ8.27 (d, *J* = 5.4 Hz, 1H), 7.38 (d, *J* = 8.8 Hz, 2H), 7.05 (d, *J* = 4.4 Hz, 1H), 6.93 (br. s., 1H), 5.30 (br. s., 3H), 3.84 (s, 2H), 3.30 (s, 2H), 2.91 (br. s., 1H), 2.02 (br. s., 2H), 1.55 (br. s., 2H) |
| **38** | 3-((4-((2-chloropyridin-4-yl)oxy)-3,5-difluorobenzyl)oxy)-7,8-dihydro-1H,6H,9H-7,8a-methanopyrrolo[1',2': 3,4] imi dazo[1,2-c]pyrimidin-1-one | 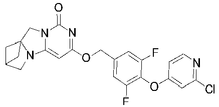 | B | 459 | DMSO-*d*₆: δ8.60 - 8.40 (m, 1H), 7.41 (br. s., 2H), 7.24 (br. s., 1H), 7.08 (br. s., 1H), 5.32 (br. s., 3H), 3.87 (br. s., 2H), 3.30 (br. s., 2H), 2.94 (br. s., 1H), 2.04 (br. s., 2H), 1.58 (br. s., 2H) |
| **39** | 3-((3,5-difluoro-4-((2-(trifluoromethyl)pyrimidin-5-yl)oxy)benzyl)oxy)-7,8-dihydro-1H,6H,9H-7,8a-methanopyrrolo[1',2': 3,4] imi dazo[1,2-c]pyrimidin-1-one | 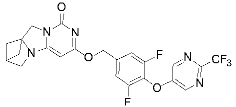 | B | 494 | DMSO-*d*₆: δ8.92 (s, 2H), 7.40 (d, *J* = 9.3 Hz, 2H), 5.34 - 5.27 (m, 3H), 3.85 (s, 2H), 3.31 (br. s., 2H), 2.92 (br. s., 1H), 2.02 (br. s., 2H), 1.55 (br. s., 2H) |
| **40** | 3-((3,5-difluoro-4-((5-(trifluoromethylpyridin-3-yl)oxy)benzyl)oxy)-7,8-dihydro-1H,6H,9H-7,8a-methanopyrrolo[1',2': 3,4] imi dazo[1,2-c]pyrimidin-1-one | 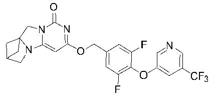 | B | 493 | DMSO-*d*₆: δ8.72 (s, 1H), 8.77 (s, 1H), 7.90 (br. s., 1H), 7.39 (d, *J* = 8.8 Hz, 2H), 5.30 (br. s., 3H), 3.85 (br. s., 2H), 3.15 (br. s., 2H), 2.93 (br. s., 1H), 2.02 (br. s., 2H), 1.56 (br. s., 2H) |
| **41** | 2-(4-chloro-3-(trifluoromethyl)phenoxy)-5-(((((1-oxo-7,8-dihydro-1H,6H,9H-7,8a-methanopyrrolo[1',2': 3,4] imi dazo[1,2-c]pyrimidin-3-yl)oxy)methyl)benzonitrile | 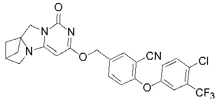 | B | 515 | DMSO-*d*₆: δ7.95 (br. s., 1H), 7.78 (d, *J* = 8.8 Hz, 1H), 7.74 - 7.67 (m, 2H), 7.48 (d, *J* = 8.8 Hz, 1H), 7.14 (d, *J =* 8.8 Hz, 1H), 5.26 (br. s., 3H), 3.84 (s, 2H), 3.29 (br. s., 2H), 2.92 (br. s., 1H), 2.02 (br. s., 2H), 1.55 (br. s., 2H) |
| **42** | 5-((((1-oxo-7,8-dihydro-1H,6H,9H-7,8a-methanopyrrolo[1',2':3,4]imi dazo[1,2-c]pyrimidin-3-yl)oxy)methyl)-2-(3-(trifluoromethyl)phenoxy)be nzonitrile | 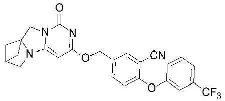 | B | 481 | DMSO-*d*₆: δ7.95 (s, 1H), 7.74 - 7.64 (m, 2H), 7.64 - 7.58 (m, 1H), 7.55 (s, 1H), 7.45 (d, *J* = 7.8 Hz, 1H), 7.08 (d, *J* = 8.8 Hz, 1H), 5.27 (s, 3H), 3.84 (s, 2H), 3.29 (s, 2H), 2.92 (br. s., 1H), 2.01 (br. s., 2H), 1.55 (d, *J =* 3.4 Hz, 2H) |
| **43** | 5-((((1-oxo-7,8-dihydro-1H,6H,9H-7,8a-methanopyrrolo[1',2':3,4]imi dazo[1,2-c]pyrimidin-3-yl)oxy)methyl)-2-((2-(trifluoromethyl)pyridin-4-yl)oxy)benzonitrile | 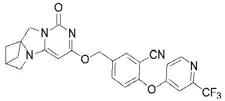 | B | 482 | CDCl₃: δ8.65 (d, *J* = 5.9 Hz, 1H), 7.81 (d, *J* = 1.5 Hz, 1H), 7.71 (dd, *J* = 2.0, 8.8 Hz, 1H), 7.29 (d, *J* = 2.4 Hz, 1H), 7.16 (d, *J* = 8.3 Hz, 1H), 7.04 (dd, *J* = 2.4, 5.4 Hz, 1H), 5.46 (s, 2H), 5.15 (s, 1H), 4.00 (s, 2H), 3.35 (s, 2H), 3.04 (br. s., 1H), 2.08 (br. s., 2H), 1.70 (dd, *J* = 1.5, 4.9 Hz, 2H) |
| **44** | 3-((2-fluorobenzyl)oxy)-7,8-dihydro-1H,6H,9H-7,8a-methanopyrrolo[1',2':3,4]imi dazo[1,2-c]pyrimidin-1-one | 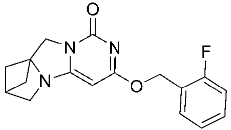 | B | 314 | DMSO-*d*₆: δ7.47 (t, *J* = 7.3 Hz, 1H), 7.43 - 7.35 (m, 1H), 7.25 - 7.16 (m, 2H), 5.28 (s, 2H), 5.25 (s, 1H), 3.84 (s, 2H), 3.29 (s, 2H), 2.92 (br. s., 1H), 2.01 (br. s., 2H), 1.57 - 1.52 (m, 2H) |
| **45** | 3-((2,3-difluorobenzyl)oxy)-7,8-dihydro-1H,6H,9H-7,8a-methanopyrrolo[1',2': 3,4] imi dazo[1,2-c]pyrimidin-1-one | 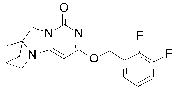 | B | 332 | DMSO-*d*₆: δ7.41 (q, *J* = 8.8 Hz, 1H), 7.33 - 7.26 (m, 1H), 7.25 - 7.17 (m, 1H), 5.32 (s, 2H), 5.26 (s, 1H), 3.84 (s, 2H), 3.29 (br. s., 2H), 2.92 (br. s., 1H), 2.01 (br. s., 2H), 1.56 (br. s., 2H) |
| **46** | 3-((3,5-difluoro-4-((2-(trifluoromethyl)pyridin-4-yl)oxy)benzyl)oxy)-7,8-dihydro-1H,6H,9H-6,8a-ethanopyrrolo[1',2':3,4]imid azo[1,2-c]pyrimidin-1-one | 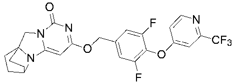 | B | 507 | DMSO-*d*₆: δ8.68 (d, *J* = 4.9 Hz, 1H), 7.66 (br. s., 1H), 7.46 (d, *J* = 9.3 Hz, 2H), 7.30 (br. s., 1H), 5.49 (s, 1H), 5.32 (br. s., 2H), 4.30 (br. s., 1H), 3.89 (br. s., 2H), 1.94 (br. s., 2H), 1.88-1.38 (m, 6H) |
| **47** | 3-((3,5-difluoro-4-((2-(trifluoromethyl)pyrimidin-5-yl)oxy)benzyl)oxy)-7,8-dihydro-1H,6H,9H-6,8a-ethanopyrrolo[1',2':3,4]imid azo[1,2-c]pyrimidin-1-one | 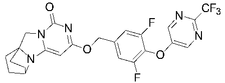 | B | 508 | DMSO-*d*₆: δ8.96 (br. s., 2H), 7.45 (br. s., 2H), 5.48 (br. s., 1H), 5.31 (br. s., 2H), 4.30 (br. s., 1H), 3.89 (br. s., 2H), 1.93 (br. s., 2H), 1.89-1.23 (m, 6H) |
| **48** | 3-((3,5-difluoro-4-((1-methyl-1H-pyrazol-4-yl)oxy)benzyl)oxy)-7,8-dihydro-1H,6H,9H-6,8a-ethanopyrrolo[1',2': 3,4] imid azo[1,2-c]pyrimidin-1-one | 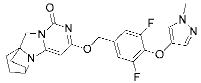 | B | 442 | DMSO-*d*₆: δ7.59 (s, 1H), 7.41 - 7.11 (m, 3H), 5.45 (s, 1H), 5.25 (br. s., 2H), 4.28 (br. s., 1H), 3.88 (s, 2H), 3.72 (s, 3H), 1.92 (br. s., 2H), 1.72 (d, *J* = 5.9 Hz, 4H), 1.64 (d, *J* = 10.3 Hz, 2H) |
| **49** | 3-((6-(4-fluoro-3-(trifluoromethyl)phenoxy)py ridin-3-yl)methoxy)-7,8-dihydro-1H,6H,9H-6,8a-ethanopyrrolo[1',2': 3,4] imid azo[1,2-c]pyrimidin-1-one | 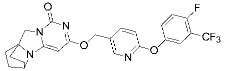 | B | 489 | DMSO-*d*₆: δ8.20 (br. s., 1H), 7.95 (d, *J* = 8.3 Hz, 1H), 7.69 - 7.42 (m, 3H), 7.14 (d, *J* = 8.8 Hz, 1H), 5.39 (s, 1H), 5.24 (br. s., 2H), 4.26 (br. s., 1H), 3.87 (br. s., 2H), 1.92 (br. s., 2H), 1.82 - 1.67 (m, 4H), 1.64 (d, *J* = 10.3 Hz, 2H) |
| **50** | 5-(((1-oxo-7,8-dihydro-1H,6H,9H-6,8a-ethanopyrrolo[1',2':3,4]imid azo[1,2-c]pyrimidin-3-yl)oxy)methyl)-2-(4-chloro-3-(trifluoromethyl)phenoxy)be nzonitrile | 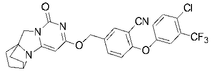 | B | 529 | DMSO-*d*₆: δ7.97 (br. s., 1H), 7.79 (d, *J* = 8.8 Hz, 1H), 7.73 (d, *J* = 9.3 Hz, 1H), 7.70 (br. s., 1H), 7.49 (d, *J* = 8.3 Hz, 1H), 7.15 (d, *J* = 8.8 Hz, 1H), 5.43 (s, 1H), 5.27 (s, 2H), 4.27 (br. s., 1H), 3.88 (s, 2H), 1.92 (br. s., 2H), 1.83 - 1.67 (m, 4H), 1.64 (d, *J =* 11.2 Hz, 2H) |
| **51** | 5-((((1-oxo-7,8-dihydro-1H,6H,9H-6,8a-ethanopyrrolo[1',2':3,4]imid azo[1,2-c]pyrimidin-3-yl)oxy)methyl)-2-((2-(trifluoromethyl)pyridin-4-yl)oxy)benzonitrile | 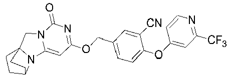 | B | 496 | DMSO-*d*₆: δ8.72 (d, *J* = 5.4 Hz, 1H), 8.06 (s, 1H), 7.85 (d, *J =* 8.8 Hz, 1H), 7.69 (s, 1H), 7.50 (d, *J =* 8.3 Hz, 1H), 7.36 (d, *J* = 3.4 Hz, 1H), 5.47 (s, 1H), 5.33 (s, 2H), 4.29 (br. s., 1H), 3.89 (s, 2H), 1.93 (br. s., 2H), 1.80 - 1.58 (m, 6H) |
| **52** | 3-((3,4,5-trifluorobenzyl)oxy)-7,8-dihydro-1H,6H,9H-6,8a-ethanopyrrolo[1',2':3,4]imid azo[1,2-c]pyrimidin-1-one | 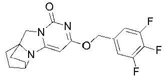 | B | 364 | DMSO-*d*₆: δ7.39 (d, *J* = 6.8 Hz, 2H), 5.46 (d, *J* = 5.4 Hz, 1H), 5.22 (br. s., 2H), 4.28 (br. s., 1H), 3.87 (br. s., 2H), 1.93 (br. s., 2H), 1.73 (br. s., 4H), 1.65 (br. s., 2H) |
| **53** | 3-((3,5-difluoro-4-((2-(trifluoromethyl)pyridin-4-yl)oxy)benzyl)oxy)-6,7,8,9-tetrahydro-1H,10H-7,9a-ethanopyrido[1',2':3,4]imida zo[1,2-c]pyrimidin-1-one | 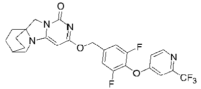 | B | 521 | CDCl₃: δ8.61 (d, *J* = 4.9 Hz, 1H), 7.31 - 7.27 (m, 1H), 7.14 (d, *J* = 8.8 Hz, 2H), 6.99 (br. s., 1H), 5.43 (s, 2H), 4.97 (s, 1H), 3.78 (s, 2H), 3.37 (br. s., 2H), 2.15 - 1.73 (m, 9H) |
| **54** | 3-((3,5-difluoro-4-((2-(trifluoromethyl)pyridin-4-yl)oxy)benzyl)oxy)-8a-methyl-7,8,8a,9-tetrahydro-1H,6H-pyrrolo[1',2':3,4]imidazo[1,2 -c]pyrimidin-1-one | 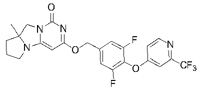 | B | 495 | DMSO-*d*₆: δ8.68 (d, *J* = 5.1 Hz, 1H), 7.66 (br. s., 1H), 7.45 (d, *J* = 9.1 Hz, 2H), 7.30 (br. s., 1H), 5.43 - 5.25 (m, 3H), 4.03 (d, *J* = 11.6 Hz, 1H), 3.73 (d, *J =* 11.6 Hz, 1H), 3.38 - 3.35 (m, 1H), 3.30 - 3.26 (m, 1H), 1.98 (br. s., 2H), 1.87 - 1.76 (m, 1H), 1.65 (d, *J* = 11.0 Hz, 1H), 1.31 (s, 3H) |
| **55** | 3-((3,5-difluoro-4-((2-(trifluoromethoxy)pyridin-4-yl)oxy)benzyl)oxy)-8a-methyl-7,8,8a,9-tetrahydro-1H,6H-pyrrolo[1',2':3,4]imidazo[1,2 -c]pyrimidin-1-one | 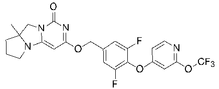 | B | 511 | CDCl₃: δ8.21 (d, *J* = 5.4 Hz, 1H), 7.11 (d, *J* = 7.8 Hz, 2H), 6.82 (br. s., 1H), 6.50 (br. s., 1H), 5.41 (d, *J* = 4.9 Hz, 2H), 5.13 (br. s., 1H), 4.17 (d, *J* = 11.7 Hz, 1H), 3.96 - 3.79 (m, 1H), 3.53 - 3.23 (m, 2H), 2.12 (br. s., 2H), 1.94 (br. s., 1H), 1.68 - 1.59 (m, 1H), 1.39 (br. s., 3H) |
| **56** | 3-((3,5-difluoro-4-((2-(trifluoromethyl)pyrimidin-5-yl)oxy)benzyl)oxy)-8a-methyl-7,8,8a,9-tetrahydro-1H,6H-pyrrolo[1',2':3,4]imidazo[1,2 -c]pyrimidin-1-one | 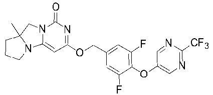 | B | 496 | CDCl₃: δ8.56 (br. s., 2H), 7.15 (d, *J* = 8.3 Hz, 2H), 5.50 - 5.33 (m, 2H), 5.13 (s, 1H), 4.17 (d, *J* = 11.7 Hz, 1H), 3.88 (d, *J =* 11.7 Hz, 1H), 3.51 - 3.26 (m, 2H), 2.13 (br. s., 2H), 1.94 (d, *J* = 2.9 Hz, 1H), 1.76 - 1.67 (m, 1H), 1.40 (br. s., 3H) |
| **57** | 3-((3,5-difluoro-4-((1-methyl-1H-pyrazol-4-yl)oxy)benzyl)oxy)-8a-methyl-7,8,8a,9-tetrahydro-1H,6Hpyrrolo[1',2':3,4]imid azo[1,2-c]pyrimidin-1-one | 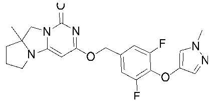 | B | 430 | CDCl₃: δ7.27 (s, 1H), 7.18 (s, 1H), 7.02 (d, *J* = 8.3 Hz, 2H), 5.43 - 5.28 (m, 2H), 5.10 (s, 1H), 4.16 (d, *J* = 12.2 Hz, 1H), 3.86 (d, *J* = 12.2 Hz, 1H), 3.81 (s, 3H), 3.47 - 3.38 (m, 1H), 3.37 - 3.24 (m, 1H), 2.10 (d, *J* = 8.8 Hz, 2H), 1.94 (d, *J =* 8.8 Hz, 1H), 1.71 (br. s., 1H), 1.39 (s, 3H) |
| **58** | 3-((6-(4-fluoro-3-(trifluoromethyl)phenoxy)py ridin-3-yl)methoxy)-8a-methyl-7,8,8a,9-tetrahydro-1H,6H-pyrrolo[1',2':3,4]imidazo[1,2 -c]pyrimidin-1-one | 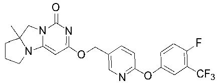 | B | 477 | CDCl₃: δ8.19 (br. s., 1H), 7.84 (d, *J* = 7.8 Hz, 1H), 7.45 - 7.29 (m, 2H), 7.26 - 7.18 (m, 1H), 6.96 (d, *J* = 8.3 Hz, 1H), 5.45 - 5.31 (m, 2H), 5.05 (s, 1H), 4.16 (d, *J* = 12.2 Hz, 1H), 3.86 (d,*J* = 12.2 Hz, 1H), 3.50 - 3.23 (m, 2H), 2.10 (br. s., 2H), 1.94 (d, *J* = 9.8 Hz, 1H), 1.77 - 1.67 (m, 1H), 1.38 (s, 3H) |
| **59** | 5-(((8a-methyl-1-oxo-7,8,8a,9-tetrahydro-1H,6H-pyrrolo[1',2':3,4]imidazo[1,2 -c]pyrimidin-3-yl)oxy)methyl)-2-(3-(trifluoromethyl)phenoxy)be nzonitrile | 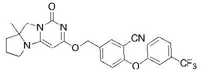 | B | 483 | CDCl₃: δ7.74 (br. s., 1H), 7.44 - 7.62 (m, 3H), 7.34 (br. s., 1H), 7.25 (br. s., 1H), 6.90 (d, *J* = 8.3 Hz, 1H), 5.31 - 5.48 (m, 2H), 5.10 (s, 1H), 4.16 (d, *J* = 11.7 Hz, 1H), 3.87 (d, *J* = 12.2 Hz, 1H), 3.39 - 3.49 (m, 1H), 3.26 - 3.36 (m, 1H), 2.05 - 2.19 (m, 2H), 1.89 - 1.99 (m, 1H), 1.70 - 1.75 (m, 1H), 1.39 (s, 3H) |
| **60** | 5-(((8a-methyl-1-oxo-7,8,8a,9-tetrahydro-1H,6H-pyrrolo[1',2':3,4]imidazo[1,2 -c]pyrimidin-3-yl)oxy)methyl)-2-(4-(trifluoromethyl)phenoxy)be nzonitrile | 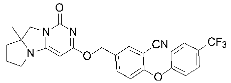 | B | 483 | CDCl₃: δ7.75 (s, 1H), 7.66 (d, *J =* 8.3 Hz, 2H), 7.58 (d, *J* = 8.8 Hz, 1H), 7.15 (d, *J* = 8.3 Hz, 2H), 6.96 (d, *J =* 8.3 Hz, 1H), 5.40 (q, *J* = 12.7 Hz, 2H), 5.10 (s, 1H), 4.16 (d, *J* = 11.7 Hz, 1H), 3.87 (d, *J* = 11.7 Hz, 1H), 3.51 - 3.25 (m, 2H), 2.11 (d, *J* = 8.3 Hz, 2H), 2.01 - 1.89 (m, 1H), 1.76 - 1.66 (m, 1H), 1.39 (s, 3H) |
| **61** | 3-(4-(4-chloro-3-(trifluoromethyl)phenoxy)ph enethoxy)-8a-methyl-7,8,8a,9-tetrahydro-1H,6H-pyrrolo[1',2':3,4]imidazo[1,2 -c]pyrimidin-1-one | 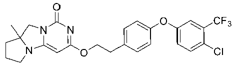 | B | 506 | DMSO-*d*₆: δ7.68 (d, *J* = 8.8 Hz, 1H), 7.40 (br. s., 1H), 7.37 - 7.31 (m, 2H), 7.23 (d, *J* = 8.8 Hz, 1H), 7.10 - 7.02 (m, 2H), 5.14 (s, 1H), 4.65 (s, 1H), 4.38 (d, *J* = 4.4 Hz, 2H), 4.24 (d, *J =* 12.2 Hz, 1H), 3.89 - 3.79 (m, 3H), 3.39 (d, *J =* 3.9 Hz, 1H), 3.25 - 3.21 (m, 1 H), 2.96 (t, *J =* 6.6 Hz, 2H), 1.87 (d, *J* = 9.8 Hz, 1H), 1.74 (d, *J* = 10.3 Hz, 1H) |
| **62** | 3-((3,4-difluorobenzyl)oxy)-8a-methyl-7,8,8a,9-tetrahydro-1H,6H-pyrrolo[1',2':3,4]imidazo[1,2 -c]pyrimidin-1-one | 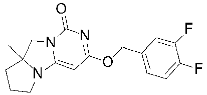 | B | 334 | CDCl₃: δ7.26 - 7.21 (m, 1H), 7.17 - 7.09 (m, 2H), 5.34 (d, *J* = 9.3 Hz, 2H), 5.08 (s, 1H), 4.16 (d, *J =* 11.7 Hz, 1H), 3.86 (d,*J* = 11.7 Hz, 1H), 3.49 - 3.24 (m, 2H), 2.09 (d, *J* = 8.8 Hz, 2H), 1.92 (d, *J* = 2.9 Hz, 1H), 1.65 (br. s., 1H), 1.38 (s, 3H) |
| **63** | 3-((3,5-difluoro-4-((2-(trifluoromethyl)pyridin-4-yl)oxy)benzyl)oxy)-8a-ethyl-7,8,8a,9-tetrahydro-1H,6H-pyrrolo[1',2':3,4]imidazo[1,2 -c]pyrimidin-1-one | 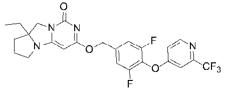 | B | 509 | DMSO-*d*₆: δ8.68 (d, *J* = 5.4 Hz, 1H), 7.66 (br. s., 1H), 7.45 (d, *J* = 9.3 Hz, 2H), 7.31 (br. s., 1H), 5.37 (s, 1H), 5.32 (br. s., 2H), 3.95 - 3.77 (m, 2H), 3.39 (br. s., 1H), 3.26 (br. s., 1H), 2.05 - 1.85 (m, 3H), 1.70 - 1.51 (m, 3H), 0.86 (t, *J* = 6.8 Hz, 3H) |
| **64** | 3-((3,5-difluoro-4-((2-(trifluoromethyl)pyrimidin-5-yl)oxy)benzyl)oxy)-8a-ethyl-7,8,8a,9-tetrahydro-1H,6H-pyrrolo[1',2':3,4]imidazo[1,2 -c]pyrimidin-1-one | 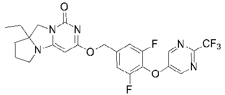 | B | 510 | CDCl₃: δ8.56 (br. s., 2H), 7.15 (d, *J* = 7.8 Hz, 2H), 5.50 - 5.35 (m, 2H), 5.14 (br. s., 1H), 4.07 - 3.94 (m, 2H), 3.36 (d, *J* = 5.4 Hz, 2H), 2.07 (br. s., 3H), 1.73 - 1.64 (m, 3H), 0.97 (br. s., 3H) |
| **65** | 3-(4-((((8a-ethyl-1-oxo-7,8,8a,9-tetrahydro-1H,6H-pyrrolo[1',2':3,4]imidazo[1,2 -c]pyrimidin-3-yl)oxy)methyl)-2-fluorophenoxy)-5-(trifluoromethyl)benzonitrile | 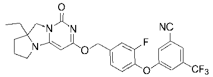 | B | 515 | DMSO-*d*₆: δ8.11 (br. s., 1H), 7.86 (br. s., 1H), 7.72 (br. s., 1H), 7.50 (d, *J* = 11.2 Hz, 1H), 7.45 - 7.18 (m, 2H), 5.55 - 5.04 (m, 3H), 4.03 - 3.63 (m, 2H), 3.38 (br. s., 1H), 3.27 (br. s., 1H), 1.92 (br. s., 3H), 1.70 - 1.52 (m, 3H), 0.86 (t, *J* = 7.1 Hz, 3H) |
| **66** | 3-((3,5-difluoro-4-((2-(trifluoromethyl)pyridin-4-yl)oxy)benzyl)oxy)-9a-methyl-6,7,8,9,9a,10-hexahydro-1H-pyrido[1',2':3,4]imidazo[1,2-c]pyrimidin-1-one | 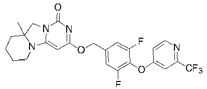 | B | 509 | DMSO-*d*₆: δ8.66 (d, *J* = 4.9 Hz, 1H), 7.64 (br. s., 1H), 7.42 (d, *J* = 9.3 Hz, 2H), 7.28 (br. s., 1H), 5.30 (br. s., 3H), 3.78 - 3.64 (m, 2H), 3.52 (d, *J =* 12.7 Hz, 1H), 3.12 (t, *J* = 13.0 Hz, 1H), 1.78 - 1.46 (m, 5H), 1.37 (br. s., 4H) |
| **67** | 3-((3,5-difluoro-4-((2-(trifluoromethyl)pyrimidin-5-yl)oxy)benzyl)oxy)-9a-methyl-6,7,8,9,9a,10-hexahydro-1H-pyrido[1',2':3,4]imidazo[1,2-c]pyrimidin-1-one | 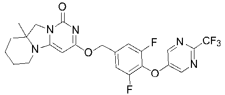 | B | 510 | DMSO-*d*₆: δ8.96 (s, 2H), 7.43 (d, *J* = 9.3 Hz, 2H), 5.31 (br. s., 3H), 3.71 (br. s., 2H), 3.54 (d, *J* = 12.2 Hz, 1H), 3.13 (t, *J* = 12.5 Hz, 1H), 1.73 - 1.53 (m, 5H), 1.39 (br. s., 4H) |
| **68** | 3-((3,5-difluoro-4-((1-methyl-1H-pyrazol-4-yl)oxy)benzyl)oxy)-9a-methyl-6,7,8,9,9a,10-hexahydro-1H-pyrido[1',2':3,4]imidazo[1,2-c]pyrimidin-1-one | 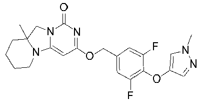 | B | 444 | DMSO-*d*₆: δ7.59 (br. s., 1H), 7.37 - 7.19 (m, 3H), 5.36 - 5.21 (m, 3H), 3.84 - 3.64 (m, 5H), 3.53 (d, *J* = 12.7 Hz, 1H), 3.12 (t, *J =* 12.7 Hz, 1H), 1.79 - 1.52 (m, 5H), 1.38 (br. s., 4H) |
| **69** | 3-((6-(4-fluoro-3-(trifluoromethyl)phenoxy)py ridin-3-yl)methoxy)-9a-methyl-6,7,8,9,9a, 10-hexahydro-1H-pyrido[1,2':3,4]imidazo[1,2-c]pyrimidin-1-one | 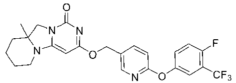 | B | 491 | DMSO-*d*₆: δ8.19 (br. s., 1H), 7.94 (d, *J =* 8.3 Hz, 1H), 7.67 - 7.50 (m, 3H), 7.19 - 7.07 (m, 1H), 5.34 - 5.15 (m, 3H), 3.78 - 3.63 (m, 2H), 3.50 (d, *J =* 12.7 Hz, 1H), 3.10 (t, *J* = 13.0 Hz, 1H), 1.79 - 1.47 (m, 5H), 1.37 (br. s., 4H) |
| **70** | 2-(4-chloro-3-(trifluoromethyl)phenoxy)-5-(((-9a-methyl-1-oxo-6,7,8,9,9a,10-hexahydro-1H-pyrido[1',2':3,4]imidazo[1,2-c]pyrimidin-3-yl)oxy)methyl)benzonitrile | 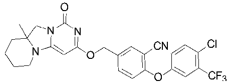 | B | 531 | DMSO-*d*₆: δ7.96 (br. s., 1H), 7.79 (br. s., 1H), 7.71 (br. s., 2H), 7.49 (br. s., 1H), 7.16 (d, *J =* 7.3 Hz, 1H), 5.27 (br. s., 3H), 3.69 (br. s., 2H), 3.50 (br. s., 1H), 3.14 (d, *J* = 13.2 Hz, 1H), 1.75 - 1.50 (m, 5H), 1.38 (br. s., 4H) |
| **71** | 5-(((-9a-methyl-1-oxo-6,7,8,9,9a,10-hexahydro-1H-pyrido[1',2':3,4]imidazo[1,2-c]pyrimidin-3-yl)oxy)methyl)-2-(3-(trifluoromethyl)phenoxy)be nzonitrile | 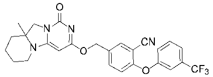 | B | 497 | DMSO-*d*₆: δ7.96 (br. s., 1H), 7.80 - 7.38 (m, 5H), 7.10 (d, *J =* 8.3 Hz, 1H), 5.36 - 5.19 (m, 3H), 3.83 - 3.60 (m, 2H), 3.52 (d, *J =* 12.7 Hz, 1H), 3.11 (t, *J* = 12.7 Hz, 1H), 1.79 - 1.51 (m, 5H), 1.38 (br. s., 4H) |
| **72** | 2-(4-chloro-3-fluorophenoxy)-5-(((-9a-methyl-1-oxo-6,7,8,9,9a,10-hexahydro-1H-pyrido[1',2':3,4]imidazo[1,2-c]pyrimidin-3-yl)oxy)methyl)benzonitrile | 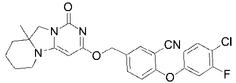 | B | 481 | DMSO-*d*₆: δ7.95 (br. s., 1H), 7.78 - 7.59 (m, 2H), 7.40 (d, *J* = 9.3 Hz, 1H), 7.15 (d, *J* = 8.3 Hz, 1H), 7.04 (d, *J* = 7.8 Hz, 1H), 5.41 - 5.08 (m, 3H), 3.69 (br. s., 2H), 3.52 (d, *J* = 12.2 Hz, 1H), 3.12 (t, *J* = 12.2 Hz, 1H), 1.75 - 1.50 (m, 5H), 1.38 (br. s., 4H) |
| **73** | 3-(4-(4-chloro-3-(trifluoromethyl)phenoxy)ph enethoxy)-9a-methyl-6,7,8,9,9a,10-hexahydro-1H-pyrido[1',2':3,4]imidazo[1,2-c]pyrimidin-1-one | 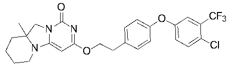 | B | 520 | DMSO-*d*₆: δ7.69 (d, *J* = 8.3 Hz, 1H), 7.18 - 7.48 (m, 4H), 7.07 (d, *J* = 7.8 Hz, 2H), 5.16 (s, 1H), 4.37 (br. s., 2H), 3.59 - 3.78 (m, 2H), 3.51 (d, *J =* 12.7 Hz, 1H), 3.09 (t, *J* = 13.0 Hz, 1H), 2.96 (br. s., 2H), 1.46 - 1.68 (m, 5H), 1.37 (br. s., 4H) |
| **74** | 3-((3,4-difluorobenzyl)oxy)-9a-methyl-6,7,8,9,9a,10-hexahydro-1H-pyrido[1',2':3,4]imidazo[1,2-c]pyrimidin-1-one | 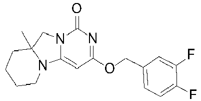 | B | 348 | DMSO-*d*₆: δ7.49 - 7.39 (m, 2H), 7.26 (br. s., 1H), 5.35 - 5.16 (m, 3H), 3.78 - 3.59 (m, 2H), 3.52 (d, *J* = 13.7 Hz, 1H), 3.11 (t, *J* = 13.0 Hz, 1H), 1.76 - 1.51 (m, 5H), 1.44 - 1.30 (m, 4H) |
| **75** | 3-((3,5-difluoro-4-((2-(trifluoromethyl)pyridin-4-yl)oxy)benzyl)oxy)-9a-ethyl-6,7,8,9,9a,10-hexahydro-1H-pyrido[1',2':3,4]imidazo[1,2-c]pyrimidin-1-one | 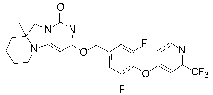 | B | 523 | DMSO-*d*₆: δ8.66 (d, *J* = 4.9 Hz, 1H), 7.64 (br. s., 1H), 7.43 (d, *J* = 9.3 Hz, 2H), 7.29 (br. s., 1H), 5.41 - 5.21 (m, 3H), 3.75 (d, *J* = 11.7 Hz, 1H), 3.63 - 3.49 (m, 2H), 3.04 (t, *J* = 13.0 Hz, 1H), 1.93 - 1.82 (m, 1H), 1.76 - 1.46 (m, 6H), 1.35 (d, *J* = 13.2 Hz, 1H), 0.78 (t, *J* = 6.8 Hz, 3H) |
| **76** | 3-((3,5-difluoro-4-((6-(trifluoromethyl)pyridin-3-yl)oxy)benzyl)oxy)-9a-ethyl-6,7,8,9,9a,10-hexahydro-1H-pyrido[1',2':3,4]imidazo[1,2-c]pyrimidin-1-one | 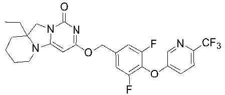 | B | 523 | DMSO-*d*₆: δ8.68 (br. s., 1H), 7.90 (d, *J* = 8.8 Hz, 1H), 7.62 (d, *J* = 8.3 Hz, 1H), 7.42 (d, *J* = 9.3 Hz, 2H), 5.31 (d, *J* = 5.9 Hz, 3H), 3.76 (d, *J* = 11.7 Hz, 1H), 3.66 - 3.51 (m, 2H), 3.11 - 2.97 (m, 1H), 1.94 - 1.80 (m, 1H), 1.78 - 1.50 (m, 6H), 1.45 - 1.33 (m, 1H), 0.80 (t, *J* = 7.1 Hz, 3H) |
| **77** | 5-(((9a-ethyl-1-oxo-6,7,8,9,9a,10-hexahydro-1H-pyrido[1',2':3,4]imidazo[1,2-c]pyrimidin-3-yl)oxy)methyl)-2-(3-(trifluoromethyl)phenoxy)be nzonitrile | 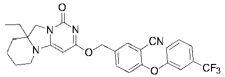 | B | 511 | DMSO-*d*₆: δ7.97 (br. s., 1H), 7.77 - 7.59 (m, 3H), 7.56 (br. s., 1H), 7.46 (d, *J* = 7.8 Hz, 1H), 7.10 (d, *J* = 8.8 Hz, 1H), 5.27 (br. s., 3H), 3.75 (d, *J =* 11.2 Hz, 1H), 3.60 (d, *J* = 11.7 Hz, 2H), 3.04 (t, *J* = 13.5 Hz, 1H), 1.94 - 1.82 (m, 1H), 1.77 - 1.47 (m, 6H), 1.44 - 1.27 (m, 1H), 0.79 (t, *J* = 6.8 Hz, 3H) |
| **78** | 7-((3,5-difluoro-4-((2-(trifluoromethyl)pyridin-4-yl)oxy)benzyl)oxy)-11a-methyl-3,4,11,11a-tetrahydro-1H,9H-pyrimido[6',1':2,3]imidazo[5 ,1-c][1,4]oxazin-9-one | 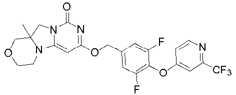 | B | 511 | DMSO-*d*₆: δ8.66 (d, *J* = 5.4 Hz, 1H), 7.63 (br. s., 1H), 7.42 (d, *J* = 9.3 Hz, 2H), 7.34 - 7.22 (m, 1H), 5.39 (s, 1H), 5.31 (s, 2H), 3.84 - 3.68 (m, 2H), 3.68 - 3.59 (m, 2H), 3.51 - 3.43 (m, 2H), 3.43 - 3.40 (m, 2H), 1.46 (s, 3H) |
| **79** | 7-((3,5-difluoro-4-((2-methyl-pyridin-4-yl)oxy)benzyl)oxy)-11a-methyl-3,4,11,11a-tetrahydro-1H,9H-pyrimido[6',1':2,3]imidazo[5 ,1-c][1,4]oxazin-9-one | 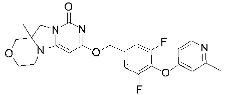 | B | 457 | DMSO-*d*₆: δ8.35 (d, *J* = 5.4 Hz, 1H), 7.39 (d, *J* = 9.3 Hz, 2H), 6.87 (br. s., 1H), 6.81 (br. s., 1H), 5.40 (s, 1H), 5.31 (s, 2H), 3.85 - 3.67 (m, 4H), 3.64 (d, *J* = 11.7 Hz, 2H), 3.59 - 3.47 (m, 2H), 2.42 (s, 3H), 1.48 (br. s., 3H) |
| **80** | 7-((3,5-difluoro-4-((5-(trifluoromethyl)pyridin-3-yl)oxy)benzyl)oxy)-11a-methyl-3,4,11,11a-tetrahydro-1H,9H-pyrimido[6', 1':2,3]imidazo[5 ,1-c][1,4]oxazin-9-one | 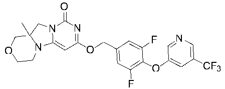 | B | 511 | DMSO-*d*₆: δ8.77 (s, 1H), 8.73 (s, 1H), 7.90 (br. s., 1H), 7.40 (d, *J* = 9.3 Hz, 2H), 5.39 (s, 1H), 5.31 (br. s., 2H), 3.82 - 3.63 (m, 4H), 3.53 - 3.41 (m, 4H), 1.48 (br. s., 3H) |
| **81** | 7-((3,5-difluoro-4-((6-(trifluoromethyl)pyridin-3-yl)oxy)benzyl)oxy)-11a-methyl-3,4,1 1,11a-tetrahydro-1H,9H-pyrimido[6',1':2,3]imidazo[5 ,1-c][1,4]oxazin-9-one | 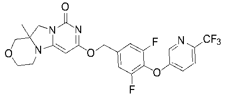 | B | 511 | DMSO-*d*₆: δ8.66 (br. s., 1H), 7.89 (d, *J* = 7.3 Hz, 1H), 7.61 (br. s., 1H), 7.41 (d, *J* = 7.8 Hz, 2H), 5.38 (br. s., 1H), 5.30 (br. s., 2H), 3.89 - 3.44 (m, 8H), 1.46 (br. s., 3H) |
| **82** | 7-((4-(4-chloro-3-(trifluoromethyl)phenoxy)-3,5-difluorobenzyl)oxy)-11a-methyl-3,4,11,11a-tetrahydro-1H,9H-pyrimido[6', 1':2,3]imidazo[5 ,1-c][1,4]oxazin-9-one | 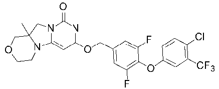 | B | 544 | DMSO-*d*₆: δ7.69 (d, *J* = 8.8 Hz, 1H), 7.51 (br. s., 1H), 7.39 (d, *J* = 9.3 Hz, 2H), 7.27 (d, *J* = 8.3 Hz, 1H), 5.39 (s, 1H), 5.30 (s, 2H), 3.80 - 3.62 (m, 4H), 3.55 - 3.41 (m, 4H), 1.48 (s, 3H) |
| **83** | 3-(2-fluoro-4-(((((11a-methyl-9-oxo-3,4,11,11a-tetrahydro-1H,9H-pyrimido[6',1':2,3]imidazo[5 ,1-c][1,4]oxazin-7-yl)oxy)methyl)phenoxy)-5-(trifluoromethyl)benzonitrile | 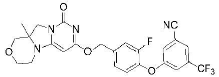 | B | 517 | DMSO-*d*₆: δ8.09 (s, 1H), 7.83 (br. s., 1H), 7.69 (br. s., 1H), 7.48 (d, *J* = 11.2 Hz, 1H), 7.37 - 7.28 (m, 2H), 5.35 (s, 1H), 5.27 (s, 2H), 3.77 - 3.59 (m, 4H), 3.51 - 3.36 (m, 4H), 1.45 (s, 3H) |
| **84** | 2-(4-chloro-3-(trifluoromethyl)phenoxy)-5-(((((11a-methyl-9-oxo-3,4,11,11a-tetrahydro-1H,9H-pyrimido[6',1':2,3]imidazo[5 ,1-c][1,4]oxazin-7-yl)oxy)methyl)benzonitrile | 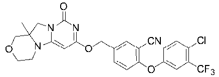 | B | 533 | DMSO-*d*₆: δ7.94 (br. s., 1H), 7.78 (d, *J* = 8.3 Hz, 1H), 7.74 - 7.66 (m, 2H), 7.47 (d, *J*=7.8 Hz, 1H), 7.14 (d, *J* = 8.3 Hz, 1H), 5.33 (br. s., 1H), 5.26 (br. s., 2H), 3.80 - 3.60 (m, 4H), 3.60 (br. s., 1H), 3.55 - 3.40 (m, 3H), 1.45 (br. s., 3H) |
| **85** | 5-(((((11a-methyl-9-oxo-3,4,11,11a-tetrahydro-1H,9H-pyrimido[6',1':2,3]imidazo[5 ,1-c][1,4]oxazin-7-yl)oxy)methyl)-2-(3-(trifluoromethyl)phenoxy)be nzonitrile | 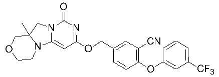 | B | 499 | DMSO-d6: δ7.96 (br. s., 1 H), 7.72 (br. s., 2 H), 7.66 - 7.60 (m, 1 H), 7.55 (br. s., 1 H), 7.47 (br. s., 1 H), 7.10 (d, J = 7.8 Hz, 1 H), 5.41 - 5.33 (m, 1 H), 5.28 (br. s., 2 H), 3.77 (d, J = 7.8 Hz, 1 H), 3.73 - 3.61 (m, 3 H), 3.50 (br. s., 4 H), 1.47 (br. s., 3 H) |
| **86** | 11a-methyl-7-(((3,4,5-trifluorobenzyl)oxy]-3,4,11,1la-tetrahydro-1H,9H-pyrimido[6',1':2,3]imidazo[5 ,1-c][1,4]oxazin-9-one | 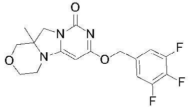 | B | 368 | DMSO-*d*₆: δ7.42 - 7.26 (m, 2H), 5.40 - 5.32 (m, 1H), 5.25 - 5.17 (m, 2H), 3.79 - 3.58 (m, 4H), 3.52 - 3.33 (m, 4H), 1.46 (s, 3H) |
| **88** | (3*S*,11*aR*)-7-((3,5-difluoro-4-((2-(trifluoromethyl)pyridin-4-yl)oxy)benzyl)oxy)-6-fluoro-3,4-dihydro-1H,9H,11H-3,11a-methanopyrimido[6',1':2,3]i midazo[5,1-c][1,4]oxazin-9-one | 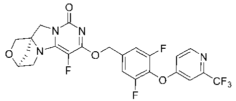 | B | 527 | CDCl₃: δ8.61 (d, *J* = 5.4 Hz, 1H), 7.27 (s, 1H), 7.18 (d, *J* = 8.3 Hz, 2H), 7.00 (br. s., 1H), 5.48 (d, *J* = 5.9 Hz, 2H), 4.77 (br. s., 1H), 4.48 (d, *J* = 12.2 Hz, 1H), 4.08 - 4.05 (m, 1H), 4.02 - 3.94 (m, 2H), 3.79 (d, *J* = 10.3 Hz, 1H), 3.61 (br. s., 1H), 2.11 (d, *J* = 9.8 Hz, 1H), 1.90 - 1.90 (m, 1H), 1.88 (d, *J* = 9.8 Hz, 1H) |
| **89** | (3*S*,11*aR*)-7-(((3,5-difluoro-4-((2-methyl-pyridin-4-yl)oxy)benzyl)oxy)-6-fluoro-3,4-dihydro-1H,9H,11H-3,11a-methanopyrimido[6',1':2,3]i midazo[5,1-c][1,4]oxazin-9-one | 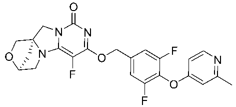 | | 473 | CDCl₃: δ8.37 (d, *J* = 4.4 Hz, 1H), 7.13 (d, *J* = 8.3 Hz, 2H), 6.69 (d, *J* = 7.3 Hz, 2H), 5.47 (d, *J* = 8.3 Hz, 2H), 4.77 (br. s., 1H), 4.48 (d, *J* = 12.2 Hz, 1H), 4.06 (d, *J* = 6.8 Hz, 1H), 4.01 - 3.94 (m, 2H), 3.78 (d, *J* = 10.3 Hz, 1H), 3.60 (d, *J* = 10.3 Hz, 1H), 2.52 (br. s., 3H), 2.11 (d, *J* = 9.8 Hz, 1H), 1.88 (d, *J* = 9.8 Hz, 1H) |
| **90** | 3-(2-fluoro-4-((((((3*S*,11a*R*)-6-fluoro-9-oxo-3,4-dihydro-1H,9H,11H-3,11a-methanopyrimido[6',1':2,3]i midazo[5,1-c][1,4]oxazin-7-yl)oxy)methyl)phenoxy)-5-(trifluoromethyl)benzonitrile | 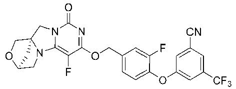 | B | 533 | CDCl₃: δ7.62 (s, 1H), 7.45 (br. s., 1H), 7.38 (d, *J* = 10.8 Hz, 1H), 7.32 (br. s., 2H), 7.20 - 7.15 (m, 1H), 5.50 (d, *J* = 6.8 Hz, 2H), 4.76 (br. s., 1H), 4.48 (d, *J* = 12.2 Hz, 1H), 4.08 - 4.04 (m, 1H), 4.01 - 3.94 (m, 2H), 3.78 (d, *J=* 10.3 Hz, 1H), 3.59 (d, *J* = 10.3 Hz, 1H), 2.10 (d, *J=* 9.8 Hz, 1H), 1.87 (d, *J* = 10.3 Hz, 1H) |
| **91** | (3*S*,11*aR*)-7-((3,5-difluoro-4-((2-(trifluoromethyl)pyridin-4-yl)oxy)benzyl)oxy)-6-chloro-3,4-dihydro-1H,9H,11H-3,11a-methanopyrimido[6',1':2,3]i midazo[5,1-c][1,4]oxazin-9-one | 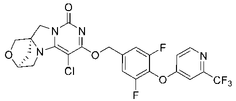 | B | 543 | DMSO-*d*₆: δ8.68 (d, *J* = 5.9 Hz, 1H), 7.68 (br. s., 1H), 7.45 (d, *J* = 8.8 Hz, 2H), 7.32 (br. s., 1H), 5.42 (s, 2H), 4.68 (br. s., 1H), 4.31 (d, *J=* 12.2 Hz, 1H), 3.95 (d, *J* = 12.2 Hz, 1H), 3.88 (q, *J* = 7.3 Hz, 2H), 3.79 (d, *J* = 9.8 Hz, 1H), 3.62 (d,*J* = 10.3 Hz, 1H), 2.06 (d, *J* = 9.8 Hz, 1H), 1.98 - 1.87 (m, 1H) |
| **92** | (3*S*,11*aR*)-7-((3,5-difluoro-4-((2-(trifluoromethyl)pyridin-4-yl)oxy)benzyl)oxy)-6-methyl-3,4-dihydro-1H,9H,11H-3,11a-methanopyrimido[6',1':2,3]i midazo[5,1-c][1,4]oxazin-9-one | 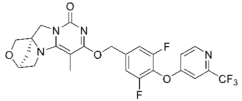 | B | 523 | CDCl₃: δ8.56 (d, *J* = 5.9 Hz, 1H), 7.24 - 7.23 (m, 1H), 7.08 (d, *J* = 8.3 Hz, 2H), 7.00 - 6.92 (m, 1H), 5.40 (d, *J* = 5.4 Hz, 2H), 4.68 (s, 1H), 4.43 (d, *J* = 12.2 Hz, 1H), 4.01 - 3.91 (m, 2H), 3.85 (d, *J* = 12.2 Hz, 1H), 3.74 (d, *J=* 9.8 Hz, 1H), 3.52 (d, *J= 9.8* Hz, 1H), 2.02 (d, *J* = 8.3 Hz, 1H), 1.93 (s, 3H), 1.77 (d, *J=* 10.3 Hz, 1H) |
| **93** | (3*S*,11*aR*)-7-(((3,5-difluoro-4-((2-(trifluoromethyl)pyridin-4-yl)oxy)benzyl)oxy)-3,4-dihydro-1H,9H,11H-3,11a-methanopyrimido[6',1';2,3]i midazo[5,1-c][1,4]oxazin-9-one-11,11-d₂ | 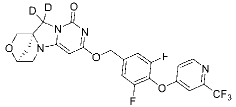 | B | 511 | CDCl₃: δ8.61 (d, *J* = 5.4 Hz, 1H), 7.27 - 7.22 (m, 1H), 7.14 (d, *J* = 7.8 Hz, 2H), 7.00 (br. s., 1H), 5.43 (d, *J* = 5.4 Hz, 2H), 5.13 (s, 1H), 4.76 (br. s., 1H), 4.01 (br. s., 2H), 3.59 (d, *J* = 9.8 Hz, 1H), 3.25 (d, *J* = 9.8 Hz, 1H), 2.06 (d, *J* = 9.8 Hz, 1H), 1.75 (d, *J=* 10.3 Hz, 1H) |
| **94** | 3-((3,5-difluoro-4-((1-methyl-1H-pyrazol-4-yl)oxy)benzyl)oxy)-7,8-dihydro-1H,6H,9H-7,8a-methanopyrrolo[1',2': 3,4] imi dazo[1,2-c]pyrimidin-1-one | 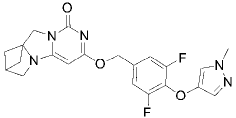 | B | 428 | DMSO-*d*₆: δ7.57 (s, 1H), 7.33 - 7.18 (m, 3H), 5.29 (s, 1H), 5.23 (s, 2H), 3.84 (s, 2H), 3.70 (s, 3H), 3.28(s, 2H) 2.92 (br. s., 1H), 2.01 (br. s., 2H), 1.55 (br. s., 2H) |
| **95** | 3-(2-fluoro-4-((((1-oxo-7,8-dihydro-1H,6H,9H-7,8a-methanopyrrolo[1',2': 3,4] imi dazo[1,2-c]pyrimidin-3-yl)oxy)methyl)phenoxy)-5-(trifluoromethyl)benzonitrile | 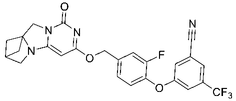 | B | 498 | DMSO-*d*₆: δ8.09 (br. s., 1H), 7.83 (br. s., 1H), 7.70 (br. s., 1H), 7.48 (d, *J =* 11.7 Hz, 1H), 7.32 (q, *J* = 8.6 Hz, 2H), 5.28 (s, 3H), 3.84 (s, 2H), 3.28 (s, 2H), 2.92 (br. s., 1H), 2.01 (br. s., 2H), 1.55 (br. s., 2H) |
| **96** | (3*S*,11*aR*)-7-((2-difluoro-4-((2-(trifluoromethyl)pyridin-4-yl)oxy)benzyl)oxy)-3,4-dihydro-1H,9H,11H-3,11a-methanopyrimido[6'-,1':2,3]i midazo[5,1-c][1,4]oxazin-9-one | 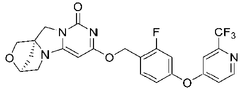 | B | 491 | DMSO-*d*₆: δ8.64 (d, *J* = 5.9 Hz, 1H), 7.62 (t, *J=* 8.3 Hz, 1H), 7.49 (d, *J* = 2.0 Hz, 1H), 7.30 (dd, *J* = 1.7, 10.5 Hz, 1H), 7.22 (dd, *J* = 2.0, 5.4 Hz, 1H), 7.12 (d, *J* = 8.3 Hz, 1H), 5.31 (br. s., 2H), 5.23 (s, 1H), 4.65 (s, 1H), 4.26 (d, *J* = 12.2 Hz, 1H), 3.92 - 3.83 (m, 2H), 3.83 - 3.77 (m, 1H), 3.38 - 3.33 (m, 1H), 3.27 - 3.21 (m, 1H), 1.87 (d, *J* = 9.3 Hz, 1H), 1.77 (d, *J* = 9.8 Hz, 1H) |
| **97** | 3-((3,4-difluorobenzyl)oxy)-7,8-dihydro-1H,6H,9H-7,8a-methanopyrrolo[1',2': 3,4] imi dazo[1,2-c]pyrimidin-1-one | 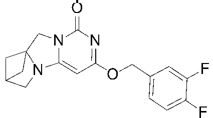 | B | 332 | DMSO-*d*₆: δ7.53 - 7.33 (m, 2H), 7.24 (br. s., 1H), 5.28 - 5.18 (m, 3H), 3.83 (br. s., 2H), 3.28 (br. s., 2H), 2.91 (br. s., 1H), 2.00 (br. s., 2H), 1.54 (br. s., 2H) |
| **98** | 3-((3,4,5-trifluorobenzyl)oxy)-7,8-dihydro-1H,6H,9H-7,8a-methanopyrrolo[1',2':3,4]imi dazo[1,2-c]pyrimidin-1-one | 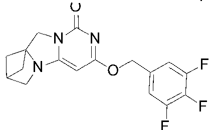 | B | 350 | DMSO-*d*₆: δ7.35 (t, *J* = 7.6 Hz, 2H), 5.28 (s, 1H), 5.21 (s, 2H), 3.83 (s, 2H), 3.28 (br. s., 2H), 2.92 (br. s., 1H), 2.01 (br. s., 2H), 1.55 (br. s., 2H) |
| **99** | (3*S*,11*aR*)-7-(3,5-difluoro-4-((2-(trifluoromethyl)pyridin-4-yl)oxy)phenethoxy)-3,4-dihydro-1H,9H,11H-3,11a-methanopyrimido[6'-,1':2,3]i midazo[5,1-c][1,4]oxazin-9-one | 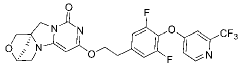 | B | 523 | CDCl₃: δ8.60 (br. s., 1H), 7.25 - 7.21 (m, 1H), 6.98 (d, *J* = 6.4 Hz, 3H), 5.01 (br. s., 1H), 4.73 (br. s., 1H), 4.60 (br. s., 2H), 4.45 (d, *J* = 12.2 Hz, 1H), 4.09 - 3.84 (m, 3H), 3.56 (d, *J* = 9.3 Hz, 1H), 3.22 (d, *J* = 9.3 Hz, 1H), 3.05 (br. s., 2H), 2.02 (d, *J* = 9.3 Hz, 1H), 1.71 (d, *J* = 9.8 Hz, 1H) |
| **100** | (3*S*,11*aR*)-7-(4-(2-(trifluoromethyl)pyridin-4-yloxy)phenethoxy)-3,4-dihydro-1H,9H,11H-3,11a-methanopyrimido[6'-,1':2,3]i midazo[5,1-c][1,4]oxazin-9-one | 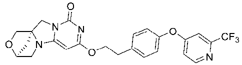 | B | 487 | DMSO-*d*₆: δ8.61 (d, *J* = 5.9 Hz, 1H), 7.43 (s, 1H), 7.41 (s, 1H), 7.40 (d, *J* = 2.4 Hz, 1H), 7.21 (s, 1H), 7.19 (s, 1H), 7.12 (dd, *J* = 2.2, 5.6 Hz, 1H), 5.16 (s, 1H), 4.71 - 4.63 (m, 1H), 4.51 - 4.34 (m, 2H), 4.26 (d, *J* = 12.2 Hz, 1H), 4.01 - 3.72 (m, 3H), 3.36 (d, *J= 11.2* Hz, 1H), 3.28 - 3.23 (m, 1H), 3.01 (t, *J* = 6.8 Hz, 2H), 1.95 - 1.83 (m, 1H), 1.76 (d, *J* = 9.8 Hz, 1H) |
| **101** | 5-(2-(((3*S*,11*aR*)-9-oxo-3,4-dihydro-1H,9H,11H-3,11a-methanopyrimido[6'-,1':2,3]i midazo[5,1-c][1,4]oxazin-7-yl)oxy)ethyl)-2-((2-(trifluoromethyl)pyridin-4-yl)oxy)benzonitrile | 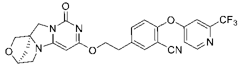 | B | 512 | CDCl₃: δ8.65 (d, *J* = 5.4 Hz, 1H), 7.63 (s, 1H), 7.57 (d, *J* = 8.3 Hz, 1H), 7.27 - 7.27 (m, 1H), 7.13 (d, *J* = 8.3 Hz, 2H), 7.02 (br. s., 2H), 5.01 (s, 1H), 4.73 (br. s., 1H), 4.65 - 4.59 (m, 1H), 4.44 (d, *J* = 12.7 Hz, 1H), 3.98 (br. s., 1H), 3.91 (d, *J=* 12.2 Hz, 1H), 3.57 (d, *J* = 9.8 Hz, 1H), 3.23 (d, *J* = 9.8 Hz, 1H), 3.13 - 3.03 (m, 2H), 2.02 (d, *J* = 9.3 Hz, 1H), 1.73 (s, 1H) |
| **102** | (3*S*,11*aR*)-7-((3,5-difluoro-4-((2-(trifluoromethyl)pyridin-4-yl)oxy)benzyl)oxy)-6-cyano-3,4-dihydro-1H,9H,11H-3,11a-methanopyrimido[6',1':2,3]i midazo[5,1-c][1,4]oxazin-9-one | 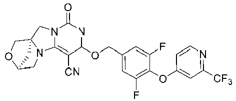 | B | 534 | DMSO-*d*₆: δ8.62 (d, *J* = 4.9 Hz, 1H), 7.26 - 7.24 (m, 1H), 7.18 (d, *J* = 8.3 Hz, 2H), 6.99 (br. s., 1H), 5.56 - 5.42 (m, 2H), 4.85 (br. s., 1H), 4.48 (d, *J* = 12.7 Hz, 1H), 4.20 - 3.97 (m, 3H), 3.95 - 3.87 (m, 1H), 3.81 (d, *J* = 10.3 Hz, 1H), 2.22 (d,*J =* 9.3 Hz, 1H), 1.89 (d, *J* = 10.3 Hz, 1H) |
| **103** | 3-(4-fluoro-3-cyanobenzyl)oxy)-7,8-dihydro-1H,6H,9H-7,8a-methanopyrrolo[1',2':3,4]imi dazo[1,2-c]pyrimidin-1-one | 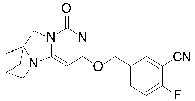 | B | 339 | CDCl₃: δ7.79 - 7.54 (m, 2H), 7.20 (t, *J=* 8.3 Hz, 1H), 5.54 - 5.33 (m, 2H), 5.12 (s, 1H), 3.99 (s, 2H), 3.34 (br. s., 2H), 3.03 (br. s., 1H), 2.08 (br. s., 2H), 1.70 (br. s., 2H) |
| **104** | (3*S*,11a*R*)-4-((3,5-difluoro-4-((2-(trifluoromethyl)pyridin-4-yl)oxy)benzyl)oxy)-6-ethyl-3,4-dihydro-1H,9H,11H-3,11a-methanopyrimido[6',1':2,3]i midazo[5,1-c][1,4]oxazin-9-one | 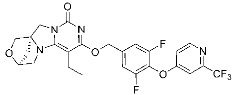 | B | 537 | DMSO-*d*₆: δ8.68 (d, *J* = 5.4 Hz, 1H), 7.67 (br. s., 1H), 7.42 (d, *J* = 8.8 Hz, 2H), 7.31 (br. s., 1H), 5.38 (s, 2H), 4.67 (br. s., 1H), 4.27 (d, *J= 12.2* Hz, 1H), 4.04 - 3.75 (m, 3H), 3.65 (d, *J* = 9.3 Hz, 1H), 3.53 (d, J=9.3 Hz, 1H), 2.44 - 2.23 (m, 2H), 1.93 (br. s., 2H), 1.07 (t, *J* = 7.1 Hz, 3H) |
| **105** | 3-((2-fluoro-4-((2-(trifluoromethyl)pyridin-4-yl)oxy)benzyl)oxy)-7,8-dihydro-1H,6H,9H-7,8a-methanopyrrolo[1',2':3,4]imi dazo[1,2-c]pyrimidin-1-one | 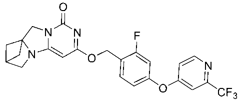 | B | 475 | DMSO-*d*₆: δ8.63 (d, *J* = 5.4 Hz, 1H), 7.61 (t, *J* = 8.3 Hz, 1H), 7.48 (br. s., 1H), 7.29 (d, *J* = 10.3 Hz, 1H), 7.21 (d, J=3.4 Hz, 1H), 7.12 (d, *J* = 8.3 Hz, 1H), 5.30 (s, 2H), 5.25 (s, 1H), 3.84 (s, 2H), 3.29 (s, 2H), 2.91 (br. s., 1H), 2.01 (br. s., 2H), 1.55 (br. s., 2H) |
| **106** | (3*S*,11a*R*)-7-((3,5-difluoro-4-((2-(trifluoromethyl)pyridin-4-yl)oxy)benzyl)oxy)-6-methoxy-3,4-dihydro-1H,9H,11H-3,11a-methanopyrimido[6',1':2,3]i midazo[5,1-c][1,4]oxazin-9-one | 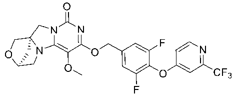 | B | 539 | DMSO-*d*₆: δ8.68 (d, *J* = 5.4 Hz, 1H), 7.67 (s, 1H), 7.46 (d, *J=* 8.8 Hz, 2H), 7.31 (d, *J* = 3.4 Hz, 1H), 5.39 (s, 2H), 4.67 (br. s., 1H), 4.27 (d, *J* = 12.2 Hz, 1H), 4.00 - 3.81 (m, 3H), 3.62 (s, 3H), 3.55 (d, *J=* 5.9 Hz, 2H), 2.05 - 1.83 (m, 2H) |
| **107** | (3*S*,11a*R*)-7-((3,5-difluoro-4-((2-(trifluoromethyl)pyridin-4-yl)oxy)benzyl)oxy)-6-isopropyl-3,4-dihydro-1H,9H,11H-3,11a-methanopyrimido[6',1':2,3]i midazo[5, 1-c][1,4]oxazin-9-one | 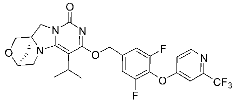 | B | 551 | CDCl₃: δ8.61 (d, *J* = 5.4 Hz, 1H), 7.27 (s, 1H), 7.15 (d, *J* = 8.3 Hz, 2H), 6.99 (d, *J* = 3.4 Hz, 1H), 5.66 - 5.36 (m, 2H), 4.72 (br. s., 1H), 4.46 (d, *J* = 12.7 Hz, lH), 4.14 - 3.93 (m, 2H), 3.91 - 3.78 (m, 2H), 3.41 (d, *J= 9.3* Hz, 1H), 2.79 (td, *J=* 6.7, 13.6 Hz, 1H), 2.06 (d, *J* = 9.8 Hz, 1H), 1.82 (d, *J* = 9.8 Hz, 1H), 1.29 (t, *J* = 8.3 Hz, 6H) |
| **108** | (3*S*,11a*R*)-7-((4-((2-(trifluoromethyl)pyridin-4-yl)oxy)benzyl)oxy)-6-methyl-3,4-dihydro-1H,9H,11H-3,11a-methanopyrimido[6',1':2,3]imidazo[5,1-c][1,4]oxazin-9-one | 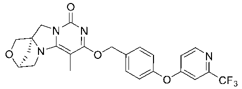 | B | 487 | DMSO-*d*₆: δ8.63 (d, *J* = 5.4 Hz, 1H), 7.56 (d, *J*= 8.3 Hz, 2H), 7.44 (s, 1H), 7.28 (d, *J* = 8.3 Hz, 2H), 7.16 (d, *J* = 3.4 Hz, 1H), 5.35 (s, 2H), 4.65 (br. s., 1H), 4.27 (d, *J* =12.2 Hz, 1H), 3.92 - 3.79 (m, 3H), 3.61 (s, 2H), 1.95 - 1.82 (m, 5H) |
| **109** | 3-((4-((2-(trifluoromethyl)pyridin-4-yl)oxy)benzyl)oxy)-7,8-dihydro-1H,6H,9H-7,8a-methanopyrrolo[1',2':3,4]imi dazo[1,2-c]pyrimidin-1-one | 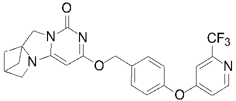 | B | 457 | DMSO-*d*₆: δ8.60 (d, *J* = 5.4 Hz, 1H), 7.56 - 7.48 (m, *J* = 8.3 Hz, 2H), 7.40 (br. s., 1H), 7.28 - 7.22 (m, *J* = 8.3 Hz, 2H), 7.14 (d, *J* = 2.9 Hz, 1H), 5.31 - 5.25 (m, 3H), 3.84 (s, 2H), 3.29 (br. s., 2H), 2.92 (br. s., 1H), 2.01 (br. s., 2H), 1.55 (br. s., 2H) |
| **110** | 5-((((3*R*,11a*S*)-9-oxo-3,4-dihydro-1H,9H,11H-3,11a-methanopyrimido[6', 1':2,3]i midazo[5, 1-c][1,4]oxazin-7-yl)oxy)methyl)-2-((2-(trifluoromethyl)pyridin-4-yl)oxy)benzonitrile | 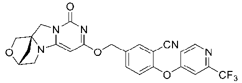 | B | 498 | DMSO-*d*₆: δ8.73 (br. s., 1H), 8.05 (br. s., 1H), 7.84 (d, *J* = 7.8 Hz, 1H), 7.69 (br. s., 1H), 7.50 (d, *J* = 7.3 Hz, 1H), 7.35 (br. s., 1H), 5.42 - 5.23 (m, 3H), 4.68 (br. s., 1H), 4.28 (d, *J* = 11.7 Hz, 1H), 3.98 - 3.77 (m, 3H), 3.56 - 3.47 (m, 1H), 3.21 - 3.15 (m, 1H), 1.90 (d, *J* = 8.8 Hz, 1H), 1.78 (d, *J* = 9.3 Hz, 1H) |
| **111** | 3-(4-((2-(trifluoromethyl)pyridin-4-yl)oxy)phenethoxy)-7,8-dihydro-1H,6H,9H-7,8a-methanopyrrolo[1',2':3,4]imi dazo[1,2-c]pyrimidin-1-one | 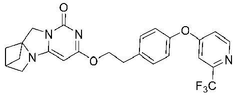 | B | 471 | CDCl₃: δ8.50 (d, *J= 4.9* Hz, 1H), 7.33 - 7.26 (m, *J* = 7.8 Hz, 2H), 7.17 (br. s., 1H), 7.02 - 6.96 (m, *J* = 8.3 Hz, 2H), 6.94 - 6.86 (m, 1H), 5.02 (s, 1H), 4.54 (t, *J* = 6.4 Hz, 2H), 3.93 (s, 2H), 3.27 (s, 2H), 3.07 - 2.94 (m, 3H), 2.01 (br. s., 2H), 1.63 (br. s., 2H) |
| **112** | 7-((3,5-difluoro-4-((2-(trifluoromethyl)pyridin-4-yl)oxy)benzyl)oxy)-3,4-dihydro-1H,9H,11H-4,11a-ethanopyrimido[6',1':2,3]imi dazo[5,1-c][1,4]oxazin-9-one | 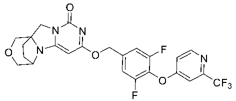 | B | 523 | DMSO-*d*₆: δ8.58 (d, *J* = 5.4 Hz, 1H), 7.24 (s, 1H), 7.11 (d, *J* = 8.3 Hz, 2H), 6.97 (d, *J* = 3.9 Hz, 1H), 5.40 (s, 2H), 5.21 (s, 1H), 4.23 (d, *J=* 11.7 Hz, 1H), 3.99 (d, *J=* 5.9 Hz, 1H), 3.91 (d, *J* = 10.3 Hz, 1H), 3.75 - 3.70 (m, 3H), 3.68 (d, *J* = 12.2 Hz, 1H), 2.37 - 2.21 (m, 1H), 2.10 - 2.01 (m, 1H), 1.94 (dd, *J* = 6.1, 12.0 Hz, 1H), 1.70 (dt, J=3.7, 12.3 Hz, 1H) |
| **113** | 3-((3-fluoro-4-((2-(trifluoromethyl)pyridin-4-yl)oxy)benzyl)oxy)-7,8-dihydro-1H,6H,9H-7,8a-methanopyrrolo[1',2': 3,4] imi dazo[1,2-c]pyrimidin-1-one | 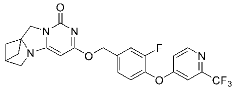 | B | 475 | DMSO-*d*₆: δ8.65 (br. s., 1H), 7.90-6.96 (m, 5H), 5.32 (br. s., 3H), 3.87 (br. s., 2H), 3.22 (br. s., 2H), 2.94 (br. s., 1H), 2.04 (br. s., 2H), 1.57 (br. s., 2H) |
| **114** | (3*S*,11a*R*)-7-((3,4,5-trifluorobenzyl)oxy)-6-methoxy-3,4-dihydro-1H,9H,11H-3,11a-methanopyrimido[6',1':2,3]i midazo[5,1-c][1,4]oxazin-9-one | 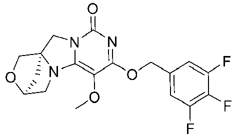 | B | 396 | DMSO-*d*₆: δ7.45 - 7.32 (m, 2H), 5.29 (s, 2H), 4.66 (s, 1H), 4.26 (d, *J=* 12.2 Hz, 1H), 3.94 - 3.82 (m, 3H), 3.58 (s, 3H), 3.54 (d, *J=* 5.4 Hz, 2H), 2.04 - 1.86 (m, 2H) |
| **115** | (3*S*,11a*R*)-7-((3,4-difluorobenzyl)oxy)-6-methoxy-3,4-dihydro-1H,9H,11H-3,11a-methanopyrimido[6',1':2,3]i midazo[5,1-c][1,4]oxazin-9-one | 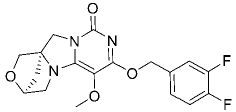 | B | 378 | DMSO-*d*₆: δ7.51 - 7.40 (m, 2H), 7.29 (br. s., 1H), 5.28 (s, 2H), 4.64 (s, 1H), 4.24 (d, *J* = 12.2 Hz, 1H), 3.94 - 3.81 (m, 3H), 3.55 (s, 3H), 3.51 (d, *J =* 4.9 Hz, 2H), 2.02 - 1.84 (m, 2H) |
| **116** | (3*R*,11a*S*)-7-((3-fluoro-4-((2-(trifluoromethyl)pyridin-4-yl)oxy)benzyl)oxy)-3,4-dihydro-1H,9H,11H-3,11a-methanopyrimido[6',1':2,3]i midazo[5,1-c][1,4]oxazin-9-one | 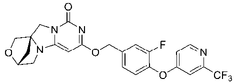 | B | 491 | DMSO-*d*₆: δ8.65 (d, *J* = 5.4 Hz, 1H), 7.58 - 7.44 (m, 3H), 7.38 (d, *J=* 8.3 Hz, 1H), 7.19 (br. s., 1H), 5.43 - 5.23 (m, 3H), 4.68 (br. s., 1H), 4.28 (d, *J* = 12.2 Hz, 1H), 3.88 - 3.88 (m, 3H), 3.40 - 3.37 (m, 1H), 3.28 (d, *J* = 9.8 Hz, 1H), 1.90 (d, *J* = 8.8 Hz, 1H), 1.79 (d, *J* = 9.8 Hz, 1H) |
| **117** | 3-((3-fluoro-4-((6-(trifluoromethyl)pyridin-3-yl)oxy)benzyl)oxy)-7,8-dihydro-1H,6H,9H-7,8a-methanopyrrolo[1',2': 3,4] imi dazo[1,2-c]pyrimidin-1-one | 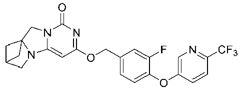 | B | 475 | CDCl₃: δ8.48 (br. s., 1H), 7.62 (d, *J* = 8.8 Hz, 1H), 7.34 - 7.27 (m, 2H), 7.24 (d, *J* = 8.3 Hz, 1H), 7.20 - 7.11 (m, 1H), 5.42 (s, 2H), 5.15 (s, 1H), 4.00 (s, 2H), 3.34 (s, 2H), 3.03 (br. s., 1H), 2.08 (br. s., 2H), 1.70 (d, *J* = 4.9 Hz, 2H) |
| **118** | 3-((3-fluoro-4-((2-(trifluoromethyl)pyridin-4-yl)oxy)benzyl)oxy)-7,8-dihydro-1H,6H,9H-6,8a-ethanopyrrolo[1',2':3,4]imid azo[1,2-c]pyrimidin-1-one | 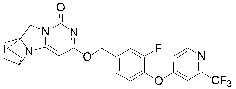 | B | 489 | CDCl₃: δ8.58 (d, *J* = 5.9 Hz, 1H), 7.34 (d, *J* = 10.8 Hz, 1H), 7.30 - 7.27 (m, 1H), 7.24 (s, 1H), 7.20 (d, *J* = 7.8 Hz, 1H), 6.96 (d, *J* = 3.9 Hz, 1H), 5.44 (s, 2H), 5.21 (s, 1H), 4.13 (t, *J* = 4.4 Hz, 1H), 4.02 (s, 2H), 2.11 (br. s., 2H), 1.82 - 1.77 (m, 6H) |
| **119** | 3-((3-fluoro-4-((2-methyl-pyridin-4-yl)oxy)benzyl)oxy)-7,8-dihydro-1H,6H,9H-7,8a-methanopyrrolo[1',2': 3,4] imi dazo[1,2-c]pyrimidin-1-one | 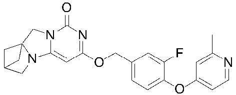 | B | 421 | CDCl₃: δ8.34 (d, *J* = 5.4 Hz, 1H), 7.34 - 7.25 (m, 1H), 7.24 - 7.18 (m, 1H), 7.18 - 7.09 (m, 1H), 6.71 - 6.61 (m, 2H), 5.41 (s, 2H), 5.14 (s, 1H), 3.99 (s, 2H), 3.33 (s, 2H), 3.02 (br. s., 1H), 2.49 (s, 3H), 2.07 (br. s., 2H), 1.74 - 1.63 (m, 2H) |
| **120** | 3-((3-fluoro-4-((2-methyl-pyridin-4-yl)oxy)benzyl)oxy)-7,8-dihydro-1H,6H,9H-6,8a-ethanopyrrolo[1',2': 3,4] imid azo[1,2-c]pyrimidin-1-one | 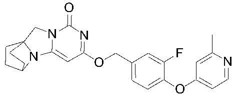 | B | 435 | CDCl₃: δ8.35 (d, *J* = 4.4 Hz, 1H), 7.31 (br. s., 1H), 7.25 - 7.13 (m, 2H), 6.68 (br. s., 2H), 5.41 (br. s., 2H), 5.20 (br. s., 1H), 4.12 (br. s., 1H), 4.01 (br. s., 2H), 2.51 (br. s., 3H), 2.10 (br. s., 2H), 1.78 (br. s., 6H) |
| **121** | 3-((3-fluoro-4-((6-(trifluoromethyl)pyridin-3-yl)oxy)benzyl)oxy)-7,8-dihydro-lH,6H,9H-6,8a-ethanopyrrolo[1',2':3,4]imid azo[1,2-c]pyrimidin-1-one | 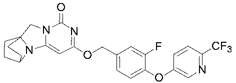 | B | 488 | CDCl₃: δ8.48 (br. s., 1H), 7.61 (s, 1H), 7.33 (br. s., 1H), 7.30 (br. s., 1H), 7.23 (br. s., 1H), 7.16 (br. s., 1H), 5.41 (s, 2H), 5.19 (s, 1H), 4.12 (br. s., 1H), 4.01 (s, 2H), 1.78 (br. s., 8H) |
| **122** | 3-((3-fluoro-4-((2-(trifluoromethyl)pyrimidin-5-yl)oxy)benzyl)oxy)-7,8-dihydro-1H,6H,9H-7,8a-methanopyrrolo[1',2':3,4]imi dazo[1,2-c]pyrimidin-1-one | 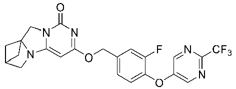 | B | 476 | CDCl₃: δ8.53 (br. s., 2H), 7.34 (d, *J= 9.3* Hz, 1H), 7.27 (br. s., 1H), 7.21 (br. s., 1H), 5.43 (br. s., 2H), 5.14 (br. s., 1H), 4.00 (br. s., 2H), 3.34 (br. s., 2H), 3.03 (br. s., 1H), 2.08 (br. s., 2H), 1.70 (br. s., 2H) |
| **123** | (7*S*,9a*R*)-3-((3,5-difluoro-4-((2-(trifluoromethyl)pyridin-4-yl)oxy)benzyl)oxy)-6,7,8,9-tetrahydro-1H,10H-7,9a-methanopyrido[1',2': 3,4]imi dazo[1,2-c]pyrimidin-1-one | 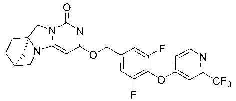 | B | 507 | CDCl₃: δ8.60 (d, *J* = 5.9 Hz, 1H), 7.27 (s, 1H), 7.13 (d, *J* = 8.3 Hz, 2H), 6.99 (dd, J=2.2, 5.6 Hz, 1H), 5.42 (d, *J* = 3.9 Hz, 2H), 5.07 (s, 1H), 4.37 (d, *J* = 12.2 Hz, 1H), 3.90 (d, *J* = 12.7 Hz, 1H), 3.24 - 3.13 (m, 2H), 2.74 (br. s., 1H), 1.98 (d, *J* = 14.7 Hz, 3H), 1.68 (d, *J* = 5.9 Hz, 1H), 1.60 - 1.56 (m, 1H), 1.54 - 1.48 (m, 1H) |
| **124** | 3-((3-fluoro-4-(3-(trifluoromethyl)phenoxy)be nzyl)oxy)-7,8-dihydro-1H,6H,9H-7,8a-methanopyrrolo[1',2':3,4]imi dazo[1,2-c]pyrimidin-1-one | 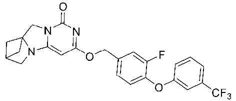 | B | 474 | DMSO-*d*₆: δ7.70 - 7.55 (m, 1H), 7.55 - 7.41 (m, 2H), 7.36 - 7.19 (m, 4H), 5.36 - 5.23 (m, 3H), 3.87 (s, 2H), 3.32 (br. s., 2H), 2.94 (br. s., 1H), 2.04 (br. s., 2H), 1.66 - 1.52 (m, 2H) |
| **125** | 3-((3-fluoro-4-(4-chloro-3-(trifluoromethyl)phenoxy)be nzyl)oxy)-7,8-dihydro-1H,6H,9H-7,8a-methanopyrrolo[1',2':3,4]imi dazo[1,2-c]pyrimidin-1-one | 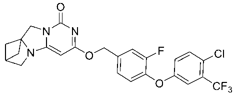 | B | 508 | DMSO-*d*₆: δ7.70 (d, *J* = 8.8 Hz, 1H), 7.55 - 7.42 (m, 2H), 7.38 - 7.22 (m, 3H), 5.30 (d, *J* = 4.4 Hz, 3H), 3.86 (s, 2H), 3.32 (br. s., 2H), 2.94 (br. s., 1H), 2.04 (br. s., 2H), 1.57 (d, *J* = 2.9 Hz, 2H) |
| **126** | 3-((3-fluoro-4-(3-(trifluoromethyl)phenoxy)be nzyl)oxy)-7,8-dihydro-1H,6H,9H-6,8a-ethanopyrrolo[1',2':3,4]imid azo[1,2-c]pyrimidin-1-one | 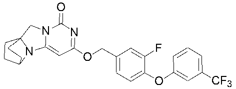 | B | 488 | DMSO-*d*₆: δ7.68 - 7.57 (m, 1H), 7.49 (d, *J* = 9.8 Hz, 2H), 7.36 - 7.20 (m, 4H), 5.45 (s, 1H), 5.28 (s, 2H), 4.28 (t, *J= 4.2* Hz, 1H), 3.88 (s, 2H), 1.92 (d, *J* = 7.8 Hz, 2H), 1.79 - 1.52 (m, 6H) |
| **127** | 3-((3-fluoro-4-(4-chloro-3-(trifluoromethyl)phenoxy)be nzyl)oxy)-7,8-dihydro-1H,6H,9H-6,8a-ethanopyrrolo[1',2':3,4]imid azo[1,2-c]pyrimidin-1-one | 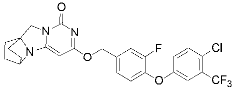 | B | 522 | DMSO-*d*₆: δ7.71 (d, *J* = 8.8 Hz, 1H), 7.58 - 7.41 (m, 2H), 7.38 - 7.21 (m, 3H), 5.45 (s, 1H), 5.28 (s, 2H), 4.28 (t, *J* = 4.2 Hz, 1H), 3.88 (s, 2H), 1.92 (d, *J* = 7.3 Hz, 2H), 1.78 - 1.54 (m, 6H) |
| **128** | 3-((3-fluoro-4-((2-(trifluoromethyl)pyrimidin-5-yl)oxy)benzyl)oxy)-7,8-dihydro-lH,6H,9H-6,8a-ethanopyrrolo[1',2':3,4]imid azo[1,2-c]pyrimidin-1-one | 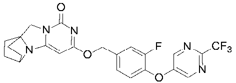 | B | 490 | CDCl₃: δ8.53 (s, 2H), 7.35 (d, *J=* 10.8 Hz, 1H), 7.29 (br. s., 1H), 7.23 (d, *J* = 7.8 Hz, 1H), 5.43 (s, 2H), 5.20 (s, 1H), 4.13 (br. s., 1H), 4.01 (s, 2H), 2.10 (br. s., 2H), 1.84 - 1.74 (m, 6H) |
| **129** | 3-((3-fluoro-4-(4-(trifluoromethyl)phenoxy)be nzyl)oxy)-7,8-dihydro-1H,6H,9H-7,8a-methanopyrrolo[1',2': 3,4] imi dazo[1,2-c]pyrimidin-1-one | 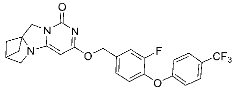 | B | 474 | DMSO-*d*₆: δ7.76 - 7.66 *(m, J=* 8.3 Hz, 2H), 7.47 (d, *J* = 11.2 Hz, 1H), 7.37 - 7.28 (m, 2H), 7.15 - 7.08 (m, *J* = 8.3 Hz, 2H), 5.29 (d, *J* = 3.9 Hz, 3H), 3.85 (s, 2H), 3.31 (br. s., 2H), 2.93 (br. s., 1H), 2.02 (br. s., 2H), 1.56 (d, *J=* 3.4 Hz, 2H) |
| **130** | 3-((3-fluoro-4-(4-(trifluoromethyl)phenoxy)be nzyl)oxy)-7,8-dihydro-1H,6H,9H-6,8a-ethanopyrrolo[1',2':3,4]imid azo[1,2-c]pyrimidin-1-one | 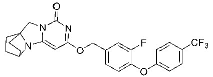 | B | 488 | DMSO-*d*₆: δ7.77 - 7.65 *(m, J=* 8.3 Hz, 2H), 7.48 (d, *J* = 11.7 Hz, 1H), 7.37 - 7.28 (m, 2H), 7.16 - 7.07 (m, *J* = 8.8 Hz, 2H), 5.44 (s, 1H), 5.27 (s, 2H), 4.27 (t, *J* = 4.2 Hz, 1H), 3.87 (s, 2H), 1.98 - 1.84 (m, 2H), 1.75 - 1.67 (m, 4H), 1.63 (d, *J= 11.7* Hz, 2H) |
| **131** | (3*S*,11a*R*)-2-benzyl-7-((3,5-difluoro-4-((2-(trifluoromethyl)pyridin-4-yl)oxy)benzyl)oxy)-3,4-dihydro-9H,11H-3,11a-methanopyrazino[1',2':3,4]i midazo[1,2-c]pyrimidin-1,9(2H)-dione | 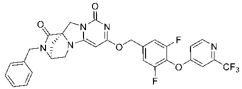 | B | 611 | CDCl₃: δ8.61 (d, *J* = 5.4 Hz, 1H), 7.37 (br. s., 3H), 7.31 - 7.27 (m, 3H), 7.12 (d, *J* = 8.3 Hz, 2H), 6.99 (br. s., 1H), 5.47 - 5.32 (m, 2H), 5.13 (s, 1H), 4.67 (d, *J* = 14.7 Hz, 1H), 4.54 - 4.31 (m, 3H), 4.21 (br. s., 1H), 3.33 - 3.08 (m, 2H), 2.12 (d, J= 8.3 Hz, 1H), 1.87 (d, *J* = 9.3 Hz, 1H) |
| **132** | (3*S*,11a*R*)-7-(4-(3-(trifluoromethyl)phenoxy)ph enethoxy)-3,4-dihydro-1H,9H,11H-3,11a-methanopyrimido[6', 1':2,3]i midazo[5, 1-c][1,4]oxazin-9-one | 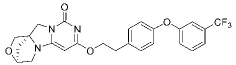 | B | 486 | DMSO-*d*₆: δ7.65 - 7.54 (m, 1H), 7.46 (d, *J* = 7.8 Hz, 1H), 7.34 (d, *J* = 7.8 Hz, 1H), 7.30 - 7.20 (m, 2H), 7.04 (d, *J* = 8.3 Hz, 2H), 5.19 (s, 1H), 4.45 - 4.44 (m, 1H), 4.38 (t, *J* = 6.6 Hz, 2H), 3.84 (s, 2H), 3.29 (s, 2H), 2.97 (t, *J* = 6.6 Hz, 2H), 2.93 (br. s., 1H), 2.03 (br. s., 2H), 1.55 (d, *J= 3.4* Hz, 2H) |
| **133** | 3-(4-(3-(trifluoromethyl)phenoxy)ph enethoxy)-7,8-dihydro-1H,6H,9H-7,8a-methanopyrrolo[1',2': 3,4] imi dazo[1,2-c]pyrimidin-1-one | 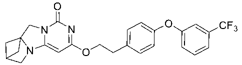 | B | 470 | DMSO-*d*₆: δ7.65 - 7.54 (m, 1H), 7.46 (d, *J* = 7.8 Hz, 1H), 7.34 (d, *J* = 7.8 Hz, 1H), 7.30 - 7.20 (m, 2H), 7.04 (d, *J* = 8.3 Hz, 2H), 5.19 (s, 1H), 4.45 - 4.44 (m, 1H), 4.38 (t, *J* = 6.6 Hz, 2H), 3.84 (s, 2H), 3.29 (s, 2H), 2.97 (t, *J* = 6.6 Hz, 2H), 2.93 (br. s., 1H), 2.03 (br. s., 2H), 1.55 (d, *J=* 3.4 Hz, 2H) |
| **134** | (3*S*,11a*R*)-7-(((3-fluoro-4-((2-(trifluoromethyl)pyridin-4-yl)oxy)benzyl)oxy)-3,4-dihydro-1H,9H,11H-3,11a-methanopyrimido[6',1':2,3]i midazo[5,1-c][1,4]oxazin-9-one-11,11-d₂ | 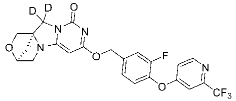 | B | 493 | CDCl₃: δ8.65 (d, *J* = 5.4 Hz, 1H), 7.60 - 7.43 (m, 3H), 7.38 (d, *J* = 8.3 Hz, 1H), 7.19 (d, *J* = 3.4 Hz, 1H), 5.45 - 5.15 (m, 3H), 4.68 (br. s., 1H), 3.95 - 3.76 (m, 2H), 3.39 - 3.36 (m, 1H), 3.30 - 3.24 (m, 1H), 1.89 (d, *J* = 9.3 Hz, 1H), 1.79 (d, *J* = 10.3 Hz, 1H) |
| **135** | (3*S*,11a*R*)-7-((3,5-difluoro-4-phenoxybenzyl)oxy)-3,4-dihydro-1H,9H,11H-3,11a-methanopyrimido[6',1':2,3]i midazo[5,1-c][1,4]oxazin-9-one | 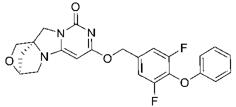 | B | 440 .2 | CDCl₃: δ7.26 - 7.20 (m, 2H), 7.02 (d, J=7.8 Hz, 3H), 6.89 (d, *J=7.8* Hz, 2H), 5.50 - 5.23 (m, 2H), 5.07 (s, 1H), 4.68 (br. s., 1H), 4.42 (d, *J= 12.7* Hz, 1H), 4.11 - 3.79 (m, 3H), 3.52 (d, *J* = 9.3 Hz, 1H), 3.19 (d, *J* = 9.8 Hz, 1H), 1.99 (d, *J* = 9.8 Hz, 1H), 1.69 (d, *J=* 10.3 Hz, 1H) |
| **136** | (3*S*,11a*R*)-7-(((4-(3,4-difluorophenoxy)-3,5-difluorobenzyl)oxy)-3,4-dihydro-1H,9H,11H-3,11a-methanopyrimido[6',1':2,3]i midazo[5,1-c][1,4]oxazin-9-one | 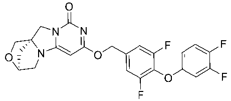 | B | 476 | CDCl₃: δ7.13 - 6.99 (m, 3H), 6.84 - 6.72 (m, 1H), 6.65 (d, *J=* 8.3 Hz, 1H), 5.44 - 5.31 (m, 2H), 5.09 (s, 1H), 4.73 (br. s., 1H), 4.45 (d, *J* = 12.7 Hz, 1H), 4.07 - 3.87 (m, 3H), 3.56 (d, *J* = 9.8 Hz, 1H), 3.23 *(d, J* = 9.8 Hz, 1H), 2.03 (d, *J=* 9.8 Hz, 1H), 1.73 (d, *J=* 10.3 Hz, 1H) |
| **137** | (3*S*,11a*R*)-7-(((3,5-difluoro-4-(3-fluorophenoxy)benzyl)oxy)-3,4-dihydro-1H,9H,11H-3,11a-methanopyrimido[6',1':2,3]i midazo[5,1-c][1,4]oxazin-9-one | 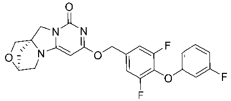 | B | 458 | CDCl₃: δ7.26 - 7.17 (m, 1H), 7.07 (d, *J=* 8.3 Hz, 2H), 6.87 - 6.69 (m, 2H), 6.65 (d, *J* = 9.8 Hz, 1H), 5.56 - 5.27 (m, 2H), 5.11 (br. s., 1H), 4.74 (br. s., 1H), 4.47 (d, *J* = 12.2 Hz, 1H), 4.15 - 3.83 (m, 3H), 3.58 (d, *J* = 9.3 Hz, 1H), 3.24 (d, *J= 9.8* Hz, 1H), 2.04 (d, *J* = 9.3 Hz, 1H), 1.74 (d, *J= 9.8* Hz, 1H) |
| **138** | 3-((3-fluoro-4-((2-(trifluoromethyl)pyridin-4-yl)oxy)benzyl)oxy)-7-(methoxymethyl)-7,8-dihydro-1H,6H,9H-7,8a-methanopyrrolo[1',2': 3,4] imi dazo[1,2-c]pyrimidin-1-one | 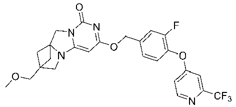 | B | 519 | CDCl₃: δ8.54 (d, *J =* 5.4 Hz, 1H), 7.30 (d, *J* = 10.8 Hz, 1H), 7.22 (d, *J* = 9.3 Hz, 2H), 7.19 - 7.11 (m, 1H), 6.93 (d, *J* = 4.4 Hz, 1H), 5.40 (s, 2H), 5.12 (s, 1H), 3.98 (s, 2H), 3.59 (s, 2H), 3.35 (s, 3H), 3.27 (s, 2H), 1.96 (br. s., 2H), 1.77 (br. s., 2H) |
| **139** | 7-(difluoromethyl)-3-((3-fluoro-4-((2-(trifluoromethyl)pyridin-4-yl)oxy)benzyl)oxy)-7,8-dihydro-1H,6H,9H-7,8a-methanopyrrolo[1',2':3,4]imi dazo[1,2-c]pyrimidin-1-one | 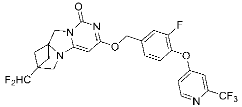 | B | 525 | CDCl₃: δ8.58 (br. s., 1H), 7.33 (d, *J* = 10.8 Hz, 2H), 7.25 - 7.15 (m, 2H), 6.96 (br. s., 1H), 6.24 - 5.81 (m, 1H), 5.44 (br. s., 2H), 5.20 (br. s., 1H), 4.06 (br. s., 2H), 3.46 (br. s., 2H), 2.22 (br. s., 2H), 1.93 (br. s., 2H) |
| **140** | 3-((4-((2-(difluoromethyl)pyridin-4-yl)oxy)-3-fluorobenzyl)oxy)-4-methoxy-7,8-dihydro-1H,6H,9H-7,8a-methanopyrrolo[1',2':3,4]imi dazo[1,2-c]pyrimidin-1-one | 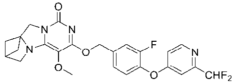 | B | 487 | CDCl₃: δ8.51 (d, *J* = 5.4 Hz, 1H), 7.36 (d, *J=* 10.8 Hz, 1H), 7.31 (br. s., 1H), 7.22 - 7.12 (m, 2H), 6.91 (d, *J* = 3.4 Hz, 1H), 6.76 - 6.41 (m, 1H), 5.49 (s, 2H), 4.00 (br. s., 2H), 3.73 (s, 3H), 3.62 (s, 2H), 3.04 (br. s., 1H), 2.07 (br. s., 2H), 1.83 - 1.72 (m, 2H) |
| **141** | 3-fluoro-5-((((3*S*,11a*R*)-9-oxo-3,4-dihydro-1H,9H,11H-3,11a-methanopyrimido[6',1':2,3]i midazo[5, 1-c][1,4]oxazin-7-yl)oxy)methyl)benzonitrile | 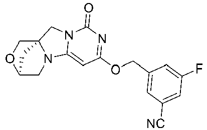 | B | 355 | CDCl₃: δ7.48 (s, 1H), 7.35 (d, *J=* 9.3 Hz, 1H), 7.30 (br. s., 1H), 5.52 - 5.36 (m, 2H), 5.11 (s, 1H), 4.75 (s, 1H), 4.46 (d, *J=* 12.7 Hz, 1H), 4.04 - 3.90 (m, 3H), 3.59 (d, *J* = 9.8 Hz, 1H), 3.26 (d, *J* = 9.8 Hz, 1H), 2.05 (d, *J* = 10.3 Hz, 1H), 1.75 (d, *J=* 10.3 Hz, 1H) |
| **142** | 2-fluoro-5-((((3*S*,11a*R*)-6-methoxy-9-oxo-3,4-dihydro-1H,9H,11H-3,11a-methanopyrimido[6',1':2,3]i midazo[5,1-c][1,4]oxazin-7-yl)oxy)methyl)benzonitrile | 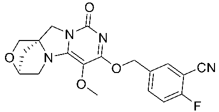 | B | 385 | CDCl₃: δ7.71 (br. s., 2H), 7.25 - 7.16 (m, 1H), 5.56 - 5.33 (m, 2H), 3.99 (s, 2H), 3.69 (s, 3H), 3.61 (s, 2H), 3.04 (br. s., 1H), 2.07 (br. s., 2H), 1.93 - 1.73 (m, 2H) |
| **143** | (3*S*,11a*R*)-7-((4-(4-chloro-3-fluorophenoxy)-3-fluorobenzyl)oxy)-3,4-dihydro-1H,9H,11H-3,11a-methanopyrimido[6',1':2,3]i midazo[5,1-c][1,4]oxazin-9-one | 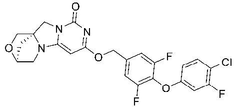 | B | 492 | CDCl₃: δ7.34 - 7.30 (m, 1H), 7.08 (d, *J* = 8.3 Hz, 2H), 6.81 - 6.66 (m, 2H), 5.40 (d, *J* = 7.8 Hz, 2H), 5.11 (s, 1H), 4.75 (br. s., 1H), 4.47 (d, *J=* 11.7 Hz, 1H), 4.08 - 3.91 (m, 3H), 3.59 (d, *J* = 9.3 Hz, 1H), 3.25 (d, *J* = 9.3 Hz, 1H), 2.05 (d,*J* = 8.3 Hz, 1H), 1.75 (d, *J* = 9.8 Hz, 1H) |
| **144** | (3*S*,11a*R*)-7-((3,5-difluoro-4-(4-(trifluoromethyl)phenoxy)be nzyl)oxy)-3,4-dihydro-1H,9H,11H-3,11a-methanopyrimido[6' 1':2,3]i midazo[5, 1-c][1,4]oxazin-9-one | 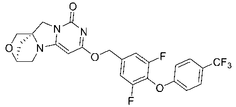 | B | 508 | CDCl₃: δ7.57 (d, *J* = 8.3 Hz, 2H), 7.14 - 6.96 (m, 4H), 5.48 - 5.32 (m, 2H), 5.12 (s, 1H), 4.75 (br. s., 1H), 4.47 (d, *J* = 12.2 Hz, 1H), 4.07 - 3.90 (m, 3H), 3.59 (d, *J* = 9.8 Hz, 1H), 3.25 (d, *J* = 9.3 Hz, 1H), 2.05 (d, *J* = 9.8 Hz, 1H), 1.75 (d, *J* = 10.3 Hz, 1H) |
| **145** | (3*S*,11a*R*)-7-((3,5-difluoro-4-(3-(trifluoromethoxy)phenoxy) benzyl)oxy)-3,4-dihydro-1H,9H,11H-3,11a-methanopyrimido[6',1':2,3]i midazo[5,1-c][1,4]oxazin-9-one | 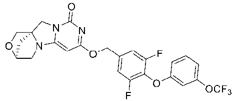 | B | 524 | CDCl₃: δ7.34 - 7.29 (m, 1H), 7.08 (d, *J* = 8.3 Hz, 2H), 6.95 (d, *J* = 7.8 Hz, 1H), 6.89 - 6.79 (m, 2H), 5.40 (d, *J* = 9.3 Hz, 2H), 5.12 (s, 1H), 4.75 (br. s., 1H), 4.47 (d, *J* = 12.2 Hz, 1H), 4.07 - 3.89 (m, 3H), 3.59 (d, *J* = 9.3 Hz, 1H), 3.25 (d, *J* = 9.3 Hz, 1H), 2.05 (d, *J* = 9.3 Hz, 1H), 1.75 (d, *J=* 10.3 Hz, 1H) |
| **146** | (3S,11a*R*)-7-((4-(3,4-difluorophenoxy)-3-fluorobenzyl)oxy)-3,4-dihydro-1H,9H,11H-3,11a-methanopyrimido[6',1':2,3]i midazo[5,1-c][1,4]oxazin-9-one | 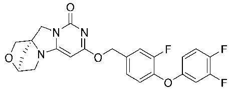 | B | 458 | CDCl₃: δ7.25 (s, 1H), 7.19 - 7.00 (m, 3H), 6.80 (dt, *J* = 3.4, 7.1 Hz, 1H), 6.69 (d, *J* = 8.8 Hz, 1H), 5.45 - 5.32 (m, 2H), 5.09 (s, 1H), 4.73 (s, 1H), 4.46 (d, *J=* 12.7 Hz, 1H), 4.04 - 3.87 (m, 3H), 3.57 (d, *J* = 9.8 Hz, 1H), 3.23 (d, *J* = 9.8 Hz, 1H), 2.03 (d, *J=* 9.8 Hz, 1H), 1.74 (br. s., 1H) |
| **147** | (3*S*,11a*R*)-7-(((3,5-difluoro-4-(4-fluorophenoxy)benzyl)oxy)-3,4-dihydro-1H,9H,11H-3,11a-methanopyrimido[6', 1':2,3]i midazo[5,1-c][1,4]oxazin-9-one | 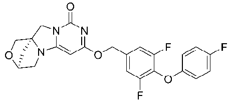 | B | 458 | CDCl₃: δ7.05 (d, *J* = 7.8 Hz, 2H), 7.02 - 6.94 (m, 2H), 6.94 - 6.87 (m, 2H), 5.44 - 5.33 (m, 2H), 5.11 (s, 1H), 4.74 (s, 1H), 4.47 (d, *J* = 12.2 Hz, 1H), 4.02 - 3.93 (m, 3H), 3.58 (d, *J* = 9.8 Hz, 1H), 3.24 (d, *J* = 9.8 Hz, 1H), 2.04 (d, *J* = 9.8 Hz, 1H), 1.74 (d, *J=* 10.3 Hz, 1H) |
| **148** | 3-((3-fluoro-4-((5-(trifluoromethyl)pyridin-3-yl)oxy)benzyl)oxy)-7,8-dihydro-1H,6H,9H-7,8a-methanopyrrolo[1',2':3,4]imi dazo[1,2-c]pyrimidin-1-one | 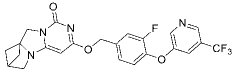 | B | 475 | CDCl₃: δ8.57 (br. s., 1H), 8.51 (br. s., 1H), 7.37 (br. s., 1H), 7.28 (d, *J* = 11.2 Hz, 1H), 7.21 (d, *J* = 8.3 Hz, 1H), 7.12 (d, *J* = 7.8 Hz, 1H), 5.38 (s, 2H), 5.11 (s, 1H), 3.96 (s, 2H), 3.30 (s, 2H), 2.99 (br. s., 1H), 2.04 (br. s., 2H), 1.72 - 1.60 (m, 2H) |
| **149** | 3-((3-fluoro-4-((2-(trifluoromethyl)pyridin-4-yl)oxy)benzyl)oxy)-7,8-dihydro-1H,6H,9H-6,8a-ethanopyrrolo[1',2': 3,4] imid azo[1,2-c]pyrimidin-1-one | 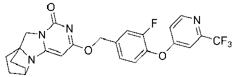 | B | 489 | CDCl₃: δ8.56 (br. s., 1H), 8.50 (br. s., 1H), 7.36 (br. s., 1H), 7.27 (d, *J* = 10.8 Hz, 1H), 7.20 (d, *J* = 7.8 Hz, 1H), 7.12 (d, *J* = 7.8 Hz, 1H), 5.36 (br. s., 2H), 5.16 (s, 1H), 4.08 (br. s., 1H), 3.96 (s, 2H), 2.04 (br. s., 2H), 1.73 (br. s., 6H) |
| **150** | 3-((3,4-difluorobenzyl)oxy)-4-methoxy-7,8-dihydro-1H,6H,9H-7,8a-methanopyrrolo[1',2':3,4]imi dazo[1,2-c]pyrimidin-1-one | 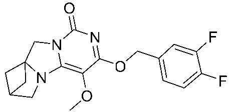 | B | 362 | CDCl₃: δ7.26 (br. s., 1H), 7.19 - 7.08 (m, 2H), 5.39 (s, 2H), 3.97 (s, 2H), 3.67 (s, 3H), 3.59 (s, 2H), 3.01 (br. s., 1H), 2.05 (br. s., 2H), 1.70 - 1.64 (m, 2H) |
| **151** | 4-methoxy-3-(((3,4,5-trifluorobenzyl)oxy)-7,8-dihydro-1H,6H,9H-7,8a-methanopyrrolo[1',2':3,4]imi dazo[1,2-c]pyrimidin-1-one | 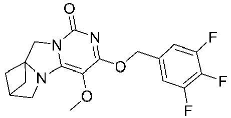 | B | 380 | CDCl₃: δ7.06 (t, *J* = 7.1 Hz, 2H), 5.37 (s, 2H), 3.97 (s, 2H), 3.69 (s, 3H), 3.60 (s, 2H), 3.02 (br. s., 1H), 2.05 (br. s., 2H), 1.71 - 1.66 (m, 2H) |
| **152** | 7-((3-fluoro-4-((2-(trifluoromethyl)pyridin-4-yl)oxy)benzyl)oxy)-3,4-dihydro-1H,9H,11H-4,11a-ethanopyrimido[6',1':2,3]imi dazo[5,1-c][1,4]oxazin-9-one | 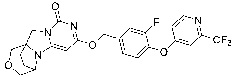 | B | 505 | CDCl₃: δ8.55 (d, *J* = 5.4 Hz, 1H), 7.31 (d, *J=* 10.8 Hz, 1H), 7.25 (d, *J* = 8.3 Hz, 1H), 7.20 (br. s., 1H), 7.20 - 7.14 (m, 1H), 6.94 (d, *J* = 3.9 Hz, 1H), 5.41 (s, 2H), 5.20 (s, 1H), 4.23 (d, *J* = 11.2 Hz, 1H), 4.00 - 3.88 (m, 2H), 3.75 - 3.65 (m, 4H), 2.32 - 2.23 (m, 1H), 2.08 - 2.00 (m, 1H), 1.92 (dd, *J* = 5.9, 11.7 Hz, 1H), 1.70 (dt, *J* = 3.4, 12.2 Hz, 1H) |
| **153** | 3-((3-fluoro-4-(((2-(trifluoromethoxy)pyridin-4-yl)oxy)benzyl)oxy)-7,8-dihydro-1H,6H,9H-7,8a-methanopyrrolo[1',2':3,4]imi dazo[1,2-c]pyrimidin-1-one | 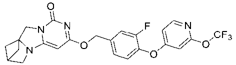 | B | 491 | CDCl₃: δ8.17 (d, *J* = 5.4 Hz, 1H), 7.31 (d, *J =* 11.2 Hz, 1H), 7.26 (br. s., 1H), 7.20 - 7.13 (m, 1H), 6.78 (d, *J* = 4.9 Hz, 1H), 6.46 (s, 1H), 5.41 (s, 2H), 5.14 (s, 1H), 3.99 (s, 2H), 3.33 (s, 2H), 3.02 (br. s., 1H), 2.07 (br. s., 2H), 1.71 - 1.67 (m, 2H) |
| **154** | 3-((3-fluoro-4-((2-(trifluoromethoxy)pyridin-4-yl)oxy)benzyl)oxy)-7,8-dihydro-1H,6H,9H-6,8a-ethanopyrrolo[1',2': 3,4] imid azo[1,2-c]pyrimidin-1-one | 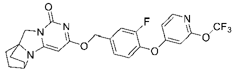 | B | 505 | CDCl₃: δ8.17 (br. s., 1H), 7.36 - 7.28 (m, 1H), 7.27 - 7.21 (m, 1H), 7.17 (br. s., 1H), 6.78 (br. s., 1H), 6.45 (br. s., 1H), 5.40 (br. s., 2H), 5.19 (br. s., 1H), 4.11 (br. s., 1H), 4.00 (br. s., 2H), 2.08 (br. s., 2H), 1.77 (br. s., 6H) |
| **155** | 3-(((4-((2-chloropyridin-4-yl)oxy)-3-fluorobenzyl)oxy)-7,8-dihydro-1H,6H,9H-7,8a-methanopyrrolo[1',2': 3,4] imi dazo[1,2-c]pyrimidin-1-one | 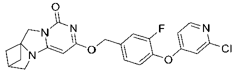 | B | 441 | CDCl₃: δ8.20 (d, *J* = 5.4 Hz, 1H), 7.27 (d, *J= 10.8* Hz, 1H), 7.21 (d, *J = 8.3* Hz, 1H), 7.12 (t, *J* = 7.8 Hz, 1H), 6.84 - 6.67 (m, 2H), 5.38 (s, 2H), 5.11 (s, 1H), 3.96 (s, 2H), 3.30 (s, 2H), 2.99 (br. s., 1H), 2.03 (br. s., 2H), 1.66 (br. s., 2H) |
| **156** | (3*S*,11a*R*)-7-(3-fluoro-4-((2-(trifluoromethyl)pyridin-4-yl)oxy)phenethyl)-3,4-dihydro-1H,9H,11H-3,11a-methanopyrimido[6',1':2,3]i midazo[5,1-c][1,4]oxazin-9-one | 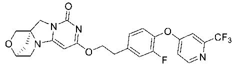 | B | 505 | DMSO-*d*₆: δ8.63 (d, *J* = 5.9 Hz, 1H), 7.52 - 7.32 (m, 3H), 7.24 (d, *J =* 8.3 Hz, 1H), 7.18 - 7.09 (m, 1H), 5.17 (s, 1H), 4.65 (br. s., 1H), 4.53 - 4.34 (m, 2H), 4.25 (d, *J =* 12.2 Hz, 1H), 3.95 - 3.75 (m, 3H), 3.32 (br. s., 1H), 3.28 - 3.18 (m, 1H), 3.02 (t, *J* = 6.1 Hz, 2H), 1.88 (d, *J* = 9.8 Hz, 1H), 1.74 (d, *J =* 10.3 Hz, 1H) |
| **157** | 7-fluoro-3-((3-fluoro-4-((2-(trifluoromethyl)pyridin-4-yl)oxy)benzyl)oxy)-7,8-dihydro-1H,6H,9H-7,8a-methanopyrrolo[1',2':3,4]imi dazo[1,2-c]pyrimidin-1-one | 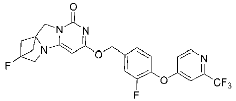 | B | 493 | CDCl₃: δ8.55 (d, *J* = 5.4 Hz, 1H), 7.31 (d, *J* = 10.8 Hz, 1H), 7.24 (br. s., 1H), 7.22 - 7.13 (m, 2H), 6.94 (d, *J* = 4.4 Hz, 1H), 5.42 (s, 2H), 5.17 (s, 1H), 4.11 (s, 2H), 3.43 (s, 2H), 2.33 (br. s., 2H), 2.20 (br. s., 2H) |
| **158** | 3-((3,5-difluoro-4-((2-(trifluoromethyl)pyridin-4-yl)oxy)benzyl)oxy)-7-fluoro-7,8-dihydro-1H,6H,9H-7,8a-methanopyrrolo[1',2':3,4]imi dazo[1,2-c]pyrimidine-1-one | 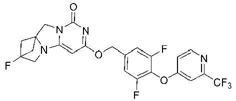 | B | 511 | CDCl₃: δ8.58 (d, *J* = 5.4 Hz, 1H), 7.24 (br. s., 1H), 7.11 (d, *J* = 8.3 Hz, 2H), 7.00 - 6.92 (m, 1H), 5.40 (s, 2H), 5.18 (s, 1H), 4.10 (s, 2H), 3.44 (s, 2H), 2.34 (br. s., 2H), 2.20 (br. s., 2H) |
| **159** | 3-fluoro-5-(((((7-fluoro-1-oxo-7,8-dihydro-1H,6H,9H-7,8a-methanopyrrolo[1',2':3,4]imi dazo[1,2-c]pyrimidin-3-yl)oxy)methyl)benzonitrile | 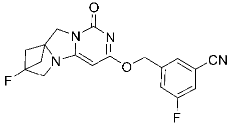 | B | 357 | CDCl₃: δ7.48 (s, 1H), 7.35 (d, *J=* 8.8 Hz, 1H), 7.30 (br. s., 1H), 5.42 (s, 2H), 5.18 (s, 1H), 4.11 (s, 2H), 3.45 (s, 2H), 2.35 (br. s., 2H), 2.22 (br. s., 2H) |
| **160** | 2-fluoro-5-(((((7-fluoro-1-oxy-7,8-dihydro-1H,6H,9H-7,8a-methanopyrrolo[1',2':3,4]imi dazo[1,2-c]pyrimidin-3-yl)oxy)methyl)benzonitrile | 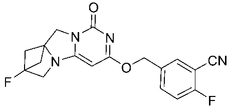 | B | 357 | CDCl₃: δ7.95 (br. s., 1H), 7.81 (br. s., 1H), 7.53 (br. s., 1H), 5.36 (br. s., 1H), 5.28 (br. s., 2H), 4.00 (br. s., 2H), 3.52 (br. s., 2H), 2.37 (br. s., 2H), 2.14 (br. s., 2H) |
| **161** | 3-(((4-((2-(difluoromethyl)pyridin-4-yl)oxy)benzyl)oxy)-7,8-dihydro-1H,6H,9H-7,8a-methanopyrrolo[1',2': 3,4] imi dazo[1,2-c]pyrimidin-1-one | 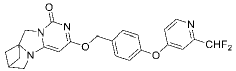 | B | 439 | DMSO-*d*₆: δ8.57 (s, 1H), 7.53 (s, 2H), 7.28 - 7.20 (m, 2H), 7.18 - 7.13 (m, 1H), 7.10 - 7.03 (m, 1H), 6.96 - 6.75 (m, 1H), 5.32 - 5.29 (m, 3H), 3.86 (s, 3H), 3.31 (s, 2H), 2.97 - 2.90 (m, 1H), 2.09 - 2.01 (m, 2H), 1.57 (br. s., 1H) |
| **162** | 3-((4-((2-(difluoromethyl)pyridin-4-yl)oxy)-3-fluorobenzyloxy)-7,8-dihydro-1H,6H,9H-7,8a-methanopyrrolo[1',2': 3,4] imi dazo[1,2-c]pyrimidin-1-one | 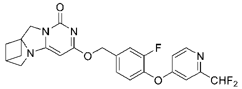 | B | 457 | CDCl₃: δ8.50 (d, *J* = 5.4 Hz, 1H), 7.33 (d, *J= 10.8* Hz, 1H), 7.25 (br. s., 1H), 7.22 - 7.11 (m, 2H), 6.91 (d, *J* = 3.9 Hz, 1H), 6.80 - 6.41 (m, 1H), 5.43 (s, 2H), 5.16 (s, 1H), 4.01 (s, 2H), 3.34 (s, 2H), 3.04 (br. s., 1H), 2.08 (br. s., 2H), 1.71 (br. s., 2H) |
| **163** | 2-fluoro-5-(((1-oxo-7,8-dihydro-1H,6H,9H-6,8a-ethanopyrrolo[1',2':3,4]imid azo[1,2-c]pyrimidin-3-yl)oxy)methyl)benzonitrile | 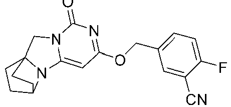 | B | 353 | CDCl₃: δ7.67 (br. s., 2H), 7.19 (t, *J=* 8.1 Hz, 1H), 5.38 (br. s., 2H), 5.17 (br. s., 1H), 4.12 (br. s., 1H), 3.99 (br. s., 2H), 2.09 (br. s., 2H), 1.78 (br. s., 6H) |
| **164** | (3*S*,11a*R*)-7-((3-fluoro-4-((2-(trifluoromethyl)pyridin-4-yl)oxy)benzyl)oxy)-6-(trifluoromethyl)-3,4-dihydro-1H,9H,11H-3,11a-methanopyrimido[6',1':2,3]i midazo[5,1-c][1,4]oxazin-9-one | 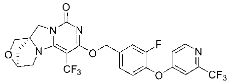 | B | 559 | CDCl₃: δ8.58 (d, *J* = 4.9 Hz, 1H), 7.33 (d, *J= 11.2* Hz, 1H), 7.26 - 7.08 (m, 3H), 6.96 (br. s., 1H), 5.63 - 5.36 (m, 2H), 4.74 (br. s., 1H), 4.46 (d, *J=* 12.7 Hz, 1H), 4.07 (d, *J* = 7.3 Hz, 1H), 4.00 (d, *J= 7.3 Hz,* 1H), 3.94 (d, *J =* 12.7 Hz, 1H), 3.82 (d, *J* = 10.3 Hz, 1H), 3.51 (d, *J=* 10.3 Hz, 1H), 2.13 (d, *J= 10.3* Hz, 1H), 1.88 (d, *J= 10.3* Hz, 1H) |
| **165** | 3-((3-fluoro-4-((2-(trifluoromethyl)pyridin-4-yl)oxy)benzyl)oxy)-6,7,8,9-tetrahydro-1H,10H-8,9a-methano-pyrido[1',2':3,4]imidazo[1,2-c]pyrimidin-1-one | 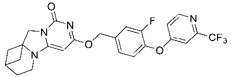 | B | 489 | CDCl₃: δ8.56 (d, *J* = 4.9 Hz, 1H), 7.32 (d, *J= 10.8* Hz, 1H), 7.24 (br. s., 1H), 7.22 (br. s., 1H), 7.20 - 7.14 (m, 1H), 6.99 - 6.90 (m, 1H), 5.48 - 5.34 (m, 2H), 5.18 (s, 1H), 4.38 (d, *J=* 12.7 Hz, 1H), 3.94 (d, *J= 12.7* Hz, 1H), 3.72 (d, *J=* 12.2 Hz, 1H), 3.62 - 3.48 (m, 1H), 2.93 (br. s., 1H), 2.55 - 2.42 (m, 1H), 2.39 - 2.25 (m, 1H), 2.22 - 2.09 (m, 1H), 1.37 - 1.31 (m, 1H), 1.28 (br. s., 2H) |
| **166** | 3-((3-fluoro-4-((2-(trifluoromethyl)pyridin-4-yl)oxy)benzyl)oxy)-7-(morpholinomethyl)-7,8-dihydro-1H,6H,9H-7,8a-methanopyrrolo[1',2':3,4]imi dazo[1,2-c]pyrimidin-1-one | 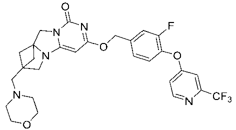 | B | 574 | CDCl₃: δ8.59 (d, *J* = 4.9 Hz, 1H), 7.35 (d, *J= 11.2* Hz, 1H), 7.30 - 7.28 (m, 1H), 7.25 (s, 1H), 7.23 - 7.16 (m, 1H), 7.01 - 6.89 (m, 1H), 5.45 (s, 2H), 5.16 (s, 1H), 4.01 (s, 2H), 3.71 (br. s., 4H), 3.34 (br. s., 2H), 2.78 - 2.66 (m, 2H), 2.47 (br. s., 4H), 1.97 (br. s., 2H), 1.81 (br. s., 2H) |
| **167** | (3*S*,11a*R*)-7-((3,5-difluoro-4-((1-methyl-1H-pyrazol-4-yl)oxy)benzyl)oxy)-2-methyl-3,4-dihydro-9H,11H-3,11a-methanopyrazino[1',2':3,4]i midazo[1,2-c]pyrimidine-1,9(2H)-dione | 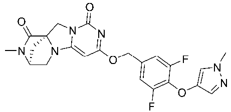 | B | 471 | CDCl₃: δ7.26 - 7.23 (m, 1H), 7.19 (br. s., 1H), 7.01 (d, *J* = 7.3 Hz, 2H), 5.46 - 5.24 (m, 2H), 5.14 (s, 1H), 4.43 - 4.15 (m, 3H), 3.82 (s, 3H), 3.50 - 3.28 (m, 2H), 2.96 (s, 3H), 2.13 (d, *J=* 9.3 Hz, 1H), 1.88 (d, *J* = 9.3 Hz, 1H) |
| **168** | 2-(4-chloro-3-(trifluoromethyl)phenoxy)-5-((((3*S*,11a*R*)-2-methyl-1,9-dioxo-1,2,3,4-tetrahydro-9H,11H-3,11a-methanopyrazino[1',2':3,4]i midazo[1,2-c]pyrimidine-7-yl)oxy)methyl)benzonitrile | 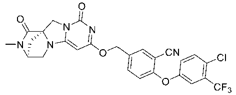 | B | 558 | CDCl₃: δ7.74 (s, 1H), 7.62 - 7.50 (m, 2H), 7.41 (br. s., 1H), 7.18 (d, *J =* 8.3 Hz, 1H), 6.91 (d, *J =* 8.3 Hz, 1H), 5.52 - 5.31 (m, 2H), 5.14 (s, 1H), 4.41 - 4.28 (m, 2H), 4.23 (br. s., 1H), 3.48 - 3.32 (m, 2H), 2.96 (s, 3H), 2.14 (d, *J* = 9.8 Hz, 1H), 1.89 (d, *J* = 9.8 Hz, 1H) |

### Example 169

### Method C

### 3-(3,5-Difluoro-4-((2-(trifluoromethyl)pyridin-4-yl)oxy)phenethoxy)-7,8-dihydro-1H,6H,9H-7, 8a-methanopyrrolo[1', 2':3, 4]imidazo[1, 2-c]pyrimidin-1-one

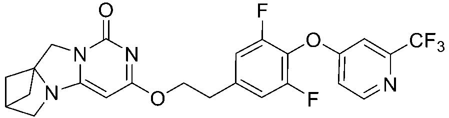

3-Chloro-7,8-dihydro-1H,6H,9H-7,8a-methanopyrrolo[1',2':3,4]imidazo[1,2-c]pyrimidin-1-one (80 mg, 0.359 mmol), 2-(3,5-difluoro-4-((2-(trifluoromethyl)pyridin-4-yl)oxy)phenyl) ethan-1-ol (137 mg, 0.43 mmol) and cesium carbonate (233 mg, 0.718 mmol) were added to toluene (10 mL) and stirred at 120°C for 16 hours. The reaction solution was concentrated and purified by thin layer chromatography to obtain the title compound (68 mg, 37 %).

LC-MS: *m*/*z* [M+H]⁺ = 507.

¹H NMR (400 MHz, CDCl₃) δ8.58 (d, *J =* 5.4 Hz, 1H), 7.24 (br. s., 1H), 6.96 (d, *J =* 8.3 Hz, 3H), 5.04 (s, 1H), 4.57 (t, *J =* 5.9 Hz, 2H), 3.96 (s, 2H), 3.31 (s, 2H), 3.03 (t, *J =* 6.4 Hz, 3H), 2.04 (br. s., 2H), 1.81 (br. s., 2H).

### Example 170

### Method D

### (3S,11aR)-6-Methoxy-7-((4-((2-(trifluoromethyl)pyridin-4-yl)oxy)phenyl)ethynyl)-3,4-dihvdro-1H,9H,11H-3,11a-methanopyrimido[6',1':2,3]imidazo[5,1-c][1,4]oxazin-9-one

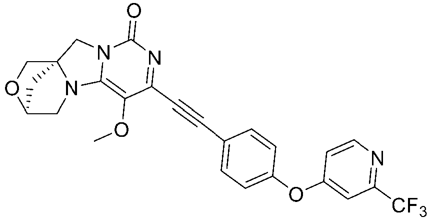

4-(4-Ethynylphenoxy)-2-(trifluoromethyl)pyridine (195.66 mg, 0.74 mmol) was dissolved in anhydrous tetrahydrofuran (10 mL), and the temperature was lowered to -50°C under argon protection, a solution of n-butyllithium in tetrahydrofuran (2 M, 0.74 mL, 1.44 mmol) was added dropwise, the reaction solution was stirred for 15 minutes, then heated to 0°C, and zinc chloride (150.96 mg, 1.11 mmol) was added. After the reaction solution was stirred for 30 minutes, (3*S*,11a*R*)-7-chloro-6-methoxy-3,4-dihydro-1H,9H,11H-3,11a-methanopyrimido[6',1':2,3]imidazo[5,1-c][1,4]oxazin-9-one (200 mg, 0.742 mmol), Pd₂(dba)₃ (67.76 mg, 0.074 mmol) and 2-dicyclohexylphosphine-2,4,6-triisopropyl-biphenyl (70.6 mg, 0.148 mmol) were added, and the reaction solution was heated to 120°C and stirred for 2 hours. The reaction solution was diluted with water and extracted with ethyl acetate. The organic phase was concentrated, and purified by preparative thin layer chromatography (dichloromethane/methanol = 20/1) to obtain the title compound (25 mg, 7 %).

LCMS: *m*/*z* [M+H]⁺= 497.

### (3S,11aR)-6-Methoxy-7-(4-((2-(trifluoromethyl)pyridin-4-yl)oxy)phenethyl)-3,4-dihydro-1H,9H,11H-3,11a-methanopyrimido[6',1':2,3]imidazo[5,1-c] [1,4]oxazin-9-one

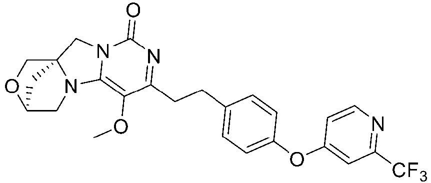

(3*S,*11*aR*)*-*6-Methoxy-7-((4-((2-(trifluoromethyl)pyridin-4-yl)oxy)phenyl)ethynyl)-3,4-dihydro-1H,9H,11H-3,11a-methanopyrimido[6',1':2,3]imidazo[5,1-c][1,4]oxazin-9-one (25 mg, 0.05 mmol) and Pd/C (10%, 5 mg) were added to methanol (5 mL) and hydrogenated at room temperature under atmospheric pressure for 1 hour. The reaction solution was filtered, and the filtrate was concentrated, and purified by preparative thin layer chromatography (dichloromethane/methanol = 20/1) to obtain the title compound (8 mg, 32 %).

LCMS: *m*/*z* [M+H]⁺ = 501.

¹H NMR (400 MHz, CDCl₃) δ8.55 (d, *J =* 5.4 Hz, 1H), 7.34 (d, *J =* 7.8 Hz, 2H), 7.19 (s, 1H), 7.02 (d, *J =* 8.3 Hz, 2H), 6.96 (d, *J =* 3.9 Hz, 1H), 4.74 (br. s., 1H), 4.52 (d, *J =* 12.7 Hz, 1H), 4.09 - 3.96 (m, 3H), 3.72 (s, 2H), 3.57 (s, 3H), 3.18 - 3.03 (m, 2H), 2.98 - 2.84 (m, 2H), 2.09 (d, *J =* 9.8 Hz, 1H), 1.86 (d, *J =* 9.8 Hz, 1H).

### Example 171

### Method E

### (3S,11aS)-7-((3-Fluoro-4-((2-(trifluoromethyl)pyridin-4-yl)oxy)benzyl)oxy)-2-methyl-1,2,3,4-tetrahydro-9H,11H-3,11a-methanopyrazino[1', 2':3, 4]imidazo[1, 2-c]pyrimidin-9-one

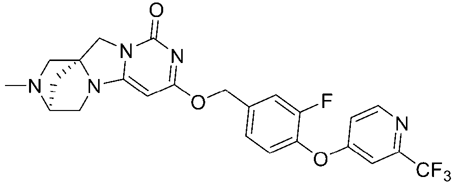

(3*S*,11a*R*)-7-(((3-Fluoro-4-((2-(trifluoromethyl)pyridin-4-yl)oxy)benzyl)oxy)-9-oxo-3,4-dihydro-9H,11H-3,11a-methanopyrazino[1',2':3,4]imidazo[1,2-c]pyrimidine-2(1H)-carboxylic acid tert-butyl ester (36 mg, 0.06 mmol) and trifluoroacetic acid (8.4 mg, 0.07 mmol) were added to dichloromethane (1 mL) and stirred at room temperature for 1 hour. The reaction solution was concentrated, and the residue was added with aqueous formaldehyde (37%, 5 mg, 0.06 mmol), sodium cyanoborohydride (12 mg, 0.18 mmol) and methanol (1 mL), and stirred at room temperature for 1 hour. The reaction solution was concentrated and purified by preparative thin layer chromatography (13 mg, 41 %).

LCMS: *m*/*z* [M+H]⁺= 504.

¹H NMR (400 MHz, CDCl₃) δ8.59 (br. s., 1H), 7.27 (br. s., 4H), 6.97 (br. s., 1H), 5.45 (br. s., 2H), 5.11 (br. s., 1H), 4.37 (br. s., 1H), 3.84 (br. s., 1H), 3.75 - 3.57 (m, 2H), 3.19 (d, *J =* 1.5 Hz, 1H), 2.73 (br. s., 1H), 2.50 (br. s., 3H), 2.00 (br. s., 1H), 0.87 (br. s., 2H).

### Example 172

### Method F

### (3S,11aS)-7-(((3-Fluoro-4-((2-(trifluoromethyl)pyridin-4-yl)oxy)benzyl)oxy)-2-(oxetan-3-yl)-1,2,3,4-tetrahydro-9H,11H-3,11a-methanopyrazino[1',2':3,4]imidazo[1,2-c]pyrimidin-9-one

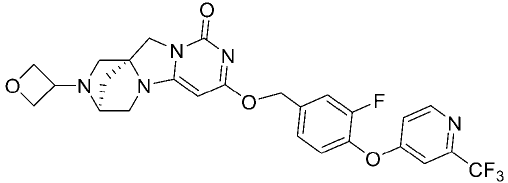

(3*S*,11a*R*)-7-(((3-Fluoro-4-((2-(trifluoromethyl)pyridin-4-yl)oxy)benzyl)oxy)-9-oxo-3,4-dihydro-9H,11H-3,11a-methanopyrazino[1',2':3,4]imidazo[1,2-c]pyrimidine-2(1H)-carboxylic acid tert-butyl ester (80 mg, 0.14 mmol) was added to a solution of hydrogen chloride in ethyl acetate (4.0 M, 1 mL) and dichloromethane (11 mL), and stirred at room temperature for 1 hour. The reaction solution was concentrated, and oxetanone (20 mg, 0.28 mmol), sodium cyanoborohydride (17 mg, 0.28 mmol) and methanol (1 mL) were added to the residue, and stirred at room temperature for 1 hour. The reaction solution was concentrated and purified by preparative thin layer chromatography to obtain the product (60 mg, 81 %).

LCMS: *m*/*z* [M+H]⁺= 546.

1H NMR (400MHz, CDCl₃) δ8.57 (d, *J =* 5.4 Hz, 1H), 7.32 (d, *J =* 10.8 Hz, 1H), 7.27 - 7.16 (m, 3H), 6.96 (d, *J* = 4.4 Hz, 1H), 5.49 - 5.38 (m, 2H), 5.09 (s, 1H), 4.75 (t, *J =* 6.4 Hz, 2H), 4.62 (t, *J* = 5.6 Hz, 1H), 4.56 (t, *J =* 5.9 Hz, 1H), 4.40 (d, *J =* 12.2 Hz, 1H), 4.04 (t, *J =* 5.9 Hz, 1H), 3.89 (d, *J =* 12.2 Hz, 1H), 3.62 (br. s., 1H), 3.43 (d, *J =* 9.8 Hz, 1H), 3.16 (d, *J =* 9.3 Hz, 1H), 3.10 (d, *J =* 9.8 Hz, 1H), 3.00 (d, *J =* 9.3 Hz, 1H), 1.97 (d, *J =* 9.8 Hz, 1H), 1.62 (d, *J =* 9.8 Hz, 1H).

### Example 173

### Method G

### (3S,11aS)-7-(((3-fluoro-4-((2-(trifluoromethyl)pyridin-4-yl)oxy)benzyl)oxy)-2-(2,2,2-trifluoroethyl)-1,2,3,4-tetrahydro-9H,11H-3,11a-methanopyrazino[1',2':3,4]imidazo[1,2-c]pyrimidin-9-one

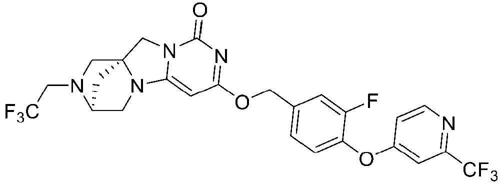

(3*S,*11a*R*)-7-(((3-Fluoro-4-((2-(trifluoromethyl)pyridin-4-yl)oxy)benzyl)oxy)-9-oxo-3,4-dihydro-9H,11H-3,11a-methanopyrazino[1',2':3,4]imidazo[1,2-c]pyrimidine-2(1H)-carboxylic acid tert-butyl ester (80 mg, 0.14 mmol) was added to a solution of hydrogen chloride in ethyl acetate (4.0 M, 1 mL) and dichloromethane (1 mL), and stirred at room temperature for 1 hour. The reaction solution was concentrated, and 2,2,2-trifluoroethyl trifluoromethanesulfonate (49 mg, 0.21 mmol), triethylamine (140 mg, 1.4 mmol) and toluene (1 mL) were added, stirred at 80°C overnight. The reaction solution was poured into saturated ammonium chloride solution, extracted with dichloromethane, the organic phase was concentrated, and purified by preparative thin layer chromatography (25 mg, 32 %).

LCMS: *m*/*z* [M+H]⁺= 572.

¹H NMR (400 MHz, CDCl₃) δ8.58 (d, *J =* 5.4 Hz, 1H), 7.33 (d, *J =* 10.8 Hz, 1H), 7.25 - 7.16 (m, 3H), 6.96 (br. s., 1H), 5.49 - 5.41 (m, 2H), 5.11 (s, 1H), 4.40 (d, *J =* 12.2 Hz, 1H), 3.88 (d, *J* = 12.7 Hz, 1H), 3.78 (br. s., 1H), 3.61 (d, *J =* 10.3 Hz, 1H), 3.44 (d, *J =* 9.3 Hz, 1H), 3.26 - 3.13 (m, 3H), 2.90 (d, *J =* 9.3 Hz, 1H), 2.04 (d, *J =* 9.3 Hz, 1H), 1.67 (d, *J =* 9.8 Hz, 1H).

### Example 174

### Method H

### (3S,11aS)-2-Benzoyl-7-(((3-fluoro-4-((2-(trifluoromethyl)pyridin-4-yl)oxy)benzyl)oxy)-1,2,3,4-tetrahydro-9H,11H-3,11a-methanopyrazino[1',2':3,4]imidazo[1,2-c]pyrimidin-9-one

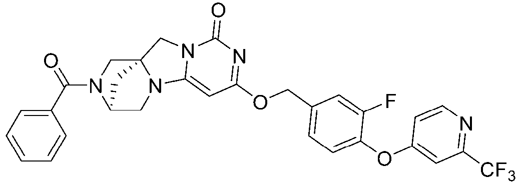

(3*S*,11a*R*)-7-(((3-Fluoro-4-((2-(trifluoromethyl)pyridin-4-yl)oxy)benzyl)oxy)-9-oxo-3,4-dihydro-9H,11H-3,11a-methanopyrazino[1',2':3,4]imidazo[1,2-c]pyrimidine-2(1H)-carboxylic acid tert-butyl ester (80 mg, 0.14 mmol) was added to a solution of hydrogen chloride in ethyl acetate (4.0 M, 1 mL) and dichloromethane (1 mL), and stirred at room temperature for 1 hour. The reaction solution was concentrated, and triethylamine (42 mg, 0.42 mmol), benzoyl chloride (29.4 mg, 0.21 mmol) and dichloromethane (1 mL) were added to the residue, stirred at room temperature overnight. The reaction solution was poured into saturated ammonium chloride solution, extracted with dichloromethane, and the organic phase was concentrated, and purified by thin layer chromatography to obtain the compound (52 mg, 65 %).

LCMS: m/z [M+H]⁺= 594.

¹H NMR (400 MHz, CDCl₃) δ8.57 (d, *J =* 5.4 Hz, 1H), 8.08 (d, *J =* 7.3 Hz, 1H), 7.55 (d, *J =* 5.4 Hz, 2H), 7.50 - 7.44 (m, 1H), 7.32 (d, *J =* 10.8 Hz, 1H), 7.27 - 7.14 (m, 4H), 6.95 (d, *J =* 3.9 Hz, 1H), 5.46 - 5.38 (m, 2H), 5.14 (d, *J =* 7.3 Hz, 1H), 4.61 (br. s., 1H), 4.51 - 4.42 (m, 1H), 4.08 (d, *J =* 12.2 Hz, 1H), 3.96 (d, *J =* 10.8 Hz, 1H), 3.82 (d, *J =* 9.3 Hz, 1H), 3.69 - 3.58 (m, 1H), 3.32 (d, *J =* 10.3 Hz, 1H), 2.12 - 2.03 (m, 1H), 1.86 - 1.75 (m, 1H).

### Example 175

### Method I

### (3S,11aR)-7-((3,4-Difluorobenzyl)amino)-6-methoxy-3,4-dihydro-1H,9H,11H-3,11a-methanopyrimido[6', 1':2, 3]imidazo[5,1-c] [1, 4]oxazin-9-one

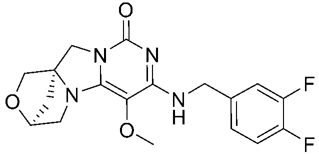

(3*S*,11a*R*)-7-Chloro-6-methoxy-3,4-dihydro-1H,9H,11H-3,11a-methanopyrimido[6',1':2,3]imidazo[5,1-c][1,4]oxazin-9-one (100 mg, 0.37 mmol), (3,4-difluorophenyl)methanamine (110 mg, 0.74 mmol) and diisopropylethylamine (480 mg, 3.7 mmol) were added to 1,4-dioxane (1 mL) and stirred at 120°C overnight. The reaction solution was poured into dichloromethane, washed with saturated ammonium chloride aqueous solution, the organic phase was concentrated, and purified by preparative thin layer chromatography to obtain the title compound (120 mg, 86 %).

LCMS: *m*/*z* [M+H]⁺= 377.

¹H NMR (400 MHz, CDCl₃) δ7.20 - 7.03 (m, 3H), 5.52 (br. s., 1H), 4.79 - 4.68 (m, 2H), 4.66 - 4.57 (m, 1H), 4.42 (d, *J =* 12.2 Hz, 1H), 4.04 - 3.95 (m, 2H), 3.86 (d, *J =* 12.2 Hz, 1H), 3.70 - 3.65 (m, 1H), 3.64 - 3.59 (m, 4H), 2.02 (d, *J = 9.8* Hz, 1H), 1.82 (d, *J =* 10.3 Hz, 1H).

Examples 176 to 200 listed in the table below were prepared by procedures similar to those described in Examples 169 to 175, starting from the corresponding intermediates:

### # Name Structure Method used m/z 1H NMR

| **Example No.** | **Name** | **Structure** | **Method used** | **m/z** | **¹H NMR (400 MHz)** |
|---|---|---|---|---|---|
| 176 | 3-((4-((2-(difluoromethyl)pyridin-4-yl)oxy)-3,5-difluorobenzyl)oxy)-7,8-dihydro-1H,6H,9H-7,8a-methanopyrrolo[1',2': 3,4]i midazo[1,2-c]pyrimidin-1-one | 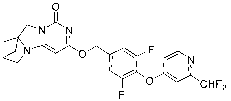 | C | 475 | CDCl₃: δ8.49 (d, *J =* 5.4 Hz, 1H), 7.18 - 7.01 (m, 3H), 6.91 (d, *J =* 3.9 Hz, 1H), 6.76 - 6.40 (m, 1H), 5.39 (s, 2H), 5.14 (s, 1H), 3.97 (s, 2H), 3.32 (s, 2H), 3.00 (br. s., 1H), 2.05 (br. s., 2H), 1.67 (d, *J =* 3.9 Hz, 2H) |
| 177 | (3*S*,11a*S*)-2-cyclobutyl-7-((3-fluoro-4-((2-(trifluoromethyl)pyridin-4-yl)oxy)benzyl)oxy)-1,2,3,4-tetrahydro-9H,11H-3,11a-methanopyrazino[1',2':3,4 ]imidazo[1,2-c]pyrimidin-9-one | 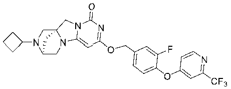 | F | 544 | CDCl₃: δ8.57 (d, *J =* 5.4 Hz, 1H), 7.32 (d, *J =* 10.8 Hz, 1H), 7.26 - 7.13 (m, 3H), 6.95 (d, *J =* 3.9 Hz, 1H), 5.42 (br. s., 2H), 5.07 (s, 1H), 4.36 (d, *J =* 12.2 Hz, 1H), 3.84 (d, *J =* 12.2 Hz, 1H), 3.63 (br. s., 1H), 3.55 (d, *J =* 8.8 Hz, 1H), 3.30 - 3.21 (m, 1H), 3.09 - 3.01 (m, 1H), 2.78 (d, *J =* 9.3 Hz, 1H), 2.05 (d, *J =* 7.8 Hz, 2H), 1.97 - 1.67 (m, 6H), 1.55 (d, *J =* 9.8 Hz, 1H) |
| 178 | (3*S*,11a*S*)-2-benzyl-7-((3-fluoro-4-((2-(trifluoromethyl)pyridin-4-yl)oxy)benzyl)oxy)-1,2,3,4-tetrahydro-9H,11H-3,11a-methanopyrazino[1',2': 3,4 ]imidazo[1,2-c]pyrimidin-9-one | 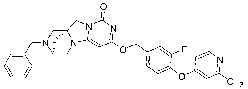 | F | 580 | CDCl₃: δ8.58 (d, *J =* 5.4 Hz, 1H), 7.39 - 7.31 (m, 5H), 7.27 (br. s., 3H), 7.23 (br. s., 1H), 7.22 - 7.15 (m, 1H), 6.96 (d, *J =* 4.4 Hz, 1H), 5.44 (d, *J =* 3.9 Hz, 2H), 5.11 (s, 1H), 4.37 (d, *J =* 12.2 Hz, 1H), 3.82 (s, 2H), 3.75 (d, *J* = 9.8 Hz, 1H), 3.68 (br. s., 1H), 3.21 - 3.05 (m, 2H), 2.76 (d, *J =* 9.3 Hz, 1H), 2.03 (d, *J =* 9.8 Hz, 1H), 1.60 (d, *J =* 9.8 Hz, 1H) |
| 179 | (3*S*,11a*R*)-7-((4-(3-(difluoromethyl)phenoxy) -3,5-difluorobenzyloxy)-3,4-dihydro-1H,9H,11H-3,11a-methanoprimido[6', 1':2,3] imidazo[5,1-c][1,4]oxazin-9-one | 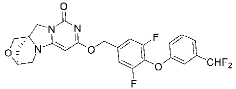 | C | 490 | CDCl₃: δ7.42 - 7.32 (m, 1H), 7.20 (d, *J =* 7.8 Hz, 1H), 7.12 - 6.93 (m, 4H), 6.78 - 6.39 (m, 1H), 5.49 - 5.27 (m, 2H), 5.10 (s, 1H), 4.72 (br. s., 1H), 4.45 (d, *J =* 12.2 Hz, 1H), 4.02 - 3.89 (m, 3H), 3.56 (d, *J =* 9.8 Hz, 1H), 3.23 (d, *J =* 9.8 Hz, 1H), 2.02 (d, *J =* 9.8 Hz, 1H), 1.73 (d, *J =* 10.3 Hz, 1H) |
| 180 | (3*S*,11a*R*)-7-((3,4-difluorobenzyl)(methyl)a mino)-6-methoxy-3,4-dihydro-1H,9H,11H-3,11a-methanopyrimido[6',1':2, 3]imidazo[5,1-c][1,4]oxazin-9-one | 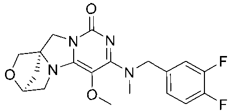 | I | 391 | CDCl₃: δ7.12 - 7.02 (m, 2H), 6.97 (br. s., 1H), 4.89 (d, *J =* 15.7 Hz, 1H), 4.75 - 4.64 (m, 2H), 4.35 (d, *J =* 11.7 Hz, 1H), 4.00 - 3.92 (m, 2H), 3.86 (d, *J =* 12.2 Hz, 1H), 3.66 - 3.61 (m, 1H), 3.59 - 3.53 (m, 1H), 3.46 (s, 3H), 3.11 (s, 3H), 2.00 (d, *J =* 9.3 Hz, 1H), 1.82 (d, *J =* 9.8 Hz, 1H) |
| 181 | (3*S*,11a*R*)-7-(3-fluoro-4-(3-(trifluoromethyl)phenoxy) phenethoxy)-3,4-dihydro-1H,9H,11H-3,11a-methanopyrimido[6',1':2, 3]imidazo[5,1-c][1,4]oxazin-9-one | 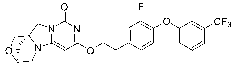 | C | 504 | DMSO-d₆: δ7.67 - 7.54 (m, 1H), 7.47 (d, *J =* 7.3 Hz, 1H), 7.38 (d, *J =* 11.7 Hz, 1H), 7.27 (br. s., 1H), 7.25 - 7.12 (m, 3H), 5.17 (s, 1H), 4.66 (br. s., 1H), 4.42 (d, *J =* 5.4 Hz, 2H), 4.26 (d, *J =* 12.2 Hz, 1H), 3.97 - 3.76 (m, 3H), 3.39 (br. s., 1H), 3.24 (d, *J =* 10.3 Hz, 1H), 3.00 (t, *J =* 6.4 Hz, 2H), 1.88 (d, *J =* 9.8 Hz, 1H), 1.75 (d, *J =* 10.3 Hz, 1H) |
| 182 | 3-(3-fluoro-4-(3-(trifluoromethyl)phenoxy) phenethoxy)-7,8-dihydro-1H,6H,9H-7,8a-methanopyrrolo[1',2':3,4]i midazo[1,2-c]pyrimidin-1-one | 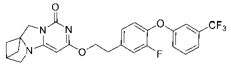 | C | 488 | CDCl₃: δ7.47 - 7.38 (m, 1H), 7.32 (d, *J =* 7.8 Hz, 1H), 7.20 (br. s., 1H), 7.12 (d, *J =* 11.2 Hz, 2H), 7.03 (br. s., 2H), 5.06 (s, 1H), 4.58 (t, *J =* 6.4 Hz, 2H), 3.98 (s, 2H), 3.32 (s, 2H), 3.08 - 2.96 (m, 3H), 2.06 (br. s., 2H), 1.68 (d, *J =* 4.4 Hz, 2H) |
| 183 | 3-(3-fluoro-4-(3-(trifluoromethyl)phenoxy) phenethoxy)-4-methoxy-7,8-dihydro-1H,6H,9H-7,8a-methanopyrrolo[1',2': 3,4]i midazo[1,2-c]pyrimidin-1-one | 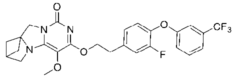 | C | 518 | CDCl₃: δ7.41 (br. s., 1H), 7.32 (br. s., 1H), 7.15 (br. s., 2H), 7.06 (br. s., 3H), 4.62 (br. s., 2H), 3.96 (br. s., 2H), 3.69 - 3.50 (m, 5H), 3.20 - 2.96 (m, 3H), 2.04 (br. s., 2H), 1.67 (br. s., 2H) |
| 184 | 4-methoxy-3-(4-(3-(trifluoromethyl)phenoxy) phenethoxy)-7,8-dihydro-1H,6H,9H-7,8a-methanopyrrolo[1',2':3,4]i midazo[1,2-c]pyrimidin-1-one | 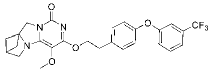 | C | 500 | CDCl₃: δ7.45 - 7.36 (m, 1H), 7.32 - 7.23 (m, 3H), 7.16 (br. s., 1H), 7.10 (d, *J =* 8.3 Hz, 1H), 6.94 (d, *J =* 7.8 Hz, 2H), 4.60 (t, *J =* 6.6 Hz, 2H), 3.94 (s, 2H), 3.55 (s, 5H), 3.07 (t, *J =* 6.6 Hz, 2H), 2.98 (br. s., 1H), 2.01 (br. s., 2H), 1.65 (d, *J =* 4.4 Hz, 2H) |
| 185 | (3*S,*11*aR*)-7-(4-(4-chloro-3-(trifluoromethyl)phenoxy) -3-fluorophenethoxy)-3,4-dihydro-1H,9H,11H-3,11a-methanopyrimido[6',1':2, 3]imidazo[5,1-c][1,4]oxazin-9-one | 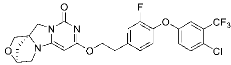 | C | 538 | CDCl₃: δ7.41 (d, *J* = 8.3 Hz, 1H), 7.27 (br. s., 1H), 7.10 (d, *J =* 11.2 Hz, 1H), 7.03 (br. s., 3H), 5.01 (br. s., 1H), 4.72 (br. s., 1H), 4.57 (br. s., 2H), 4.44 (d, *J =* 12.2 Hz, 1H), 4.06 - 3.84 (m, 3H), 3.55 (d, *J =* 9.8 Hz, 1H), 3.21 (d, *J =* 9.8 Hz, 1H), 3.03 (br. s., 2H), 2.02 (d, *J =* 9.8 Hz, 1H), 1.71 (d, *J =* 9.8 Hz, 1H) |
| 186 | 3-(4-(4-chloro-3-(trifluoromethyl)phenoxy) -3-fluorophenethoxy)-7,8-dihydro-1H,6H,9H-7,8a-methanopyrrolo[1',2': 3,4]i midazo[1,2-c]pyrimidin-1-one | 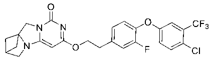 | C | 522 | CDCl₃: δ7.40 (d, *J* = 8.3 Hz, 1H), 7.28 (br. s., 1H), 7.11 (d, *J* = 11.2 Hz, 1H), 7.06 - 6.96 (m, 3H), 5.05 (s, 1H), 4.57 (t, *J =* 6.1 Hz, 2H), 3.97 (s, 2H), 3.31 (s, 2H), 3.13 - 2.97 (m, 3H), 2.05 (br. s., 2H), 1.67 (br. s., 2H) |
| 187 | 3-(4-(4-chloro-3-(trifluoromethyl)phenoxy) -3-fluorophenethoxy)-4-methoxy-7,8-dihydro-1H,6H,9H-7,8a-methanopyrrolo[1',2': 3,4]i midazo[1,2-c]pyrimidin-1-one | 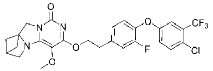 | C | 552 | CDCl₃: δ7.40 (d, *J* = 8.8 Hz, 1H), 7.25 - 7.23 (m, 1H), 7.15 (d, *J =* 11.2 Hz, 1H), 7.10 - 6.95 (m, 3H), 4.71 - 4.58 (m, 2H), 3.96 (s, 2H), 3.57 (s, 5H), 3.08 (br. s., 2H), 3.00 (br. s., 1H), 2.03 (br. s., 2H), 1.74 - 1.67 (m, 2H) |
| 188 | 4-methoxy-3-(4-(((2-(trifluoromethyl)pyridin-4-yl)oxy)phenethoxy)-7,8-dihydro-1H,6H,9H-7,8a-methanopyrrolo[1',2': 3,4]i midazo[1,2-c]pyrimidin-1-one | 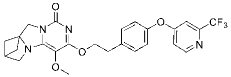 | C | 501 | CDCl₃: δ8.54 (d, *J* = 5.4 Hz, 1H), 7.40 - 7.34 (m, *J* = 8.3 Hz, 2H), 7.18 (s, 1H), 7.05 - 7.01 (m, *J =* 8.3 Hz, 2H), 6.94 (d, *J* = 4.9 Hz, 1H), 4.64 (t, *J* = 6.6 Hz, 2H), 3.96 (s, 2H), 3.58 (s, 5H), 3.13 (t, *J =* 6.6 Hz, 2H), 3.01 (br. s., 1H), 2.04 (br. s., 2H), 1.68 (br. s., 2H) |
| 189 | 3-(3,5-difluoro-4-((2-(trifluoromethyl)pyridin-4-yl)oxy)phenethoxy)-4-methoxy-7,8-dihydro-1H,6H,9H-7,8a-methanopyrrolo[1',2': 3,4]i midazo[1,2-c]pyrimidin-1-one | 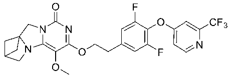 | C | 537 | CDCl₃: δ= 8.59 (d, *J =* 4.9 Hz, 1H), 7.21 (br. s., 1H), 7.02 (d, *J =* 8.8 Hz, 2H), 6.97 (br. s., 1H), 4.63 (br. s., 2H), 3.96 (s, 2H), 3.61 - 3.56 (m, 5H), 3.10 (br. s., 2H), 3.01 (br. s., 1H), 2.04 (br. s., 2H), 1.71 (br. s., 2H) |
| 190 | 3-(3-fluoro-4-((2-(trifluoromethyl)pyridin-4-yl)oxy)phenethoxy)-7,8-dihydro-1H,6H,9H-7,8a-methanopyrrolo[1',2': 3,4]i midazo[1,2-c]pyrimidin-1-one | 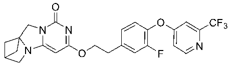 | C | 489 | DMSO-d₆: δ8.63 (d, *J =* 5.4 Hz, 1H), 7.50 - 7.32 (m, 3H), 7.25 (br. s., 1H), 7.18 - 7.09 (m, 1H), 5.19 (s, 1H), 4.41 (t, *J =* 6.4 Hz, 2H), 3.83 (s, 2H), 3.28 (s, 2H), 3.02 (t, *J =* 6.1 Hz, 2H), 2.91 (br. s., 1H), 2.01 (br. s., 2H), 1.60 - 1.48 (m, 2H) |
| 191 | 3-(3-fluoro-4-((2-(trifluoromethyl)pyridin-4-yl)oxy)phenethoxy)-4-methoxy-7,8-dihydro-1H,6H,9H-7,8a-methanopyrrolo[1',2': 3,4]i midazo[1,2-c]pyrimidin-1-one | 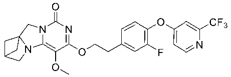 | C | 519 | DMSO-d₆: δ8.63 (d, *J =* 5.4 Hz, 1H), 7.49 - 7.35 (m, 3H), 7.27 (d, *J =* 8.3 Hz, 1H), 7.13 (d, *J =* 4.9 Hz, 1H), 4.47 (t, *J =* 6.1 Hz, 2H), 3.83 (s, 2H), 3.55 - 3.42 (m, 5H), 3.07 (t, *J* = 6.1 Hz, 2H), 2.93 (br. s., 1H), 2.08 - 1.93 (m, 2H), 1.65 - 1.53 (m, 2H) |
| 192 | (3*S*,11a*R*)-7-(((3-fluoro-4-(3-(trifluoromethyl)phenoxy) benzyl)oxy)-3,4-dihydro-1H,9H,11H-3,11a-methanopyrimido[6',1':2, 3]imidazo[5,1-c][1,4]oxazin-9-one | 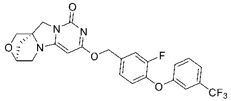 | C | 490 | CDCl₃: δ7.48 -7.40 (m, 1H), 7.34 (d, *J* = 7.3 Hz, 1H), 7.30 (br. s., 1H), 7.20 (d, *J =* 13.7 Hz, 2H), 7.16 - 7.05 (m, 2H), 5.47 - 5.33 (m, 2H), 5.10 (s, 1H), 4.74 (br. s., 1H), 4.47 (d, *J =* 12.2 Hz, 1H), 4.06 - 3.89 (m, 3H), 3.57 (d, *J =* 9.8 Hz, 1H), 3.23 (d, *J =* 9.8 Hz, 1H), 2.04 (d, *J =* 10.3 Hz, 1H), 1.74 (d, *J =* 10.3 Hz, 1H) |
| 193 | 2-fluoro-5-((((((3*S*,11a*R*)-9-keto-3,4-dihydro-1H,9H,11H-3,11a-methanopyrimido[6',1':2, 3]imidazo[5,1-c][1,4]oxazin-7-yl)oxy)methyl)benzonitril e | 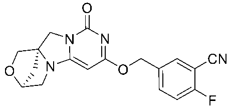 | C | 355 | CDCl₃: δ7.72 - 7.61 (m, 2H), 7.21 (t, *J =* 8.6 Hz, 1H), 5.48 - 5.34 (m, 2H), 5.08 (s, 1H), 4.75 (br. s., 1H), 4.46 (d, *J =* 12.7 Hz, 1H), 4.02 - 3.91 (m, 3H), 3.58 (d, *J =* 9.8 Hz, 1H), 3.24 (d, *J =* 9.8 Hz, 1H), 2.04 (d, *J =* 9.8 Hz, 1H), 1.74 (d, *J =* 10.3 Hz, 1H) |
| 194 | (3*S*,11a*R*)-7-(((3,5-difluoro-4-(3-(trifluoromethyl)phenoxy) benzyl)oxy)-3,4-dihydro-1H,9H,11H-3,11a-methanopyrimido[6',1':2, 3]imidazo[5,1-c][1,4]oxazin-9-one | 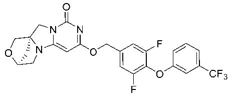 | C | 508 | CDCl₃: δ7.46 - 7.39 (m, 1H), 7.37 - 7.32 (m, 1H), 7.20 (br. s., 1H), 7.09 (d, *J =* 7.8 Hz, 3H), 5.47 - 5.34 (m, 2H), 5.12 (s, 1H), 4.75 (s, 1H), 4.47 (d, *J =* 12.2 Hz, 1H), 4.05 - 3.92 (m, 3H), 3.59 (d, *J =* 10.3 Hz, 1H), 3.25 (d, *J =* 9.8 Hz, 1H), 2.05 (d, *J =* 9.8 Hz, 1H), 1.75 (d, *J=* 10.3 Hz, 1H) |
| 195 | (3*S*,11a*R*)-7-(3,4-difluorophenethoxy)-3,4-dihydro-1H,9H,11H-3,11a-methanopyrimido[6',1':2, 3]imidazo[5,1-c][1,4]oxazin-9-one | 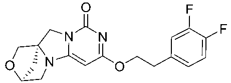 | C | 362 | CDCl₃: δ7.06 (d, *J =* 8.8 Hz, 2H), 6.94 (br. s., 1H), 4.98 (s, 1H), 4.71 (s, 1H), 4.53 (t, *J =* 5.4 Hz, 2H), 4.43 (d, *J =* 12.2 Hz, 1H), 3.97 (s, 2H), 3.90 (d, *J =* 12.2 Hz, 1H), 3.54 (d, *J* = 9.8 Hz, 1H), 3.20 (d, *J =* 9.8 Hz, 1H), 2.96 (t*, J =* 6.4 Hz, 2H), 2.01 (d, *J =* 9.8 Hz, 1H), 1.70 (d, *J =* 9.8 Hz, 1H) |
| 196 | 3-((4-((2-(difluoromethyl)pyridin-4-yl)oxy)-3-fluorobenzyloxy)-7,8-dihydro-1H,6H,9H-6,8a-ethanopyrrolo[1',2':3,4]im idazo[1,2-c]pyrimidin-1-one | 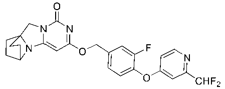 | C | 471 | CDCl₃: δ8.51 (d, *J =* 5.4 Hz, 1H), 7.33 (d, *J =* 10.8 Hz, 1H), 7.25 (br. s., 1H), 7.22 - 7.15 (m, 2H), 6.92 (d, *J =* 3.9 Hz, 1H), 6.76 - 6.44 (m, 1H), 5.43 (s, 2H), 5.21 (s, 1H), 4.13 (t, *J =* 4.2 Hz, 1H), 4.02 (s, 2H), 2.10 (br. s., 2H), 1.82 - 1.76 (m, 6H) |
| 197 | (3*S*,11a*R*)-7-((3-fluoro-4-((2-(trifluoromethyl)pyridin-4-yl)oxy)benzyl)oxy)-2-((1-methyl-1H-pyrazol-3-yl)methyl)-3,4-dihydro-9H,11H-3,11a-methanopyrazino[1',2':3,4 ]imidazo[1,2-c]pyrimidin-1,9(2H)-dione | 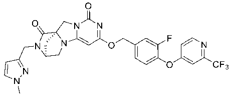 | C | 598 | CDCl₃: δ8.58 (d, *J* = 5.4 Hz, 1H), 7.37 - 7.29 (m, 2H), 7.26 - 7.15 (m, 3H), 6.96 (d, *J =* 3.4 Hz, 1H), 6.19 (s, 1H), 5.44 (br. s., 2H), 5.13 (br. s., 1H), 4.62 (d, *J =* 15.2 Hz, 1H), 4.49 - 4.24 (m, 4H), 3.90 (s, 3H), 3.28 (br. s., 2H), 2.09 (br. s., 1H), 1.85 (br. s., 1H) |
| 198 | (3S,11aR)-2-(bicyclo[1.1.1]pent-1-ylmethyl)-7-((3-fluoro-4-((2-(trifluoromethyl)pyridin-4-yl)oxy)benzyl)oxy)-3,4-dihydro-9H,11H-3,11a-methanopyrazino[1',2': 3,4 ]imidazo[1,2-c]pyrimidin-1,9(2H)-dione | 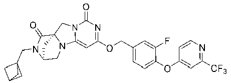 | C | 584 | CDCl₃: δ8.58 (d, *J* = 5.4 Hz, 1H), 7.33 (d, *J =* 11.2 Hz, 2H), 7.25 - 7.15 (m, 2H), 6.96 (d, *J* = 2.9 Hz, 1H), 5.45 (br. s., 2H), 5.16 (br. s., 1H), 4.49 - 4.26 (m, 3H), 3.69 (d, *J =* 14.7 Hz, 1H), 3.50 - 3.30 (m, 2H), 3.01 (d, *J =* 14.2 Hz, 1H), 2.67 - 2.51 (m, 1H), 2.15 (br. s., 1H), 2.00 - 1.87 (m, 1H), 1.79 (s, 4H), 1.68 (br. s., 2H) |
| 199 | N-benzyl-3-((3-fluoro-4-((2-(trifluoromethyl)pyridin-4-yl)oxy)benzyl)oxy)-1-oxo-1H,6H,9H-7,8a-methanopyrrolo[1',2': 3,4]i midazo[1,2-c]pyrimidine-7(8H)-carboxamide | 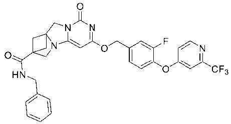 | C | 608 | CDCl₃: δ8.58 (d, *J =* 4.9 Hz, 1H), 7.39 - 7.32 (m, 5H), 7.24 - 7.15 (m, 4H), 6.96 (br. s., 1H), 6.03 (br. s., 1H), 5.43 (br. s., 2H), 5.21 (br. s., 1H), 4.49 (br. s., 2H), 4.03 (br. s., 2H), 3.61 (br. s., 2H), 2.38 - 2.26 (m, 2H), 2.10 (br. s., 2H) |
| 200 | 3-((3-fluoro-4-((2-(trifluoromethyl)pyridin-4-yl)oxy)benzyl)oxy)-1-oxo-1H,6H,9H-7,8a-methanopyrrolo[1',2': 3,4]i midazo[1,2-c]pyrimidine-7(8H)-carbonitrile | 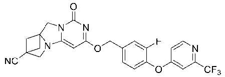 | C | 500 | CDCl₃: δ8.59 (d, *J* = 5.9 Hz, 1H), 7.33 (d, *J* = 10.8 Hz, 2H), 7.25 - 7.16 (m, 2H), 6.97 (d, *J =* 3.9 Hz, 1H), 5.45 (s, 2H), 5.24 (s, 1H), 4.08 (s, 2H), 3.63 (s, 2H), 2.56 (d, *J =* 4.4 Hz, 2H), 2.28 - 2.15 (m, 2H) |

### Example 201

### 3-((3-Fluoro-4-((2-trifluoromethylpyridin-4-yl)oxy)benzyl)oxy)-7-(2-hydroxypropyl-2-yl)-7,8-dihydro-1H,6H,9H-7,8a-methanopyrrolo[1', 2':3, 4]imidazo[1, 2-c]pyrimidin-1-one

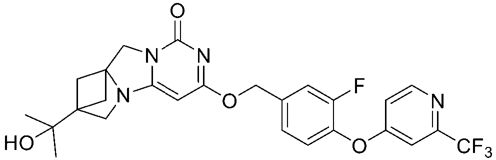

3-((3-Fluoro-4-((2-(trifluoromethyl)pyridin-4-yl)oxy)benzyl)oxy)-1-oxo-1H,6H,9H-7,8a-methanopyrrolo[1',2':3,4]imidazo[1,2-c]pyrimidine-7(8H)-carboxylic acid methyl ester (20 mg, 0.038 mmol) was added to tetrahydrofuran (4 mL). The temperature was lowered to 0°C under argon protection, and a solution of methylmagnesium chloride in tetrahydrofuran (3.0 M, 0.13 mL) was added, and stirred at room temperature for 2 hours. The reaction solution was quenched with saturated ammonium chloride solution, extracted with ethyl acetate, and the organic phase was concentrated, and purified by preparative thin layer chromatography (dichloromethane/methanol = 10/1) to obtain the title compound (1 mg, 5 %).

LC-MS: *m*/*z* [M+H]⁺= 533*.*

¹H NMR (400 MHz, CDCl₃) δ8.58 (d, *J =* 5.4 Hz, 1H), 7.34 (d, *J =* 10.8 Hz, 1H), 7.30 - 7.28 (m, 1H), 7.24 (br. s., 1H), 7.22 - 7.15 (m, 1H), 6.96 (d, *J =* 5.4 Hz, 1H), 5.45 (s, 2H), 5.17 (br. s., 1H), 4.03 (s, 2H), 3.82 - 3.74 (m, 1H), 3.35 (d, *J =* 3.9 Hz, 2H), 2.07 - 1.99 (m, 2H), 1.76 (br. s., 2H), 1.44 (s, 3H), 1.31 (s, 3H).

### Example 202

### 7-Amino-3-((3-fluoro-4-((2-(trifluoromethyl)pyridin-4-yl)oxy)benzyl)oxy)-7,8-dihydro-1H,6H,9H-7,8a-methanopyrrolo[1',2':3,4]imidazo[1, 2-c]pyrimidin-1-one

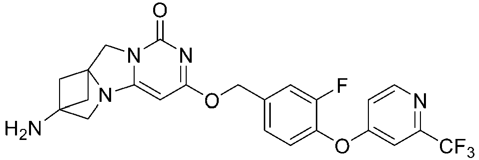

(3-((3-Fluoro-4-((2-(trifluoromethyl)pyridin-4-yl)oxy)benzyl)oxy)-1-oxo-1H,6H,9H-7,8a-methanopyrrolo[1',2':3,4]imidazo[1,2-c]pyrimidin-7(8H)-yl)carbamic acid tert-butyl ester (90 mg, 0.15 mmol) was added to ethyl acetate (4 mL). A solution of hydrogen chloride in ethyl acetate (4.0 M, 2 mL) was added under stirring, and stirred at room temperature for 10 minutes. The reaction solution was adjusted to pH 8 with solid sodium carbonate, filtered, and the filtrate was concentrated, and purified by preparative thin layer chromatography (dichloromethane/methanol = 10/1) to obtain the title compound (6 mg, 7 %).

LC-MS: *m*/*z* [M+H]⁺= 490.

¹H NMR (400 MHz, CDCl₃) δ8.58 (d, *J =* 5.9 Hz, 1H), 7.34 (d, *J =* 11.2 Hz, 2H), 7.25 - 7.15 (m, 2H), 6.96 (d, *J =* 3.4 Hz, 1H), 5.44 (s, 2H), 5.15 (s, 1H), 4.04 (s, 2H), 3.25 (s, 2H), 1.97 (s, 4H).

### Biological Tests and Data

The compound of the present invention is Lp-PLA₂ inhibitor and can be used for the treatment and prevention of a Lp-PLA₂-mediated disease. The biological activity of the compound of the present invention can be determined using any suitable assay for determining the activity of compound as a Lp-PLA₂ inhibitor, as well as tissue and in vivo models.

Biological activity data for each compound is reported as the average of at least one experiment or multiple experiments. It is to be understood that the data described herein may vary reasonably depending upon the particular conditions and methodology employed by the person conducting the experiments.

### Lipoprotein-Associated Phospholipase A2 (Lp-PLA₂) Human Plasma Assay

The human plasma assay utilizes a thioester analog of PAF (phosphatidylcholine), in which hydrolysis results in the formation of phospholipids containing free sulfhydryl groups. The amount of sulfhydryl groups is continuously determined by the reaction with CPM (7-diethylamino-3-(4'-maleimidophenyl)-4-methylcoumarin), and the CPM is a maleimide of which fluorescence increases after Michael addition of sulfhydryl group. This assay can detect Lp-PLA₂ activity in human plasma, as determined by specific inhibition of Lp-PLA₂ inhibitors.

Thio-PAF assay was performed as quenched 75 µL assay. Compound source plate was prepared for each compound by preparing 1:3 (by volume) serial dilutions in pure DMSO in a 96-well microplate. The compound on the source plate was diluted 20-fold by transferring 3 µL of the compound from the compound source plate into a 96-well microplate pre-added with 57 µL of assay buffer by Rainin multichannel pipette. The assay buffer described above consisted of 50 mM HEPES, pH 7.4, 150 mM NaCl, 1 mM CHAPS. 1 µL of the 20-fold diluted compound was transferred by Rainin multichannel pipette into a 96-well Greiner 655076 (black) microplate to which aliquoted and thawed 40 µL of pooled human plasma was previously added. The plate was shaken for 20 seconds on a microplate shaker to mix well. After a 30-min pre-incubation at room temperature, 10 µL of substrate solution was added to the 96-well Greiner 655076 (black) microplate by Rainin multichannel pipette, in which the substrate solution contained 2.5 mM 2-thio-PAF [from ethanol stock], 32 µM CPM [from DMSO stock], and 3.2 mM NEM (N-ethylmaleimide) [freshly prepared in DMSO per experiment] in assay buffer consisting of 50 mM HEPES, pH 7.4; 150 mM NaCl; 1mM CHAPS. After 2 min, the reaction was quenched with 25 µL of 5% trifluoroacetic acid (TFA) in water. The plate was centrifuged at 2000 rpm for 1 minute. The plate was read at ex:380/em:485 using a Biotek Synergy H1 (H1MF) microplate reader. IC₅₀ data, curves and QC analysis were performed using GraphPad Prism 6.0 and Excel.

### Determined Activity of Examples

| **Example** | IC₅₀(nM) | **Example** | IC₅₀(nM) | **Example** | IC₅₀(nM) |
|---|---|---|---|---|---|
| **1** | 4.4 | **69** | 325.3 | **137** | 5.8 |
| **2** | 2.6 | **70** | 6.9 | **138** | 2.4 |
| **4** | 2.5 | **71** | 7.9 | **139** | 2.6 |
| **5** | 4.2 | **72** | 7.6 | **140** | 3.9 |
| **6** | 4.9 | **73** | 4.8 | **141** | 6.1 |
| **7** | 2.5 | **74** | 22.3 | **142** | 3.6 |
| **8** | 4.9 | **75** | 23.4 | **143** | 2.5 |
| **9** | 6.5 | **76** | 42.2 | **144** | 2.4 |
| **10** | 6.7 | **77** | 5.7 | **145** | 4.3 |
| **11** | 10.8 | **78** | 5.0 | **146** | 2.7 |
| **12** | 4.3 | **79** | 6.1 | **147** | 6.5 |
| **13** | 6.7 | **80** | 3.3 | **148** | 4.3 |
| **14** | 4.6 | **81** | 5.1 | **149** | 4.1 |
| **15** | 6.6 | **82** | 2.0 | **150** | 4.8 |
| **16** | 5.2 | **83** | 4.9 | **151** | 5.7 |
| **17** | 32.4 | **84** | 6.2 | **152** | 3.9 |
| **18** | 3.5 | **85** | 6.7 | **153** | 4.1 |
| **19** | 3.9 | **86** | 6.1 | **154** | 3.6 |
| **20** | 6.8 | **88** | 5.2 | **155** | 3.7 |
| **21** | 6.4 | **89** | 10.9 | **156** | 5.7 |
| **22** | 3.0 | **90** | 4.8 | **157** | 3.9 |
| **23** | 5.0 | **91** | 4.8 | **158** | 2.1 |
| **24** | 13.2 | **92** | 5.6 | **159** | 128.2 |
| **25** | 10.4 | **93** | 3.7 | **160** | 3.8 |
| **26** | 5.5 | **94** | 92.7 | **161** | 18.1 |
| **27** | 2.5 | **95** | 7.4 | **162** | 6.5 |
| **28** | 7.3 | **96** | 4.7 | **163** | 2.4 |
| **29** | 3.4 | **97** | 14.2 | **164** | 1.5 |
| **30** | 6.8 | **98** | 30.2 | **165** | 3.1 |
| **31** | 7.1 | **99** | 5.6 | **166** | 5.8 |
| **32** | 163.8 | **100** | 5.9 | **167** | 19.4 |
| **33** | 6.0 | **101** | 15.1 | **168** | 3.6 |
| **34** | 3.6 | **102** | 7.6 | **169** | 4.9 |
| **35** | 38.8 | **103** | 6.2 | **170** | 177.8 |
| **36** | 9.6 | **104** | 8.6 | **171** | 2.1 |
| **37** | 5.8 | **105** | 10.8 | **172** | 2.2 |
| **38** | 8.5 | **106** | 1.0 | **173** | 3.4 |
| **39** | 13.8 | **107** | 9.5 | **174** | 5.0 |
| **40** | 6.4 | **108** | 5.0 | **175** | 167.6 |
| **41** | 3.9 | **109** | 14.0 | **176** | 13.9 |
| **42** | 4.9 | **110** | 5.3 | **177** | 5.5 |
| **43** | 4.0 | **111** | 11.4 | **178** | 6.2 |
| **44** | 342.1 | **112** | 5.1 | **179** | 4.1 |
| **45** | 71.8 | **113** | 5.7 | **180** | 87.2 |
| **46** | 15.3 | **114** | 5.7 | **181** | 6.7 |
| **47** | 9.4 | **115** | 5.2 | **182** | 9.4 |
| **48** | 62.8 | **116** | 4.7 | **183** | 13.8 |
| **49** | 168.3 | **117** | 9.1 | **184** | 23.8 |
| **50** | 2.7 | **118** | 6.0 | **185** | 4.7 |
| **51** | 3.3 | **119** | 8.2 | **186** | 7.4 |
| **52** | 16.0 | **120** | 8.7 | **187** | 7.8 |
| **53** | 14.3 | **121** | 8.6 | **188** | 9.2 |
| **54** | 7.6 | **122** | 9.4 | **189** | 8.6 |
| **55** | 6.5 | **123** | 4.4 | **190** | 5.9 |
| **56** | 7.7 | **124** | 6.1 | **191** | 6.2 |
| **57** | 62.2 | **125** | 3.0 | **192** | 6.3 |
| **58** | 204.0 | **126** | 5.0 | **193** | 5.2 |
| **59** | 2.4 | **127** | 2.6 | **194** | 4.0 |
| **60** | 6.6 | **128** | 8.4 | **195** | 62.7 |
| **61** | 3.4 | **129** | 6.5 | **196** | 15.4 |
| **62** | 13.0 | **130** | 4.8 | **197** | 2.9 |
| **63** | 8.7 | **131** | 7.9 | **198** | 5.1 |
| **64** | 22.6 | **132** | 5.9 | **199** | 7.9 |
| **65** | 12.1 | **133** | 36.6 | **200** | 4.4 |
| **66** | 6.9 | **134** | 5.0 | **201** | 4.4 |
| **67** | 8.7 | **135** | 17.2 | **202** | 1.9 |
| **68** | 50.0 | **136** | 3.8 | | |

### Comparison of activity data (I)

| **Example** | **Structure** | **IC₅₀ (nM)** | **Example** | **Structure** | **IC₅₀ (nM)** |
|---|---|---|---|---|---|
| | 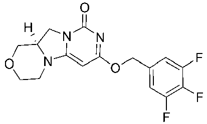 **Example 123 in WO2016011930A1** | 75.3 | | 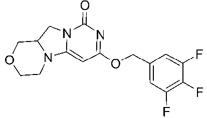 **Corresponding racemate of Example 123 in WO2016011930A1** | 63.3 |
| **86** | 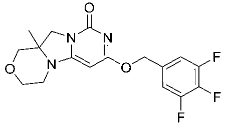 | 6.1 | **52** | 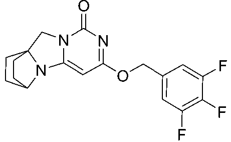 | 16 |

It could be seen from the comparative data that the compounds of the present patent application had significantly higher activity than the compounds in the prior art.

## Claims

1. A compound shown in Formula I, cis-trans isomer thereof, enantiomer thereof, diastereomer thereof, racemate thereof, solvate thereof, hydrate thereof or pharmaceutically acceptable salt or prodrug thereof, 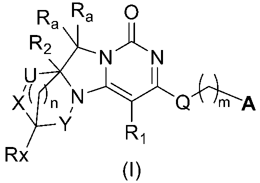 wherein
n is 0, 1 or 2; and when n is 0, R₂ is methyl or ethyl; when n is 1 or 2, R₂ is absent;
R₁ is H, halogen, cyano, C₁₋₆ alkyl, C₁₋₆ alkoxy, C₃₋₈ cycloalkyl or 3- to 8-membered heterocyclyl, R₁ may be optionally substituted with one or more of the following substituents: halogen, cyano, C₁₋₆ alkoxy, C₃₋₈ cycloalkyl, 3- to 8-membered heterocyclyl or 6- to 10-membered heteroaryl;
Rₐ independently is H or D;
m is 1 or 2;
Rₓ is H, halogen, hydroxyl, carboxyl, cyano, amino, C₁₋₆ alkyl, C₁₋₆ alkoxy, C₃₋₈ cycloalkyl, 3- to 8-membered heterocyclyl, 6- to 10-membered aryl, 6- to 10-membered heteroaryl, -C(O)NR_{b}R_{c} or -S(O)₂NR_{b}R_{c}, Rₓ may be optionally substituted with one or more of the following substituents: halogen, hydroxyl, C₁₋₆ alkoxy, cyano, C₃₋₈ cycloalkyl, 3- to 8-membered heterocyclyl, 6- to 10-membered aryl or 6- to 10-membered heteroaryl;
Q is -O-, -S-, -CH₂- or -NR_{b}-;
X is -O-, -CH₂-, -NR_{c}-, -OCH₂-, or is absent;
R_{b} is H, C₁₋₆ alkyl or C₃₋₈ cycloalkyl or 3- to 8-membered heterocyclyl;
R_{c} is L, L-C(O)-, L-CH₂- or L-S(O)₂-, wherein L is H, C₁₋₆ alkyl, C₃₋₆ cycloalkyl, 3- to 8-membered heterocyclyl, 6- to 10-membered aryl or 6- to 10-membered heteroaryl, L may be optionally substituted with one or more of the following groups: halogen, hydroxy, C₁₋₆ alkoxy, cyano, C₃₋₈ cycloalkyl, 3- to 8-membered heterocyclyl, 6- to 10-membered aryl or 6- to 10-membered heteroaryl;
Y is -CH₂-, -CH₂CH₂-, or is absent;
U is -CH₂-, -C(O)-, or is absent;
X and U are not absent at the same time;
Y and U may be optionally substituted with one or more of the following substituents: halogen, hydroxy, C₁₋₆ alkyl, C₁₋₆ alkoxy, cyano, C₃₋₈ cycloalkyl, 3- to 8-membered heterocyclyl, 6- to 10-membered aryl or 6- to 10-membered heteroaryl;
A is 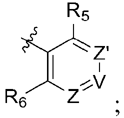
Z is N or CR₃;
Z' is N or CR₄;
R₃, R₄, R₅, R₆ are each independently H, cyano, halogen or C₁₋₃ haloalkyl;
V is N or CR₉, wherein R₉ is H, cyano, halogen, C₁₋₃ alkyl, C₁₋₃ haloalkyl or -O-W;
W is phenyl or 5- or 6-membered heteroaryl, which may be optionally substituted with one or more of the following substituents: halogen, cyano, C₁₋₆ alkyl, C₁₋₃ alkoxy, C₁₋₃ haloalkyl and C₁₋₃ haloalkoxy.

2. The compound according to claim 1, the compound having Formula I' 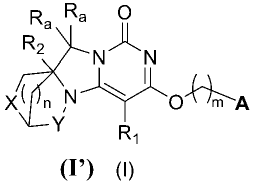 wherein
R₁ is H, cyano, halogen, C₁₋₆ alkyl, C₁₋₃ alkoxy or C₁₋₃ haloalkyl;
X is -O-, -CH₂-, -NR_{c}-, or is absent;
R_{c} is L or L-C(O)-, wherein L is H, C₁₋₃ alkyl, C₃₋₆ cycloalkyl, C₃₋₆ heterocycloalkyl, C₁₋₃ haloalkyl, or benzyl;
Y is -CH₂-, or is absent;
n, R₂, Ra, A and m are as defined in claim 1.

3. The compound according to claim 1, wherein n=1 or 2; and R₂ is absent.

4. The compound of claim 3, wherein n is 2; U is -CH₂-; X is -CH₂- or -O-; Y is - CH₂-, or is absent; Rₓ is H, halogen, cyano, amino, difluoromethyl, 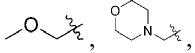 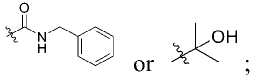 R₁ is H, cyano, halogen, C₁₋₃ alkyl, C₁₋₃ haloalkyl or C₁₋₆ alkoxy; and Q is -O-.

5. The compound according to any one of claims 1-4, wherein m=2; R₁ is H, cyano or C₁₋₃ haloalkyl; and Rₓ is H.

6. The compound according to any one of claims 1-4, wherein m=1; R₁ is H, cyano, halogen, C₁₋₃ alkyl, C₁₋₃ haloalkyl or C₁₋₆ alkoxy; Rₓ is H.

7. The compound according to any one of claims 1 to 6, wherein A is 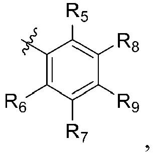 and R₅, R₆, R₇, R₈, R₉ are each independently H, F or cyano;
or wherein A is 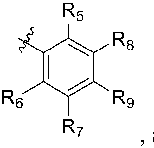 and R₅, R₆, R₇ and R₈ are each independently H, F or cyano; R₉ is -O-W; W is 5- or 6-membered heteroaryl or phenyl, which can be optionally substituted with one or more of the following substituents: C₁₋₃ haloalkyl, C₁₋₃ haloalkoxy, cyano, halogen and C₁₋₆ alkyl;
or wherein A is 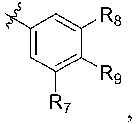 and R₇ and R₈ are each independently H, F or cyano; R₉ is -O-W; and W is pyridyl, pyrimidinyl, pyrazolyl or phenyl, which can be optionally substituted with one or more substituents independently selected from the following substituents: halogen, cyano, CF₃, -OCF₃, CHF₂ and CH₃.

8. The compound according to any one of claims 1 to 7, which is the following compound 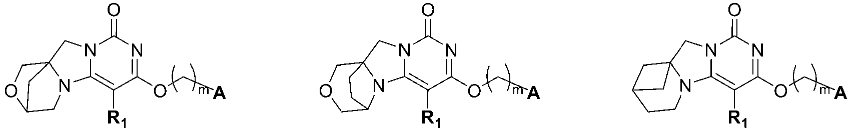 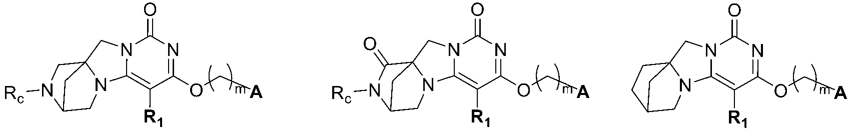 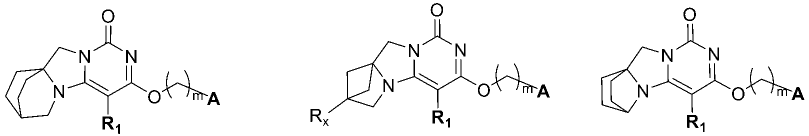 wherein, R₁ is H, halogen, cyano, C₁₋₆ alkyl, C₁₋₃ haloalkyl or C₁₋₆ alkoxy; R_{c} is C₁₋₆ alkyl, 3- to 8-membered heterocyclyl, C₁₋₃ haloalkyl, benzoyl, C₃₋₈ cycloalkyl, benzyl or 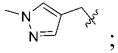 Rₓ is H, cyano, halogen, C₁₋₃ haloalkyl, amino,

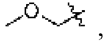

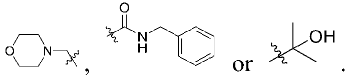

9. The compound according to claim 8, wherein R₁ is H, fluorine, chlorine, cyano, methyl, ethyl, isopropyl, trifluoromethyl or methoxy; R₂ is methyl or ethyl; R_{c} is methyl, oxetanyl, trifluoroethyl, benzoyl, cyclobutyl, benzyl or 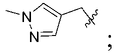 Rₓ is H, cyano, fluorine, difluoromethyl, amino,

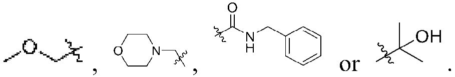

10. The compound according to any one of claims 1 to 6 and 8-9, wherein A is selected from the group consisting of:

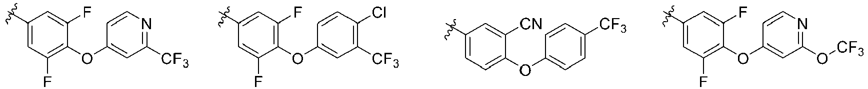

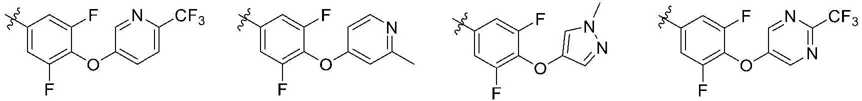

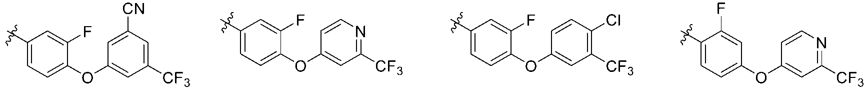

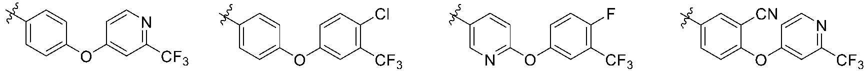

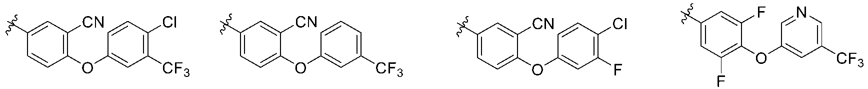

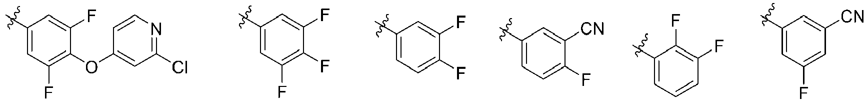

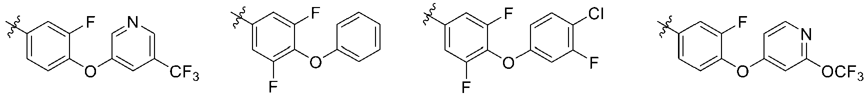

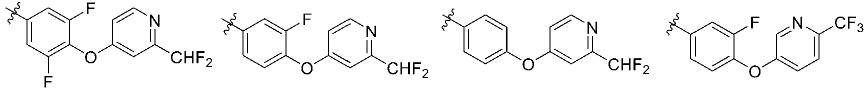

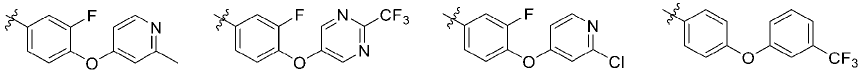

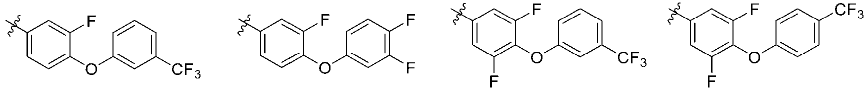

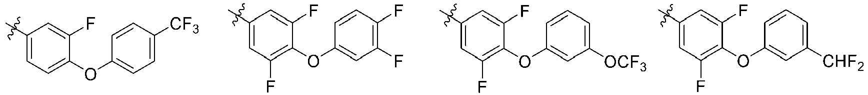

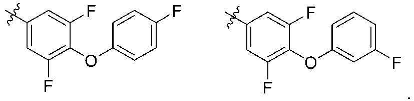

11. The compound according to any one of claims 1 to 10, wherein the compound is the following compound:
| **Example** | **Structure** | **Example** | **Structure** |
|---|---|---|---|
| **1** | 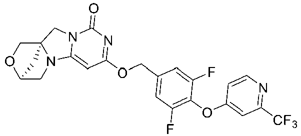 | **2** | 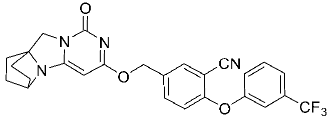 |
| **4** | 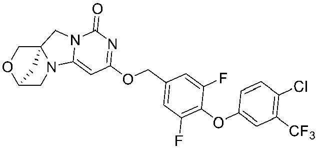 | **5** | 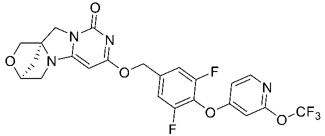 |
| **6** | 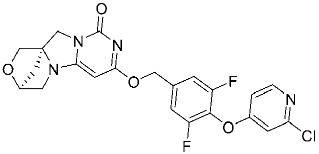 | **7** | 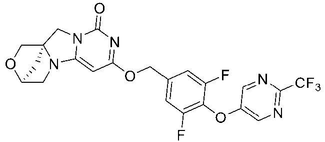 |
| **8** | 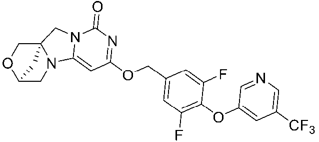 | **9** | 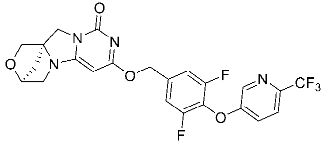 |
| **10** | 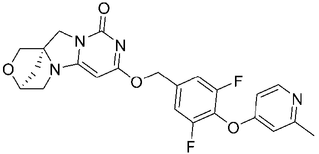 | **11** | 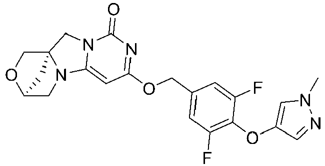 |
| **12** | 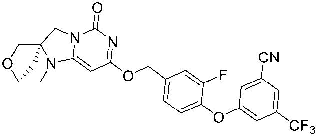 | **13** | 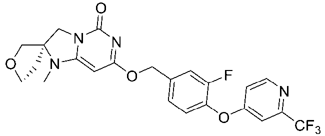 |
| **14** | 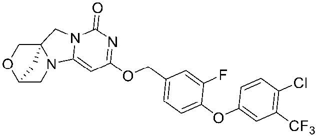 | **15** | 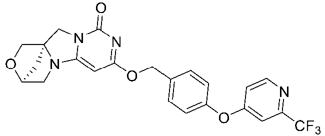 |
| **16** | 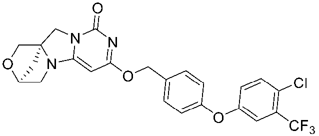 | **17** | 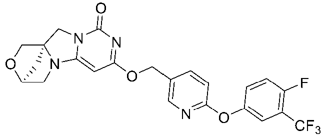 |
| **18** | 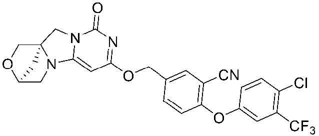 | **19** | 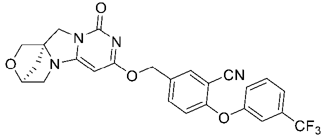 |
| **20** | 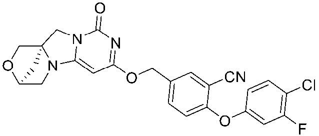 | **21** | 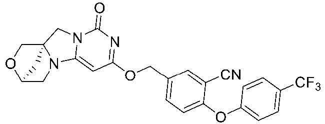 |
| **22** | 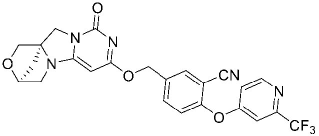 | **23** | 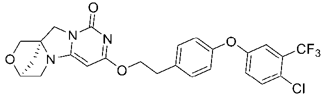 |
| **24** | 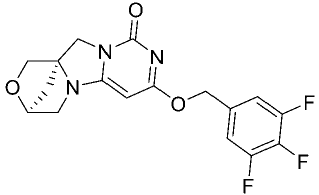 | **25** | 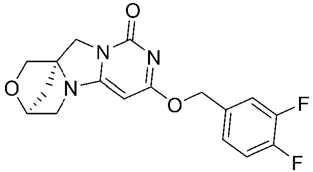 |
| **26** | 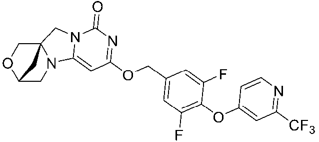 | **27** | 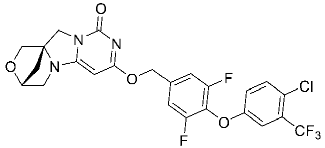 |
| **28** | 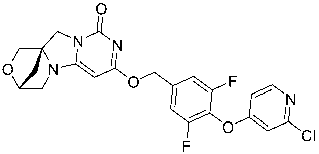 | **29** | 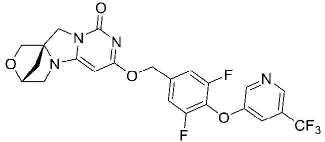 |
| **30** | 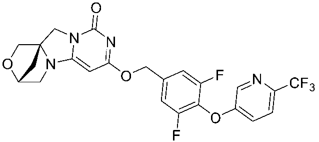 | **31** | 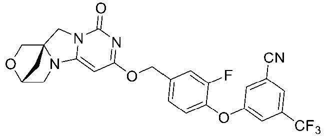 |
| **32** | 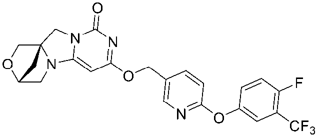 | **33** | 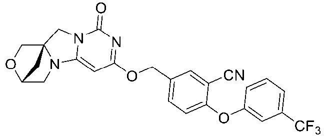 |
| **34** | 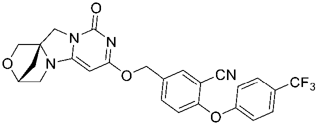 | **35** | 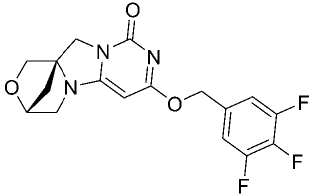 |
| **36** | 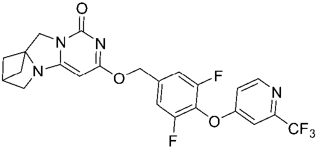 | **37** | 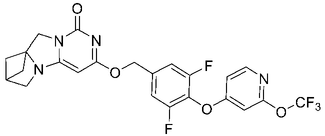 |
| **38** | 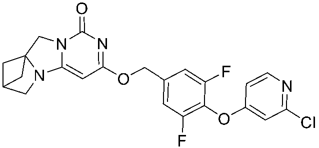 | **39** | 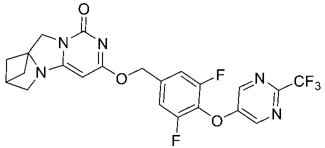 |
| **40** | 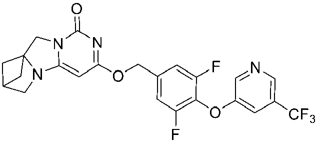 | **41** | 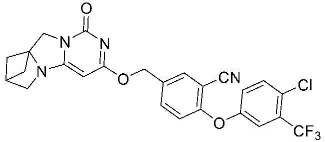 |
| **42** | 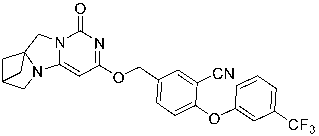 | **43** | 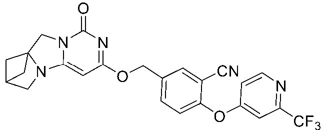 |
| **44** | 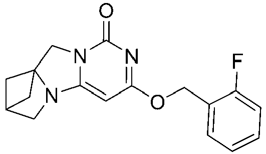 | **45** | 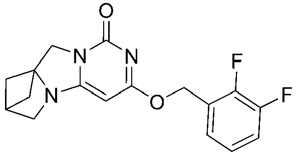 |
| **46** | 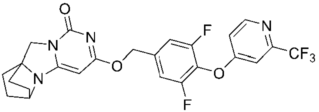 | **47** | 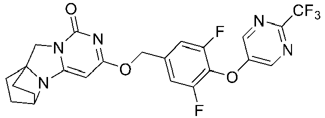 |
| **48** | 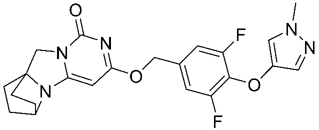 | **49** | 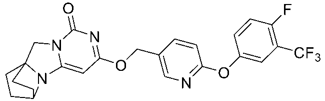 |
| **50** | 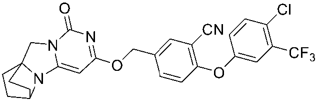 | **51** | 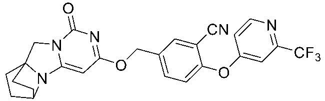 |
| **52** | 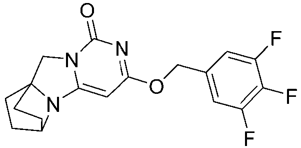 | **53** | 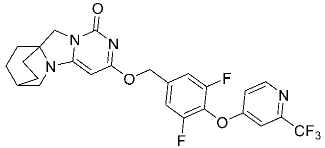 |
| **54** | 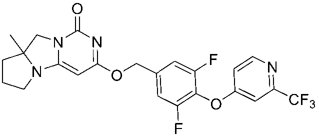 | **55** | 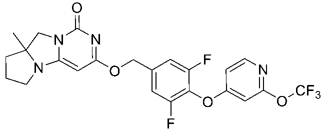 |
| **56** | 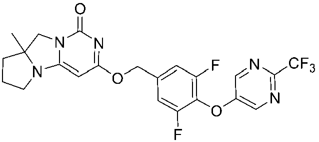 | **57** | 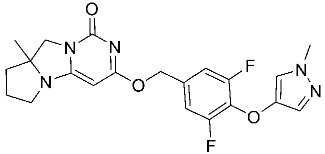 |
| **58** | 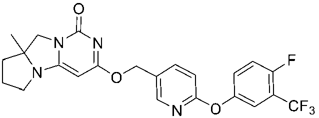 | **59** | 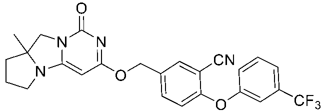 |
| **60** | 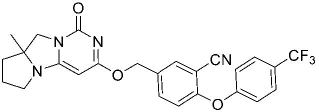 | **61** | 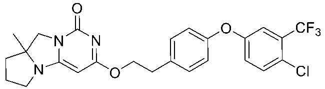 |
| **62** | 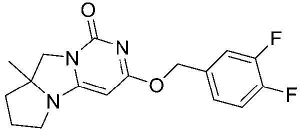 | **63** | 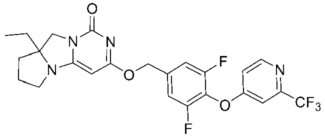 |
| **64** | 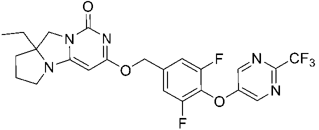 | **65** | 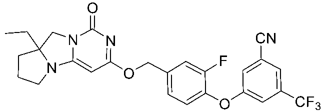 |
| **66** | 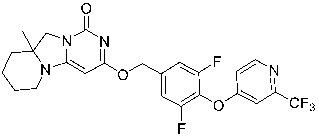 | **67** | 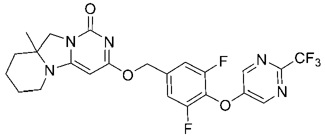 |
| **68** | 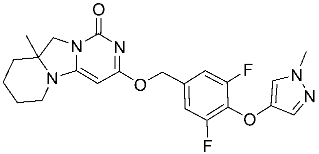 | **69** | 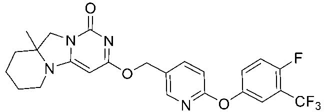 |
| **70** | 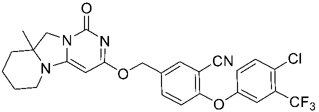 | **71** | 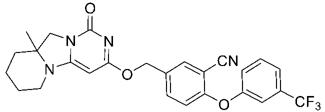 |
| **72** | 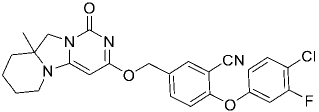 | **73** | 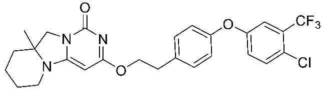 |
| **74** | 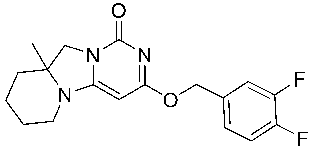 | **75** | 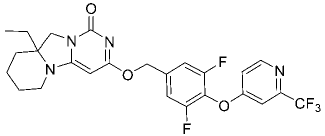 |
| **76** | 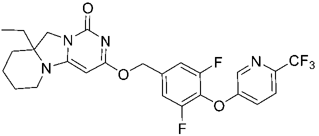 | **77** | 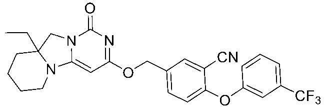 |
| **78** | 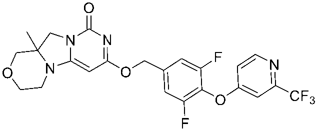 | **79** | 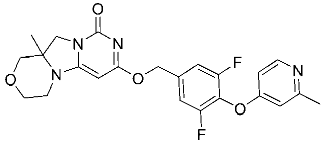 |
| **80** | 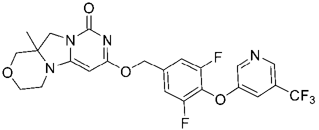 | **81** | 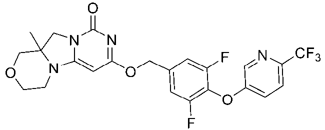 |
| **82** | 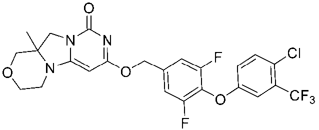 | **83** | 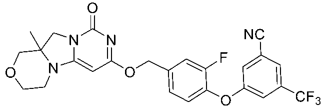 |
| **84** | 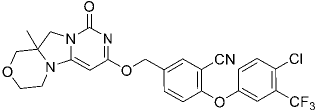 | **85** | 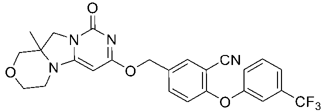 |
| **86** | 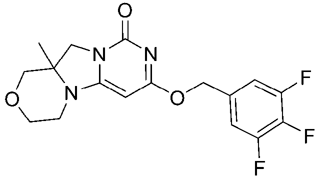 | **88** | 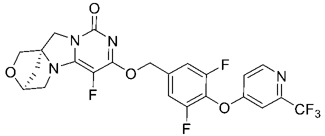 |
| **89** | 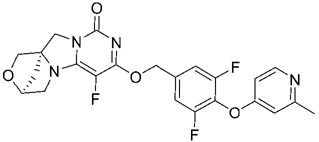 | **90** | 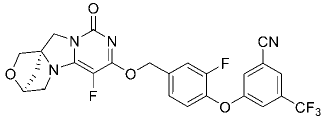 |
| **91** | 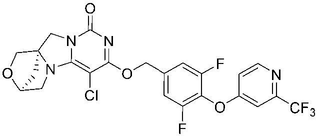 | **92** | 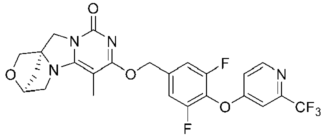 |
| **93** | 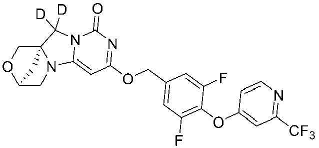 | **94** | 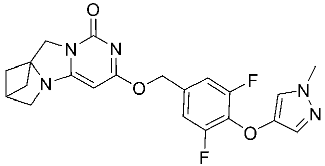 |
| **95** | 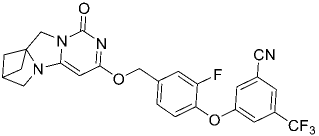 | **96** | 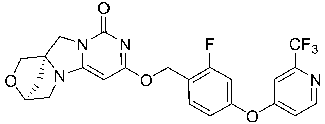 |
| **97** | 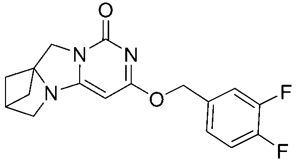 | **98** | 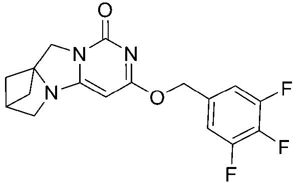 |
| **99** | 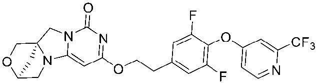 | **100** | 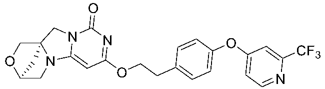 |
| **101** | 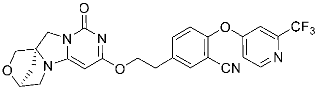 | **102** | 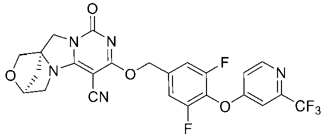 |
| **103** | 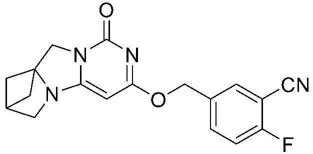 | **104** | 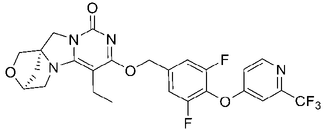 |
| **105** | 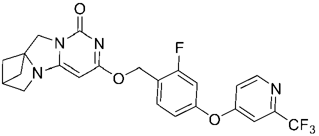 | **106** | 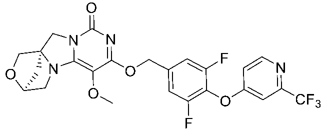 |
| **107** | 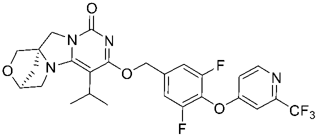 | **108** | 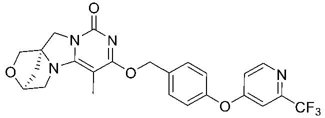 |
| **109** | 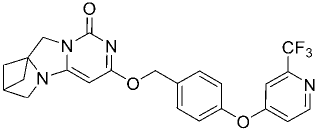 | **110** | 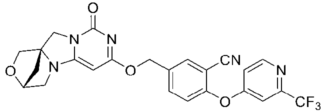 |
| **111** | 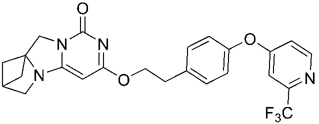 | **112** | 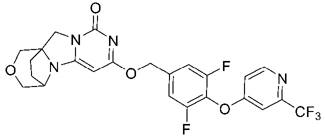 |
| **113** | 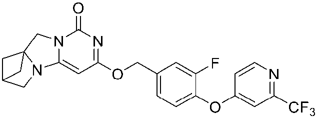 | **114** | 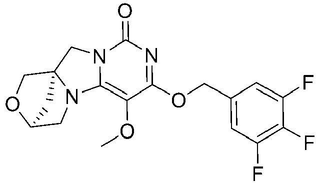 |
| **115** | 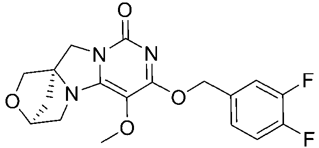 | **116** | 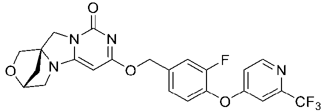 |
| **117** | 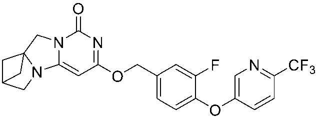 | **118** | 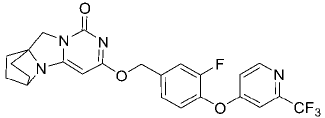 |
| **119** | 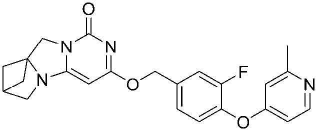 | **120** | 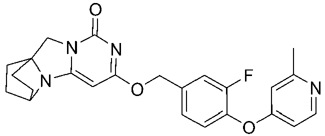 |
| **121** | 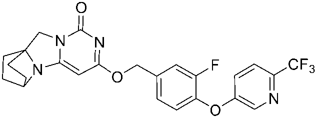 | **122** | 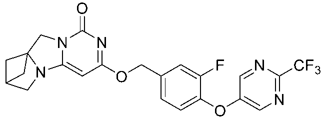 |
| **123** | 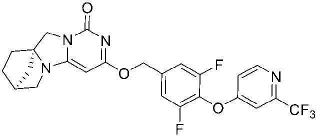 | **124** | 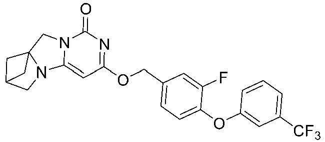 |
| **125** | 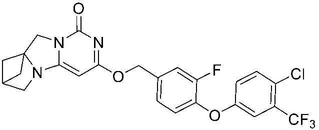 | **126** | 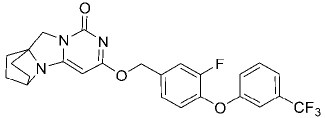 |
| **127** | 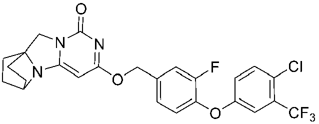 | **128** | 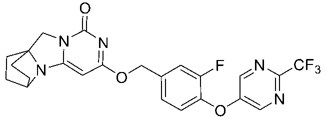 |
| **129** | 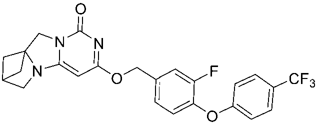 | **130** | 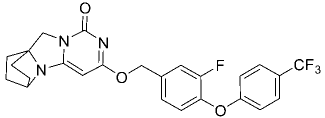 |
| **131** | 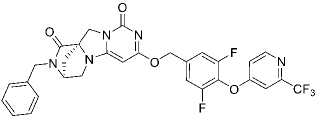 | **132** | 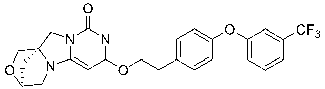 |
| **133** | 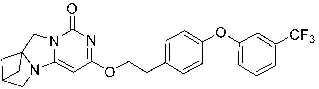 | **134** | 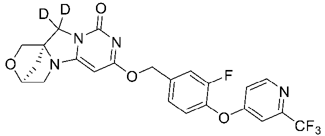 |
| **135** | 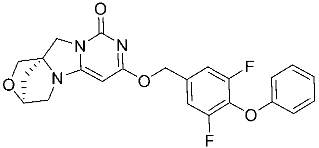 | **136** | 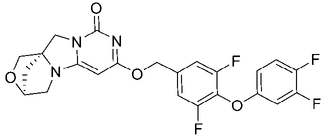 |
| **137** | 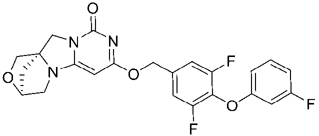 | **138** | 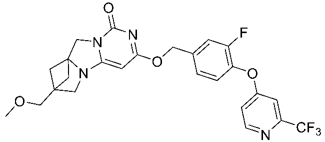 |
| **139** | 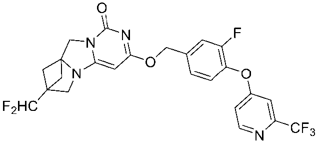 | **140** | 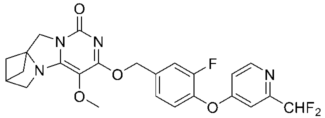 |
| **141** | 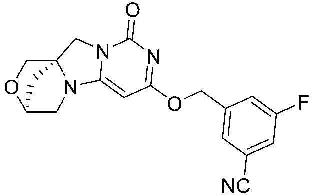 | **142** | 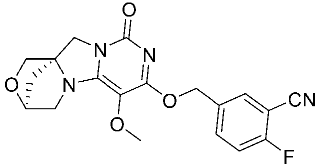 |
| **143** | 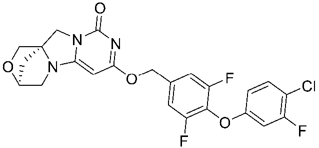 | **144** | 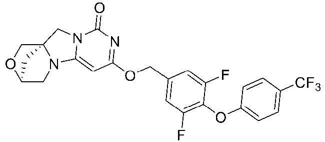 |
| **145** | 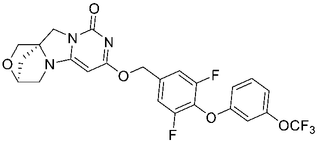 | **146** | 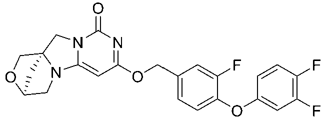 |
| **147** | 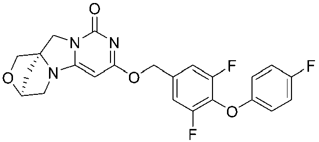 | **148** | 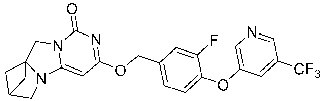 |
| **149** | 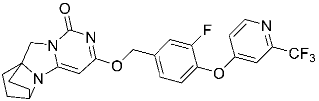 | **150** | 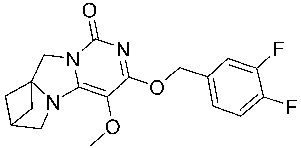 |
| **151** | 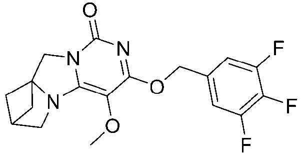 | **152** | 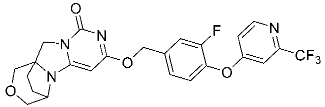 |
| **153** | 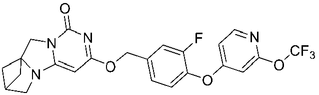 | **154** | 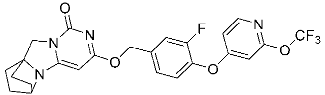 |
| **155** | 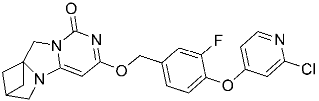 | **156** | 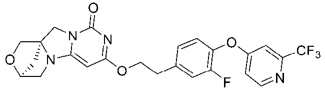 |
| **157** | 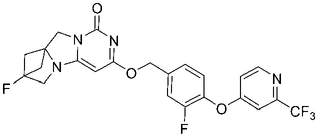 | **158** | 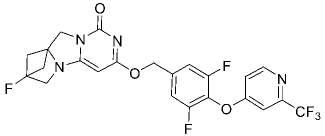 |
| **159** | 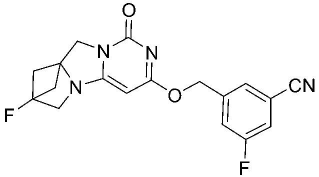 | **160** | 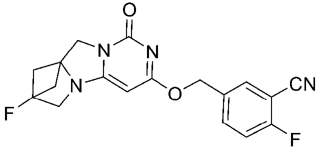 |
| **161** | 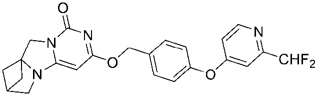 | **162** | 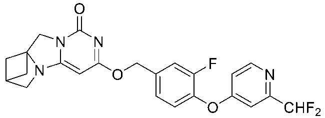 |
| **163** | 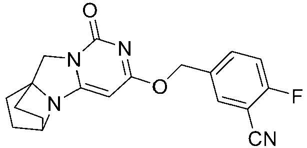 | **164** | 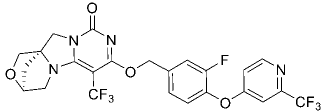 |
| **165** | 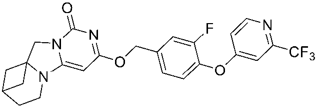 | **166** | 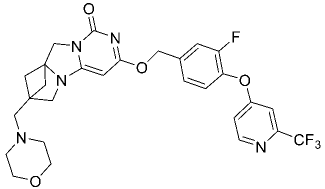 |
| **167** | 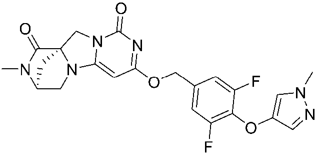 | **168** | 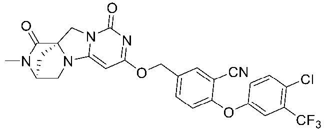 |
| **169** | 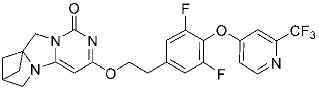 | **170** | 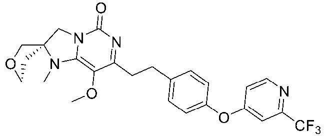 |
| **171** | 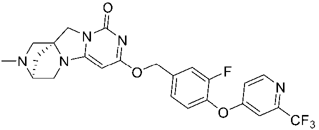 | **172** | 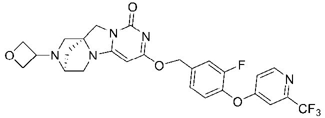 |
| **173** | 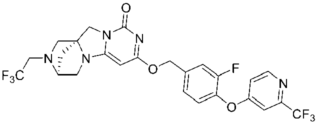 | **174** | 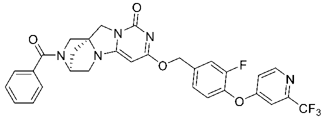 |
| **175** | 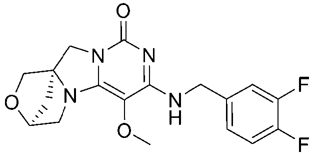 | **176** | 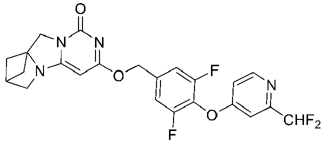 |
| **177** | 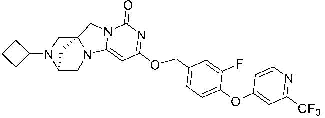 | **178** | 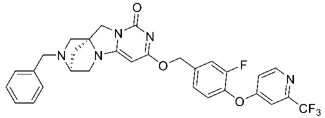 |
| **179** | 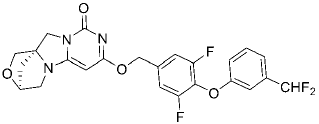 | **180** | 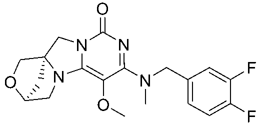 |
| **181** | 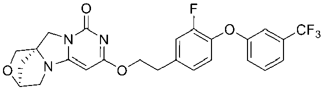 | **182** | 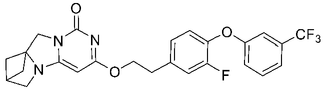 |
| **183** | 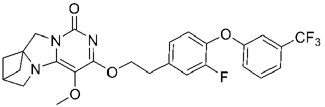 | **184** | 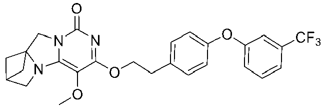 |
| **185** | 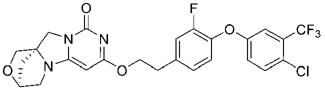 | **186** | 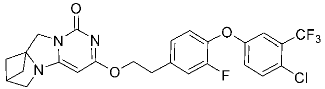 |
| **187** | 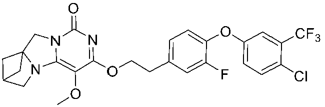 | **188** | 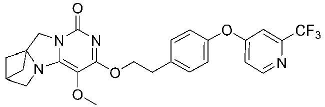 |
| **189** | 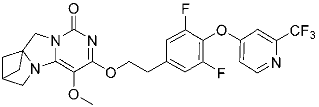 | **190** | 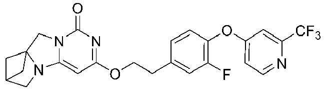 |
| **191** | 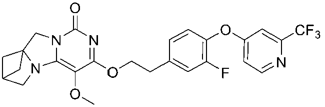 | **192** | 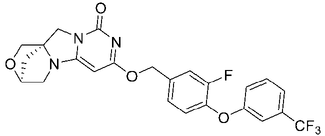 |
| **193** | 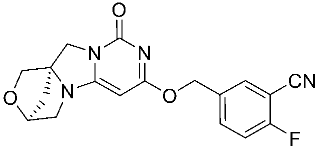 | **194** | 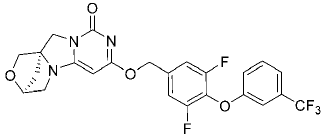 |
| **195** | 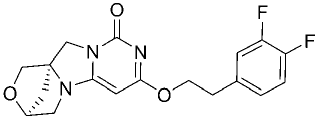 | **196** | 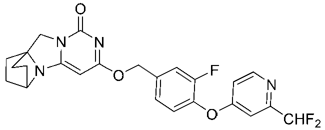 |
| **197** | 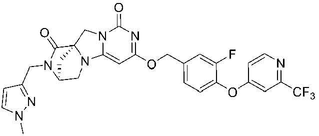 | **198** | 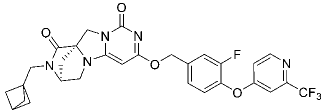 |
| **199** | 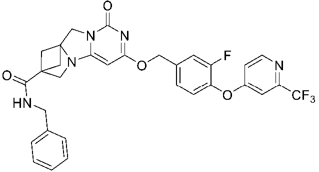 | **200** | 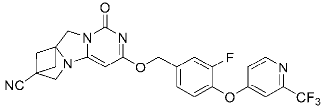 |
| **201** | 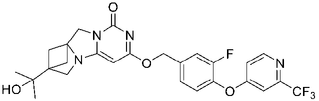 | **202** | 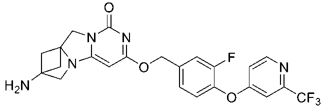 |

12. A composition, **characterized in** comprising the compound, cis-trans isomer thereof, enantiomer thereof, diastereomer thereof, racemate thereof, solvate thereof, hydrate thereof or pharmaceutically acceptable salt thereof or prodrug thereof according to any one of claims 1 to 11, and a pharmaceutically acceptable excipient.

13. The compound according to any one of claims 1 to 11 or the composition according to claim 12 for use in the treatment or prevention of a disease associated with Lp-PLA₂.

14. The compound according to any one of claims 1 to 11 or the composition according to claim 12 for use in the treatment or prevention of a diabetic complication, preferably diabetic retinopathy/diabetic macular edema, diabetic nephropathy, diabetic neuropathy, diabetic peripheral neuropathy pain or diabetic foot; a neuroinflammation-related disease, preferably Alzheimer's disease, multiple sclerosis, amyotrophic lateral sclerosis, or Parkinson's disease; or atherosclerosis.
